# EUROPEAN PATENT APPLICATION

(11) **EP 3 797 764 A1**
(43) Date of publication of application: **31.03.2021**
(21) Application number: 19460051.6
(22) Date of filing: 27.09.2019
(51) Int. Cl.: A61K 31/133, A61K 31/19, A61K 9/00

(54) **PHARMACOLOGICAL COMPOSITION FOR TREATMENT OF NERVOUS TISSUE DYSFUNCTION, IN PARTICULAR VIRAL LATENCY, AND THE USE OF J05 ATC GROUP SUBSTANCES IN TREATMENT OF NERVOUS TISSUE DYSFUNCTION, IN PARTICULAR VIRAL LATENCY**

(71) Applicant: Janusz Chupty Contissi, 39-200 Debica (PL)
(72) Inventor: Chuptys, Piotr, 39-200 Debica (PL)
(74) Representative: Warzybok, Tadeusz

(57) **Abstract**

The subject of the invention is a pharmacological composition for treatment of nervous tissue dysfunction, in particular viral latency caused by herpes viruses, which contains at least one antiepileptic active substance selected from the NO3 ATC group and at least one antiviral active substance selected from the J05 ATC group.

The invention also concerns the use of J05 ATC group substances in treatment of nervous tissue dysfunction, in particular viral latency.

## Description

The subject of the invention is a pharmacological composition for treatment of nervous tissue dysfunction, in particular viral latency caused by herpes viruses and the use of J05 ATC group substances in treatment of nervous tissue dysfunction, in particular viral latency caused by herpes viruses.

Viruses are organic molecules composed mainly of nucleic acids and proteins. Viruses are not capable of independent replication. In order to replicate their own genes, they multiply by infecting healthy cells. They can infect all types of organisms, from animals and plants to bacteria and archaea.
A given virus species contains only one type of nucleic acid (DNA or RNA), although another type of acid is usually synthesized during intracellular development. Due to their parasitic nature, viruses bear proteins on their outer surface that allow them to attack the right cells. Numerous studies have shown that viruses can regulate the functioning of our brain and affect the risk of various mental or neurological disorders.

Herpes viruses are pathogens that are particularly prevalent in the human population, eight of which can cause diseases such as: herpes labialis, genital herpes, varicella, herpes zoster and cytomegalovirus. Based on the genome capsid structure, three subfamilies of the virus were distinguished: alpha, beta and gamma, whereas alpha-herpesviruses can be found mainly in neurons, beta-herpseviruses in subpopulated hematopoietic cells and gamma-herpesviruses in B lymphocytes.
A common feature of herpesviruses is their ability to enter into latency, which can be defined as the absence of infectious viral particles in the host cell, whilst being able to produce them under favourable conditions. During latency, the entire viral genome is in the cell and its expression is very limited. For a given infection to qualify as a latent infection, certain conditions must be met. The infection must be persistent, i.e. maintained despite responses from the host immune system and other unfavourable signals in the cell environment. It must be a reversible infection, which means that in certain circumstances the virus reactivates, i.e. there is a viral gene expression with simultaneous production of daughter cells, which is referred to as a productive infection. Latency is a specific cellular phenomenon, which means that the virus causes a latent infection only in certain cells. One of the defensive mechanisms directed against intracellular pathogens is programmed cell death, i.e. apoptosis. During apoptosis, proteins and nucleic acids of both the cell and the virus present therein undergo degradation. Thereby, the death of an infected cell prevents the virus from spreading, however, Herpesviruses have developed the ability to suppress apoptosis. (source: work entitled: "Strategie przetrwania herpeswirusów - latencja i apoptoza" written by Dariusz Miszczak, Anna S 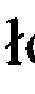 ońska, Anna Golke, Joanna Cymerys -publication: Postepy Hig Dosw (online), 2013: 67:276:287: www.phmd.pl).
Infections caused by herpes viruses (HSV, Herpes simplex, Herpesvirus hominis) belong to the most common human infections. It is estimated that as many as 80-90 percent of people are potentially the carriers of this virus. It can be found in human ganglia and for many years stays in a latent form without any symptoms. It is activated by stress, fatigue or reduced immunity caused by infections. Medicine distinguishes two types of herpes viruses: type 1 (HSV-1) causing changes in the oral cavity, conjunctiva, cornea and skin on the upper part of the body, and type 2 (HSV-2) causing changes mainly in the genital area.
Herpes simplex virus type 2 is particularly dangerous for pregnant women, as it increases the risk of miscarriage. In addition, the risk of infecting the foetus is particularly high during delivery, and in the case of intrauterine infection before the 20th week of pregnancy, this virus could result in damage to the unborn child, such as: microphthalmia, hydrocephalus, microcephalus or damage to the central nervous system.
Nervous tissue damaged by the virus does not regenerate, and even the smallest loss of nervous tissue can lead to permanent brain damage.

Research has shown that the primary HSV type 1 (HSV-1) infection in children from 1 to 3 years of age is usually asymptomatic. It rarely takes the form of herpetic inflammation of the oral cavity and is most often manifested by blisters formed on the skin around the mouth and nose, which after a few days turn into a scab and fall off. In most cases, the symptoms resolve spontaneously. After primary infection, the virus remains in the nerve cells in a latent state and becomes active under stress, fatigue, ultraviolet rays, or reduced immunity. The administered antiviral drugs do not destroy the virus, but prevent its multiplication.

According to the above mentioned specialised literature, in spite of knowledge about the ability of viruses to enter into latency, viruses were not treated in this phase, but only after they were activated, which resulted, for example, in herpes labialis, acting symptomatically and removing only their effects.
In addition, scientific literature also lists various causes of brain dysfunction, one of which is undoubtedly epilepsy. The causes of epilepsy may be genetically conditioned or acquired. Some families may have a genetic predisposition to this disorder. Epilepsy may also develop as a result of neuronal damage following head injury, inflammatory diseases, vascular diseases or metabolic diseases. It is not always possible to determine the cause of this disorder, therefore physicians often diagnose it as "epilepsy of unknown cause".
Standard treatment of epilepsy is a symptomatic treatment, and the administered antiepileptic drugs act on the central nervous system preventing or alleviating epileptic seizures. The choice of a drug is based on knowledge of its effectiveness and efficiency in various types of epileptic seizures or epileptic syndromes as well as knowledge of possible side effects. The kind of medication should be adapted to the type of seizure. If seizures are not controlled with one drug, another drug is gradually introduced. (*Source:* Halczuk I.: Postepy w leczeniu padaczki. Neurol Prakt 2015; 3(84): 5-11.)
Antiepileptic drugs include numerous first- and second-generation drugs as well as third-generation drugs. First-generation drugs include: phenobarbital, phenytoin, primidone, ethosuximide. Second-generation drugs include: benzodiazepine, carbamazepine and valproate, and third-generation drugs include: vigabatrin, zonisamide, lamotrigine, felbamate, gabapentin, topiramate, fosphenytoin, tiagabine, oxcarbazepine and levetiracetam.

The most well-known and used antiepileptic drug is "Depakine". It contains an active substance in the form of sodium valproate, which has an effect on the central nervous system. It selectively increases the concentration of GABA (gamma aminobutyric acid) in synapses in the CNS and reduces the consumption of GABA by glial cells and nerve endings by inhibiting GABA aminotransferase and semialdehyde dehydrogenase. The drug also affects excitatory neurotransmitters and can act directly on sodium and potassium channels in neuronal membranes. It also has anticonvulsant effect in different seizure types.

Patent No. EP1466606 describes the use of inosine in the manufacture of drugs to stimulate the growth of axons in the central nervous system in neuroproliferative disorders, for example in cases with the occurrence of abnormal or excessive growth of axons, such as in epilepsy or neuropathic pain.

The use of inosine pranobex as an antiviral and immunostimulating agent in infections with herpes simplex viruses (HSV) and other viral infections is also known.

J05 ATC group substances, qualified as antiviral drugs by WHO, are usually used for 14 days. However, it is far too short a time to effectively treat the virus after its transition from the active to the latent phase. In the present state of scientific knowledge, there is no treatment of viruses in the latent phase, in particular Herpes viruses, taking the view that they are not dangerous for the human body during latency. Until now, no brain disorders, including epileptic disorders, have been associated with the latency of viruses in human brain cells, treating them symptomatically and qualifying them as "disorders of unknown cause". It has been found that J05 ATC group substances are also effective against latent viruses, and their activity is different than in the case of an active virus. The use of this group of substances is recommended for patients who have recovered from a viral infection confirmed by laboratory tests, i.e. when HSV-1 or HSV-2 IgM and IgG antibodies are negative or low.
Hence the new and previously unknown use of J05 ATC group substances for treatment of viral infections in the latent phase.

The aim of the invention is to develop such a pharmacological composition consisting of at least two active substances to treat nervous tissue dysfunction, in particular brain dysfunctions of viral origin, with at least one substance controlling symptoms to eliminate any impaired brain functions diagnosed as epileptic seizures of unknown cause, and the other active substance acting as an antiviral agent to prevent the proliferation of pathogenic viruses.

The aim of the invention is also to use J05 ATC group substances to treat nervous tissue dysfunction, especially viral infections in the latent phase.

A pharmacological composition used in treatment of nervous tissue dysfunction, especially in the latent phase, consistent with the invention, contains at least one active substance with antiepileptic effect selected from the NO3 ATC group and at least one active substance with antiviral effect selected from the J05 ATC group, whereas the preferable active substance of the NO3 ATC group is: methylphenobarbital or phenobarbital or primidone or barbexaclone or metharbital or ethotoin or phenytoin or amino(diphenylhydantoin) valeric acid or mephenytoin or fosphenytoin or phenytoin combinations or mephenytoin combinations or paramethadione or trimethadione or ethadione or ethosuximide or phensuximide or mesuximide or ethosuximide combinations or clonazepam or carbamazepine or oxcarbazepine or rufinamide or eslicarbazepine or valproic acid or valpromide or aminobutyric acid or vigabatrin or progabide or tiagabine or sultiame or phenacemide or lamotrigine or felbamate or topiramate or gabapentin or pheneturide or levetiracetam or zonisamide or pregabalin or stiripentol or lacosamide or carisbamate or retigabine or perampanel or brivaracetam or cannabidiol or beclamide, whereas the preferable active substance of the J05 ATC group is: metisazone or aciclovir or idoxuridine or vidarabine or ganciclovir or famciclovir or valaciclovir or cidofovir or penciclovir or valganciclovir or brivudine or rimantadine or tromantadine or foscarnet or fosfonet or saquinavir or indinavir or ritonavir or nelfinavir or amprenavir or fosamprenavir or atazanavir or tipranavir or darunavir or zidovudine or didanosine or zalcitabine or stavudine or lamivudine or abacavir or tenofovir disoproxil or adefovir dipivoxil or emtricitabine or entecavir or telbivudine or clevudine or tenofovir alafenamide or nevirapine or delavirdine or efavirenz or etravirine or rilpivirine or doravirine or zanamivir or oseltamivir or peramivir or ribavirin or telaprevir or boceprevir or faldaprevir or simeprevir or asunaprevir or daclatasvir or sofosbuvir or dasabuvir or sofosbuvir and ledipasvir or dasabuvir, ombitasvir, paritaprevir and ritonavir or ombitasvir, paritaprevir and ritonavir or elbasvir and grazoprevir or sofosbuvir and velpatasvir or sofosbuvir, velpatasvir and voxilaprevir or glecaprevir and pibrentasvir or zidovudine and lamivudine or lamivudine and abacavir or tenofovir disoproxil and emtricitabine or zidovudine, lamivudine and abacavir or zidovudine, lamivudine and nevirapine or emtricitabine, tenofovir disoproxil and efavirenz or stavudine, lamivudine and nevirapine or emtricitabine, tenofovir disoproxil and rilpivirine or emtricitabine, tenofovir disoproxil, elvitegravir and cobicistat lopinavir and ritonavir or lamivudine, tenofovir disoproxil and efavirenz or lamivudine and tenofovir disoproxil or lamivudine, abacavir and dolutegravir or darunavir and cobicistat or atazanavir and cobicistat or lamivudine and raltegravir or emtricitabine and tenofovir alafenamide or emtricitabine, tenofovir alafenamide, elvitegravir and cobicistat or emtricitabine, tenofovir alafenamide and rilpivirine or emtricitabine, tenofovir alafenamide and bictegravir or dolutegravir and rilpivirine or emtricitabine, tenofovir or alafenamide, darunavir and cobicistat or atazanavir and ritonavir or lamivudine, tenofovir disoproxil and doravirine or moroxydine or lysozyme or inosine pranobex or pleconaril or enfuvirtide or raltegravir or maraviroc or maribavir or elvitegravir or dolutegravir or umifenovir or enisamium iodide or letermovir or tilorone or pentanedioic acid imidazolyl ethanamide or ibalizumab.

It is also preferable when the active substance with antiepileptic effect is valproic acid (sodium valproate) or vigabatrin, while the active substance with antiviral effect is inosine pranobex.

It is also preferable when the weight ratio in [mg] of active substances selected from the NO3 ATC group to active substances selected from the J05 ATC group is as follows: 10.000.000 to 1:1; 1.000.000 to 1:1; 100.000 to 1:1; 10.000 to 1:1; 1.000 to 1:1; 100 to 1:1; 10 to 1:1; 1:1 to 10.000.000; 1:1 to 1.000.000; 1:1 to 100.000; 1:1 to 10.000; 1 to 1: 1.000; 1:1 to 100; 1:1 to 10 preferably 1:2, most preferably 1:2.13.

The preferable combination of active substances in the composition is:
N03AA01 and J05AA01 or N03AA02 and J05AA01 or N03AA03 and J05AA01 or N03AA04 and J05AA01 or N03AA30 and J05AA01 or N03AB01 and J05AA01 or N03AB02 and J05AA01 or N03AB03 and J05AA01 or N03AB04 and J05AA01 or N03AB05 and J05AA01 or N03AB52 and J05AA01 or N03AB54 and J05AA01 or N03AC01 and J05AA01 or N03AC02 and J05AA01 or N03AC03 and J05AA01 or N03AD01 and J05AA01 or N03AD02 and J05AA01 or N03AD03 and J05AA01 or N03AD51 and J05AA01 or N03AE01 and J05AA01 or N03AF01 and J05AA01 or N03AF02 and J05AA01 or N03AF03 and J05AA01 or N03AF04 and J05AA01 or N03AG01 and J05AA01 or N03AG02 and J05AA01 or N03AG03 and J05AA01 or N03AG04 and J05AA01 or N03AG05 and J05AA01 or N03AG06 and J05AA01 or N03AX03 and J05AA01 or N03AX07 and J05AA01 or N03AX09 and J05AA01 or N03AX10 and J05AA01 or N03AX11 and J05AA01 or N03AX12 and J05AA01 or N03AX13 and J05AA01 or N03AX14 and J05AA01 or N03AX15 and J05AA01 or N03AX16 and J05AA01 or N03AX17 and J05AA01 or N03AX18 and J05AA01 or N03AX19 and J05AA01 or N03AX21 and J05AA01 or N03AX22 and J05AA01 or N03AX23 and J05AA01 or N03AX24 and J05AA01 or N03AX30 and J05AA01 or N03AA01 and J05AB01 or N03AA02 and J05AB01 or N03AA03 and J05AB01 or N03AA04 and J05AB01 or N03AA30 and J05AB01 or N03AB01 and J05AB01 or N03AB02 and J05AB01 or N03AB03 and J05AB01 or N03AB04 and J05AB01 or N03AB05 and J05AB01 or N03AB52 and J05AB01 or N03AB54 and J05AB01 or N03AC01 and J05AB01 or N03AC02 and J05AB01 or N03AC03 and J05AB01 or N03AD01 and J05AB01 or N03AD02 and J05AB01 or N03AD03 and J05AB01 or N03AD51 and J05AB01 or N03AE01 and J05AB01 or N03AF01 and J05AB01 or N03AF02 and J05AB01 or N03AF03 and J05AB01 or N03AF04 and J05AB01 or N03AG01 and J05AB01 or N03AG02 and J05AB01 or N03AG03 and J05AB01 or N03AG04 and J05AB01 or N03AG05 and J05AB01 or N03AG06 and J05AB01 or N03AX03 and J05AB01 or N03AX07 and J05AB01 or N03AX09 and J05AB01 or N03AX10 and J05AB01 or N03AX11 and J05AB01 or N03AX12 and J05AB01 or N03AX13 and J05AB01 or N03AX14 and J05AB01 or N03AX15 and J05AB01 or N03AX16 and J05AB01 or N03AX17 and J05AB01 or N03AX18 and J05AB01 or N03AX19 and J05AB01 or N03AX21 and J05AB01 or N03AX22 and J05AB01 or N03AX23 and J05AB01 or N03AX24 and J05AB01 or N03AX30 and J05AB01 or N03AA01 and J05AB02 or N03AA02 and J05AB02 or N03AA03 and J05AB02 or N03AA04 and J05AB02 or N03AA30 and J05AB02 or N03AB01 and J05AB02 or N03AB02 and J05AB02 or N03AB03 and J05AB02 or N03AB04 and J05AB02 or N03AB05 and J05AB02 or N03AB52 and J05AB02 or N03AB54 and J05AB02 or N03AC01 and J05AB02 or N03AC02 and J05AB02 or N03AC03 and J05AB02 or N03AD01 and J05AB02 or N03AD02 and J05AB02 or N03AD03 and J05AB02 or N03AD51 and J05AB02 or N03AE01 and J05AB02 or N03AF01 and J05AB02 or N03AF02 and J05AB02 or N03AF03 and J05AB02 or N03AF04 and J05AB02 or N03AG01 and J05AB02 or N03AG02 and J05AB02 or N03AG03 and J05AB02 or N03AG04 and J05AB02 or N03AG05 and J05AB02 or N03AG06 and J05AB02 or N03AX03 and J05AB02 or N03AX07 and J05AB02 or N03AX09 and J05AB02 or N03AX10 and J05AB02 or N03AX11 and J05AB02 or N03AX12 and J05AB02 or N03AX13 and J05AB02 or N03AX14 and J05AB02 or N03AX15 and J05AB02 or N03AX16 and J05AB02 or N03AX17 and J05AB02 or N03AX18 and J05AB02 or N03AX19 and J05AB02 or N03AX21 and J05AB02 or N03AX22 and J05AB02 or N03AX23 and J05AB02 or N03AX24 and J05AB02 or N03AX30 and J05AB02 or N03AA01 and J05AB03 or N03AA02 and J05AB03 or N03AA03 and J05AB03 or N03AA04 and J05AB03 or N03AA30 and J05AB03 or N03AB01 and J05AB03 or N03AB02 and J05AB03 or N03AB03 and J05AB03 or N03AB04 and J05AB03 or N03AB05 and J05AB03 or N03AB52 and J05AB03 or N03AB54 and J05AB03 or N03AC01 and J05AB03 or N03AC02 and J05AB03 or N03AC03 and J05AB03 or N03AD01 and J05AB03 or N03AD02 and J05AB03 or N03AD03 and J05AB03 or N03AD51 and J05AB03 or N03AE01 and J05AB03 or N03AF01 and J05AB03 or N03AF02 and J05AB03 or N03AF03 and J05AB03 or N03AF04 and J05AB03 or N03AG01 and J05AB03 or N03AG02 and J05AB03 or N03AG03 and J05AB03 or N03AG04 and J05AB03 or N03AG05 and J05AB03 or N03AG06 and J05AB03 or N03AX03 and J05AB03 or N03AX07 and J05AB03 or N03AX09 and J05AB03 or N03AX10 and J05AB03 or N03AX11 and J05AB03 or N03AX12 and J05AB03 or N03AX13 and J05AB03 or N03AX14 and J05AB03 or N03AX15 and J05AB03 or N03AX16 and J05AB03 or N03AX17 and J05AB03 or N03AX18 and J05AB03 or N03AX19 and J05AB03 or N03AX21 and J05AB03 or N03AX22 and J05AB03 or N03AX23 and J05AB03 or N03AX24 and J05AB03 or N03AX30 and J05AB03 or N03AA01 and J05AB06 or N03AA02 and J05AB06 or N03AA03 and J05AB06 or N03AA04 and J05AB06 or N03AA30 and J05AB06 or N03AB01 and J05AB06 or N03AB02 and J05AB06 or N03AB03 and J05AB06 or N03AB04 and J05AB06 or N03AB05 and J05AB06 or N03AB52 and J05AB06 or N03AB54 and J05AB06 or N03AC01 and J05AB06 or N03AC02 and J05AB06 or N03AC03 and J05AB06 or N03AD01 and J05AB06 or N03AD02 and J05AB06 or N03AD03 and J05AB06 or N03AD51 and J05AB06 or N03AE01 and J05AB06 or N03AF01 and J05AB06 or N03AF02 and J05AB06 or N03AF03 and J05AB06 or N03AF04 and J05AB06 or N03AG01 and J05AB06 or N03AG02 and J05AB06 or N03AG03 and J05AB06 or N03AG04 and J05AB06 or N03AG05 and J05AB06 or N03AG06 and J05AB06 or N03AX03 and J05AB06 or N03AX07 and J05AB06 or N03AX09 and J05AB06 or N03AX10 and J05AB06 or N03AX11 and J05AB06 or N03AX12 and J05AB06 or N03AX13 and J05AB06 or N03AX14 and J05AB06 or N03AX15 and J05AB06 or N03AX16 and J05AB06 or N03AX17 and J05AB06 or N03AX18 and J05AB06 or N03AX19 and J05AB06 or N03AX21 and J05AB06 or N03AX22 and J05AB06 or N03AX23 and J05AB06 or N03AX24 and J05AB06 or N03AX30 and J05AB06 or N03AA01 and J05AB09 or N03AA02 and J05AB09 or N03AA03 and J05AB09 or N03AA04 and J05AB09 or N03AA30 and J05AB09 or N03AB01 and J05AB09 or N03AB02 and J05AB09 or N03AB03 and J05AB09 or N03AB04 and J05AB09 or N03AB05 and J05AB09 or N03AB52 and J05AB09 or N03AB54 and J05AB09 or N03AC01 and J05AB09 or N03AC02 and J05AB09 or N03AC03 and J05AB09 or N03AD01 and J05AB09 or N03AD02 and J05AB09 or N03AD03 and J05AB09 or N03AD51 and J05AB09 or N03AE01 and J05AB09 or N03AF01 and J05AB09 or N03AF02 and J05AB09 or N03AF03 and J05AB09 or N03AF04 and J05AB09 or N03AG01 and J05AB09 or N03AG02 and J05AB09 or N03AG03 and J05AB09 or N03AG04 and J05AB09 or N03AG05 and J05AB09 or N03AG06 and J05AB09 or N03AX03 and J05AB09 or N03AX07 and J05AB09 or N03AX09 and J05AB09 or N03AX10 and J05AB09 or N03AX11 and J05AB09 or N03AX12 and J05AB09 or N03AX13 and J05AB09 or N03AX14 and J05AB09 or N03AX15 and J05AB09 or N03AX16 and J05AB09 or N03AX17 and J05AB09 or N03AX18 and J05AB09 or N03AX19 and J05AB09 or N03AX21 and J05AB09 or N03AX22 and J05AB09 or N03AX23 and J05AB09 or N03AX24 and J05AB09 or N03AX30 and J05AB09 or N03AA01 and J05AB11 or N03AA02 and J05AB11 or N03AA03 and J05AB11 or N03AA04 and J05AB11 or N03AA30 and J05AB11 or N03AB01 and J05AB11 or N03AB02 and J05AB11 or N03AB03 and J05AB11 or N03AB04 and J05AB11 or N03AB05 and J05AB11 or N03AB52 and J05AB11 or N03AB54 and J05AB11 or N03AC01 and J05AB1 1 or N03AC02 and J05AB1 1 or N03AC03 and J05AB11 or N03AD01 and J05AB11 or N03AD02 and J05AB11 or N03AD03 and J05AB11 or N03AD51 and J05AB1 1 or N03AE01 and J05AB1 1 or N03AF01 and J05AB11 or N03AF02 and J05AB11 or N03AF03 and J05AB11 or N03AF04 and J05AB11 or N03AG01 and J05AB11 or N03AG02 and J05AB11 or N03AG03 and J05AB11 or N03AG04 and J05AB1 1 or N03AG05 and J05AB1 1 or N03AG06 and J05AB11 or N03AX03 and J05AB11 or N03AX07 and J05AB11 or N03AX09 and J05AB11 or N03AX10 and J05AB11 or N03AX11 and J05AB11 or N03AX12 and J05AB11 or N03AX13 and J05AB11 or N03AX14 and J05AB11 or N03AX15 and J05AB11 or N03AX16 and J05AB11 or N03AX17 and J05AB11 or N03AX18 and J05AB11 or N03AX19 and J05AB11 or N03AX21 and J05AB11 or N03AX22 and J05AB11 or N03AX23 and J05AB11 or N03AX24 and J05AB11 or N03AX30 and J05AB11 or N03AA01 and J05AB12 or N03AA02 and J05AB12 or N03AA03 and J05AB12 or N03AA04 and J05AB12 or N03AA30 and J05AB12 or N03AB01 and J05AB12 or N03AB02 and J05AB12 or N03AB03 and J05AB12 or N03AB04 and J05AB12 or N03AB05 and J05AB12 or N03AB52 and J05AB12 or N03AB54 and J05AB12 or N03AC01 and J05AB12 or N03AC02 and J05AB12 or N03AC03 and J05AB12 or N03AD01 and J05AB12 or N03AD02 and J05AB12 or N03AD03 and J05AB12 or N03AD51 and J05AB12 or N03AE01 and J05AB12 or N03AF01 and J05AB12 or N03AF02 and J05AB12 or N03AF03 and J05AB12 or N03AF04 and J05AB12 or N03AG01 and J05AB12 or N03AG02 and J05AB12 or N03AG03 and J05AB12 or N03AG04 and J05AB12 or N03AG05 and J05AB12 or N03AG06 and J05AB12 or N03AX03 and J05AB12 or N03AX07 and J05AB12 or N03AX09 and J05AB12 or N03AX10 and J05AB12 or N03AX11 and J05AB12 or N03AX12 and J05AB12 or N03AX13 and J05AB12 or N03AX14 and J05AB12 or N03AX15 and J05AB12 or N03AX16 and J05AB12 or N03AX17 and J05AB12 or N03AX18 and J05AB12 or N03AX19 and J05AB12 or N03AX21 and J05AB12 or N03AX22 and J05AB12 or N03AX23 and J05AB12 or N03AX24 and J05AB12 or N03AX30 and J05AB12 or N03AA01 and J05AB13 or N03AA02 and J05AB13 or N03AA03 and J05AB13 or N03AA04 and J05AB13 or N03AA30 and J05AB13 or N03AB01 and J05AB13 or N03AB02 and J05AB13 or N03AB03 and J05AB13 or N03AB04 and J05AB13 or N03AB05 and J05AB13 or N03AB52 and J05AB13 or N03AB54 and J05AB13 or N03AC01 and J05AB13 or N03AC02 and J05AB13 or N03AC03 and J05AB13 or N03AD01 and J05AB13 or N03AD02 and J05AB13 or N03AD03 and J05AB13 or N03AD51 and J05AB13 or N03AE01 and J05AB13 or N03AF01 and J05AB13 or N03AF02 and J05AB13 or N03AF03 and J05AB13 or N03AF04 and J05AB13 or N03AG01 and J05AB13 or N03AG02 and J05AB13 or N03AG03 and J05AB13 or N03AG04 and J05AB13 or N03AG05 and J05AB13 or N03AG06 and J05AB13 or N03AX03 and J05AB13 or N03AX07 and J05AB13 or N03AX09 and J05AB13 or N03AX10 and J05AB13 or N03AX11 and J05AB13 or N03AX12 and J05AB13 or N03AX13 and J05AB13 or N03AX14 and J05AB13 or N03AX15 and J05AB13 or N03AX16 and J05AB13 or N03AX17 and J05AB13 or N03AX18 and J05AB13 or N03AX19 and J05AB13 or N03AX21 and J05AB13 or N03AX22 and J05AB13 or N03AX23 and J05AB13 or N03AX24 and J05AB13 or N03AX30 and J05AB13 or N03AA01 and J05AB14 or N03AA02 and J05AB14 or N03AA03 and J05AB14 or N03AA04 and J05AB14 or N03AA30 and J05AB14 or N03AB01 and J05AB14 or N03AB02 and J05AB14 or N03AB03 and J05AB14 or N03AB04 and J05AB14 or N03AB05 and J05AB14 or N03AB52 and J05AB14 or N03AB54 and J05AB14 or N03AC01 and J05AB14 or N03AC02 and J05AB14 or N03AC03 and J05AB14 or N03AD01 and J05AB14 or N03AD02 and J05AB14 or N03AD03 and J05AB14 or N03AD51 and J05AB14 or N03AE01 and J05AB14 or N03AF01 and J05AB14 or N03AF02 and J05AB14 or N03AF03 and J05AB14 or N03AF04 and J05AB14 or N03AG01 and J05AB14 or N03AG02 and J05AB14 or N03AG03 and J05AB14 or N03AG04 and J05AB14 or N03AG05 and J05AB14 or N03AG06 and J05AB14 or N03AX03 and J05AB14 or N03AX07 and J05AB14 or N03AX09 and J05AB14 or N03AX10 and J05AB14 or N03AX11 and J05AB14 or N03AX12 and J05AB14 or N03AX13 and J05AB14 or N03AX14 and J05AB14 or N03AX15 and J05AB14 or N03AX16 and J05AB14 or N03AX17 and J05AB14 or N03AX18 and J05AB14 or N03AX19 and J05AB14 or N03AX21 and J05AB14 or N03AX22 and J05AB14 or N03AX23 and J05AB14 or N03AX24 and J05AB14 or N03AX30 and J05AB14 or
N03AA01 and J05AB15 or N03AA02 and J05AB15 or N03AA03 and J05AB15 or N03AA04 and J05AB15 or N03AA30 and J05AB15 or N03AB01 and J05AB15 or N03AB02 and J05AB15 or N03AB03 and J05AB15 or N03AB04 and J05AB15 or N03AB05 and J05AB15 or N03AB52 and J05AB15 or N03AB54 and J05AB15 or N03AC01 and J05AB15 or N03AC02 and J05AB15 or N03AC03 and J05AB15 or N03AD01 and J05AB15 or N03AD02 and J05AB15 or N03AD03 and J05AB15 or N03AD51 and J05AB15 or N03AE01 and J05AB15 or N03AF01 and J05AB15 or N03AF02 and J05AB15 or N03AF03 and J05AB15 or N03AF04 and J05AB15 or N03AG01 and J05AB15 or N03AG02 and J05AB15 or N03AG03 and J05AB15 or N03AG04 and J05AB15 or N03AG05 and J05AB15 or N03AG06 and J05AB15 or N03AX03 and J05AB15 or N03AX07 and J05AB15 or N03AX09 and J05AB15 or N03AX10 and J05AB15 or N03AX11 and J05AB15 or N03AX12 and J05AB15 or N03AX13 and J05AB15 or N03AX14 and J05AB15 or N03AX15 and J05AB15 or N03AX16 and J05AB15 or N03AX17 and J05AB15 or N03AX18 and J05AB15 or N03AX19 and J05AB15 or N03AX21 and J05AB15 or N03AX22 and J05AB15 or N03AX23 and J05AB15 or N03AX24 and J05AB15 or N03AX30 and J05AB15 or N03AA01 and J05AC02 or N03AA02 and J05AC02 or N03AA03 and J05AC02 or N03AA04 and J05AC02 or N03AA30 and J05AC02 or N03AB01 and J05AC02 or N03AB02 and J05AC02 or N03AB03 and J05AC02 or N03AB04 and J05AC02 or N03AB05 and J05AC02 or N03AB52 and J05AC02 or N03AB54 and J05AC02 or N03AC01 and J05AC02 or N03AC02 and J05AC02 or N03AC03 and J05AC02 or N03AD01 and J05AC02 or N03AD02 and J05AC02 or N03AD03 and J05AC02 or N03AD51 and J05AC02 or N03AE01 and J05AC02 or N03AF01 and J05AC02 or N03AF02 and J05AC02 or N03AF03 and J05AC02 or N03AF04 and J05AC02 or N03AG01 and J05AC02 or N03AG02 and J05AC02 or N03AG03 and J05AC02 or N03AG04 and J05AC02 or N03AG05 and J05AC02 or N03AG06 and J05AC02 or N03AX03 and J05AC02 or N03AX07 and J05AC02 or N03AX09 and J05AC02 or N03AX10 and J05AC02 or N03AX11 and J05AC02 or N03AX12 and J05AC02 or N03AX13 and J05AC02 or N03AX14 and J05AC02 or N03AX15 and J05AC02 or N03AX16 and J05AC02 or N03AX17 and J05AC02 or N03AX18 and J05AC02 or N03AX19 and J05AC02 or N03AX21 and J05AC02 or N03AX22 and J05AC02 or N03AX23 and J05AC02 or N03AX24 and J05AC02 or N03AX30 and J05AC02 or N03AA01 and J05AC03 or N03AA02 and J05AC03 or N03AA03 and J05AC03 or N03AA04 and J05AC03 or N03AA30 and J05AC03 or N03AB01 and J05AC03 or N03AB02 and J05AC03 or N03AB03 and J05AC03 or N03AB04 and J05AC03 or N03AB05 and J05AC03 or N03AB52 and J05AC03 or N03AB54 and J05AC03 or N03AC01 and J05AC03 or N03AC02 and J05AC03 or N03AC03 and J05AC03 or N03AD01 and J05AC03 or N03AD02 and J05AC03 or N03AD03 and J05AC03 or N03AD51 and J05AC03 or N03AE01 and J05AC03 or N03AF01 and J05AC03 or N03AF02 and J05AC03 or N03AF03 and J05AC03 or N03AF04 and J05AC03 or N03AG01 and J05AC03 or N03AG02 and J05AC03 or N03AG03 and J05AC03 or N03AG04 and J05AC03 or N03AG05 and J05AC03 or N03AG06 and J05AC03 or N03AX03 and J05AC03 or N03AX07 and J05AC03 or N03AX09 and J05AC03 or N03AX10 and J05AC03 or N03AX11 and J05AC03 or N03AX12 and J05AC03 or N03AX13 and J05AC03 or N03AX14 and J05AC03 or N03AX15 and J05AC03 or N03AX16 and J05AC03 or N03AX17 and J05AC03 or N03AX18 and J05AC03 or N03AX19 and J05AC03 or N03AX21 and J05AC03 or N03AX22 and J05AC03 or N03AX23 and J05AC03 or N03AX24 and J05AC03 or N03AX30 and J05AC03 or N03AA01 and J05AD01 or N03AA02 and J05AD01 or N03AA03 and J05AD01 or N03AA04 and J05AD01 or N03AA30 and J05AD01 or N03AB01 and J05AD01 or N03AB02 and J05AD01 or N03AB03 and J05AD01 or N03AB04 and J05AD01 or N03AB05 and J05AD01 or N03AB52 and J05AD01 or N03AB54 and J05AD01 or N03AC01 and J05AD01 or N03AC02 and J05AD01 or N03AC03 and J05AD01 or N03AD01 and J05AD01 or N03AD02 and J05AD01 or N03AD03 and J05AD01 or N03AD51 and J05AD01 or N03AE01 and J05AD01 or N03AF01 and J05AD01 or N03AF02 and J05AD01 or N03AF03 and J05AD01 or N03AF04 and J05AD01 or N03AG01 and J05AD01 or N03AG02 and J05AD01 or N03AG03 and J05AD01 or N03AG04 and J05AD01 or N03AG05 and J05AD01 or N03AG06 and J05AD01 or N03AX03 and J05AD01 or N03AX07 and J05AD01 or N03AX09 and J05AD01 or N03AX10 and J05AD01 or N03AX11 and J05AD01 or N03AX12 and J05AD01 or N03AX13 and J05AD01 or N03AX14 and J05AD01 or N03AX15 and J05AD01 or N03AX16 and J05AD01 or N03AX17 and J05AD01 or N03AX18 and J05AD01 or N03AX19 and J05AD01 or N03AX21 and J05AD01 or N03AX22 and J05AD01 or N03AX23 and J05AD01 or N03AX24 and J05AD01 or N03AX30 and J05AD01 or N03AA01 and J05AD02 or N03AA02 and J05AD02 or N03AA03 and J05AD02 or N03AA04 and J05AD02 or N03AA30 and J05AD02 or N03AB01 and J05AD02 or N03AB02 and J05AD02 or N03AB03 and J05AD02 or N03AB04 and J05AD02 or N03AB05 and J05AD02 or N03AB52 and J05AD02 or N03AB54 and J05AD02 or N03AC01 and J05AD02 or N03AC02 and J05AD02 or N03AC03 and J05AD02 or N03AD01 and J05AD02 or N03AD02 and J05AD02 or N03AD03 and J05AD02 or N03AD51 and J05AD02 or N03AE01 and J05AD02 or N03AF01 and J05AD02 or N03AF02 and J05AD02 or N03AF03 and J05AD02 or N03AF04 and J05AD02 or N03AG01 and J05AD02 or N03AG02 and J05AD02 or N03AG03 and J05AD02 or N03AG04 and J05AD02 or N03AG05 and J05AD02 or N03AG06 and J05AD02 or N03AX03 and J05AD02 or N03AX07 and J05AD02 or N03AX09 and J05AD02 or N03AX10 and J05AD02 or N03AX11 and J05AD02 or N03AX12 and J05AD02 or N03AX13 and J05AD02 or N03AX14 and J05AD02 or N03AX15 and J05AD02 or N03AX16 and J05AD02 or N03AX17 and J05AD02 or N03AX18 and J05AD02 or N03AX19 and J05AD02 or N03AX21 and J05AD02 or N03AX22 and J05AD02 or N03AX23 and J05AD02 or N03AX24 and J05AD02 or N03AX30 and J05AD02 or N03AA01 and J05AE01 or N03AA02 and J05AE01 or N03AA03 and J05AE01 or N03AA04 and J05AE01 or N03AA30 and J05AE01 or N03AB01 and J05AE01 or N03AB02 and J05AE01 or N03AB03 and J05AE01 or N03AB04 and J05AE01 or N03AB05 and J05AE01 or N03AB52 and J05AE01 or N03AB54 and J05AE01 or N03AC01 and J05AE01 or N03AC02 and J05AE01 or N03AC03 and J05AE01 or N03AD01 and J05AE01 or N03AD02 and J05AE01 or N03AD03 and J05AE01 or N03AD51 and J05AE01 or N03AE01 and J05AE01 or N03AF01 and J05AE01 or N03AF02 and J05AE01 or N03AF03 and J05AE01 or N03AF04 and J05AE01 or N03AG01 and J05AE01 or N03AG02 and J05AE01 or N03AG03 and J05AE01 or N03AG04 and J05AE01 or N03AG05 and J05AE01 or N03AG06 and J05AE01 or N03AX03 and J05AE01 or N03AX07 and J05AE01 or N03AX09 and J05AE01 or N03AX10 and J05AE01 or N03AX11 and J05AE01 or N03AX12 and J05AE01 or N03AX13 and J05AE01 or N03AX14 and J05AE01 or N03AX15 and J05AE01 or N03AX16 and J05AE01 or N03AX17 and J05AE01 or N03AX18 and J05AE01 or N03AX19 and J05AE01 or N03AX21 and J05AE01 or N03AX22 and J05AE01 or N03AX23 and J05AE01 or N03AX24 and J05AE01 or N03AX30 and J05AE01 or N03AA01 and J05AE02 or N03AA02 and J05AE02 or N03AA03 and J05AE02 or N03AA04 and J05AE02 or N03AA30 and J05AE02 or N03AB01 and J05AE02 or N03AB02 and J05AE02 or N03AB03 and J05AE02 or N03AB04 and J05AE02 or N03AB05 and J05AE02 or N03AB52 and J05AE02 or N03AB54 and J05AE02 or N03AC01 and J05AE02 or N03AC02 and J05AE02 or N03AC03 and J05AE02 or N03AD01 and J05AE02 or N03AD02 and J05AE02 or N03AD03 and J05AE02 or N03AD51 and J05AE02 or N03AE01 and J05AE02 or N03AF01 and J05AE02 or N03AF02 and J05AE02 or N03AF03 and J05AE02 or N03AF04 and J05AE02 or N03AG01 and J05AE02 or N03AG02 and J05AE02 or N03AG03 and J05AE02 or N03AG04 and J05AE02 or N03AG05 and J05AE02 or N03AG06 and J05AE02 or N03AX03 and J05AE02 or N03AX07 and J05AE02 or N03AX09 and J05AE02 or N03AX10 and J05AE02 or N03AX11 and J05AE02 or N03AX12 and J05AE02 or N03AX13 and J05AE02 or N03AX14 and J05AE02 or N03AX15 and J05AE02 or N03AX16 and J05AE02 or N03AX17 and J05AE02 or N03AX18 and J05AE02 or N03AX19 and J05AE02 or N03AX21 and J05AE02 or N03AX22 and J05AE02 or N03AX23 and J05AE02 or N03AX24 and J05AE02 or N03AX30 and J05AE02 or N03AA01 and J05AE03 or N03AA02 and J05AE03 or N03AA03 and J05AE03 or N03AA04 and J05AE03 or N03AA30 and J05AE03 or N03AB01 and J05AE03 or N03AB02 and J05AE03 or N03AB03 and J05AE03 or N03AB04 and J05AE03 or N03AB05 and J05AE03 or N03AB52 and J05AE03 or N03AB54 and J05AE03 or N03AC01 and J05AE03 or N03AC02 and J05AE03 or N03AC03 and J05AE03 or N03AD01 and J05AE03 or N03AD02 and J05AE03 or N03AD03 and J05AE03 or N03AD51 and J05AE03 or N03AE01 and J05AE03 or N03AF01 and J05AE03 or N03AF02 and J05AE03 or N03AF03 and J05AE03 or N03AF04 and J05AE03 or N03AG01 and J05AE03 or N03AG02 and J05AE03 or N03AG03 and J05AE03 or N03AG04 and J05AE03 or N03AG05 and J05AE03 or N03AG06 and J05AE03 or N03AX03 and J05AE03 or N03AX07 and J05AE03 or N03AX09 and J05AE03 or N03AX10 and J05AE03 or N03AX11 and J05AE03 or N03AX12 and J05AE03 or N03AX13 and J05AE03 or N03AX14 and J05AE03 or N03AX15 and J05AE03 or N03AX16 and J05AE03 or N03AX17 and J05AE03 or N03AX18 and J05AE03 or N03AX19 and J05AE03 or N03AX21 and J05AE03 or N03AX22 and J05AE03 or N03AX23 and J05AE03 or N03AX24 and J05AE03 or N03AX30 and J05AE03 or N03AA01 and J05AE04 or N03AA02 and J05AE04 or N03AA03 and J05AE04 or N03AA04 and J05AE04 or N03AA30 and J05AE04 or N03AB01 and J05AE04 or N03AB02 and J05AE04 or N03AB03 and J05AE04 or N03AB04 and J05AE04 or N03AB05 and J05AE04 or N03AB52 and J05AE04 or N03AB54 and J05AE04 or N03AC01 and J05AE04 or N03AC02 and J05AE04 or N03AC03 and J05AE04 or N03AD01 and J05AE04 or N03AD02 and J05AE04 or N03AD03 and J05AE04 or N03AD51 and J05AE04 or N03AE01 and J05AE04 or N03AF01 and J05AE04 or N03AF02 and J05AE04 or N03AF03 and J05AE04 or N03AF04 and J05AE04 or N03AG01 and J05AE04 or N03AG02 and J05AE04 or N03AG03 and J05AE04 or N03AG04 and J05AE04 or N03AG05 and J05AE04 or N03AG06 and J05AE04 or N03AX03 and J05AE04 or N03AX07 and J05AE04 or N03AX09 and J05AE04 or N03AX10 and J05AE04 or N03AX11 and J05AE04 or N03AX12 and J05AE04 or N03AX13 and J05AE04 or N03AX14 and J05AE04 or N03AX15 and J05AE04 or N03AX16 and J05AE04 or N03AX17 and J05AE04 or N03AX18 and J05AE04 or N03AX19 and J05AE04 or N03AX21 and J05AE04 or N03AX22 and J05AE04 or N03AX23 and J05AE04 or N03AX24 and J05AE04 or N03AX30 and J05AE04 or N03AA01 and J05AE05 or N03AA02 and J05AE05 or N03AA03 and J05AE05 or N03AA04 and J05AE05 or N03AA30 and J05AE05 or N03AB01 and J05AE05 or N03AB02 and J05AE05 or N03AB03 and J05AE05 or N03AB04 and J05AE05 or N03AB05 and J05AE05 or N03AB52 and J05AE05 or N03AB54 and J05AE05 or N03AC01 and J05AE05 or N03AC02 and J05AE05 or N03AC03 and J05AE05 or N03AD01 and J05AE05 or N03AD02 and J05AE05 or N03AD03 and J05AE05 or N03AD51 and J05AE05 or N03AE01 and J05AE05 or N03AF01 and J05AE05 or N03AF02 and J05AE05 or N03AF03 and J05AE05 or N03AF04 and J05AE05 or N03AG01 and J05AE05 or N03AG02 and J05AE05 or N03AG03 and J05AE05 or N03AG04 and J05AE05 or N03AG05 and J05AE05 or N03AG06 and J05AE05 or N03AX03 and J05AE05 or N03AX07 and J05AE05 or N03AX09 and J05AE05 or N03AX10 and J05AE05 or N03AX11 and J05AE05 or N03AX12 and J05AE05 or N03AX13 and J05AE05 or N03AX14 and J05AE05 or N03AX15 and J05AE05 or N03AX16 and J05AE05 or N03AX17 and J05AE05 or N03AX18 and J05AE05 or N03AX19 and J05AE05 or N03AX21 and J05AE05 or N03AX22 and J05AE05 or N03AX23 and J05AE05 or N03AX24 and J05AE05 or N03AX30 and J05AE05 or N03AA01 and J05AE07 or N03AA02 and J05AE07 or N03AA03 and J05AE07 or N03AA04 and J05AE07 or N03AA30 and J05AE07 or N03AB01 and J05AE07 or N03AB02 and J05AE07 or N03AB03 and J05AE07 or N03AB04 and J05AE07 or N03AB05 and J05AE07 or N03AB52 and J05AE07 or N03AB54 and J05AE07 or N03AC01 and J05AE07 or N03AC02 and J05AE07 or N03AC03 and J05AE07 or N03AD01 and J05AE07 or N03AD02 and J05AE07 or N03AD03 and J05AE07 or N03AD51 and J05AE07 or N03AE01 and J05AE07 or N03AF01 and J05AE07 or N03AF02 and J05AE07 or N03AF03 and J05AE07 or N03AF04 and J05AE07 or N03AG01 and J05AE07 or N03AG02 and J05AE07 or N03AG03 and J05AE07 or N03AG04 and J05AE07 or N03AG05 and J05AE07 or N03AG06 and J05AE07 or N03AX03 and J05AE07 or N03AX07 and J05AE07 or N03AX09 and J05AE07 or N03AX10 and J05AE07 or N03AX11 and J05AE07 or N03AX12 and J05AE07 or N03AX13 and J05AE07 or N03AX14 and J05AE07 or N03AX15 and J05AE07 or N03AX16 and J05AE07 or N03AX17 and J05AE07 or N03AX18 and J05AE07 or N03AX19 and J05AE07 or N03AX21 and J05AE07 or N03AX22 and J05AE07 or N03AX23 and J05AE07 or N03AX24 and J05AE07 or N03AX30 and J05AE07 or N03AA01 and J05AE08 or N03AA02 and J05AE08 or N03AA03 and J05AE08 or N03AA04 and J05AE08 or N03AA30 and J05AE08 or N03AB01 and J05AE08 or N03AB02 and J05AE08 or N03AB03 and J05AE08 or N03AB04 and J05AE08 or N03AB05 and J05AE08 or N03AB52 and J05AE08 or N03AB54 and J05AE08 or N03AC01 and J05AE08 or N03AC02 and J05AE08 or N03AC03 and J05AE08 or N03AD01 and J05AE08 or N03AD02 and J05AE08 or N03AD03 and J05AE08 or N03AD51 and J05AE08 or N03AE01 and J05AE08 or N03AF01 and J05AE08 or N03AF02 and J05AE08 or N03AF03 and J05AE08 or N03AF04 and J05AE08 or N03AG01 and J05AE08 or N03AG02 and J05AE08 or N03AG03 and J05AE08 or N03AG04 and J05AE08 or N03AG05 and J05AE08 or N03AG06 and J05AE08 or N03AX03 and J05AE08 or N03AX07 and J05AE08 or N03AX09 and J05AE08 or N03AX10 and J05AE08 or N03AX11 and J05AE08 or N03AX12 and J05AE08 or N03AX13 and J05AE08 or N03AX14 and J05AE08 or N03AX15 and J05AE08 or N03AX16 and J05AE08 or N03AX17 and J05AE08 or N03AX18 and J05AE08 or N03AX19 and J05AE08 or N03AX21 and J05AE08 or N03AX22 and J05AE08 or N03AX23 and J05AE08 or N03AX24 and J05AE08 or N03AX30 and J05AE08 or N03AA01 and J05AE09 or N03AA02 and J05AE09 or N03AA03 and J05AE09 or N03AA04 and J05AE09 or N03AA30 and J05AE09 or N03AB01 and J05AE09 or N03AB02 and J05AE09 or N03AB03 and J05AE09 or N03AB04 and J05AE09 or N03AB05 and J05AE09 or N03AB52 and J05AE09 or N03AB54 and J05AE09 or N03AC01 and J05AE09 or N03AC02 and J05AE09 or N03AC03 and J05AE09 or N03AD01 and J05AE09 or N03AD02 and J05AE09 or N03AD03 and J05AE09 or N03AD51 and J05AE09 or N03AE01 and J05AE09 or N03AF01 and J05AE09 or N03AF02 and J05AE09 or N03AF03 and J05AE09 or N03AF04 and J05AE09 or N03AG01 and J05AE09 or N03AG02 and J05AE09 or N03AG03 and J05AE09 or N03AG04 and J05AE09 or N03AG05 and J05AE09 or N03AG06 and J05AE09 or N03AX03 and J05AE09 or N03AX07 and J05AE09 or N03AX09 and J05AE09 or N03AX10 and J05AE09 or N03AX11 and J05AE09 or N03AX12 and J05AE09 or N03AX13 and J05AE09 or N03AX14 and J05AE09 or N03AX15 and J05AE09 or N03AX16 and J05AE09 or N03AX17 and J05AE09 or N03AX18 and J05AE09 or N03AX19 and J05AE09 or N03AX21 and J05AE09 or N03AX22 and J05AE09 or N03AX23 and J05AE09 or N03AX24 and J05AE09 or N03AX30 and J05AE09 or N03AA01 and J05AE10 or N03AA02 and J05AE10 or N03AA03 and J05AE10 or N03AA04 and JO5AE10 or N03AA30 and JO5AE10 or N03AB01 and J05AE10 or N03AB02 and J05AE10 or N03AB03 and J05AE10 or N03AB04 and J05AE10 or N03AB05 and J05AE10 or N03AB52 and J05AE10 or N03AB54 and J05AE10 or N03AC01 and J05AE10 or N03AC02 and JO5AE10 or N03AC03 and J05AE10 or N03AD01 and J05AE10 or N03AD02 and J05AE10 or N03AD03 and J05AE10 or N03AD51 and J05AE10 or N03AE01 and J05AE10 or N03AF01 and J05AE10 or N03AF02 and J05AE10 or N03AF03 and J05AE10 or N03AF04 and J05AE10 or N03AG01 and J05AE10 or N03AG02 and J05AE10 or N03AG03 and J05AE10 or N03AG04 and J05AE10 or N03AG05 and J05AE10 or N03AG06 and J05AE10 or N03AX03 and J05AE10 or N03AX07 and J05AE10 or N03AX09 and J05AE10 or N03AX10 and J05AE10 or N03AX11 and J05AE10 or N03AX12 and J05AE10 or N03AX13 and J05AE10 or N03AX14 and J05AE10 or N03AX15 and J05AE10 or N03AX16 and J05AE10 or N03AX17 and J05AE10 or N03AX18 and J05AE10 or N03AX19 and J05AE10 or N03AX21 and J05AE10 or N03AX22 and J05AE10 or N03AX23 and J05AE10 or N03AX24 and J05AE10 or N03AX30 and J05AE10 or N03AA01 and J05AF01 or N03AA02 and J05AF01 or N03AA03 and J05AF01 or N03AA04 and J05AF01 or N03AA30 and J05AF01 or N03AB01 and J05AF01 or N03AB02 and J05AF01 or N03AB03 and J05AF01 or N03AB04 and JOSAF01 or N03AB05 and J05AF01 or N03AB52 and J05AF01 or N03AB54 and J05AF01 or N03AC01 and J05AF01 or N03AC02 and J05AF01 or N03AC03 and J05AF01 or N03AD01 and J05AF01 or N03AD02 and J05AF01 or N03AD03 and J05AF01 or N03AD51 and J05AF01 or N03AE01 and J05AF01 or N03AF01 and J05AF01 or N03AF02 and J05AF01 or N03AF03 and J05AF01 or N03AF04 and J05AF01 or N03AG01 and J05AF01 or N03AG02 and J05AF01 or N03AG03 and J05AF01 or N03AG04 and J05AF01 or N03AG05 and J05AF01 or N03AG06 and J05AF01 or N03AX03 and J05AF01 or N03AX07 and J05AF01 or N03AX09 and J05AF01 or N03AX10 and J05AF01 or N03AX11 and J05AF01 or N03AX12 and J05AF01 or N03AX13 and J05AF01 or N03AX14 and J05AF01 or N03AX15 and J05AF01 or N03AX16 and J05AF01 or N03AX17 and J05AF01 or N03AX18 and J05AF01 or N03AX19 and J05AF01 or N03AX21 and J05AF01 or N03AX22 and J05AF01 or N03AX23 and J05AF01 or N03AX24 and J05AF01 or N03AX30 and J05AF01 or N03AA01 and J05AF02 or N03AA02 and J05AF02 or N03AA03 and J05AF02 or N03AA04 and J05AF02 or N03AA30 and J05AF02 or N03AB01 and J05AF02 or N03AB02 and J05AF02 or N03AB03 and J05AF02 or N03AB04 and J05AF02 or N03AB05 and J05AF02 or N03AB52 and J05AF02 or N03AB54 and J05AF02 or N03AC01 and J05AF02 or N03AC02 and J05AF02 or N03AC03 and J05AF02 or N03AD01 and J05AF02 or N03AD02 and J05AF02 or N03AD03 and J05AF02 or N03AD51 and J05AF02 or N03AE01 and J05AF02 or N03AF01 and J05AF02 or N03AF02 and J05AF02 or N03AF03 and J05AF02 or N03AF04 and J05AF02 or N03AG01 and J05AF02 or N03AG02 and J05AF02 or N03AG03 and J05AF02 or N03AG04 and J05AF02 or N03AG05 and J05AF02 or N03AG06 and J05AF02 or N03AX03 and J05AF02 or N03AX07 and J05AF02 or N03AX09 and J05AF02 or N03AX10 and J05AF02 or N03AX11 and J05AF02 or N03AX12 and J05AF02 or N03AX13 and J05AF02 or N03AX14 and J05AF02 or N03AX15 and J05AF02 or N03AX16 and J05AF02 or N03AX17 and J05AF02 or N03AX18 and J05AF02 or N03AX19 and J05AF02 or N03AX21 and J05AF02 or N03AX22 and J05AF02 or N03AX23 and J05AF02 or N03AX24 and J05AF02 or N03AX30 and J05AF02 or N03AA01 and J05AF03 or N03AA02 and J05AF03 or N03AA03 and J05AF03 or N03AA04 and J05AF03 or N03AA30 and J05AF03 or N03AB01 and J05AF03 or N03AB02 and J05AF03 or N03AB03 and J05AF03 or N03AB04 and J05AF03 or N03AB05 and J05AF03 or N03AB52 and J05AF03 or N03AB54 and J05AF03 or N03AC01 and J05AF03 or N03AC02 and J05AF03 or N03AC03 and J05AF03 or N03AD01 and J05AF03 or N03AD02 and J05AF03 or N03AD03 and J05AF03 or N03AD51 and J05AF03 or N03AE01 and J05AF03 or N03AF01 and J05AF03 or N03AF02 and J05AF03 or N03AF03 and J05AF03 or N03AF04 and J05AF03 or N03AG01 and J05AF03 or N03AG02 and J05AF03 or N03AG03 and J05AF03 or N03AG04 and J05AF03 or N03AG05 and J05AF03 or N03AG06 and J05AF03 or N03AX03 and J05AF03 or N03AX07 and J05AF03 or N03AX09 and J05AF03 or N03AX10 and J05AF03 or N03AX11 and J05AF03 or N03AX12 and J05AF03 or N03AX13 and J05AF03 or N03AX14 and J05AF03 or N03AX15 and J05AF03 or N03AX16 and J05AF03 or N03AX17 and J05AF03 or N03AX18 and J05AF03 or N03AX19 and J05AF03 or N03AX21 and J05AF03 or N03AX22 and J05AF03 or N03AX23 and J05AF03 or N03AX24 and J05AF03 or N03AX30 and J05AF03 or N03AA01 and J05AF04 or N03AA02 and J05AF04 or N03AA03 and J05AF04 or N03AA04 and J05AF04 or N03AA30 and J05AF04 or N03AB01 and J05AF04 or N03AB02 and J05AF04 or N03AB03 and J05AF04 or N03AB04 and J05AF04 or N03AB05 and J05AF04 or N03AB52 and J05AF04 or N03AB54 and J05AF04 or N03AC01 and J05AF04 or N03AC02 and J05AF04 or N03AC03 and J05AF04 or N03AD01 and J05AF04 or N03AD02 and J05AF04 or N03AD03 and J05AF04 or N03AD51 and J05AF04 or N03AE01 and J05AF04 or N03AF01 and J05AF04 or N03AF02 and J05AF04 or N03AF03 and J05AF04 or N03AF04 and J05AF04 or N03AG01 and J05AF04 or N03AG02 and J05AF04 or N03AG03 and J05AF04 or N03AG04 and J05AF04 or N03AG05 and J05AF04 or N03AG06 and J05AF04 or N03AX03 and J05AF04 or N03AX07 and J05AF04 or N03AX09 and J05AF04 or N03AX10 and J05AF04 or N03AX11 and J05AF04 or N03AX12 and J05AF04 or N03AX13 and J05AF04 or N03AX14 and J05AF04 or N03AX15 and J05AF04 or N03AX16 and J05AF04 or N03AX17 and J05AF04 or N03AX18 and J05AF04 or N03AX19 and J05AF04 or N03AX21 and J05AF04 or N03AX22 and J05AF04 or N03AX23 and J05AF04 or N03AX24 and J05AF04 or N03AX30 and J05AF04 or N03AA01 and J05AF05 or N03AA02 and J05AF05 or N03AA03 and J05AF05 or N03AA04 and J05AF05 or N03AA30 and J05AF05 or N03AB01 and J05AF05 or N03AB02 and J05AF05 or N03AB03 and J05AF05 or N03AB04 and J05AF05 or N03AB05 and J05AF05 or N03AB52 and J05AF05 or N03AB54 and J05AF05 or N03AC01 and J05AF05 or N03AC02 and J05AF05 or N03AC03 and J05AF05 or N03AD01 and J05AF05 or N03AD02 and J05AF05 or N03AD03 and J05AF05 or N03AD51 and J05AF05 or N03AE01 and J05AF05 or N03AF01 and J05AF05 or N03AF02 and J05AF05 or N03AF03 and J05AF05 or N03AF04 and J05AF05 or N03AG01 and J05AF05 or N03AG02 and J05AF05 or N03AG03 and J05AF05 or N03AG04 and J05AF05 or N03AG05 and J05AF05 or N03AG06 and J05AF05 or N03AX03 and J05AF05 or N03AX07 and J05AF05 or N03AX09 and J05AF05 or N03AX10 and J05AF05 or N03AX11 and J05AF05 or N03AX12 and J05AF05 or N03AX13 and J05AF05 or N03AX14 and J05AF05 or N03AX15 and J05AF05 or N03AX16 and J05AF05 or N03AX17 and J05AF05 or N03AX18 and J05AF05 or N03AX19 and J05AF05 or N03AX21 and J05AF05 or N03AX22 and J05AF05 or N03AX23 and J05AF05 or N03AX24 and J05AF05 or N03AX30 and J05AF05 or N03AA01 and J05AF06 or N03AA02 and J05AF06 or N03AA03 and J05AF06 or N03AA04 and J05AF06 or N03AA30 and J05AF06 or N03AB01 and J05AF06 or N03AB02 and J05AF06 or N03AB03 and J05AF06 or N03AB04 and J05AF06 or N03AB05 and J05AF06 or N03AB52 and J05AF06 or N03AB54 and J05AF06 or N03AC01 and J05AF06 or N03AC02 and J05AF06 or N03AC03 and J05AF06 or N03AD01 and J05AF06 or N03AD02 and J05AF06 or N03AD03 and J05AF06 or N03AD51 and J05AF06 or N03AE01 and J05AF06 or N03AF01 and J05AF06 or N03AF02 and J05AF06 or N03AF03 and J05AF06 or N03AF04 and J05AF06 or N03AG01 and J05AF06 or N03AG02 and J05AF06 or N03AG03 and J05AF06 or N03AG04 and J05AF06 or N03AG05 and J05AF06 or N03AG06 and J05AF06 or N03AX03 and J05AF06 or N03AX07 and J05AF06 or N03AX09 and J05AF06 or N03AX10 and J05AF06 or N03AX11 and J05AF06 or N03AX12 and J05AF06 or N03AX13 and J05AF06 or N03AX14 and J05AF06 or N03AX15 and J05AF06 or N03AX16 and J05AF06 or N03AX17 and J05AF06 or N03AX18 and J05AF06 or N03AX19 and J05AF06 or N03AX21 and J05AF06 or N03AX22 and J05AF06 or N03AX23 and J05AF06 or N03AX24 and J05AF06 or N03AX30 and J05AF06 or N03AA01 and J05AF07 or N03AA02 and J05AF07 or N03AA03 and J05AF07 or N03AA04 and J05AF07 or N03AA30 and J05AF07 or N03AB01 and J05AF07 or N03AB02 and J05AF07 or N03AB03 and J05AF07 or N03AB04 and J05AF07 or N03AB05 and J05AF07 or N03AB52 and J05AF07 or N03AB54 and J05AF07 or N03AC01 and J05AF07 or N03AC02 and J05AF07 or N03AC03 and J05AF07 or N03AD01 and J05AF07 or N03AD02 and J05AF07 or N03AD03 and J05AF07 or N03AD51 and J05AF07 or N03AE01 and J05AF07 or N03AF01 and J05AF07 or N03AF02 and J05AF07 or N03AF03 and J05AF07 or N03AF04 and J05AF07 or N03AG01 and J05AF07 or N03AG02 and J05AF07 or N03AG03 and J05AF07 or N03AG04 and J05AF07 or N03AG05 and J05AF07 or N03AG06 and J05AF07 or N03AX03 and J05AF07 or N03AX07 and J05AF07 or N03AX09 and J05AF07 or N03AX10 and J05AF07 or N03AX11 and J05AF07 or N03AX12 and J05AF07 or N03AX13 and J05AF07 or N03AX14 and J05AF07 or N03AX15 and J05AF07 or N03AX16 and J05AF07 or N03AX17 and J05AF07 or N03AX18 and J05AF07 or N03AX19 and J05AF07 or N03AX21 and J05AF07 or N03AX22 and J05AF07 or N03AX23 and J05AF07 or N03AX24 and J05AF07 or N03AX30 and J05AF07 or N03AA01 and J05AF08 or N03AA02 and J05AF08 or N03AA03 and J05AF08 or N03AA04 and J05AF08 or N03AA30 and J05AF08 or N03AB01 and J05AF08 or N03AB02 and J05AF08 or N03AB03 and J05AF08 or N03AB04 and J05AF08 or N03AB05 and J05AF08 or N03AB52 and J05AF08 or N03AB54 and J05AF08 or N03AC01 and J05AF08 or N03AC02 and J05AF08 or N03AC03 and J05AF08 or N03AD01 and J05AF08 or N03AD02 and J05AF08 or N03AD03 and J05AF08 or N03AD51 and J05AF08 or N03AE01 and J05AF08 or N03AF01 and J05AF08 or N03AF02 and J05AF08 or N03AF03 and J05AF08 or N03AF04 and J05AF08 or N03AG01 and J05AF08 or N03AG02 and J05AF08 or N03AG03 and J05AF08 or N03AG04 and J05AF08 or N03AG05 and J05AF08 or N03AG06 and J05AF08 or N03AX03 and J05AF08 or N03AX07 and J05AF08 or N03AX09 and J05AF08 or N03AX10 and J05AF08 or N03AX11 and J05AF08 or N03AX12 and J05AF08 or N03AX13 and J05AF08 or N03AX14 and J05AF08 or N03AX15 and J05AF08 or N03AX16 and J05AF08 or N03AX17 and J05AF08 or N03AX18 and J05AF08 or N03AX19 and J05AF08 or N03AX21 and J05AF08 or N03AX22 and J05AF08 or N03AX23 and J05AF08 or N03AX24 and J05AF08 or N03AX30 and J05AF08 or N03AA01 and J05AF09 or N03AA02 and J05AF09 or N03AA03 and J05AF09 or N03AA04 and J05AF09 or N03AA30 and J05AF09 or N03AB01 and J05AF09 or N03AB02 and J05AF09 or N03AB03 and J05AF09 or N03AB04 and J05AF09 or N03AB05 and J05AF09 or N03AB52 and J05AF09 or N03AB54 and J05AF09 or N03AC01 and J05AF09 or N03AC02 and J05AF09 or N03AC03 and J05AF09 or N03AD01 and J05AF09 or N03AD02 and J05AF09 or N03AD03 and J05AF09 or N03AD51 and J05AF09 or N03AE01 and J05AF09 or N03AF01 and J05AF09 or N03AF02 and J05AF09 or N03AF03 and J05AF09 or N03AF04 and J05AF09 or N03AG01 and J05AF09 or N03AG02 and J05AF09 or N03AG03 and J05AF09 or N03AG04 and J05AF09 or N03AG05 and J05AF09 or N03AG06 and J05AF09 or N03AX03 and J05AF09 or N03AX07 and J05AF09 or N03AX09 and J05AF09 or N03AX10 and J05AF09 or N03AX11 and J05AF09 or N03AX12 and J05AF09 or N03AX13 and J05AF09 or N03AX14 and J05AF09 or N03AX15 and J05AF09 or N03AX16 and J05AF09 or N03AX17 and J05AF09 or N03AX18 and J05AF09 or N03AX19 and J05AF09 or N03AX21 and J05AF09 or N03AX22 and J05AF09 or N03AX23 and J05AF09 or N03AX24 and J05AF09 or N03AX30 and J05AF09 or N03AA01 and J05AF10 or N03AA02 and J05AF10 or N03AA03 and J05AF10 or N03AA04 and J05AF10 or N03AA30 and J05AF10 or N03AB01 and J05AF10 or N03AB02 and J05AF10 or N03AB03 and J05AF10 or N03AB04 and J05AF10 or N03AB05 and J05AF10 or N03AB52 and J05AF10 or N03AB54 and J05AF10 or N03AC01 and J05AF10 or N03AC02 and J05AF10 or N03AC03 and J05AF10 or N03AD01 and J05AF10 or N03AD02 and JO5AF10 or N03AD03 and J05AF10 or N03AD51 and J05AF10 or N03AE01 and J05AF10 or N03AF01 and J05AF10 or N03AF02 and J05AF10 or N03AF03 and J05AF10 or N03AF04 and J05AF10 or N03AG01 and J05AF10 or N03AG02 and J05AF10 or N03AG03 and J05AF10 or N03AG04 and J05AF10 or N03AG05 and J05AF10 or N03AG06 and J05AF10 or N03AX03 and J05AF10 or N03AX07 and J05AF10 or N03AX09 and J05AF10 or N03AX10 and J05AF10 or N03AX11 and J05AF10 or N03AX12 and J05AF10 or N03AX13 and J05AF10 or N03AX14 and J05AF10 or N03AX15 and J05AF10 or N03AX16 and J05AF10 or N03AX17 and J05AF10 or N03AX18 and J05AF10 or N03AX19 and J05AF10 or N03AX21 and J05AF10 or N03AX22 and J05AF10 or N03AX23 and J05AF10 or N03AX24 and J05AF10 or N03AX30 and J05AF10 or N03AA01 and J05AF11 or N03AA02 and J05AF11 or N03AA03 and J05AF11 or N03AA04 and J05AF11 or N03AA30 and J05AF11 or N03AB01 and J05AF11 or N03AB02 and J05AF11 or N03AB03 and J05AF11 or N03AB04 and J05AF11 or N03AB05 and J05AF11 or N03AB52 and J05AF11 or N03AB54 and J05AF11 or N03AC01 and J05AF11 or N03AC02 and J05AF11 or N03AC03 and J05AF11 or N03AD01 and J05AF11 or N03AD02 and J05AF11 or N03AD03 and J05AF11 or N03AD51 and J05AF11 or N03AE01 and J05AF11 or N03AF01 and J05AF11 or N03AF02 and J05AF11 or N03AF03 and J05AF11 or N03AF04 and J05AF11 or N03AG01 and J05AF11 or N03AG02 and J05AF11 or N03AG03 and J05AF11 or N03AG04 and J05AF11 or N03AG05 and J05AF11 or N03AG06 and J05AF11 or N03AX03 and J05AF11 or N03AX07 and J05AF11 or N03AX09 and J05AF11 or N03AX10 and J05AF11 or N03AX11 and J05AF11 or N03AX12 and J05AF11 or N03AX13 and J05AF11 or N03AX14 and J05AF11 or N03AX15 and J05AF11 or N03AX16 and J05AF11 or N03AX17 and J05AF11 or N03AX18 and J05AF11 or N03AX19 and J05AF11 or N03AX21 and J05AF11 or N03AX22 and J05AF11 or N03AX23 and J05AF11 or N03AX24 and J05AF11 or N03AX30 and J05AF11 or N03AA01 and J05AF12 or N03AA02 and J05AF12 or N03AA03 and J05AF12 or N03AA04 and J05AF12 or N03AA30 and J05AF12 or N03AB01 and J05AF12 or N03AB02 and J05AF12 or N03AB03 and J05AF12 or N03AB04 and J05AF12 or N03AB05 and J05AF12 or N03AB52 and J05AF12 or N03AB54 and J05AF12 or N03AC01 and J05AF12 or N03AC02 and J05AF12 or N03AC03 and J05AF12 or N03AD01 and J05AF12 or N03AD02 and J05AF12 or N03AD03 and J05AF12 or N03AD51 and J05AF12 or N03AE01 and J05AF12 or N03AF01 and J05AF12 or N03AF02 and J05AF12 or N03AF03 and J05AF12 or N03AF04 and J05AF12 or N03AG01 and J05AF12 or N03AG02 and J05AF12 or N03AG03 and J05AF12 or N03AG04 and J05AF12 or N03AG05 and J05AF12 or N03AG06 and J05AF12 or N03AX03 and J05AF12 or N03AX07 and J05AF12 or N03AX09 and J05AF12 or N03AX10 and J05AF12 or N03AX11 and J05AF12 or N03AX12 and J05AF12 or N03AX13 and J05AF12 or N03AX14 and J05AF12 or N03AX15 and J05AF12 or N03AX16 and J05AF12 or N03AX17 and J05AF12 or N03AX18 and J05AF12 or N03AX19 and J05AF12 or N03AX21 and J05AF12 or N03AX22 and J05AF12 or N03AX23 and J05AF12 or N03AX24 and J05AF12 or N03AX30 and J05AF12 or N03AA01 and J05AF13 or N03AA02 and J05AF13 or N03AA03 and J05AF13 or N03AA04 and J05AF13 or N03AA30 and J05AF13 or N03AB01 and J05AF13 or N03AB02 and J05AF13 or N03AB03 and J05AF13 or N03AB04 and J05AF13 or N03AB05 and J05AF13 or N03AB52 and J05AF13 or N03AB54 and J05AF13 or N03AC01 and J05AF13 or N03AC02 and J05AF13 or N03AC03 and J05AF13 or N03AD01 and J05AF13 or N03AD02 and J05AF13 or N03AD03 and J05AF13 or N03AD51 and J05AF13 or N03AE01 and J05AF13 or N03AF01 and J05AF13 or N03AF02 and J05AF13 or N03AF03 and J05AF13 or N03AF04 and J05AF13 or N03AG01 and J05AF13 or N03AG02 and J05AF13 or N03AG03 and J05AF13 or N03AG04 and J05AF13 or N03AG05 and J05AF13 or N03AG06 and J05AF13 or N03AX03 and J05AF13 or N03AX07 and J05AF13 or N03AX09 and J05AF13 or N03AX10 and J05AF13 or N03AX11 and J05AF13 or N03AX12 and J05AF13 or N03AX13 and J05AF13 or N03AX14 and J05AF13 or N03AX15 and J05AF13 or N03AX16 and J05AF13 or N03AX17 and J05AF13 or N03AX18 and J05AF13 or N03AX19 and J05AF13 or N03AX21 and J05AF13 or N03AX22 and J05AF13 or N03AX23 and J05AF13 or N03AX24 and J05AF13 or N03AX30 and J05AF13 or N03AA01 and J05AG01 or N03AA02 and J05AG01 or N03AA03 and J05AG01 or N03AA04 and J05AG01 or N03AA30 and J05AG01 or N03AB01 and J05AG01 or N03AB02 and J05AG01 or N03AB03 and J05AG01 or N03AB04 and J05AG01 or N03AB05 and J05AG01 or N03AB52 and J05AG01 or N03AB54 and J05AG01 or N03AC01 and J05AG01 or N03AC02 and J05AG01 or N03AC03 and J05AG01 or N03AD01 and J05AG01 or N03AD02 and J05AG01 or N03AD03 and J05AG01 or N03AD51 and J05AG01 or N03AE01 and J05AG01 or N03AF01 and J05AG01 or N03AF02 and J05AG01 or N03AF03 and J05AG01 or N03AF04 and J05AG01 or N03AG01 and J05AG01 or N03AG02 and J05AG01 or N03AG03 and J05AG01 or N03AG04 and J05AG01 or N03AG05 and J05AG01 or N03AG06 and J05AG01 or N03AX03 and J05AG01 or N03AX07 and J05AG01 or N03AX09 and J05AG01 or N03AX10 and J05AG01 or N03AX11 and J05AG01 or N03AX12 and J05AG01 or N03AX13 and J05AG01 or N03AX14 and J05AG01 or N03AX15 and J05AG01 or N03AX16 and J05AG01 or N03AX17 and J05AG01 or N03AX18 and J05AG01 or N03AX19 and J05AG01 or N03AX21 and J05AG01 or N03AX22 and J05AG01 or N03AX23 and J05AG01 or N03AX24 and J05AG01 or N03AX30 and J05AG01 or N03AA01 and J05AG02 or N03AA02 and J05AG02 or N03AA03 and J05AG02 or N03AA04 and J05AG02 or N03AA30 and J05AG02 or N03AB01 and J05AG02 or N03AB02 and J05AG02 or N03AB03 and J05AG02 or N03AB04 and J05AG02 or N03AB05 and J05AG02 or N03AB52 and J05AG02 or N03AB54 and J05AG02 or N03AC01 and J05AG02 or N03AC02 and J05AG02 or N03AC03 and J05AG02 or N03AD01 and J05AG02 or N03AD02 and J05AG02 or N03AD03 and J05AG02 or N03AD51 and J05AG02 or N03AE01 and J05AG02 or N03AF01 and J05AG02 or N03AF02 and J05AG02 or N03AF03 and J05AG02 or N03AF04 and J05AG02 or N03AG01 and J05AG02 or N03AG02 and J05AG02 or N03AG03 and J05AG02 or N03AG04 and J05AG02 or N03AG05 and J05AG02 or N03AG06 and J05AG02 or N03AX03 and J05AG02 or N03AX07 and J05AG02 or N03AX09 and J05AG02 or N03AX10 and J05AG02 or N03AX11 and J05AG02 or N03AX12 and J05AG02 or N03AX13 and J05AG02 or N03AX14 and J05AG02 or N03AX15 and J05AG02 or N03AX16 and J05AG02 or N03AX17 and J05AG02 or N03AX18 and J05AG02 or N03AX19 and J05AG02 or N03AX21 and J05AG02 or N03AX22 and J05AG02 or N03AX23 and J05AG02 or N03AX24 and J05AG02 or N03AX30 and J05AG02 or N03AA01 and J05AG03 or N03AA02 and J05AG03 or N03AA03 and J05AG03 or N03AA04 and J05AG03 or N03AA30 and J05AG03 or N03AB01 and J05AG03 or N03AB02 and J05AG03 or N03AB03 and J05AG03 or N03AB04 and J05AG03 or N03AB05 and J05AG03 or N03AB52 and J05AG03 or N03AB54 and J05AG03 or N03AC01 and J05AG03 or N03AC02 and J05AG03 or N03AC03 and J05AG03 or N03AD01 and J05AG03 or N03AD02 and J05AG03 or N03AD03 and J05AG03 or N03AD51 and J05AG03 or N03AE01 and J05AG03 or N03AF01 and J05AG03 or N03AF02 and J05AG03 or N03AF03 and J05AG03 or N03AF04 and J05AG03 or N03AG01 and J05AG03 or N03AG02 and J05AG03 or N03AG03 and J05AG03 or N03AG04 and J05AG03 or N03AG05 and J05AG03 or N03AG06 and J05AG03 or N03AX03 and J05AG03 or N03AX07 and J05AG03 or N03AX09 and J05AG03 or N03AX10 and J05AG03 or N03AX11 and J05AG03 or N03AX12 and J05AG03 or N03AX13 and J05AG03 or N03AX14 and J05AG03 or N03AX15 and J05AG03 or N03AX16 and J05AG03 or N03AX17 and J05AG03 or N03AX18 and J05AG03 or _{.}N03AX19 and J05AG03 or N03AX21 and J05AG03 or N03AX22 and J05AG03 or N03AX23 and J05AG03 or N03AX24 and J05AG03 or N03AX30 and J05AG03 or N03AA01 and J05AG04 or N03AA02 and J05AG04 or N03AA03 and J05AG04 or N03AA04 and J05AG04 or N03AA30 and J05AG04 or N03AB01 and J05AG04 or N03AB02 and J05AG04 or N03AB03 and J05AG04 or N03AB04 and J05AG04 or N03AB05 and J05AG04 or N03AB52 and J05AG04 or N03AB54 and J05AG04 or N03AC01 and J05AG04 or N03AC02 and J05AG04 or N03AC03 and J05AG04 or N03AD01 and J05AG04 or N03AD02 and J05AG04 or N03AD03 and J05AG04 or N03AD51 and J05AG04 or N03AE01 and J05AG04 or N03AF01 and J05AG04 or N03AF02 and J05AG04 or N03AF03 and J05AG04 or N03AF04 and J05AG04 or N03AG01 and J05AG04 or N03AG02 and J05AG04 or N03AG03 and J05AG04 or N03AG04 and J05AG04 or N03AG05 and J05AG04 or N03AG06 and J05AG04 or N03AX03 and J05AG04 or N03AX07 and J05AG04 or N03AX09 and J05AG04 or N03AX10 and J05AG04 or N03AX11 and J05AG04 or N03AX12 and J05AG04 or N03AX13 and J05AG04 or N03AX14 and J05AG04 or N03AX15 and J05AG04 or N03AX16 and J05AG04 or N03AX17 and J05AG04 or N03AX18 and J05AG04 or N03AX19 and J05AG04 or N03AX21 and J05AG04 or N03AX22 and J05AG04 or N03AX23 and J05AG04 or N03AX24 and J05AG04 or N03AX30 and J05AG04 or N03AA01 and J05AG05 or N03AA02 and J05AG05 or N03AA03 and J05AG05 or N03AA04 and J05AG05 or N03AA30 and J05AG05 or N03AB01 and J05AG05 or N03AB02 and J05AG05 or N03AB03 and J05AG05 or N03AB04 and J05AG05 or N03AB05 and J05AG05 or N03AB52 and J05AG05 or N03AB54 and J05AG05 or N03AC01 and J05AG05 or N03AC02 and J05AG05 or N03AC03 and J05AG05 or N03AD01 and J05AG05 or N03AD02 and J05AG05 or N03AD03 and J05AG05 or N03AD51 and J05AG05 or N03AE01 and J05AG05 or N03AF01 and J05AG05 or N03AF02 and J05AG05 or N03AF03 and J05AG05 or N03AF04 and J05AG05 or N03AG01 and J05AG05 or N03AG02 and J05AG05 or N03AG03 and J05AG05 or N03AG04 and J05AG05 or N03AG05 and J05AG05 or N03AG06 and J05AG05 or N03AX03 and J05AG05 or N03AX07 and J05AG05 or N03AX09 and J05AG05 or N03AX10 and J05AG05 or N03AX11 and J05AG05 or N03AX12 and J05AG05 or N03AX13 and J05AG05 or N03AX14 and J05AG05 or N03AX15 and J05AG05 or N03AX16 and J05AG05 or N03AX17 and J05AG05 or N03AX18 and J05AG05 or N03AX19 and J05AG05 or N03AX21 and J05AG05 or N03AX22 and J05AG05 or N03AX23 and J05AG05 or N03AX24 and J05AG05 or N03AX30 and J05AG05 or N03AA01 and J05AG06 or N03AA02 and J05AG06 or N03AA03 and J05AG06 or N03AA04 and J05AG06 or N03AA30 and J05AG06 or N03AB01 and J05AG06 or N03AB02 and J05AG06 or N03AB03 and J05AG06 or N03AB04 and J05AG06 or N03AB05 and J05AG06 or N03AB52 and J05AG06 or N03AB54 and J05AG06 or N03AC01 and J05AG06 or N03AC02 and J05AG06 or N03AC03 and J05AG06 or N03AD01 and J05AG06 or N03AD02 and J05AG06 or N03AD03 and J05AG06 or N03AD51 and J05AG06 or N03AE01 and J05AG06 or N03AF01 and J05AG06 or N03AF02 and J05AG06 or N03AF03 and J05AG06 or N03AF04 and J05AG06 or N03AG01 and J05AG06 or N03AG02 and J05AG06 or N03AG03 and J05AG06 or N03AG04 and J05AG06 or N03AG05 and J05AG06 or N03AG06 and J05AG06 or N03AX03 and J05AG06 or N03AX07 and J05AG06 or N03AX09 and J05AG06 or N03AX10 and J05AG06 or N03AX11 and J05AG06 or N03AX12 and J05AG06 or N03AX13 and J05AG06 or N03AX14 and J05AG06 or N03AX15 and J05AG06 or N03AX16 and J05AG06 or N03AX17 and J05AG06 or N03AX18 and J05AG06 or N03AX19 and J05AG06 or N03AX21 and J05AG06 or N03AX22 and J05AG06 or N03AX23 and J05AG06 or N03AX24 and J05AG06 or N03AX30 and J05AG06 or N03AA01 and J05AH01 or N03AA02 and J05AH01 or N03AA03 and J05AH01 or N03AA04 and J05AH01 or N03AA30 and J05AH01 or N03AB01 and J05AH01 or N03AB02 and J05AH01 or N03AB03 and J05AH01 or N03AB04 and J05AH01 or N03AB05 and J05AH01 or N03AB52 and J05AH01 or N03AB54 and J05AH01 or N03AC01 and J05AH01 or N03AC02 and J05AH01 or N03AC03 and J05AH01 or N03AD01 and J05AH01 or N03AD02 and J05AH01 or N03AD03 and J05AH01 or N03AD51 and J05AH01 or N03AE01 and J05AH01 or N03AF01 and J05AH01 or N03AF02 and J05AH01 or N03AF03 and J05AH01 or N03AF04 and J05AH01 or N03AG01 and J05AH01 or N03AG02 and J05AH01 or N03AG03 and J05AH01 or N03AG04 and J05AH01 or N03AG05 and J05AH01 or N03AG06 and J05AH01 or N03AX03 and J05AH01 or N03AX07 and J05AH01 or N03AX09 and J05AH01 or N03AX10 and J05AH01 or N03AX11 and J05AH01 or N03AX12 and J05AH01 or N03AX13 and J05AH01 or N03AX14 and J05AH01 or N03AX15 and J05AH01 or N03AX16 and J05AH01 or N03AX17 and J05AH01 or N03AX18 and J05AH01 or N03AX19 and J05AH01 or N03AX21 and J05AH01 or N03AX22 and J05AH01 or N03AX23 and J05AH01 or N03AX24 and J05AH01 or N03AX30 and J05AH01 or N03AA01 and J05AH02 or N03AA02 and J05AH02 or N03AA03 and J05AH02 or N03AA04 and J05AH02 or N03AA30 and J05AH02 or N03AB01 and J05AH02 or N03AB02 and J05AH02 or N03AB03 and J05AH02 or N03AB04 and J05AH02 or N03AB05 and J05AH02 or N03AB52 and J05AH02 or N03AB54 and J05AH02 or N03AC01 and J05AH02 or N03AC02 and J05AH02 or N03AC03 and J05AH02 or N03AD01 and J05AH02 or N03AD02 and J05AH02 or N03AD03 and J05AH02 or N03AD51 and J05AH02 or N03AE01 and J05AH02 or N03AF01 and J05AH02 or N03AF02 and J05AH02 or N03AF03 and J05AH02 or N03AF04 and J05AH02 or N03AG01 and J05AH02 or N03AG02 and J05AH02 or N03AG03 and J05AH02 or N03AG04 and J05AH02 or N03AG05 and J05AH02 or N03AG06 and J05AH02 or N03AX03 and J05AH02 or N03AX07 and J05AH02 or N03AX09 and J05AH02 or N03AX10 and J05AH02 or N03AX11 and J05AH02 or N03AX12 and J05AH02 or N03AX13 and J05AH02 or N03AX14 and J05AH02 or N03AX15 and J05AH02 or N03AX16 and J05AH02 or N03AX17 and J05AH02 or N03AX18 and J05AH02 or N03AX19 and J05AH02 or N03AX21 and J05AH02 or N03AX22 and J05AH02 or N03AX23 and J05AH02 or N03AX24 and J05AH02 or N03AX30 and J05AH02 or N03AA01 and J05AH03 or N03AA02 and J05AH03 or N03AA03 and J05AH03 or N03AA04 and J05AH03 or N03AA30 and J05AH03 or N03AB01 and J05AH03 or N03AB02 and J05AH03 or N03AB03 and J05AH03 or N03AB04 and J05AH03 or N03AB05 and J05AH03 or N03AB52 and J05AH03 or N03AB54 and J05AH03 or N03AC01 and J05AH03 or N03AC02 and J05AH03 or N03AC03 and J05AH03 or N03AD01 and J05AH03 or N03AD02 and J05AH03 or N03AD03 and J05AH03 or N03AD51 and J05AH03 or N03AE01 and J05AH03 or N03AF01 and J05AH03 or N03AF02 and J05AH03 or N03AF03 and J05AH03 or N03AF04 and J05AH03 or N03AG01 and J05AH03 or N03AG02 and J05AH03 or N03AG03 and J05AH03 or N03AG04 and J05AH03 or N03AG05 and J05AH03 or N03AG06 and J05AH03 or N03AX03 and J05AH03 or N03AX07 and J05AH03 or N03AX09 and J05AH03 or N03AX10 and J05AH03 or N03AX11 and J05AH03 or N03AX12 and J05AH03 or N03AX13 and J05AH03 or N03AX14 and J05AH03 or N03AX15 and J05AH03 or N03AX16 and J05AH03 or N03AX17 and J05AH03 or N03AX18 and J05AH03 or N03AX19 and J05AH03 or N03AX21 and J05AH03 or N03AX22 and J05AH03 or N03AX23 and J05AH03 or N03AX24 and J05AH03 or N03AX30 and J05AH03 or N03AA01 and J05AP01 or N03AA02 and J05AP01 or N03AA03 and J05AP01 or N03AA04 and J05AP01 or N03AA30 and J05AP01 or N03AB01 and J05AP01 or N03AB02 and J05AP01 or N03AB03 and J05AP01 or N03AB04 and J05AP01 or N03AB05 and J05AP01 or N03AB52 and J05AP01 or N03AB54 and J05AP01 or N03AC01 and J05AP01 or N03AC02 and J05AP01 or N03AC03 and J05AP01 or N03AD01 and J05AP01 or N03AD02 and J05AP01 or N03AD03 and J05AP01 or N03AD51 and J05AP01 or N03AE01 and J05AP01 or N03AF01 and J05AP01 or N03AF02 and J05AP01 or N03AF03 and J05AP01 or N03AF04 and J05AP01 or N03AG01 and J05AP01 or N03AG02 and J05AP01 or N03AG03 and J05AP01 or N03AG04 and J05AP01 or N03AG05 and J05AP01 or N03AG06 and J05AP01 or N03AX03 and J05AP01 or N03AX07 and J05AP01 or N03AX09 and J05AP01 or N03AX10 and J05AP01 or N03AX11 and J05AP01 or N03AX12 and J05AP01 or N03AX13 and J05AP01 or N03AX14 and J05AP01 or N03AX15 and J05AP01 or N03AX16 and J05AP01 or N03AX17 and J05AP01 or N03AX18 and J05AP01 or N03AX19 and J05AP01 or N03AX21 and J05AP01 or N03AX22 and J05AP01 or N03AX23 and J05AP01 or N03AX24 and J05AP01 or N03AX30 and J05AP01 or N03AA01 and J05AP02 or N03AA02 and J05AP02 or N03AA03 and J05AP02 or N03AA04 and J05AP02 or N03AA30 and J05AP02 or N03AB01 and J05AP02 or N03AB02 and J05AP02 or N03AB03 and J05AP02 or N03AB04 and J05AP02 or N03AB05 and J05AP02 or N03AB52 and J05AP02 or N03AB54 and J05AP02 or N03AC01 and J05AP02 or N03AC02 and J05AP02 or N03AC03 and J05AP02 or N03AD01 and J05AP02 or N03AD02 and J05AP02 or N03AD03 and J05AP02 or N03AD51 and J05AP02 or N03AE01 and J05AP02 or N03AF01 and J05AP02 or N03AF02 and J05AP02 or N03AF03 and J05AP02 or N03AF04 and J05AP02 or N03AG01 and J05AP02 or N03AG02 and J05AP02 or N03AG03 and J05AP02 or N03AG04 and J05AP02 or N03AG05 and J05AP02 or N03AG06 and J05AP02 or N03AX03 and J05AP02 or N03AX07 and J05AP02 or N03AX09 and J05AP02 or N03AX10 and J05AP02 or N03AX11 and J05AP02 or N03AX12 and J05AP02 or N03AX13 and J05AP02 or N03AX14 and J05AP02 or N03AX15 and J05AP02 or N03AX16 and J05AP02 or N03AX17 and J05AP02 or N03AX18 and J05AP02 or N03AX19 and J05AP02 or N03AX21 and J05AP02 or N03AX22 and J05AP02 or N03AX23 and J05AP02 or N03AX24 and J05AP02 or N03AX30 and J05AP02 or N03AA01 and J05AP03 or N03AA02 and J05AP03 or N03AA03 and J05AP03 or N03AA04 and J05AP03 or N03AA30 and J05AP03 or N03AB01 and J05AP03 or N03AB02 and J05AP03 or N03AB03 and J05AP03 or N03AB04 and J05AP03 or N03AB05 and J05AP03 or N03AB52 and J05AP03 or N03AB54 and J05AP03 or N03AC01 and J05AP03 or N03AC02 and J05AP03 or N03AC03 and J05AP03 or N03AD01 and J05AP03 or N03AD02 and J05AP03 or N03AD03 and J05AP03 or N03AD51 and J05AP03 or N03AE01 and J05AP03 or N03AF01 and J05AP03 or N03AF02 and J05AP03 or N03AF03 and J05AP03 or N03AF04 and J05AP03 or N03AG01 and J05AP03 or N03AG02 and J05AP03 or N03AG03 and J05AP03 or N03AG04 and J05AP03 or N03AG05 and J05AP03 or N03AG06 and J05AP03 or N03AX03 and J05AP03 or N03AX07 and J05AP03 or N03AX09 and J05AP03 or N03AX10 and J05AP03 or N03AX11 and J05AP03 or N03AX12 and J05AP03 or N03AX13 and J05AP03 or N03AX14 and J05AP03 or N03AX15 and J05AP03 or N03AX16 and J05AP03 or N03AX17 and J05AP03 or N03AX18 and J05AP03 or N03AX19 and J05AP03 or N03AX21 and J05AP03 or N03AX22 and J05AP03 or N03AX23 and J05AP03 or N03AX24 and J05AP03 or N03AX30 and J05AP03 or N03AA01 and J05AP04 or N03AA02 and J05AP04 or N03AA03 and J05AP04 or N03AA04 and J05AP04 or N03AA30 and J05AP04 or N03AB01 and J05AP04 or N03AB02 and J05AP04 or N03AB03 and J05AP04 or N03AB04 and J05AP04 or N03AB05 and J05AP04 or N03AB52 and J05AP04 or N03AB54 and J05AP04 or N03AC01 and J05AP04 or N03AC02 and J05AP04 or N03AC03 and J05AP04 or N03AD01 and J05AP04 or N03AD02 and J05AP04 or N03AD03 and J05AP04 or N03AD51 and J05AP04 or N03AE01 and J05AP04 or N03AF01 and J05AP04 or N03AF02 and J05AP04 or N03AF03 and J05AP04 or N03AF04 and J05AP04 or N03AG01 and J05AP04 or N03AG02 and J05AP04 or N03AG03 and J05AP04 or N03AG04 and J05AP04 or N03AG05 and J05AP04 or N03AG06 and J05AP04 or N03AX03 and J05AP04 or N03AX07 and J05AP04 or N03AX09 and J05AP04 or N03AX10 and J05AP04 or N03AX11 and J05AP04 or N03AX12 and J05AP04 or N03AX13 and J05AP04 or N03AX14 and J05AP04 or N03AX15 and J05AP04 or N03AX16 and J05AP04 or N03AX17 and J05AP04 or N03AX18 and J05AP04 or N03AX19 and J05AP04 or N03AX21 and J05AP04 or N03AX22 and J05AP04 or N03AX23 and J05AP04 or N03AX24 and J05AP04 or N03AX30 and J05AP04 or N03AA01 and J05AP05 or N03AA02 and J05AP05 or N03AA03 and J05AP05 or N03AA04 and J05AP05 or N03AA30 and J05AP05 or N03AB01 and J05AP05 or N03AB02 and J05AP05 or N03AB03 and J05AP05 or N03AB04 and J05AP05 or N03AB05 and J05AP05 or N03AB52 and J05AP05 or N03AB54 and J05AP05 or N03AC01 and J05AP05 or N03AC02 and J05AP05 or N03AC03 and J05AP05 or N03AD01 and J05AP05 or N03AD02 and J05AP05 or N03AD03 and J05AP05 or N03AD51 and J05AP05 or N03AE01 and J05AP05 or N03AF01 and J05AP05 or N03AF02 and J05AP05 or N03AF03 and J05AP05 or N03AF04 and J05AP05 or N03AG01 and J05AP05 or N03AG02 and J05AP05 or N03AG03 and J05AP05 or N03AG04 and J05AP05 or N03AG05 and J05AP05 or N03AG06 and J05AP05 or N03AX03 and J05AP05 or N03AX07 and J05AP05 or N03AX09 and J05AP05 or N03AX10 and J05AP05 or N03AX11 and J05AP05 or N03AX12 and J05AP05 or N03AX13 and J05AP05 or N03AX14 and J05AP05 or N03AX15 and J05AP05 or N03AX16 and J05AP05 or N03AX17 and J05AP05 or N03AX18 and J05AP05 or N03AX19 and J05AP05 or N03AX21 and J05AP05 or N03AX22 and J05AP05 or N03AX23 and J05AP05 or N03AX24 and J05AP05 or N03AX30 and J05AP05 or N03AA01 and J05AP06 or N03AA02 and J05AP06 or N03AA03 and J05AP06 or N03AA04 and J05AP06 or N03AA30 and J05AP06 or N03AB01 and J05AP06 or N03AB02 and J05AP06 or N03AB03 and J05AP06 or N03AB04 and J05AP06 or N03AB05 and J05AP06 or N03AB52 and J05AP06 or N03AB54 and J05AP06 or N03AC01 and J05AP06 or N03AC02 and J05AP06 or N03AC03 and J05AP06 or N03AD01 and J05AP06 or N03AD02 and J05AP06 or N03AD03 and J05AP06 or N03AD51 and J05AP06 or N03AE01 and J05AP06 or N03AF01 and J05AP06 or N03AF02 and J05AP06 or N03AF03 and J05AP06 or N03AF04 and J05AP06 or N03AG01 and J05AP06 or N03AG02 and J05AP06 or N03AG03 and J05AP06 or N03AG04 and J05AP06 or N03AG05 and J05AP06 or N03AG06 and J05AP06 or N03AX03 and J05AP06 or N03AX07 and J05AP06 or N03AX09 and J05AP06 or N03AX10 and J05AP06 or N03AX11 and J05AP06 or N03AX12 and J05AP06 or N03AX13 and J05AP06 or N03AX14 and J05AP06 or N03AX15 and J05AP06 or N03AX16 and J05AP06 or N03AX17 and J05AP06 or N03AX18 and J05AP06 or N03AX19 and J05AP06 or N03AX21 and J05AP06 or N03AX22 and J05AP06 or N03AX23 and J05AP06 or N03AX24 and J05AP06 or N03AX30 and J05AP06 or N03AA01 and J05AP07 or N03AA02 and J05AP07 or N03AA03 and J05AP07 or N03AA04 and J05AP07 or N03AA30 and J05AP07 or N03AB01 and J05AP07 or N03AB02 and J05AP07 or N03AB03 and J05AP07 or N03AB04 and J05AP07 or N03AB05 and J05AP07 or N03AB52 and J05AP07 or N03AB54 and J05AP07 or N03AC01 and J05AP07 or N03AC02 and J05AP07 or N03AC03 and J05AP07 or N03AD01 and J05AP07 or N03AD02 and J05AP07 or N03AD03 and J05AP07 or N03AD51 and J05AP07 or N03AE01 and J05AP07 or N03AF01 and J05AP07 or N03AF02 and J05AP07 or N03AF03 and J05AP07 or N03AF04 and J05AP07 or N03AG01 and J05AP07 or N03AG02 and J05AP07 or N03AG03 and J05AP07 or N03AG04 and J05AP07 or N03AG05 and J05AP07 or N03AG06 and J05AP07 or N03AX03 and J05AP07 or N03AX07 and J05AP07 or N03AX09 and J05AP07 or N03AX10 and J05AP07 or N03AX11 and J05AP07 or N03AX12 and J05AP07 or N03AX13 and J05AP07 or N03AX14 and J05AP07 or N03AX15 and J05AP07 or N03AX16 and J05AP07 or N03AX17 and J05AP07 or N03AX18 and J05AP07 or N03AX19 and J05AP07 or N03AX21 and J05AP07 or N03AX22 and J05AP07 or N03AX23 and J05AP07 or N03AX24 and J05AP07 or N03AX30 and J05AP07 or N03AA01 and J05AP08 or N03AA02 and J05AP08 or N03AA03 and J05AP08 or N03AA04 and J05AP08 or N03AA30 and J05AP08 or N03AB01 and J05AP08 or N03AB02 and J05AP08 or N03AB03 and J05AP08 or N03AB04 and J05AP08 or N03AB05 and J05AP08 or N03AB52 and J05AP08 or N03AB54 and J05AP08 or N03AC01 and J05AP08 or N03AC02 and J05AP08 or N03AC03 and J05AP08 or N03AD01 and J05AP08 or N03AD02 and J05AP08 or N03AD03 and J05AP08 or N03AD51 and J05AP08 or N03AE01 and J05AP08 or N03AF01 and J05AP08 or N03AF02 and J05AP08 or N03AF03 and J05AP08 or N03AF04 and J05AP08 or N03AG01 and J05AP08 or N03AG02 and J05AP08 or N03AG03 and J05AP08 or N03AG04 and J05AP08 or N03AG05 and J05AP08 or N03AG06 and J05AP08 or N03AX03 and J05AP08 or N03AX07 and J05AP08 or N03AX09 and J05AP08 or N03AX10 and J05AP08 or N03AX11 and J05AP08 or N03AX12 and J05AP08 or N03AX13 and J05AP08 or N03AX14 and J05AP08 or N03AX15 and J05AP08 or N03AX16 and J05AP08 or N03AX17 and J05AP08 or N03AX18 and J05AP08 or N03AX19 and J05AP08 or N03AX21 and J05AP08 or N03AX22 and J05AP08 or N03AX23 and J05AP08 or N03AX24 and J05AP08 or N03AX30 and J05AP08 or N03AA01 and J05AP09 or N03AA02 and J05AP09 or N03AA03 and J05AP09 or N03AA04 and J05AP09 or N03AA30 and J05AP09 or N03AB01 and J05AP09 or N03AB02 and J05AP09 or N03AB03 and J05AP09 or N03AB04 and J05AP09 or N03AB05 and J05AP09 or N03AB52 and J05AP09 or N03AB54 and J05AP09 or N03AC01 and J05AP09 or N03AC02 and J05AP09 or N03AC03 and J05AP09 or N03AD01 and J05AP09 or N03AD02 and J05AP09 or N03AD03 and J05AP09 or N03AD51 and J05AP09 or N03AE01 and J05AP09 or N03AF01 and J05AP09 or N03AF02 and J05AP09 or N03AF03 and J05AP09 or N03AF04 and J05AP09 or N03AG01 and J05AP09 or N03AG02 and J05AP09 or N03AG03 and J05AP09 or N03AG04 and J05AP09 or N03AG05 and J05AP09 or N03AG06 and J05AP09 or N03AX03 and J05AP09 or N03AX07 and J05AP09 or N03AX09 and J05AP09 or N03AX10 and J05AP09 or N03AX11 and J05AP09 or N03AX12 and J05AP09 or N03AX13 and J05AP09 or N03AX14 and J05AP09 or N03AX15 and J05AP09 or N03AX16 and J05AP09 or N03AX17 and J05AP09 or N03AX18 and J05AP09 or N03AX19 and J05AP09 or N03AX21 and J05AP09 or N03AX22 and J05AP09 or N03AX23 and J05AP09 or N03AX24 and J05AP09 or N03AX30 and J05AP09 or N03AA01 and J05AP51 or N03AA02 and J05AP51 or N03AA03 and J05AP51 or N03AA04 and J05AP51 or N03AA30 and J05AP51 or N03AB01 and J05AP51 or N03AB02 and J05AP51 or N03AB03 and J05AP51 or N03AB04 and J05AP51 or N03AB05 and J05AP51 or N03AB52 and J05AP51 or N03AB54 and J05AP51 or N03AC01 and J05AP51 or N03AC02 and J05AP51 or N03AC03 and J05AP51 or N03AD01 and J05AP51 or N03AD02 and J05AP51 or N03AD03 and J05AP51 or N03AD51 and J05AP51 or N03AE01 and J05AP51 or N03AF01 and J05AP51 or N03AF02 and J05AP51 or N03AF03 and J05AP51 or N03AF04 and J05AP51 or N03AG01 and J05AP51 or N03AG02 and J05AP51 or N03AG03 and J05AP51 or N03AG04 and J05AP51 or N03AG05 and J05AP51 or N03AG06 and J05AP51 or N03AX03 and J05AP51 or N03AX07 and J05AP51 or N03AX09 and J05AP51 or N03AX10 and J05AP51 or N03AX11 and J05AP51 or N03AX12 and J05AP51 or N03AX13 and J05AP51 or N03AX14 and J05AP51 or N03AX15 and J05AP51 or N03AX16 and J05AP51 or N03AX17 and J05AP51 or N03AX18 and J05AP51 or N03AX19 and J05AP51 or N03AX21 and J05AP51 or N03AX22 and J05AP51 or N03AX23 and J05AP51 or N03AX24 and J05AP51 or N03AX30 and J05AP51 or N03AA01 and J05AP52 or N03AA02 and J05AP52 or N03AA03 and J05AP52 or N03AA04 and J05AP52 or N03AA30 and J05AP52 or N03AB01 and J05AP52 or N03AB02 and J05AP52 or N03AB03 and J05AP52 or N03AB04 and J05AP52 or N03AB05 and J05AP52 or N03AB52 and J05AP52 or N03AB54 and J05AP52 or N03AC01 and J05AP52 or N03AC02 and J05AP52 or N03AC03 and J05AP52 or N03AD01 and J05AP52 or N03AD02 and J05AP52 or N03AD03 and J05AP52 or N03AD51 and J05AP52 or N03AE01 and J05AP52 or N03AF01 and J05AP52 or N03AF02 and J05AP52 or N03AF03 and J05AP52 or N03AF04 and J05AP52 or N03AG01 and J05AP52 or N03AG02 and J05AP52 or N03AG03 and J05AP52 or N03AG04 and J05AP52 or N03AG05 and J05AP52 or N03AG06 and J05AP52 or N03AX03 and J05AP52 or N03AX07 and J05AP52 or N03AX09 and J05AP52 or N03AX10 and J05AP52 or N03AX11 and J05AP52 or N03AX12 and J05AP52 or N03AX13 and J05AP52 or N03AX14 and J05AP52 or N03AX15 and J05AP52 or N03AX16 and J05AP52 or N03AX17 and J05AP52 or N03AX18 and J05AP52 or N03AX19 and J05AP52 or N03AX21 and J05AP52 or N03AX22 and J05AP52 or N03AX23 and J05AP52 or N03AX24 and J05AP52 or N03AX30 and J05AP52 or N03AA01 and J05AP53 or N03AA02 and J05AP53 or N03AA03 and J05AP53 or N03AA04 and J05AP53 or N03AA30 and J05AP53 or N03AB01 and J05AP53 or N03AB02 and J05AP53 or N03AB03 and J05AP53 or N03AB04 and J05AP53 or N03AB05 and J05AP53 or N03AB52 and J05AP53 or N03AB54 and J05AP53 or N03AC01 and J05AP53 or N03AC02 and J05AP53 or N03AC03 and J05AP53 or N03AD01 and J05AP53 or N03AD02 and J05AP53 or N03AD03 and J05AP53 or N03AD51 and J05AP53 or N03AE01 and J05AP53 or N03AF01 and J05AP53 or N03AF02 and J05AP53 or N03AF03 and J05AP53 or N03AF04 and J05AP53 or N03AG01 and J05AP53 or N03AG02 and J05AP53 or N03AG03 and J05AP53 or N03AG04 and J05AP53 or N03AG05 and J05AP53 or N03AG06 and J05AP53 or N03AX03 and J05AP53 or N03AX07 and J05AP53 or N03AX09 and J05AP53 or N03AX10 and J05AP53 or N03AX11 and J05AP53 or N03AX12 and J05AP53 or N03AX13 and J05AP53 or N03AX14 and J05AP53 or N03AX15 and J05AP53 or N03AX16 and J05AP53 or N03AX17 and J05AP53 or N03AX18 and J05AP53 or N03AX19 and J05AP53 or N03AX21 and J05AP53 or N03AX22 and J05AP53 or N03AX23 and J05AP53 or N03AX24 and J05AP53 or N03AX30 and J05AP53 or N03AA01 and J05AP54 or N03AA02 and J05AP54 or N03AA03 and J05AP54 or N03AA04 and J05AP54 or N03AA30 and J05AP54 or N03AB01 and J05AP54 or N03AB02 and J05AP54 or N03AB03 and J05AP54 or N03AB04 and J05AP54 or N03AB05 and J05AP54 or N03AB52 and J05AP54 or N03AB54 and J05AP54 or N03AC01 and J05AP54 or N03AC02 and J05AP54 or N03AC03 and J05AP54 or N03AD01 and J05AP54 or N03AD02 and J05AP54 or N03AD03 and J05AP54 or N03AD51 and J05AP54 or N03AE01 and J05AP54 or N03AF01 and J05AP54 or N03AF02 and J05AP54 or N03AF03 and J05AP54 or N03AF04 and J05AP54 or N03AG01 and J05AP54 or N03AG02 and J05AP54 or N03AG03 and J05AP54 or N03AG04 and J05AP54 or N03AG05 and J05AP54 or N03AG06 and J05AP54 or N03AX03 and J05AP54 or N03AX07 and J05AP54 or N03AX09 and J05AP54 or N03AX10 and J05AP54 or N03AX11 and J05AP54 or N03AX12 and J05AP54 or N03AX13 and J05AP54 or N03AX14 and J05AP54 or N03AX15 and J05AP54 or N03AX16 and J05AP54 or N03AX17 and J05AP54 or N03AX18 and J05AP54 or N03AX19 and J05AP54 or N03AX21 and J05AP54 or N03AX22 and J05AP54 or N03AX23 and J05AP54 or N03AX24 and J05AP54 or N03AX30 and J05AP54 or N03AA01 and J05AP55 or N03AA02 and J05AP55 or N03AA03 and J05AP55 or N03AA04 and J05AP55 or N03AA30 and J05AP55 or N03AB01 and J05AP55 or N03AB02 and J05AP55 or N03AB03 and J05AP55 or N03AB04 and J05AP55 or N03AB05 and J05AP55 or N03AB52 and J05AP55 or N03AB54 and J05AP55 or N03AC01 and J05AP55 or N03AC02 and J05AP55 or N03AC03 and J05AP55 or N03AD01 and J05AP55 or N03AD02 and J05AP55 or N03AD03 and J05AP55 or N03AD51 and J05AP55 or N03AE01 and J05AP55 or N03AF01 and J05AP55 or N03AF02 and J05AP55 or N03AF03 and J05AP55 or N03AF04 and J05AP55 or N03AG01 and J05AP55 or N03AG02 and J05AP55 or N03AG03 and J05AP55 or N03AG04 and J05AP55 or N03AG05 and J05AP55 or N03AG06 and J05AP55 or N03AX03 and J05AP55 or N03AX07 and J05AP55 or N03AX09 and J05AP55 or N03AX10 and J05AP55 or N03AX11 and J05AP55 or N03AX12 and J05AP55 or N03AX13 and J05AP55 or N03AX14 and J05AP55 or N03AX15 and J05AP55 or N03AX16 and J05AP55 or N03AX17 and J05AP55 or N03AX18 and J05AP55 or N03AX19 and J05AP55 or N03AX21 and J05AP55 or N03AX22 and J05AP55 or N03AX23 and J05AP55 or N03AX24 and J05AP55 or N03AX30 and J05AP55 or N03AA01 and J05AP56 or N03AA02 and J05AP56 or N03AA03 and J05AP56 or N03AA04 and J05AP56 or N03AA30 and J05AP56 or N03AB01 and J05AP56 or N03AB02 and J05AP56 or N03AB03 and J05AP56 or N03AB04 and J05AP56 or N03AB05 and J05AP56 or N03AB52 and J05AP56 or N03AB54 and J05AP56 or N03AC01 and J05AP56 or N03AC02 and J05AP56 or N03AC03 and J05AP56 or N03AD01 and J05AP56 or N03AD02 and J05AP56 or N03AD03 and J05AP56 or N03AD51 and J05AP56 or N03AE01 and J05AP56 or N03AF01 and J05AP56 or N03AF02 and J05AP56 or N03AF03 and J05AP56 or N03AF04 and J05AP56 or N03AG01 and J05AP56 or N03AG02 and J05AP56 or N03AG03 and J05AP56 or N03AG04 and J05AP56 or N03AG05 and J05AP56 or N03AG06 and J05AP56 or N03AX03 and J05AP56 or N03AX07 and J05AP56 or N03AX09 and J05AP56 or N03AX10 and J05AP56 or N03AX11 and J05AP56 or N03AX12 and J05AP56 or N03AX13 and J05AP56 or N03AX14 and J05AP56 or N03AX15 and J05AP56 or N03AX16 and J05AP56 or N03AX17 and J05AP56 or N03AX18 and J05AP56 or N03AX19 and J05AP56 or N03AX21 and J05AP56 or N03AX22 and J05AP56 or N03AX23 and J05AP56 or N03AX24 and J05AP56 or N03AX30 and J05AP56 or N03AA01 and J05AP57 or N03AA02 and J05AP57 or N03AA03 and J05AP57 or N03AA04 and J05AP57 or N03AA30 and J05AP57 or N03AB01 and J05AP57 or N03AB02 and J05AP57 or N03AB03 and J05AP57 or N03AB04 and J05AP57 or N03AB05 and J05AP57 or N03AB52 and J05AP57 or N03AB54 and J05AP57 or N03AC01 and J05AP57 or N03AC02 and J05AP57 or N03AC03 and J05AP57 or N03AD01 and J05AP57 or N03AD02 and J05AP57 or N03AD03 and J05AP57 or N03AD51 and J05AP57 or N03AE01 and J05AP57 or N03AF01 and J05AP57 or N03AF02 and J05AP57 or N03AF03 and J05AP57 or N03AF04 and J05AP57 or N03AG01 and J05AP57 or N03AG02 and J05AP57 or N03AG03 and J05AP57 or N03AG04 and J05AP57 or N03AG05 and J05AP57 or N03AG06 and J05AP57 or N03AX03 and J05AP57 or N03AX07 and J05AP57 or N03AX09 and J05AP57 or N03AX10 and J05AP57 or N03AX11 and J05AP57 or N03AX12 and J05AP57 or N03AX13 and J05AP57 or N03AX14 and J05AP57 or N03AX15 and J05AP57 or N03AX16 and J05AP57 or N03AX17 and J05AP57 or N03AX18 and J05AP57 or N03AX19 and J05AP57 or N03AX21 and J05AP57 or N03AX22 and J05AP57 or N03AX23 and J05AP57 or N03AX24 and J05AP57 or N03AX30 and J05AP57 or N03AA01 and J05AR01 or N03AA02 and J05AR01 or N03AA03 and J05AR01 or N03AA04 and J05AR01 or N03AA30 and J05AR01 or N03AB01 and J05AR01 or N03AB02 and J05AR01 or N03AB03 and J05AR01 or N03AB04 and J05AR01 or N03AB05 and J05AR01 or N03AB52 and J05AR01 or N03AB54 and J05AR01 or N03AC01 and J05AR01 or N03AC02 and J05AR01 or N03AC03 and J05AR01 or N03AD01 and J05AR01 or N03AD02 and J05AR01 or N03AD03 and J05AR01 or N03AD51 and J05AR01 or N03AE01 and J05AR01 or N03AF01 and J05AR01 or N03AF02 and J05AR01 or N03AF03 and J05AR01 or N03AF04 and J05AR01 or N03AG01 and J05AR01 or N03AG02 and J05AR01 or N03AG03 and J05AR01 or N03AG04 and J05AR01 or N03AG05 and J05AR01 or N03AG06 and J05AR01 or N03AX03 and J05AR01 or N03AX07 and J05AR01 or N03AX09 and J05AR01 or N03AX10 and J05AR01 or N03AX11 and J05AR01 or N03AX12 and J05AR01 or N03AX13 and J05AR01 or N03AX14 and J05AR01 or N03AX15 and J05AR01 or N03AX16 and J05AR01 or N03AX17 and J05AR01 or N03AX18 and J05AR01 or N03AX19 and J05AR01 or N03AX21 and J05AR01 or N03AX22 and J05AR01 or N03AX23 and J05AR01 or N03AX24 and J05AR01 or N03AX30 and J05AR01 or N03AA01 and J05AR02 or N03AA02 and J05AR02 or N03AA03 and J05AR02 or N03AA04 and J05AR02 or N03AA30 and J05AR02 or N03AB01 and J05AR02 or N03AB02 and J05AR02 or N03AB03 and J05AR02 or N03AB04 and J05AR02 or N03AB05 and J05AR02 or N03AB52 and J05AR02 or N03AB54 and J05AR02 or N03AC01 and J05AR02 or N03AC02 and J05AR02 or N03AC03 and J05AR02 or N03AD01 and J05AR02 or N03AD02 and J05AR02 or N03AD03 and J05AR02 or N03AD51 and J05AR02 or N03AE01 and J05AR02 or N03AF01 and J05AR02 or N03AF02 and J05AR02 or N03AF03 and J05AR02 or N03AF04 and J05AR02 or N03AG01 and J05AR02 or N03AG02 and J05AR02 or N03AG03 and J05AR02 or N03AG04 and J05AR02 or N03AG05 and J05AR02 or N03AG06 and J05AR02 or N03AX03 and J05AR02 or N03AX07 and J05AR02 or N03AX09 and J05AR02 or N03AX10 and J05AR02 or N03AX11 and J05AR02 or N03AX12 and J05AR02 or N03AX13 and J05AR02 or N03AX14 and J05AR02 or N03AX15 and J05AR02 or N03AX16 and J05AR02 or N03AX17 and J05AR02 or N03AX18 and J05AR02 or N03AX19 and J05AR02 or N03AX21 and J05AR02 or N03AX22 and J05AR02 or N03AX23 and J05AR02 or N03AX24 and J05AR02 or N03AX30 and J05AR02 or
N03AA01 and J05AR03 or N03AA02 and J05AR03 or N03AA03 and J05AR03 or N03AA04 and J05AR03 or N03AA30 and J05AR03 or N03AB01 and J05AR03 or N03AB02 and J05AR03 or N03AB03 and J05AR03 or N03AB04 and J05AR03 or N03AB05 and J05AR03 or N03AB52 and J05AR03 or N03AB54 and J05AR03 or N03AC01 and J05AR03 or N03AC02 and J05AR03 or N03AC03 and J05AR03 or N03AD01 and J05AR03 or N03AD02 and J05AR03 or N03AD03 and J05AR03 or N03AD51 and J05AR03 or N03AE01 and J05AR03 or N03AF01 and J05AR03 or N03AF02 and J05AR03 or N03AF03 and J05AR03 or N03AF04 and J05AR03 or N03AG01 and J05AR03 or N03AG02 and J05AR03 or N03AG03 and J05AR03 or N03AG04 and J05AR03 or N03AG05 and J05AR03 or N03AG06 and J05AR03 or N03AX03 and J05AR03 or N03AX07 and J05AR03 or N03AX09 and J05AR03 or N03AX10 and J05AR03 or N03AX11 and J05AR03 or N03AX12 and J05AR03 or N03AX13 and J05AR03 or N03AX14 and J05AR03 or N03AX15 and J05AR03 or N03AX16 and J05AR03 or N03AX17 and J05AR03 or N03AX18 and J05AR03 or N03AX19 and J05AR03 or N03AX21 and J05AR03 or N03AX22 and J05AR03 or N03AX23 and J05AR03 or N03AX24 and J05AR03 or N03AX30 and J05AR03 or N03AA01 and J05AR04 or N03AA02 and J05AR04 or N03AA03 and J05AR04 or N03AA04 and J05AR04 or N03AA30 and J05AR04 or N03AB01 and J05AR04 or N03AB02 and J05AR04 or N03AB03 and J05AR04 or N03AB04 and J05AR04 or N03AB05 and J05AR04 or N03AB52 and J05AR04 or N03AB54 and J05AR04 or N03AC01 and J05AR04 or N03AC02 and J05AR04 or N03AC03 and J05AR04 or N03AD01 and J05AR04 or N03AD02 and J05AR04 or N03AD03 and J05AR04 or N03AD51 and J05AR04 or N03AE01 and J05AR04 or N03AF01 and J05AR04 or N03AF02 and J05AR04 or N03AF03 and J05AR04 or N03AF04 and J05AR04 or N03AG01 and J05AR04 or N03AG02 and J05AR04 or N03AG03 and J05AR04 or N03AG04 and J05AR04 or N03AG05 and J05AR04 or N03AG06 and J05AR04 or N03AX03 and J05AR04 or N03AX07 and J05AR04 or N03AX09 and J05AR04 or N03AX10 and J05AR04 or N03AX11 and J05AR04 or N03AX12 and J05AR04 or N03AX13 and J05AR04 or N03AX14 and J05AR04 or N03AX15 and J05AR04 or N03AX16 and J05AR04 or N03AX17 and J05AR04 or N03AX18 and J05AR04 or N03AX19 and J05AR04 or N03AX21 and J05AR04 or N03AX22 and J05AR04 or N03AX23 and J05AR04 or N03AX24 and J05AR04 or N03AX30 and J05AR04 or N03AA01 and J05AR05 or N03AA02 and J05AR05 or N03AA03 and J05AR05 or N03AA04 and J05AR05 or N03AA30 and J05AR05 or N03AB01 and J05AR05 or N03AB02 and J05AR05 or N03AB03 and J05AR05 or N03AB04 and J05AR05 or N03AB05 and J05AR05 or N03AB52 and J05AR05 or N03AB54 and J05AR05 or N03AC01 and J05AR05 or N03AC02 and J05AR05 or N03AC03 and J05AR05 or N03AD01 and J05AR05 or N03AD02 and J05AR05 or N03AD03 and J05AR05 or N03AD51 and J05AR05 or N03AE01 and J05AR05 or N03AF01 and J05AR05 or N03AF02 and J05AR05 or N03AF03 and J05AR05 or N03AF04 and J05AR05 or N03AG01 and J05AR05 or N03AG02 and J05AR05 or N03AG03 and J05AR05 or N03AG04 and J05AR05 or N03AG05 and J05AR05 or N03AG06 and J05AR05 or N03AX03 and J05AR05 or N03AX07 and J05AR05 or N03AX09 and J05AR05 or N03AX10 and J05AR05 or N03AX11 and J05AR05 or N03AX12 and J05AR05 or N03AX13 and J05AR05 or N03AX14 and J05AR05 or N03AX15 and J05AR05 or N03AX16 and J05AR05 or N03AX17 and J05AR05 or N03AX18 and J05AR05 or N03AX19 and J05AR05 or N03AX21 and J05AR05 or N03AX22 and J05AR05 or N03AX23 and J05AR05 or N03AX24 and J05AR05 or N03AX30 and J05AR05 or N03AA01 and J05AR06 or N03AA02 and J05AR06 or N03AA03 and J05AR06 or N03AA04 and J05AR06 or N03AA30 and J05AR06 or N03AB01 and J05AR06 or N03AB02 and J05AR06 or N03AB03 and J05AR06 or N03AB04 and J05AR06 or N03AB05 and J05AR06 or N03AB52 and J05AR06 or N03AB54 and J05AR06 or N03AC01 and J05AR06 or N03AC02 and J05AR06 or N03AC03 and J05AR06 or N03AD01 and J05AR06 or N03AD02 and J05AR06 or N03AD03 and J05AR06 or N03AD51 and J05AR06 or N03AE01 and J05AR06 or N03AF01 and J05AR06 or N03AF02 and J05AR06 or N03AF03 and J05AR06 or N03AF04 and J05AR06 or N03AG01 and J05AR06 or N03AG02 and J05AR06 or N03AG03 and J05AR06 or N03AG04 and J05AR06 or N03AG05 and J05AR06 or N03AG06 and J05AR06 or N03AX03 and J05AR06 or N03AX07 and J05AR06 or N03AX09 and J05AR06 or N03AX10 and J05AR06 or N03AX11 and J05AR06 or N03AX12 and J05AR06 or N03AX13 and J05AR06 or N03AX14 and J05AR06 or N03AX15 and J05AR06 or N03AX16 and J05AR06 or N03AX17 and J05AR06 or N03AX18 and J05AR06 or N03AX19 and J05AR06 or N03AX21 and J05AR06 or N03AX22 and J05AR06 or N03AX23 and J05AR06 or N03AX24 and J05AR06 or N03AX30 and J05AR06 or N03AA01 and J05AR07 or N03AA02 and J05AR07 or N03AA03 and J05AR07 or N03AA04 and J05AR07 or N03AA30 and J05AR07 or N03AB01 and J05AR07 or N03AB02 and J05AR07 or N03AB03 and J05AR07 or N03AB04 and J05AR07 or N03AB05 and J05AR07 or N03AB52 and J05AR07 or N03AB54 and J05AR07 or N03AC01 and J05AR07 or N03AC02 and J05AR07 or N03AC03 and J05AR07 or N03AD01 and J05AR07 or N03AD02 and J05AR07 or N03AD03 and J05AR07 or N03AD51 and J05AR07 or N03AE01 and J05AR07 or N03AF01 and J05AR07 or N03AF02 and J05AR07 or N03AF03 and J05AR07 or N03AF04 and J05AR07 or N03AG01 and J05AR07 or N03AG02 and J05AR07 or N03AG03 and J05AR07 or N03AG04 and J05AR07 or N03AG05 and J05AR07 or N03AG06 and J05AR07 or N03AX03 and J05AR07 or N03AX07 and J05AR07 or N03AX09 and J05AR07 or N03AX10 and J05AR07 or N03AX11 and J05AR07 or N03AX12 and J05AR07 or N03AX13 and J05AR07 or N03AX14 and J05AR07 or N03AX15 and J05AR07 or N03AX16 and J05AR07 or N03AX17 and J05AR07 or N03AX18 and J05AR07 or N03AX19 and J05AR07 or N03AX21 and J05AR07 or N03AX22 and J05AR07 or N03AX23 and J05AR07 or N03AX24 and J05AR07 or N03AX30 and J05AR07 or N03AA01 and J05AR08 or N03AA02 and J05AR08 or N03AA03 and J05AR08 or N03AA04 and J05AR08 or N03AA30 and J05AR08 or N03AB01 and J05AR08 or N03AB02 and J05AR08 or N03AB03 and J05AR08 or N03AB04 and J05AR08 or N03AB05 and J05AR08 or N03AB52 and J05AR08 or N03AB54 and J05AR08 or N03AC01 and J05AR08 or N03AC02 and J05AR08 or N03AC03 and J05AR08 or N03AD01 and J05AR08 or N03AD02 and J05AR08 or N03AD03 and J05AR08 or N03AD51 and J05AR08 or N03AE01 and J05AR08 or N03AF01 and J05AR08 or N03AF02 and J05AR08 or N03AF03 and J05AR08 or N03AF04 and J05AR08 or N03AG01 and J05AR08 or N03AG02 and J05AR08 or N03AG03 and J05AR08 or N03AG04 and J05AR08 or N03AG05 and J05AR08 or N03AG06 and J05AR08 or N03AX03 and J05AR08 or N03AX07 and J05AR08 or N03AX09 and J05AR08 or N03AX10 and J05AR08 or N03AX11 and J05AR08 or N03AX12 and J05AR08 or N03AX13 and J05AR08 or N03AX14 and J05AR08 or N03AX15 and J05AR08 or N03AX16 and J05AR08 or N03AX17 and J05AR08 or N03AX18 and J05AR08 or N03AX19 and J05AR08 or N03AX21 and J05AR08 or N03AX22 and J05AR08 or N03AX23 and J05AR08 or N03AX24 and J05AR08 or N03AX30 and J05AR08 or N03AA01 and J05AR09 or N03AA02 and J05AR09 or N03AA03 and J05AR09 or N03AA04 and J05AR09 or N03AA30 and J05AR09 or N03AB01 and J05AR09 or N03AB02 and J05AR09 or N03AB03 and J05AR09 or N03AB04 and J05AR09 or N03AB05 and J05AR09 or N03AB52 and J05AR09 or N03AB54 and J05AR09 or N03AC01 and J05AR09 or N03AC02 and J05AR09 or N03AC03 and J05AR09 or N03AD01 and J05AR09 or N03AD02 and J05AR09 or N03AD03 and J05AR09 or N03AD51 and J05AR09 or N03AE01 and J05AR09 or N03AF01 and J05AR09 or N03AF02 and J05AR09 or N03AF03 and J05AR09 or N03AF04 and J05AR09 or N03AG01 and J05AR09 or N03AG02 and J05AR09 or N03AG03 and J05AR09 or N03AG04 and J05AR09 or N03AG05 and J05AR09 or N03AG06 and J05AR09 or N03AX03 and J05AR09 or N03AX07 and J05AR09 or N03AX09 and J05AR09 or N03AX10 and J05AR09 or N03AX11 and J05AR09 or N03AX12 and J05AR09 or N03AX13 and J05AR09 or N03AX14 and J05AR09 or N03AX15 and J05AR09 or N03AX16 and J05AR09 or N03AX17 and J05AR09 or N03AX18 and J05AR09 or N03AX19 and J05AR09 or N03AX21 and J05AR09 or N03AX22 and J05AR09 or N03AX23 and J05AR09 or N03AX24 and J05AR09 or N03AX30 and J05AR09 or N03AA01 and J05AR10 or N03AA02 and J05AR10 or N03AA03 and J05AR10 or N03AA04 and J05AR10 or N03AA30 and J05AR10 or N03AB01 and J05AR10 or N03AB02 and J05AR10 or N03AB03 and J05AR10 or N03AB04 and J05AR10 or N03AB05 and J05AR10 or N03AB52 and J05AR10 or N03AB54 and J05AR10 or N03AC01 and J05AR10 or N03AC02 and J05AR10 or N03AC03 and J05AR10 or N03AD01 and J05AR10 or N03AD02 and J05AR10 or N03AD03 and J05AR10 or N03AD51 and J05AR10 or N03AE01 and J05AR10 or N03AF01 and J05AR10 or N03AF02 and J05AR10 or N03AF03 and J05AR10 or N03AF04 and J05AR10 or N03AG01 and J05AR10 or N03AG02 and J05AR10 or N03AG03 and J05AR10 or N03AG04 and J05AR10 or N03AG05 and J05AR10 or N03AG06 and J05AR10 or N03AX03 and J05AR10 or N03AX07 and J05AR10 or N03AX09 and J05AR10 or N03AX10 and J05AR10 or N03AX11 and J05AR10 or N03AX12 and J05AR10 or N03AX13 and J05AR10 or N03AX14 and J05AR10 or N03AX15 and J05AR10 or N03AX16 and J05AR10 or N03AX17 and J05AR10 or N03AX18 and J05AR10 or N03AX19 and J05AR10 or N03AX21 and J05AR10 or N03AX22 and J05AR10 or N03AX23 and J05AR10 or N03AX24 and J05AR10 or N03AX30 and J05AR10 or N03AA01 and J05AR11 or N03AA02 and J05AR11 or N03AA03 and J05AR11 or N03AA04 and J05AR11 or N03AA30 and J05AR11 or N03AB01 and J05AR11 or N03AB02 and J05AR11 or N03AB03 and J05AR11 or N03AB04 and J05AR11 or N03AB05 and J05AR11 or N03AB52 and J05AR11 or N03AB54 and J05AR11 or N03AC01 and J05AR11 or N03AC02 and J05AR11 or N03AC03 and J05AR11 or N03AD01 and J05AR11 or N03AD02 and J05AR11 or N03AD03 and J05AR11 or N03AD51 and J05AR11 or N03AE01 and J05AR11 or N03AF01 and J05AR11 or N03AF02 and J05AR11 or N03AF03 and J05AR11 or N03AF04 and J05AR11 or N03AG01 and J05AR11 or N03AG02 and J05AR11 or N03AG03 and J05AR11 or N03AG04 and J05AR11 or N03AG05 and J05AR11 or N03AG06 and J05AR11 or N03AX03 and J05AR11 or N03AX07 and J05AR11 or N03AX09 and J05AR11 or N03AX10 and J05AR11 or N03AX11 and J05AR11 or N03AX12 and J05AR11 or N03AX13 and J05AR11 or N03AX14 and J05AR11 or N03AX15 and J05AR11 or N03AX16 and J05AR11 or N03AX17 and J05AR11 or N03AX18 and J05AR11 or N03AX19 and J05AR11 or N03AX21 and J05AR11 or N03AX22 and J05AR11 or N03AX23 and J05AR11 or N03AX24 and J05AR11 or N03AX30 and J05AR11 or N03AA01 and J05AR12 or N03AA02 and J05AR12 or N03AA03 and J05AR12 or N03AA04 and J05AR12 or N03AA30 and J05AR12 or N03AB01 and J05AR12 or N03AB02 and J05AR12 or N03AB03 and J05AR12 or N03AB04 and J05AR12 or N03AB05 and J05AR12 or N03AB52 and J05AR12 or N03AB54 and J05AR12 or N03AC01 and J05AR12 or N03AC02 and J05AR12 or N03AC03 and J05AR12 or N03AD01 and J05AR12 or N03AD02 and J05AR12 or N03AD03 and J05AR12 or N03AD51 and J05AR12 or N03AE01 and J05AR12 or N03AF01 and J05AR12 or N03AF02 and J05AR12 or N03AF03 and J05AR12 or N03AF04 and J05AR12 or N03AG01 and J05AR12 or N03AG02 and J05AR12 or N03AG03 and J05AR12 or N03AG04 and J05AR12 or N03AG05 and J05AR12 or N03AG06 and J05AR12 or N03AX03 and J05AR12 or N03AX07 and J05AR12 or N03AX09 and J05AR12 or N03AX10 and J05AR12 or N03AX11 and J05AR12 or N03AX12 and J05AR12 or N03AX13 and J05AR12 or N03AX14 and J05AR12 or N03AX15 and J05AR12 or N03AX16 and J05AR12 or N03AX17 and J05AR12 or N03AX18 and J05AR12 or N03AX19 and J05AR12 or N03AX21 and J05AR12 or N03AX22 and J05AR12 or N03AX23 and J05AR12 or N03AX24 and J05AR12 or N03AX30 and J05AR12 or N03AA01 and J05AR13 or N03AA02 and J05AR13 or N03AA03 and J05AR13 or N03AA04 and J05AR13 or N03AA30 and J05AR13 or N03AB01 and J05AR13 or N03AB02 and J05AR13 or N03AB03 and J05AR13 or N03AB04 and J05AR13 or N03AB05 and J05AR13 or N03AB52 and J05AR13 or N03AB54 and J05AR13 or N03AC01 and J05AR13 or N03AC02 and J05AR13 or N03AC03 and J05AR13 or N03AD01 and J05AR13 or N03AD02 and J05AR13 or N03AD03 and J05AR13 or N03AD51 and J05AR13 or N03AE01 and J05AR13 or N03AF01 and J05AR13 or N03AF02 and J05AR13 or N03AF03 and J05AR13 or N03AF04 and J05AR13 or N03AG01 and J05AR13 or N03AG02 and J05AR13 or N03AG03 and J05AR13 or N03AG04 and J05AR13 or N03AG05 and J05AR13 or N03AG06 and J05AR13 or N03AX03 and J05AR13 or N03AX07 and J05AR13 or N03AX09 and J05AR13 or N03AX10 and J05AR13 or N03AX11 and J05AR13 or N03AX12 and J05AR13 or N03AX13 and J05AR13 or N03AX14 and J05AR13 or N03AX15 and J05AR13 or N03AX16 and J05AR13 or N03AX17 and J05AR13 or N03AX18 and J05AR13 or N03AX19 and J05AR13 or N03AX21 and J05AR13 or N03AX22 and J05AR13 or N03AX23 and J05AR13 or N03AX24 and J05AR13 or N03AX30 and J05AR13 or N03AA01 and J05AR14 or N03AA02 and J05AR14 or N03AA03 and J05AR14 or N03AA04 and J05AR14 or N03AA30 and J05AR14 or N03AB01 and J05AR14 or N03AB02 and J05AR14 or N03AB03 and J05AR14 or N03AB04 and J05AR14 or N03AB05 and J05AR14 or N03AB52 and J05AR14 or N03AB54 and J05AR14 or N03AC01 and J05AR14 or N03AC02 and J05AR14 or N03AC03 and J05AR14 or N03AD01 and J05AR14 or N03AD02 and J05AR14 or N03AD03 and J05AR14 or N03AD51 and J05AR14 or N03AE01 and J05AR14 or N03AF01 and J05AR14 or N03AF02 and J05AR14 or N03AF03 and J05AR14 or N03AF04 and J05AR14 or N03AG01 and J05AR14 or N03AG02 and J05AR14 or N03AG03 and J05AR14 or N03AG04 and J05AR14 or N03AG05 and J05AR14 or N03AG06 and J05AR14 or N03AX03 and J05AR14 or N03AX07 and J05AR14 or N03AX09 and J05AR14 or N03AX10 and J05AR14 or N03AX11 and J05AR14 or N03AX12 and J05AR14 or N03AX13 and J05AR14 or N03AX14 and J05AR14 or N03AX15 and J05AR14 or N03AX16 and J05AR14 or N03AX17 and J05AR14 or N03AX18 and J05AR14 or N03AX19 and J05AR14 or N03AX21 and J05AR14 or N03AX22 and J05AR14 or N03AX23 and J05AR14 or N03AX24 and J05AR14 or N03AX30 and J05AR14 or N03AA01 and J05AR15 or N03AA02 and J05AR15 or N03AA03 and J05AR15 or N03AA04 and J05AR15 or N03AA30 and J05AR15 or N03AB01 and J05AR15 or N03AB02 and J05AR15 or N03AB03 and J05AR15 or N03AB04 and J05AR15 or N03AB05 and J05AR15 or N03AB52 and J05AR15 or N03AB54 and J05AR15 or N03AC01 and J05AR15 or N03AC02 and J05AR15 or N03AC03 and J05AR15 or N03AD01 and J05AR15 or N03AD02 and J05AR15 or N03AD03 and J05AR15 or N03AD51 and J05AR15 or N03AE01 and J05AR15 or N03AF01 and J05AR15 or N03AF02 and J05AR15 or N03AF03 and J05AR15 or N03AF04 and J05AR15 or N03AG01 and J05AR15 or N03AG02 and J05AR15 or N03AG03 and J05AR15 or N03AG04 and J05AR15 or N03AG05 and J05AR15 or N03AG06 and J05AR15 or N03AX03 and J05AR15 or N03AX07 and J05AR15 or N03AX09 and J05AR15 or N03AX10 and J05AR15 or N03AX11 and J05AR15 or N03AX12 and J05AR15 or N03AX13 and J05AR15 or N03AX14 and J05AR15 or N03AX15 and J05AR15 or N03AX16 and J05AR15 or N03AX17 and J05AR15 or N03AX18 and J05AR15 or N03AX19 and J05AR15 or N03AX21 and J05AR15 or N03AX22 and J05AR15 or N03AX23 and J05AR15 or N03AX24 and J05AR15 or N03AX30 and J05AR15 or N03AA01 and J05AR16 or N03AA02 and J05AR16 or N03AA03 and J05AR16 or N03AA04 and J05AR16 or N03AA30 and J05AR16 or N03AB01 and J05AR16 or N03AB02 and J05AR16 or N03AB03 and J05AR16 or N03AB04 and J05AR16 or N03AB05 and J05AR16 or N03AB52 and J05AR16 or N03AB54 and J05AR16 or N03AC01 and J05AR16 or N03AC02 and J05AR16 or N03AC03 and J05AR16 or N03AD01 and J05AR16 or N03AD02 and J05AR16 or N03AD03 and J05AR16 or N03AD51 and J05AR16 or N03AE01 and J05AR16 or N03AF01 and J05AR16 or N03AF02 and J05AR16 or N03AF03 and J05AR16 or N03AF04 and J05AR16 or N03AG01 and J05AR16 or N03AG02 and J05AR16 or N03AG03 and J05AR16 or N03AG04 and J05AR16 or N03AG05 and J05AR16 or N03AG06 and J05AR16 or N03AX03 and J05AR16 or N03AX07 and J05AR16 or N03AX09 and J05AR16 or N03AX10 and J05AR16 or N03AX11 and J05AR16 or N03AX12 and J05AR16 or N03AX13 and J05AR16 or N03AX14 and J05AR16 or N03AX15 and J05AR16 or N03AX16 and J05AR16 or N03AX17 and J05AR16 or N03AX18 and J05AR16 or N03AX19 and J05AR16 or N03AX21 and J05AR16 or N03AX22 and J05AR16 or N03AX23 and J05AR16 or N03AX24 and J05AR16 or N03AX30 and J05AR16 or N03AA01 and J05AR17 or N03AA02 and J05AR17 or N03AA03 and J05AR17 or N03AA04 and J05AR17 or N03AA30 and J05AR17 or N03AB01 and J05AR17 or N03AB02 and J05AR17 or N03AB03 and J05AR17 or N03AB04 and J05AR17 or N03AB05 and J05AR17 or N03AB52 and J05AR17 or N03AB54 and J05AR17 or N03AC01 and J05AR17 or N03AC02 and J05AR17 or N03AC03 and J05AR17 or N03AD01 and J05AR17 or N03AD02 and J05AR17 or N03AD03 and J05AR17 or N03AD51 and J05AR17 or N03AE01 and J05AR17 or N03AF01 and J05AR17 or N03AF02 and J05AR17 or N03AF03 and J05AR17 or N03AF04 and J05AR17 or N03AG01 and J05AR17 or N03AG02 and J05AR17 or N03AG03 and J05AR17 or N03AG04 and J05AR17 or N03AG05 and J05AR17 or N03AG06 and J05AR17 or N03AX03 and J05AR17 or N03AX07 and J05AR17 or N03AX09 and J05AR17 or N03AX10 and J05AR17 or N03AX11 and J05AR17 or N03AX12 and J05AR17 or N03AX13 and J05AR17 or N03AX14 and J05AR17 or N03AX15 and J05AR17 or N03AX16 and J05AR17 or N03AX17 and J05AR17 or N03AX18 and J05AR17 or N03AX19 and J05AR17 or N03AX21 and J05AR17 or N03AX22 and J05AR17 or N03AX23 and J05AR17 or N03AX24 and J05AR17 or N03AX30 and J05AR17 or
N03AA01 and J05AR18 or N03AA02 and J05AR18 or N03AA03 and J05AR18 or N03AA04 and J05AR18 or N03AA30 and J05AR18 or N03AB01 and J05AR18 or N03AB02 and J05AR18 or N03AB03 and J05AR18 or N03AB04 and J05AR18 or N03AB05 and J05AR18 or N03AB52 and J05AR18 or N03AB54 and J05AR18 or N03AC01 and J05AR18 or N03AC02 and J05AR18 or N03AC03 and J05AR18 or N03AD01 and J05AR18 or N03AD02 and J05AR18 or N03AD03 and J05AR18 or N03AD51 and J05AR18 or N03AE01 and J05AR18 or N03AF01 and J05AR18 or N03AF02 and J05AR18 or N03AF03 and J05AR18 or N03AF04 and J05AR18 or N03AG01 and J05AR18 or N03AG02 and J05AR18 or N03AG03 and J05AR18 or N03AG04 and J05AR18 or N03AG05 and J05AR18 or N03AG06 and J05AR18 or N03AX03 and J05AR18 or N03AX07 and J05AR18 or N03AX09 and J05AR18 or N03AX10 and J05AR18 or N03AX11 and J05AR18 or N03AX12 and J05AR18 or N03AX13 and J05AR18 or N03AX14 and J05AR18 or N03AX15 and J05AR18 or N03AX16 and J05AR18 or N03AX17 and J05AR18 or N03AX18 and J05AR18 or N03AX19 and J05AR18 or N03AX21 and J05AR18 or N03AX22 and J05AR18 or N03AX23 and J05AR18 or N03AX24 and J05AR18 or N03AX30 and J05AR18 or N03AA01 and J05AR19 or N03AA02 and J05AR19 or N03AA03 and J05AR19 or N03AA04 and J05AR19 or N03AA30 and J05AR19 or N03AB01 and J05AR19 or N03AB02 and J05AR19 or N03AB03 and J05AR19 or N03AB04 and J05AR19 or N03AB05 and J05AR19 or N03AB52 and J05AR19 or N03AB54 and J05AR19 or N03AC01 and J05AR19 or N03AC02 and J05AR19 or N03AC03 and J05AR19 or N03AD01 and J05AR19 or N03AD02 and J05AR19 or N03AD03 and J05AR19 or N03AD51 and J05AR19 or N03AE01 and J05AR19 or N03AF01 and J05AR19 or N03AF02 and J05AR19 or N03AF03 and J05AR19 or N03AF04 and J05AR19 or N03AG01 and J05AR19 or N03AG02 and J05AR19 or N03AG03 and J05AR19 or N03AG04 and J05AR19 or N03AG05 and J05AR19 or N03AG06 and J05AR19 or N03AX03 and J05AR19 or N03AX07 and J05AR19 or N03AX09 and J05AR19 or N03AX10 and J05AR19 or N03AX11 and J05AR19 or N03AX12 and J05AR19 or N03AX13 and J05AR19 or N03AX14 and J05AR19 or N03AX15 and J05AR19 or N03AX16 and J05AR19 or N03AX17 and J05AR19 or N03AX18 and J05AR19 or N03AX19 and J05AR19 or N03AX21 and J05AR19 or N03AX22 and J05AR19 or N03AX23 and J05AR19 or N03AX24 and J05AR19 or N03AX30 and J05AR19 or N03AA01 and J05AR20 or N03AA02 and J05AR20 or N03AA03 and J05AR20 or N03AA04 and J05AR20 or N03AA30 and J05AR20 or N03AB01 and J05AR20 or N03AB02 and J05AR20 or N03AB03 and J05AR20 or N03AB04 and J05AR20 or N03AB05 and J05AR20 or N03AB52 and J05AR20 or N03AB54 and J05AR20 or N03AC01 and J05AR20 or N03AC02 and J05AR20 or N03AC03 and J05AR20 or N03AD01 and J05AR20 or N03AD02 and J05AR20 or N03AD03 and J05AR20 or N03AD51 and J05AR20 or N03AE01 and J05AR20 or N03AF01 and J05AR20 or N03AF02 and J05AR20 or N03AF03 and J05AR20 or N03AF04 and J05AR20 or N03AG01 and J05AR20 or N03AG02 and J05AR20 or N03AG03 and J05AR20 or N03AG04 and J05AR20 or N03AG05 and J05AR20 or N03AG06 and J05AR20 or N03AX03 and J05AR20 or N03AX07 and J05AR20 or N03AX09 and J05AR20 or N03AX10 and J05AR20 or N03AX11 and J05AR20 or N03AX12 and J05AR20 or N03AX13 and J05AR20 or N03AX14 and J05AR20 or N03AX15 and J05AR20 or N03AX16 and J05AR20 or N03AX17 and J05AR20 or N03AX18 and J05AR20 or N03AX19 and J05AR20 or N03AX21 and J05AR20 or N03AX22 and J05AR20 or N03AX23 and J05AR20 or N03AX24 and J05AR20 or N03AX30 and J05AR20 or N03AA01 and J05AR21 or N03AA02 and J05AR21 or N03AA03 and J05AR21 or N03AA04 and J05AR21 or N03AA30 and J05AR21 or N03AB01 and J05AR21 or N03AB02 and J05AR21 or N03AB03 and J05AR21 or N03AB04 and J05AR21 or N03AB05 and J05AR21 or N03AB52 and J05AR21 or N03AB54 and J05AR21 or N03AC01 and J05AR21 or N03AC02 and J05AR21 or N03AC03 and J05AR21 or N03AD01 and J05AR21 or N03AD02 and J05AR21 or N03AD03 and J05AR21 or N03AD51 and J05AR21 or N03AE01 and J05AR21 or N03AF01 and J05AR21 or N03AF02 and J05AR21 or N03AF03 and J05AR21 or N03AF04 and J05AR21 or N03AG01 and J05AR21 or N03AG02 and J05AR21 or N03AG03 and J05AR21 or N03AG04 and J05AR21 or N03AG05 and J05AR21 or N03AG06 and J05AR21 or N03AX03 and J05AR21 or N03AX07 and J05AR21 or N03AX09 and J05AR21 or N03AX10 and J05AR21 or N03AX11 and J05AR21 or N03AX12 and J05AR21 or N03AX13 and J05AR21 or N03AX14 and J05AR21 or N03AX15 and J05AR21 or N03AX16 and J05AR21 or N03AX17 and J05AR21 or N03AX18 and J05AR21 or N03AX19 and J05AR21 or N03AX21 and J05AR21 or N03AX22 and J05AR21 or N03AX23 and J05AR21 or N03AX24 and J05AR21 or N03AX30 and J05AR21 or N03AA01 and J05AR22 or N03AA02 and J05AR22 or N03AA03 and J05AR22 or N03AA04 and J05AR22 or N03AA30 and J05AR22 or N03AB01 and J05AR22 or N03AB02 and J05AR22 or N03AB03 and J05AR22 or N03AB04 and J05AR22 or N03AB05 and J05AR22 or N03AB52 and J05AR22 or N03AB54 and J05AR22 or N03AC01 and J05AR22 or N03AC02 and J05AR22 or N03AC03 and J05AR22 or N03AD01 and J05AR22 or N03AD02 and J05AR22 or N03AD03 and J05AR22 or N03AD51 and J05AR22 or N03AE01 and J05AR22 or N03AF01 and J05AR22 or N03AF02 and J05AR22 or N03AF03 and J05AR22 or N03AF04 and J05AR22 or N03AG01 and J05AR22 or N03AG02 and J05AR22 or N03AG03 and J05AR22 or N03AG04 and J05AR22 or N03AG05 and J05AR22 or N03AG06 and J05AR22 or N03AX03 and J05AR22 or N03AX07 and J05AR22 or N03AX09 and J05AR22 or N03AX10 and J05AR22 or N03AX11 and J05AR22 or N03AX12 and J05AR22 or N03AX13 and J05AR22 or N03AX14 and J05AR22 or N03AX15 and J05AR22 or N03AX16 and J05AR22 or N03AX17 and J05AR22 or N03AX18 and J05AR22 or N03AX19 and J05AR22 or N03AX21 and J05AR22 or N03AX22 and J05AR22 or N03AX23 and J05AR22 or N03AX24 and J05AR22 or N03AX30 and J05AR22 or
N03AA01 and J05AR23 or N03AA02 and J05AR23 or N03AA03 and J05AR23 or N03AA04 and J05AR23 or N03AA30 and J05AR23 or N03AB01 and J05AR23 or N03AB02 and J05AR23 or N03AB03 and J05AR23 or N03AB04 and J05AR23 or N03AB05 and J05AR23 or N03AB52 and J05AR23 or N03AB54 and J05AR23 or N03AC01 and J05AR23 or N03AC02 and J05AR23 or N03AC03 and J05AR23 or N03AD01 and J05AR23 or N03AD02 and J05AR23 or N03AD03 and J05AR23 or N03AD51 and J05AR23 or N03AE01 and J05AR23 or N03AF01 and J05AR23 or N03AF02 and J05AR23 or N03AF03 and J05AR23 or N03AF04 and J05AR23 or N03AG01 and J05AR23 or N03AG02 and J05AR23 or N03AG03 and J05AR23 or N03AG04 and J05AR23 or N03AG05 and J05AR23 or N03AG06 and J05AR23 or N03AX03 and J05AR23 or N03AX07 and J05AR23 or N03AX09 and J05AR23 or N03AX10 and J05AR23 or N03AX11 and J05AR23 or N03AX12 and J05AR23 or N03AX13 and J05AR23 or N03AX14 and J05AR23 or N03AX15 and J05AR23 or N03AX16 and J05AR23 or N03AX17 and J05AR23 or N03AX18 and J05AR23 or N03AX19 and J05AR23 or N03AX21 and J05AR23 or N03AX22 and J05AR23 or N03AX23 and J05AR23 or N03AX24 and J05AR23 or N03AX30 and J05AR23 or N03AA01 and J05AR24 or N03AA02 and J05AR24 or N03AA03 and J05AR24 or N03AA04 and J05AR24 or N03AA30 and J05AR24 or N03AB01 and J05AR24 or N03AB02 and J05AR24 or N03AB03 and J05AR24 or N03AB04 and J05AR24 or N03AB05 and J05AR24 or N03AB52 and J05AR24 or N03AB54 and J05AR24 or N03AC01 and J05AR24 or N03AC02 and J05AR24 or N03AC03 and J05AR24 or N03AD01 and J05AR24 or N03AD02 and J05AR24 or N03AD03 and J05AR24 or N03AD51 and J05AR24 or N03AE01 and J05AR24 or N03AF01 and J05AR24 or N03AF02 and J05AR24 or N03AF03 and J05AR24 or N03AF04 and J05AR24 or N03AG01 and J05AR24 or N03AG02 and J05AR24 or N03AG03 and J05AR24 or N03AG04 and J05AR24 or N03AG05 and J05AR24 or N03AG06 and J05AR24 or N03AX03 and J05AR24 or N03AX07 and J05AR24 or N03AX09 and J05AR24 or N03AX10 and J05AR24 or N03AX11 and J05AR24 or N03AX12 and J05AR24 or N03AX13 and J05AR24 or N03AX14 and J05AR24 or N03AX15 and J05AR24 or N03AX16 and J05AR24 or N03AX17 and J05AR24 or N03AX18 and J05AR24 or N03AX19 and J05AR24 or N03AX21 and J05AR24 or N03AX22 and J05AR24 or N03AX23 and J05AR24 or N03AX24 and J05AR24 or N03AX30 and J05AR24 or N03AA01 and J05AX01 or N03AA02 and J05AX01 or N03AA03 and J05AX01 or N03AA04 and J05AX01 or N03AA30 and J05AX01 or N03AB01 and J05AX01 or N03AB02 and J05AX01 or N03AB03 and J05AX01 or N03AB04 and J05AX01 or N03AB05 and J05AX01 or N03AB52 and J05AX01 or N03AB54 and J05AX01 or N03AC01 and J05AX01 or N03AC02 and J05AX01 or N03AC03 and J05AX01 or N03AD01 and J05AX01 or N03AD02 and J05AX01 or N03AD03 and J05AX01 or N03AD51 and J05AX01 or N03AE01 and J05AX01 or N03AF01 and J05AX01 or N03AF02 and J05AX01 or N03AF03 and J05AX01 or N03AF04 and J05AX01 or N03AG01 and J05AX01 or N03AG02 and J05AX01 or N03AG03 and J05AX01 or N03AG04 and J05AX01 or N03AG05 and J05AX01 or N03AG06 and J05AX01 or N03AX03 and J05AX01 or N03AX07 and J05AX01 or N03AX09 and J05AX01 or N03AX10 and J05AX01 or N03AX11 and J05AX01 or N03AX12 and J05AX01 or N03AX13 and J05AX01 or N03AX14 and J05AX01 or N03AX15 and J05AX01 or N03AX16 and J05AX01 or N03AX17 and J05AX01 or N03AX18 and J05AX01 or N03AX19 and J05AX01 or N03AX21 and J05AX01 or N03AX22 and J05AX01 or N03AX23 and J05AX01 or N03AX24 and J05AX01 or N03AX30 and J05AX01 or N03AA01 and J05AX02 or N03AA02 and J05AX02 or N03AA03 and J05AX02 or N03AA04 and J05AX02 or N03AA30 and J05AX02 or N03AB01 and J05AX02 or N03AB02 and J05AX02 or N03AB03 and J05AX02 or N03AB04 and J05AX02 or N03AB05 and J05AX02 or N03AB52 and J05AX02 or N03AB54 and J05AX02 or N03AC01 and J05AX02 or N03AC02 and J05AX02 or N03AC03 and J05AX02 or N03AD01 and J05AX02 or N03AD02 and J05AX02 or N03AD03 and J05AX02 or N03AD51 and J05AX02 or N03AE01 and J05AX02 or N03AF01 and J05AX02 or N03AF02 and J05AX02 or N03AF03 and J05AX02 or N03AF04 and J05AX02 or N03AG01 and J05AX02 or N03AG02 and J05AX02 or N03AG03 and J05AX02 or N03AG04 and J05AX02 or N03AG05 and J05AX02 or N03AG06 and J05AX02 or N03AX03 and J05AX02 or N03AX07 and J05AX02 or N03AX09 and J05AX02 or N03AX10 and J05AX02 or N03AX11 and J05AX02 or N03AX12 and J05AX02 or N03AX13 and J05AX02 or N03AX14 and J05AX02 or N03AX15 and J05AX02 or N03AX16 and J05AX02 or N03AX17 and J05AX02 or N03AX18 and J05AX02 or N03AX19 and J05AX02 or N03AX21 and J05AX02 or N03AX22 and J05AX02 or N03AX23 and J05AX02 or N03AX24 and J05AX02 or N03AX30 and J05AX02 or N03AA01 and J05AX05 or N03AA02 and J05AX05 or N03AA03 and J05AX05 or N03AA04 and J05AX05 or N03AA30 and J05AX05 or N03AB01 and J05AX05 or N03AB02 and J05AX05 or N03AB03 and J05AX05 or N03AB04 and J05AX05 or N03AB05 and J05AX05 or N03AB52 and J05AX05 or N03AB54 and J05AX05 or N03AC01 and J05AX05 or N03AC02 and J05AX05 or N03AC03 and J05AX05 or N03AD01 and J05AX05 or N03AD02 and J05AX05 or N03AD03 and J05AX05 or N03AD51 and J05AX05 or N03AE01 and J05AX05 or N03AF01 and J05AX05 or N03AF02 and J05AX05 or N03AF03 and J05AX05 or N03AF04 and J05AX05 or N03AG01 and J05AX05 or N03AG02 and J05AX05 or N03AG03 and J05AX05 or N03AG04 and J05AX05 or N03AG05 and J05AX05 or N03AG06 and J05AX05 or N03AX03 and J05AX05 or N03AX07 and J05AX05 or N03AX09 and J05AX05 or N03AX10 and J05AX05 or N03AX11 and J05AX05 or N03AX12 and J05AX05 or N03AX13 and J05AX05 or N03AX14 and J05AX05 or N03AX15 and J05AX05 or N03AX16 and J05AX05 or N03AX17 and J05AX05 or N03AX18 and J05AX05 or N03AX19 and J05AX05 or N03AX21 and J05AX05 or N03AX22 and J05AX05 or N03AX23 and J05AX05 or N03AX24 and J05AX05 or N03AX30 and J05AX05 or N03AA01 and J05AX06 or N03AA02 and J05AX06 or N03AA03 and J05AX06 or N03AA04 and J05AX06 or N03AA30 and J05AX06 or N03AB01 and J05AX06 or N03AB02 and J05AX06 or N03AB03 and J05AX06 or N03AB04 and J05AX06 or N03AB05 and J05AX06 or N03AB52 and J05AX06 or N03AB54 and J05AX06 or N03AC01 and J05AX06 or N03AC02 and J05AX06 or N03AC03 and J05AX06 or N03AD01 and J05AX06 or N03AD02 and J05AX06 or N03AD03 and J05AX06 or N03AD51 and J05AX06 or N03AE01 and J05AX06 or N03AF01 and J05AX06 or N03AF02 and J05AX06 or N03AF03 and J05AX06 or N03AF04 and J05AX06 or N03AG01 and J05AX06 or N03AG02 and J05AX06 or N03AG03 and J05AX06 or N03AG04 and J05AX06 or N03AG05 and J05AX06 or N03AG06 and J05AX06 or N03AX03 and J05AX06 or N03AX07 and J05AX06 or N03AX09 and J05AX06 or N03AX10 and J05AX06 or N03AX11 and J05AX06 or N03AX12 and J05AX06 or N03AX13 and J05AX06 or N03AX14 and J05AX06 or N03AX15 and J05AX06 or N03AX16 and J05AX06 or N03AX17 and J05AX06 or N03AX18 and J05AX06 or N03AX19 and J05AX06 or N03AX21 and J05AX06 or N03AX22 and J05AX06 or N03AX23 and J05AX06 or N03AX24 and J05AX06 or N03AX30 and J05AX06 or N03AA01 and J05AX07 or N03AA02 and J05AX07 or N03AA03 and J05AX07 or N03AA04 and J05AX07 or N03AA30 and J05AX07 or N03AB01 and J05AX07 or N03AB02 and J05AX07 or N03AB03 and J05AX07 or N03AB04 and J05AX07 or N03AB05 and J05AX07 or N03AB52 and J05AX07 or N03AB54 and J05AX07 or N03AC01 and J05AX07 or N03AC02 and J05AX07 or N03AC03 and J05AX07 or N03AD01 and J05AX07 or N03AD02 and J05AX07 or N03AD03 and J05AX07 or N03AD51 and J05AX07 or N03AE01 and J05AX07 or N03AF01 and J05AX07 or N03AF02 and J05AX07 or N03AF03 and J05AX07 or N03AF04 and J05AX07 or N03AG01 and J05AX07 or N03AG02 and J05AX07 or N03AG03 and J05AX07 or N03AG04 and J05AX07 or N03AG05 and J05AX07 or N03AG06 and J05AX07 or N03AX03 and J05AX07 or N03AX07 and J05AX07 or N03AX09 and J05AX07 or N03AX10 and J05AX07 or N03AX11 and J05AX07 or N03AX12 and J05AX07 or N03AX13 and J05AX07 or N03AX14 and J05AX07 or N03AX15 and J05AX07 or N03AX16 and J05AX07 or N03AX17 and J05AX07 or N03AX18 and J05AX07 or N03AX19 and J05AX07 or N03AX21 and J05AX07 or N03AX22 and J05AX07 or N03AX23 and J05AX07 or N03AX24 and J05AX07 or N03AX30 and J05AX07 or N03AA01 and J05AX08 or N03AA02 and J05AX08 or N03AA03 and J05AX08 or N03AA04 and J05AX08 or N03AA30 and J05AX08 or N03AB01 and J05AX08 or N03AB02 and J05AX08 or N03AB03 and J05AX08 or N03AB04 and J05AX08 or N03AB05 and J05AX08 or N03AB52 and J05AX08 or N03AB54 and J05AX08 or N03AC01 and J05AX08 or N03AC02 and J05AX08 or N03AC03 and J05AX08 or N03AD01 and J05AX08 or N03AD02 and J05AX08 or N03AD03 and J05AX08 or N03AD51 and J05AX08 or N03AE01 and J05AX08 or N03AF01 and J05AX08 or N03AF02 and J05AX08 or N03AF03 and J05AX08 or N03AF04 and J05AX08 or N03AG01 and J05AX08 or N03AG02 and J05AX08 or N03AG03 and J05AX08 or N03AG04 and J05AX08 or N03AG05 and J05AX08 or N03AG06 and J05AX08 or N03AX03 and J05AX08 or N03AX07 and J05AX08 or N03AX09 and J05AX08 or N03AX10 and J05AX08 or N03AX11 and J05AX08 or N03AX12 and J05AX08 or N03AX13 and J05AX08 or N03AX14 and J05AX08 or N03AX15 and J05AX08 or N03AX16 and J05AX08 or N03AX17 and J05AX08 or N03AX18 and J05AX08 or N03AX19 and J05AX08 or N03AX21 and J05AX08 or N03AX22 and J05AX08 or N03AX23 and J05AX08 or N03AX24 and J05AX08 or N03AX30 and J05AX08 or N03AA01 and J05AX09 or N03AA02 and J05AX09 or N03AA03 and J05AX09 or N03AA04 and J05AX09 or N03AA30 and J05AX09 or N03AB01 and J05AX09 or N03AB02 and J05AX09 or N03AB03 and J05AX09 or N03AB04 and J05AX09 or N03AB05 and J05AX09 or N03AB52 and J05AX09 or N03AB54 and J05AX09 or N03AC01 and J05AX09 or N03AC02 and J05AX09 or N03AC03 and J05AX09 or N03AD01 and J05AX09 or N03AD02 and J05AX09 or N03AD03 and J05AX09 or N03AD51 and J05AX09 or N03AE01 and J05AX09 or N03AF01 and J05AX09 or N03AF02 and J05AX09 or N03AF03 and J05AX09 or N03AF04 and J05AX09 or N03AG01 and J05AX09 or N03AG02 and J05AX09 or N03AG03 and J05AX09 or N03AG04 and J05AX09 or N03AG05 and J05AX09 or N03AG06 and J05AX09 or N03AX03 and J05AX09 or N03AX07 and J05AX09 or N03AX09 and J05AX09 or N03AX10 and J05AX09 or N03AX11 and J05AX09 or N03AX12 and J05AX09 or N03AX13 and J05AX09 or N03AX14 and J05AX09 or N03AX15 and J05AX09 or N03AX16 and J05AX09 or N03AX17 and J05AX09 or N03AX18 and J05AX09 or N03AX19 and J05AX09 or N03AX21 and J05AX09 or N03AX22 and J05AX09 or N03AX23 and J05AX09 or N03AX24 and J05AX09 or N03AX30 and J05AX09 or N03AA01 and J05AX10 or N03AA02 and J05AX10 or N03AA03 and J05AX10 or N03AA04 and J05AX10 or N03AA30 and J05AX10 or N03AB01 and J05AX10 or N03AB02 and J05AX10 or N03AB03 and J05AX10 or N03AB04 and J05AX10 or N03AB05 and J05AX10 or N03AB52 and J05AX10 or N03AB54 and J05AX10 or N03AC01 and J05AX10 or N03AC02 and J05AX10 or N03AC03 and J05AX10 or N03AD01 and J05AX10 or N03AD02 and J05AX10 or N03AD03 and J05AX10 or N03AD51 and J05AX10 or N03AE01 and J05AX10 or N03AF01 and J05AX10 or N03AF02 and J05AX10 or N03AF03 and J05AX10 or N03AF04 and J05AX10 or N03AG01 and J05AX10 or N03AG02 and J05AX10 or N03AG03 and J05AX10 or N03AG04 and J05AX10 or N03AG05 and J05AX10 or N03AG06 and J05AX10 or N03AX03 and J05AX10 or N03AX07 and J05AX10 or N03AX09 and J05AX10 or N03AX10 and J05AX10 or N03AX11 and J05AX10 or N03AX12 and J05AX10 or N03AX13 and J05AX10 or N03AX14 and J05AX10 or N03AX15 and J05AX10 or N03AX16 and J05AX10 or N03AX17 and J05AX10 or N03AX18 and J05AX10 or N03AX19 and J05AX10 or N03AX21 and J05AX10 or N03AX22 and J05AX10 or N03AX23 and J05AX10 or N03AX24 and J05AX10 or N03AX30 and J05AX10 or
N03AA01 and J05AX11 or N03AA02 and J05AX1 1 or N03AA03 and J05AX11 or N03AA04 and J05AX11 or N03AA30 and J05AX11 or N03AB01 and J05AX11 or N03AB02 and J05AX11 or N03AB03 and J05AX11 or N03AB04 and J05AX11 or N03AB05 and J05AX11 or N03AB52 and J05AX11 or N03AB54 and J05AX11 or N03AC01 and J05AX11 or N03AC02 and J05AX11 or N03AC03 and J05AX11 or N03AD01 and J05AX11 or N03AD02 and J05AX11 or N03AD03 and J05AX11 or N03AD51 and J05AX11 or N03AE01 and J05AX11 or N03AF01 and J05AX11 or N03AF02 and J05AX11 or N03AF03 and J05AX11 or N03AF04 and J05AX11 or N03AG01 and J05AX11 or N03AG02 and J05AX11 or N03AG03 and J05AX11 or N03AG04 and J05AX11 or N03AG05 and J05AX11 or N03AG06 and J05AX11 or N03AX03 and J05AX11 or N03AX07 and J05AX11 or N03AX09 and J05AX11 or N03AX10 and J05AX11 or N03AX11 and J05AX11 or N03AX12 and J05AX11 or N03AX13 and J05AX11 or N03AX14 and J05AX11 or N03AX15 and J05AX11 or N03AX16 and J05AX11 or N03AX17 and J05AX11 or N03AX18 and J05AX11 or N03AX19 and J05AX11 or N03AX21 and J05AX11 or N03AX22 and J05AX11 or N03AX23 and J05AX11 or N03AX24 and J05AX11 or N03AX30 and J05AX11 or N03AA01 and J05AX12 or N03AA02 and J05AX12 or N03AA03 and J05AX12 or N03AA04 and J05AX12 or N03AA30 and J05AX12 or N03AB01 and J05AX12 or N03AB02 and J05AX12 or N03AB03 and J05AX12 or N03AB04 and J05AX12 or N03AB05 and J05AX12 or N03AB52 and J05AX12 or N03AB54 and J05AX12 or N03AC01 and J05AX12 or N03AC02 and J05AX12 or N03AC03 and J05AX12 or N03AD01 and J05AX12 or N03AD02 and J05AX12 or N03AD03 and J05AX12 or N03AD51 and J05AX12 or N03AE01 and J05AX12 or N03AF01 and J05AX12 or N03AF02 and J05AX12 or N03AF03 and J05AX12 or N03AF04 and J05AX12 or N03AG01 and J05AX12 or N03AG02 and J05AX12 or N03AG03 and J05AX12 or N03AG04 and J05AX12 or N03AG05 and J05AX12 or N03AG06 and J05AX12 or N03AX03 and J05AX12 or N03AX07 and J05AX12 or N03AX09 and J05AX12 or N03AX10 and J05AX12 or N03AX11 and J05AX12 or N03AX12 and J05AX12 or N03AX13 and J05AX12 or N03AX14 and J05AX12 or N03AX15 and J05AX12 or N03AX16 and J05AX12 or N03AX17 and J05AX12 or N03AX18 and J05AX12 or N03AX19 and J05AX12 or N03AX21 and J05AX12 or N03AX22 and J05AX12 or N03AX23 and J05AX12 or N03AX24 and J05AX12 or N03AX30 and J05AX12 or N03AA01 and J05AX13 or N03AA02 and J05AX13 or N03AA03 and J05AX13 or N03AA04 and J05AX13 or N03AA30 and J05AX13 or N03AB01 and J05AX13 or N03AB02 and J05AX13 or N03AB03 and J05AX13 or N03AB04 and J05AX13 or N03AB05 and J05AX13 or N03AB52 and J05AX13 or N03AB54 and J05AX13 or N03AC01 and J05AX13 or N03AC02 and J05AX13 or N03AC03 and J05AX13 or N03AD01 and J05AX13 or N03AD02 and J05AX13 or N03AD03 and J05AX13 or N03AD51 and J05AX13 or N03AE01 and J05AX13 or N03AF01 and J05AX13 or N03AF02 and J05AX13 or N03AF03 and J05AX13 or N03AF04 and J05AX13 or N03AG01 and J05AX13 or N03AG02 and J05AX13 or N03AG03 and J05AX13 or N03AG04 and J05AX13 or N03AG05 and J05AX13 or N03AG06 and J05AX13 or N03AX03 and J05AX13 or N03AX07 and J05AX13 or N03AX09 and J05AX13 or N03AX10 and J05AX13 or N03AX11 and J05AX13 or N03AX12 and J05AX13 or N03AX13 and J05AX13 or N03AX14 and J05AX13 or N03AX15 and J05AX13 or N03AX16 and J05AX13 or N03AX17 and J05AX13 or N03AX18 and J05AX13 or N03AX19 and J05AX13 or N03AX21 and J05AX13 or N03AX22 and J05AX13 or N03AX23 and J05AX13 or N03AX24 and J05AX13 or N03AX30 and J05AX13 or N03AA01 and J05AX17 or N03AA02 and J05AX17 or N03AA03 and J05AX17 or N03AA04 and J05AX17 or N03AA30 and J05AX17 or N03AB01 and J05AX17 or N03AB02 and J05AX17 or N03AB03 and J05AX17 or N03AB04 and J05AX17 or N03AB05 and J05AX17 or N03AB52 and J05AX17 or N03AB54 and J05AX17 or N03AC01 and J05AX17 or N03AC02 and J05AX17 or N03AC03 and J05AX17 or N03AD01 and J05AX17 or N03AD02 and J05AX17 or N03AD03 and J05AX17 or N03AD51 and J05AX17 or N03AE01 and J05AX17 or N03AF01 and J05AX17 or N03AF02 and J05AX17 or N03AF03 and J05AX17 or N03AF04 and J05AX17 or N03AG01 and J05AX17 or N03AG02 and J05AX17 or N03AG03 and J05AX17 or N03AG04 and J05AX17 or N03AG05 and J05AX17 or N03AG06 and J05AX17 or N03AX03 and J05AX17 or N03AX07 and J05AX17 or N03AX09 and J05AX17 or N03AX10 and J05AX17 or N03AX11 and J05AX17 or N03AX12 and J05AX17 or N03AX13 and J05AX17 or N03AX14 and J05AX17 or N03AX15 and J05AX17 or N03AX16 and J05AX17 or N03AX17 and J05AX17 or N03AX18 and J05AX17 or N03AX19 and J05AX17 or N03AX21 and J05AX17 or N03AX22 and J05AX17 or N03AX23 and J05AX17 or N03AX24 and J05AX17 or N03AX30 and J05AX17 or N03AA01 and J05AX18 or N03AA02 and J05AX18 or N03AA03 and J05AX18 or N03AA04 and J05AX18 or N03AA30 and J05AX18 or N03AB01 and J05AX18 or N03AB02 and J05AX18 or N03AB03 and J05AX18 or N03AB04 and J05AX18 or N03AB05 and J05AX18 or N03AB52 and J05AX18 or N03AB54 and J05AX18 or N03AC01 and J05AX18 or N03AC02 and J05AX18 or N03AC03 and J05AX18 or N03AD01 and J05AX18 or N03AD02 and J05AX18 or N03AD03 and J05AX18 or N03AD51 and J05AX18 or N03AE01 and J05AX18 or N03AF01 and J05AX18 or N03AF02 and J05AX18 or N03AF03 and J05AX18 or N03AF04 and J05AX18 or N03AG01 and J05AX18 or N03AG02 and J05AX18 or N03AG03 and J05AX18 or N03AG04 and J05AX18 or N03AG05 and J05AX18 or N03AG06 and J05AX18 or N03AX03 and J05AX18 or N03AX07 and J05AX18 or N03AX09 and J05AX18 or N03AX10 and J05AX18 or N03AX11 and J05AX18 or N03AX12 and J05AX18 or N03AX13 and J05AX18 or N03AX14 and J05AX18 or N03AX15 and J05AX18 or N03AX16 and J05AX18 or N03AX17 and J05AX18 or N03AX18 and J05AX18 or N03AX19 and J05AX18 or N03AX21 and J05AX18 or N03AX22 and J05AX18 or N03AX23 and J05AX18 or N03AX24 and J05AX18 or N03AX30 and J05AX18 or N03AA01 and J05AX19 or N03AA02 and J05AX19 or N03AA03 and J05AX19 or N03AA04 and J05AX19 or N03AA30 and J05AX19 or N03AB01 and J05AX19 or N03AB02 and J05AX19 or N03AB03 and J05AX19 or N03AB04 and J05AX19 or N03AB05 and J05AX19 or N03AB52 and J05AX19 or N03AB54 and J05AX19 or N03AC01 and J05AX19 or N03AC02 and J05AX19 or N03AC03 and J05AX19 or N03AD01 and J05AX19 or N03AD02 and J05AX19 or N03AD03 and J05AX19 or N03AD51 and J05AX19 or N03AE01 and J05AX19 or N03AF01 and J05AX19 or N03AF02 and J05AX19 or N03AF03 and J05AX19 or N03AF04 and J05AX19 or N03AG01 and J05AX19 or N03AG02 and J05AX19 or N03AG03 and J05AX19 or N03AG04 and J05AX19 or N03AG05 and J05AX19 or N03AG06 and J05AX19 or N03AX03 and J05AX19 or N03AX07 and J05AX19 or N03AX09 and J05AX19 or N03AX10 and J05AX19 or N03AX11 and J05AX19 or N03AX12 and J05AX19 or N03AX13 and J05AX19 or N03AX14 and J05AX19 or N03AX15 and J05AX19 or N03AX16 and J05AX19 or N03AX17 and J05AX19 or N03AX18 and J05AX19 or N03AX19 and J05AX19 or N03AX21 and J05AX19 or N03AX22 and J05AX19 or N03AX23 and J05AX19 or N03AX24 and J05AX19 or N03AX30 and J05AX19 or N03AA01 and J05AX21 or N03AA02 and J05AX21 or N03AA03 and J05AX21 or N03AA04 and J05AX21 or N03AA30 and J05AX21 or N03AB01 and J05AX21 or N03AB02 and J05AX21 or N03AB03 and J05AX21 or N03AB04 and J05AX21 or N03AB05 and J05AX21 or N03AB52 and J05AX21 or N03AB54 and J05AX21 or N03AC01 and J05AX21 or N03AC02 and J05AX21 or N03AC03 and J05AX21 or N03AD01 and J05AX21 or N03AD02 and J05AX21 or N03AD03 and J05AX21 or N03AD51 and J05AX21 or N03AE01 and J05AX21 or N03AF01 and J05AX21 or N03AF02 and J05AX21 or N03AF03 and J05AX21 or N03AF04 and J05AX21 or N03AG01 and J05AX21 or N03AG02 and J05AX21 or N03AG03 and J05AX21 or N03AG04 and J05AX21 or N03AG05 and J05AX21 or N03AG06 and J05AX21 or N03AX03 and J05AX21 or N03AX07 and J05AX21 or N03AX09 and J05AX21 or N03AX10 and J05AX21 or N03AX11 and J05AX21 or N03AX12 and J05AX21 or N03AX13 and J05AX21 or N03AX14 and J05AX21 or N03AX15 and J05AX21 or N03AX16 and J05AX21 or N03AX17 and J05AX21 or N03AX18 and J05AX21 or N03AX19 and J05AX21 or N03AX21 and J05AX21 or N03AX22 and J05AX21 or N03AX23 and J05AX21 or N03AX24 and J05AX21 or N03AX30 and J05AX21 or N03AA01 and J05AX23 or N03AA02 and J05AX23 or N03AA03 and J05AX23 or N03AA04 and J05AX23 or N03AA30 and J05AX23 or N03AB01 and J05AX23 or N03AB02 and J05AX23 or N03AB03 and J05AX23 or N03AB04 and J05AX23 or N03AB05 and J05AX23 or N03AB52 and J05AX23 or N03AB54 and J05AX23 or N03AC01 and J05AX23 or N03AC02 and J05AX23 or N03AC03 and J05AX23 or N03AD01 and J05AX23 or N03AD02 and J05AX23 or N03AD03 and J05AX23 or N03AD51 and J05AX23 or N03AE01 and J05AX23 or N03AF01 and J05AX23 or N03AF02 and J05AX23 or N03AF03 and J05AX23 or N03AF04 and J05AX23 or N03AG01 and J05AX23 or N03AG02 and J05AX23 or N03AG03 and J05AX23 or N03AG04 and J05AX23 or N03AG05 and J05AX23 or N03AG06 and J05AX23 or N03AX03 and J05AX23 or N03AX07 and J05AX23 or N03AX09 and J05AX23 or N03AX10 and J05AX23 or N03AX11 and J05AX23 or N03AX12 and J05AX23 or N03AX13 and J05AX23 or N03AX14 and J05AX23 or N03AX15 and J05AX23 or N03AX16 and J05AX23 or N03AX17 and J05AX23 or N03AX18 and J05AX23 or N03AX19 and J05AX23 or N03AX21 and J05AX23 or N03AX22 and J05AX23 or N03AX23 and J05AX23 or N03AX24 and J05AX23 or N03AX30 and J05AX23.

It is also preferable when this composition is composed of any combination of active substances selected from the NO3 ATC group and active substances selected from the J05 ATC group.

The use of J05 ATC group substances to treat nervous tissue dysfunction, in particular viral latency, where the preferable J05 ATC group substance is: metisazone or aciclovir or idoxuridine or vidarabine or ganciclovir or famciclovir or valaciclovir or cidofovir or penciclovir or valganciclovir or brivudine or rimantadine or tromantadine or foscarnet or fosfonet or saquinavir or indinavir or ritonavir or nelfinavir or amprenavir or fosamprenavir or atazanavir or tipranavir or darunavir or zidovudine or didanosine or zalcitabine or stavudine or lamivudine or abacavir or tenofovir disoproxil or adefovir dipivoxil or emtricitabine or entecavir or telbivudine or clevudine or tenofovir alafenamide or nevirapine or delavirdine or efavirenz or etravirine or rilpivirine or doravirine or zanamivir or oseltamivir or peramivir or ribavirin or telaprevir or boceprevir or faldaprevir or simeprevir or asunaprevir or daclatasvir or sofosbuvir or dasabuvir or sofosbuvir and ledipasvir or dasabuvir, ombitasvir, paritaprevir and ritonavir or ombitasvir, paritaprevir and ritonavir or elbasvir and grazoprevir or sofosbuvir and velpatasvir or sofosbuvir, velpatasvir and voxilaprevir or glecaprevir and pibrentasvir or zidovudine and lamivudine or lamivudine and abacavir or tenofovir disoproxil and emtricitabine or zidovudine, lamivudine and abacavir or zidovudine, lamivudine and nevirapine or emtricitabine, tenofovir disoproxil and efavirenz or stavudine, lamivudine and nevirapine or emtricitabine, tenofovir disoproxil and rilpivirine or emtricitabine, tenofovir disoproxil, elvitegravir and cobicistat lopinavir and ritonavir or lamivudine, tenofovir disoproxil and efavirenz or lamivudine and tenofovir disoproxil or lamivudine, abacavir and dolutegravir or darunavir and cobicistat or atazanavir and cobicistat or lamivudine and raltegravir or emtricitabine and tenofovir alafenamide or emtricitabine, tenofovir alafenamide, elvitegravir and cobicistat or emtricitabine or tenofovir alafenamide and rilpivirine or emtricitabine, tenofovir alafenamide and bictegravir or dolutegravir and rilpivirine or emtricitabine, tenofovir or alafenamide, darunavir and cobicistat or atazanavir and ritonavir or lamivudine, tenofovir disoproxil and doravirine or moroxydine or lysozyme or inosine pranobex or pleconaril or enfuvirtide or raltegravir or maraviroc or maribavir or elvitegravir or dolutegravir or umifenovir or enisamium iodide or letermovir or tilorone or pentanedioic acid imidazolyl ethanamide or ibalizumab.
Unexpectedly, it turned out that the combination of at least two active substances, with at least one having antiviral effect, for example inosine pranobex and the other having antiepileptic effect, for example sodium valproate (valproic acid), with an appropriate quantitative selection in the composition, resulted in a more effective therapeutic effect in the case of diagnosed childhood epilepsy, as confirmed by the cases described below.

It was also found that J05 ATC group substances are effective in treatment of viral infections during the latent phase, in particular Herpes viruses, which cause neurological disorders such as epilepsy.

A healthy 3 year old girl suddenly started experiencing problems with cognitive, sensory and motor performance due to unknown causes. She struggled with learning new words and speaking correctly, her vocabulary was limited to only a few nouns, she did not use any verbs. After she turned 5, contrary to the opinions of doctors (laryngologist and speech therapist), the girl did not start talking, and her development deficit kept widening. After visiting a paediatric neurologist and based on the performed EEG, the child was diagnosed with childhood epilepsy of unknown cause. The child took an anti-epileptic drug "Depakine" for one year, with sodium valproate as the active substance, at a dose of 200 mg in the morning and 200 mg in the evening. At the same time, the child went to daily appointments with a speech therapist that each lasted for 30 minutes. After a year, the child's progress in speech and motor skills were insignificant. Although the number of words used by the girl increased slightly, she still could not speak in complex sentences typical for children her age. In the meantime, the child developed a viral infection and was given a commonly available, over-the-counter antiviral drug called "Neosine Forte" at doses of 4 ml in the morning and 4 ml in the evening, with inosine pranobex as the active substance. After a few days of treatment with this drug together with the antiepileptic drug, it was unexpectedly noted that the girl started acquiring words that she had struggled with before, she started speaking in complex sentences, as well as singing, riding a bike and acting more freely in a group of peers. It should be noted that at that time the child was not participating in speech therapy. The use of these drugs for the next dozen or so days resulted in further psychophysical development of the child. The previously experienced problems with short-term memory disappeared, and the performed control tests, including liver and kidney functions, did not deviate from the norm.

On the other hand, in the case of a 5-year old girl with the same symptoms as those described above and a diagnosis of childhood epilepsy of unknown cause, treated for about 10 months with a typical antiepileptic drug, it was decided to administer a composition consistent with the invention, in which the active substance was valproic acid and inosine pranobex in a ratio of 1:2.13. The composition was administered as a syrup at two daily doses containing 187.33 mg of valproic acid and 400 mg of inosine pranobex. After a few days, the girl's psychophysical development improved significantly. The girl remembered the words she had learned the day before, she spoke in sentences using verbs, and established relationships with her peers. At the same time, it was observed that the administration of a composition containing ¾ of the recommended dose of active substance with antiviral effect (inosine pranobex) caused a small loss of sensory and motor skills of the child, and a return to the earlier dose of the composition caused the child to advance more quickly in the development. Also in this case, liver and kidney function tests carried out after three months of treatment with the composition showed normal parameters.
As seen from the above, the effect of a composition consistent with the invention is not accidental and isolated. The examples described above may prove that epileptic symptoms are of viral nature, as EEG studies of patients with symptomatic and asymptomatic seizure disorders point to the focal nature of brain dysfunction, which is related to latency of viruses, especially Herpes viruses, in human nerve cells.
Therefore, it should be stated that the active substance used in the composition consistent with the invention - inosine pranobex - does not act as its second active substance, for example valproic acid, preventing impaired brain function due to excessive and violent discharge of electrical impulses in nerve cells, but has antiviral effect, preventing the proliferation of pathogenic viruses, including Herpes viruses. The use of the composition had a surprising and lasting effect, which manifested itself in an improvement of short-term memory and motor skills of the analysed underage patients. This proves the existence of a viral focus in brain cells and its effects on the human brain, manifested by memory dysfunction and diagnosed as epilepsy.

Pharmacological composition consistent with the invention contains at least one antiepileptic active substance selected from the NO3 ATC group (in accordance with the WHO classification) and at least one antiviral active substance selected from the J05 ATC group (in accordance with the WHO classification). Active substances of the NO3 and J05 groups with examples of their defined daily dose (a statistical measure of drug consumption in adult patients) according to WHO are shown in Tables 1 and 2 respectively.

**Table 1**

| **Antiepileptic active substances contained in the composition together with their DDDs according to WHO** | | | | |
|---|---|---|---|---|
| **DDD** | **UM** | **Route of administration** | **Name of the active substance** | **ATC Code** |
| 0.5 | g | O | methylphenobarbital | N03AA01 |
| 0.1 | g | O | phenobarbital | N03AA02 |
| 0.1 | g | P | | |
| 1.25 | g | O | primidone | N03AA03 |
| | | | barbexaclone | N03AA04 |
| 0.2 | g | O | metharbital | N03AA30 |
| 2.5 | g | O | ethotoin | N03AB01 |
| 0.3 | g | O | phenytoin | N03AB02 |
| 0.3 | g | P | | |
| 0.3 | g | O | amino(diphenylhydantoin) valeric acid | N03AB03 |
| 0.4 | g | O | mephenytoin | N03AB04 |
| 0.45 | g | P | fosphenytoin | N03AB05 |
| | | | phenytoin, combinations | N03AB52 |
| | | | mephenytoin combinations | N03AB54 |
| 0.9 | g | O | paramethadione | N03AC01 |
| 1.5 | g | O | trimethadione | |
| | | | ethadione | N03AC03 |
| 1.25 | g | O | ethosuximide | N03AD01 |
| 2.0 | g | O | phensuximide | N03AD02 |
| 0.9 | g | O | mesuximide | N03AD03 |
| | | | ethosuximide combinations | N03AD51 |
| 8.0 | mg | O | clonazepam | N03AE01 |
| 8.0 | mg | P | | |
| 1.0 | g | O | carbamazepine | N03AF01 |
| 1.0 | g | R | | |
| 1.0 | g | O | oxcarbazepine | N03AF02 |
| 1.4 | g | O | rufinamide | N03AF03 |
| 0.8 | g | O | eslicarbazepine | N03AF04 |
| 1.5 | g | O | valproic acid | N03AG01 |
| 1.5 | g | P | | |
| 1.5 | g | R | | |
| 1.5 | g | O | valpromide | N03AG02 |
| 1.0 | g | O | aminobutyric acid | N03AG03 |
| 1.0 | g | P | | |
| 2.0 | g | O | vigabatrin | N03AG04 |
| | | | progabide | N03AG05 |
| 30.0 | mg | O | tiagabine | N03AG06 |
| 0.4 | g | O | sultiame | N03AX03 |
| 1.5 | g | O | phenacemide | N03AX07 |
| 0.3 | g | O | lamotrigine | N03AX09 |
| 2.4 | g | O | felbamate | N03AX10 |
| 0.3 | g | O | topiramate | N03AX11 |
| 1.8 | g | O | gabapentin | N03AX12 |
| | | | pheneturide | N03AX13 |
| 1.5 | g | O | levetiracetam | N03AX14 |
| 1.5 | g | P | | |
| 0.2 | g | O | zonisamide | N03AX15 |
| 0.3 | g | O | pregabalin | N03AX16 |
| 1.0 | g | O | stiripentol | N03AX17 |
| 0.3 | g | O | lacosamide | N03AX18 |
| 0.3 | g | P | | |
| | | | carisbamate | N03AX19 |
| 0.9 | g | O | retigabine | N03AX21 |
| 8.0 | mg | O | perampanel | N03AX22 |
| 0.1 | g | O | brivaracetam | N03AX23 |
| 0.1 | g | P | | |
| | | | cannabidiol | N03AX24 |
| | | | beclamide | N03AX30 |

**Table 2**

| **Antiepileptic active substances contained in the composition together with their DDDs according to WHO** | | | | |
|---|---|---|---|---|
| **DDD** | **UM** | **Route of administration** | **Name of the active substance** | **ATC Code** |
| | | | metisazone | J05AA01 |
| 4.0 | g | O | aciclovir | J05AB01 |
| 4.0 | g | P | | |
| | | | idoxuridine | J05AB02 |
| | | | vidarabine | J05AB03 |
| 3.0 | g | O | ganciclovir | J05AB06 |
| 0.5 | g | P | | |
| 0.75 | g | O | famciclovir | J05AB09 |
| 3.0 | g | O | valaciclovir | J05AB11 |
| 25.0 | mg | P | cidofovir | J05AB12 |
| | | | penciclovir | J05AB13 |
| 0.9 | g | O | valganciclovir | J05AB 14 |
| 0.125 | g | O | brivudine | J05AB15 |
| 0.2 | g | O | rimantadine | J05AC02 |
| | | | tromantadine | J05AC03 |
| 6.5 | g | P | foscarnet | J05AD01 |
| | | | fosfonet | J05AD02 |
| 1.8 | g | O | saquinavir | J05AE01 |
| 2.4 | g | O | indinavir | J05AE02 |
| 1.2 | g | O | ritonavir | J05AE03 |
| 2.25 | g | O | nelfinavir | J05AE04 |
| 1.2 | g | O | amprenavir | J05AE05 |
| 1.4 | g | O | fosamprenavir | J05AE07 |
| 0.3 | g | O | atazanavir | J05AE08 |
| 1.0 | g | O | tipranavir | J05AE09 |
| 1.2 | g | O | darunavir | J05AE10 |
| 0.6 | g | O | zidovudine | J05AF01 |
| 0.6 | g | P | | |
| 0.4 | g | O | didanosine | J0SAF02 |
| 2.25 | mg | O | zalcitabine | J05AF03 |
| 80 | mg | O | stavudine | J05AF04 |
| 0.3 | g | O | lamivudine | J05AF05 |
| 0.6 | g | O | abacavir | J05AF06 |
| 0.245 | g | O | tenofovir disoproxil | J05AF07 |
| 10.0 | mg | O | adefovir dipivoxil | J05AF08 |
| 0.2 | g | O | emtricitabine | J05AF09 |
| 0.5 | mg | O | entecavir | J05AF10 |
| 0.6 | g | O | telbivudine | J0SAF11 |
| 30 | mg | O | clevudine | J05AF12 |
| 25.0 | mg | O | tenofovir alafenamide | J05AF13 |
| 0.4 | g | O | nevirapine | J05AG01 |
| 1.2 | g | O | delavirdine | J05AG02 |
| 0.6 | g | O | efavirenz | J05AG03 |
| 0.4 | g | O | etravirine | J05AG04 |
| 25.0 | mg | O | rilpivirine | J05AG05 |
| | | | doravirine | J05AG06 |
| 20.0 | mg | Inhal.powder | zanamivir | J05AH01 |
| 0.15 | g | O | oseltamivir | J05AH02 |
| | | | peramivir | J05AH03 |
| 6.0 | g | Inhal.solution | ribavirin | J05AP01 |
| 1.0 | g | O | | |
| 2.25 | g | O | telaprevir | J05AP02 |
| 2.4 | g | O | boceprevir | J05AP03 |
| | | | faldaprevir | J05AP04 |
| 0.15 | g | O | simeprevir | J05AP05 |
| | | | asunaprevir | J05AP06 |
| 60.0 | mg | O | daclatasvir | J05AP07 |
| 0.4 | g | O | sofosbuvir | J05AP08 |
| 0.5 | g | O | dasabuvir | J05AP09 |
| | | | sofosbuvir and ledipasvir | J05AP51 |
| | | | dasabuvir ombitasvir, paritaprevir and ritonavir | J05AP52 |
| | | | ombitasvir, paritaprevir and ritonavir | J05AP53 |
| | | | elbasvir and grazoprevir | J05AP54 |
| | | | sofosbuvir and velpatasvir | J05AP55 |
| | | | sofosbuvir, velpatasvir and voxilaprevir | J05AP56 |
| | | | glecaprevir and pibrentasvir | J05AP57 |
| | | | zidovudine and lamivudine | J05AR01 |
| | | | lamivudine and abacavir | J05AR02 |
| | | | tenofovir disoproxil and emtricitabine | J05AR03 |
| | | | zidovudine, lamivudine and abacavir | J05AR04 |
| | | | zidovudine, lamivudine and nevirapine | J05AR05 |
| | | | emtricitabine, tenofovir disoproxil and efavirenz | J05AR06 |
| | | | stavudine, lamivudine and nevirapine | J05AR07 |
| | | | emtricitabine, tenofovir disoproxil and rilpivirine | J05AR08 |
| | | | emtricitabine, tenofovir disoproxil, elvitegravir and cobicistat | J05AR09 |
| 0.8 | g | O | lopinavir and ritonavir | J05AR10 |
| | | | lamivudine, tenofovir disoproxil and efavirenz | J05AR11 |
| | | | lamivudine and tenofovir disoproxil | J05AR12 |
| | | | lamivudine, abacavir and dolutegravir | J05AR13 |
| | | | darunavir and cobicistat | J05AR14 |
| | | | atazanavir and cobicistat | J05AR15 |
| | | | lamivudine and raltearavir | J05AR16 |
| | | | emtricitabine and tenofovir alafenamide | J05AR17 |
| | | | emtricitabine. tenofovir alafenamide, elvitegravir and cobicistat | J05AR18 |
| | | | emtricitabine, tenofovir | J05AR19 |
| | | | alafenamide and rilpivirine | |
| | | | emtricitabine, tenofovir | J05AR20 |
| | | | alafenamide and bictegravir | |
| | | | dolutegravir and rilpivirine | J05AR21 |
| | | | emtricitabine, tenofovir alafenamide, darunavir and cobicistat | J05AR22 |
| 0.3 | g | O | atazanavir and ritonavir | J05AR23 |
| | | | lamivudine, tenofovir disoproxil and doravirine | J05AR24 |
| 0.3 | g | O | moroxydine | J05AX01 |
| | | | lysozyme | J05AX02 |
| 3.0 | g | O | inosine pranobex | J05AX05 |
| | | | pleconaril | J05AX06 |
| 0.18 | g | P | enfuvirtide | J05AX07 |
| 0.8 | g | O | raltegravir | J05AX08 |
| 0.6 | g | O | maraviroc | J05AX09 |
| | | | maribavir | J05AX10 |
| | | | elvitegravir | J05AX11 |
| 50.0 | mg | O | dolutegravir | J05AX12 |
| 0.8 | g | O | umifenovir | J05AX13 |
| 1.5 | g | O | enisamium iodide | J05AX17 |
| 0.48 | g | O | letermovir | J05AX18 |
| 0.48 | g | P | | |
| 0.125 | g | O | tilorone | J05AX19 |
| 90.0 | mg | O | pentanedioic acid imidazolyl ethanamide | J05AX21 |
| | | | ibalizumab | J05AX23 |

The letter "O" used in Tables 1 and 2 means oral administration and the letter "P" means parenteral administration.

The composition consistent with the invention can take the form of a tablet, capsule, powder, granules, solution, suspension, emulsion, syrup, injection, aerosol, etc., with the use of the accepted pharmaceutical auxiliaries, such as: excipent, carrier, thinner, stabilizer, flavour and fragrance, etc. Regardless of the form of the composition, the dose and frequency of administration is adjusted according to age, weight and symptoms experienced by the patient. The weight ratio in [mg] of the antiepileptic active substance to the antiviral active substance is as follows: 10.000.000 to 1:1; 1.000.000 to 1:1; 100.000 to 1:1; 10.000 to 1:1; 1.000 to 1:1; 100 to 1:1; 10 to 1:1; 1:1 to 10.000.000; 1:1 to 1.000.000; 1:1 to 100.000; 1:1 to 10.000; 1 to 1: 1.000; 1:1 to 100; 1:1 to 10 preferably 1:2, most preferably 1:2.13.

Table 3 shows examples of the amounts of active substances contained in the composition consistent with the invention, which can be administered in one or more split doses per day, adjusted to the symptoms.
The abbreviation "b.w." used in the table means body weight.

**Table 3**

| **Active substances contained in the composition** | | | |
|---|---|---|---|
| Antiepileptic and antisymptomatic active substance (according to ATC classification) | Quantity in mg/kg b.w./24h | Antiviral active substance (according to ATC classification) | Quantity in mg/kg b.w./24h |
| Valproic acid - sodium valproate (N 03 AG 01) | 20-30 | Inosine pranobex (J 05 AX 05) | 750-2700 |
| Valproic acid - sodium valproate (N 03 AG 01) | 20-30 | Ganciclovir (J 05 AB 06) | 10-12 |
| Valproic acid - sodium valproate (N 03 AG 01) | 20-30 | Ritonavir (J 05 AE 03) | 100 |
| Valproic acid - sodium valproate (N 03 AG 01) | 20-30 | Amprenavir (J 05 AE 05) | 600-1200 |
| Valproic acid - sodium valproate (N 03 AG 01) | 20-30 | Atazanavir (J 05 AE 08) | 300 |
| Valproic acid - sodium valproate (N 03 AG 01) | 20-30 | Typranavir (J 05 AE 09) | 500 |
| Valproic acid - sodium valproate (N 03 AG 01) | 20-30 | Abacavir (J 05 AF 06) | 8-600 |
| Valproic acid - sodium valproate (N 03 AG 01) | 20-30 | Entecavir (J 05 AF 10) | 0.5 |
| Valproic acid - sodium valproate (N 03 AG 01) | 20-30 | Efavirenz (J 05 AG 03) | 600 |
| Valproic acid - sodium valproate (N 03 AG 01) | 20-30 | Moroxidine (J 05 AX 01) | 100-600 |
| Valproic acid - sodium valproate (N 03 AG 01) | 20-30 | Maraviroc (J 05 AX 09) | 150-300 |
| Vigabatrin (N 03 AG 04) | 50-3000 | Inosine pranobex (J 05 AX 05) | 750-2700 |
| Vigabatrin (N 03 AG 04) | 50-3000 | Ganciclovir (J 05 AB 06) | 10-12 |
| Vigabatrin (N 03 AG 04) | 50-3000 | Ritonavir (J 05 AE 03) | 100 |
| Vigabatrin (N 03 AG 04) | 50-3000 | Amprenavir (J 05 AE 05) | 600-1200 |
| Vigabatrin (N 03 AG 04) | 50-3000 | Atazanavir (J 05 AE 08) | 300 |
| Vigabatrin (N 03 AG 04) | 50-3000 | Typranavir (J 05 AE 09) | 500 |
| Vigabatrin (N 03 AG 04) | 50-3000 | Abacavir (J 05 AF 06) | 8-600 |
| Vigabatrin (N 03 AG 04) | 50-3000 | Entecavir (J 05 AF 10) | 0.5 |
| Vigabatrin (N 03 AG 04) | 50-3000 | Efavirenz (J 05 AG 03) | 600 |
| Vigabatrin (N 03 AG 04) | 50-3000 | Moroxidine (J 05 AX 01) | 100-600 |
| Vigabatrin (N 03 AG 04) | 50-3000 | Maraviroc (J 05 AX 09) | 150-300 |

In the next example, the composition consistent with the invention contains phenobarbital in the amount of 60 mg/kg of body weight/24h and valproic acid in the amount of 400 mg/kg of body weight/24h as the antiepileptic active substances as well as inosine pranobex in the amount of 800 mg/kg of body weight/24h as the antiviral active substance.
In yet another example, the composition consistent with the invention contains phenobarbital in the amount of 60 mg/kg of body weight/24h and vigabatrin in the amount of 50 mg/kg of body weight/24h as the antiepileptic active substances as well as inosine pranobex in the amount of 800 mg/kg of body weight/24h as the antiviral active substance.
In the next example, the composition consistent with the invention contains vigabatrin in the amount of 100 mg/kg of body weight/24h and inosine pranobex in the amount of 400 mg/kg of body weight/24h as the antiepileptic active substances as well as atazanavir in the amount of 300 mg/kg of body weight/24h and entecavir in the amount of 0.5 mg/kg of body mass/24h as the antiviral active substances.

It turned out that J05 ATC group substances, for example inosine pranobex, are effective against latent viruses, and their activity is different than in the case of an active virus.

When administering inosine pranobex to treat nervous tissue dysfunction, in particular viral latency, manifested as epileptic disorders, 400 mg of inosine pranobex were administered twice a day, resulting in a visible improvement in psychophysical development of the patient.
In other examples of administering inosine pranobex to treat nervous tissue dysfunction, in particular viral latency, especially HERPES viruses, 750 - 2700 mg/kg of inosine pranobex were administered once or several times a day, depending on the symptoms, age and weight of the patient, to achieve the desired effect.

## Claims

1. A pharmacological composition used in treatment of nervous tissue dysfunction, in particular viral latency, consistent with the invention, contains at least one active substance with antiepileptic effect selected from the NO3 ATC group and at least one active substance with antiviral effect selected from the J05 ATC group.

2. A composition according to claim 1, **characterised in that** the active substance of the NO3 ATC group is: methylphenobarbital or phenobarbital or primidone or barbexaclone or metharbital or ethotoin or phenytoin or amino(diphenylhydantoin) valeric acid or mephenytoin or fosphenytoin or phenytoin combinations or mephenytoin combinations or paramethadione or trimethadione or ethadione or ethosuximide or phensuximide or mesuximide or ethosuximide combinations or clonazepam or carbamazepine or oxcarbazepine or rufinamide or eslicarbazepine or valproic acid or valpromide or aminobutyric acid or vigabatrin or progabide or tiagabine or sultiame or phenacemide or lamotrigine or felbamate or topiramate or gabapentin or pheneturide or levetiracetam or zonisamide or pregabalin or stiripentol or lacosamide or carisbamate or retigabine or perampanel or brivaracetam or cannabidiol or beclamide.

3. A composition according to claim 1, **characterised in that** the active substance of the J05 ATC group is: metisazone or aciclovir or idoxuridine or vidarabine or ganciclovir or famciclovir or valaciclovir or cidofovir or penciclovir or valganciclovir or brivudine or rimantadine or tromantadine or foscarnet or fosfonet or saquinavir or indinavir or ritonavir or nelfinavir or amprenavir or fosamprenavir or atazanavir or tipranavir or darunavir or zidovudine or didanosine or zalcitabine or stavudine or lamivudine or abacavir or tenofovir disoproxil or adefovir dipivoxil or emtricitabine or entecavir or telbivudine or clevudine or tenofovir alafenamide or nevirapine or delavirdine or efavirenz or etravirine or rilpivirine or doravirine or zanamivir or oseltamivir or peramivir or ribavirin or telaprevir or boceprevir or faldaprevir or simeprevir or asunaprevir or daclatasvir or sofosbuvir or dasabuvir or sofosbuvir and ledipasvir or dasabuvir, ombitasvir, paritaprevir and ritonavir or ombitasvir, paritaprevir and ritonavir or elbasvir and grazoprevir or sofosbuvir and velpatasvir or sofosbuvir, velpatasvir and voxilaprevir or glecaprevir and pibrentasvir or zidovudine and lamivudine or lamivudine and abacavir or tenofovir disoproxil and emtricitabine or zidovudine, lamivudine and abacavir or zidovudine, lamivudine and nevirapine or emtricitabine, tenofovir disoproxil and efavirenz or stavudine, lamivudine and nevirapine or emtricitabine, tenofovir disoproxil and rilpivirine or emtricitabine, tenofovir disoproxil, elvitegravir and cobicistat lopinavir and ritonavir or lamivudine, tenofovir disoproxil and efavirenz or lamivudine and tenofovir disoproxil or lamivudine, abacavir and dolutegravir or darunavir and cobicistat or atazanavir and cobicistat or lamivudine and raltegravir or emtricitabine and tenofovir alafenamide or emtricitabine, tenofovir alafenamide, elvitegravir and cobicistat or emtricitabine or tenofovir alafenamide and rilpivirine or emtricitabine, tenofovir alafenamide and bictegravir or dolutegravir and rilpivirine or emtricitabine, tenofovir or alafenamide, darunavir and cobicistat or atazanavir and ritonavir or lamivudine, tenofovir disoproxil and doravirine or moroxydine or lysozyme or inosine pranobex or pleconaril or enfuvirtide or raltegravir or maraviroc or maribavir or elvitegravir or dolutegravir or umifenovir or enisamium iodide or letermovir or tilorone or pentanedioic acid imidazolyl ethanamide or ibalizumab.

4. A composition according to claims 1 or 2, **characterised in that** the preferable active substance with antiepileptic effect is valproic acid (sodium valproate) or vigabatrin.

5. A composition according to claims 1 or 3, **characterised in that** the preferable active substance with antiviral effect is inosine pranobex.

6. A composition according to any of the previous claims, **characterised in that** the weight ratio in [mg] of active substances selected from the NO3 ATC group to active substances selected from the J05 ATC group is as follows: 10.000.000 to 1:1; 1.000.000 to 1:1; 100.000 to 1:1; 10.000 to 1:1; 1.000 to 1:1; 100 to 1 :1; 10 to 1:1; 1 :1 to 10.000.000; 1:1 to 1.000.000; 1:1 to 100. 000; 1:1 to 10.000; 1 to 1: 1.000; 1:1 to 100; 1:1 to 10 preferably 1:2, most preferably 1:2.13.

7. A composition according to any of the previous claims, **characterised in that** the preferable combination of active substances in the composition is:
N03AA01 and J05AA01 or N03AA02 and J05AA01 or N03AA03 and J05AA01 or N03AA04 and J05AA01 or N03AA30 and J05AA01 or N03AB01 and J05AA01 or N03AB02 and J05AA01 or N03AB03 and J05AA01 or N03AB04 and J05AA01 or N03AB05 and J05AA01 or N03AB52 and J05AA01 or N03AB54 and J05AA01 or N03AC01 and J05AA01 or N03AC02 and J05AA01 or N03AC03 and J05AA01 or N03AD01 and J05AA01 or N03AD02 and J05AA01 or N03AD03 and J05AA01 or N03AD51 and J05AA01 or N03AE01 and J05AA01 or N03AF01 and J05AA01 or N03AF02 and J05AA01 or N03AF03 and J05AA01 or N03AF04 and J05AA01 or N03AG01 and J05AA01 or N03AG02 and J05AA01 or N03AG03 and J05AA01 or N03AG04 and J05AA01 or N03AG05 and J05AA01 or N03AG06 and J05AA01 or N03AX03 and J05AA01 or N03AX07 and J05AA01 or N03AX09 and J05AA01 or N03AX10 and J05AA01 or N03AX11 and J05AA01 or N03AX12 and J05AA01 or N03AX13 and J05AA01 or N03AX14 and J05AA01 or N03AX15 and J05AA01 or N03AX16 and J05AA01 or N03AX17 and J05AA01 or N03AX18 and J05AA01 or N03AX19 and J05AA01 or N03AX21 and J05AA01 or N03AX22 and J05AA01 or N03AX23 and J05AA01 or N03AX24 and J05AA01 or N03AX30 and J05AA01 or N03AA01 and J05AB01 or N03AA02 and J05AB01 or N03AA03 and J05AB01 or N03AA04 and J05AB01 or N03AA30 and J05AB01 or N03AB01 and J05AB01 or N03AB02 and J05AB01 or N03AB03 and J05AB01 or N03AB04 and J05AB01 or N03AB05 and J05AB01 or N03AB52 and J05AB01 or N03AB54 and J05AB01 or N03AC01 and J05AB01 or N03AC02 and J05AB01 or N03AC03 and J05AB01 or N03AD01 and J05AB01 or N03AD02 and J05AB01 or N03AD03 and J05AB01 or N03AD51 and J05AB01 or N03AE01 and J05AB01 or N03AF01 and J05AB01 or N03AF02 and J05AB01 or N03AF03 and J05AB01 or N03AF04 and J05AB01 or N03AG01 and J05AB01 or N03AG02 and J05AB01 or N03AG03 and J05AB01 or N03AG04 and J05AB01 or N03AG05 and J05AB01 or N03AG06 and J05AB01 or N03AX03 and J05AB01 or N03AX07 and J05AB01 or N03AX09 and J05AB01 or N03AX10 and J05AB01 or N03AX11 and J05AB01 or N03AX12 and J05AB01 or N03AX13 and J05AB01 or N03AX14 and J05AB01 or N03AX15 and J05AB01 or N03AX16 and J05AB01 or N03AX17 and J05AB01 or N03AX18 and J05AB01 or N03AX19 and J05AB01 or N03AX21 and J05AB01 or N03AX22 and J05AB01 or N03AX23 and J05AB01 or N03AX24 and J05AB01 or N03AX30 and J05AB01 or N03AA01 and J05AB02 or N03AA02 and J05AB02 or N03AA03 and J05AB02 or N03AA04 and J05AB02 or N03AA30 and J05AB02 or N03AB01 and J05AB02 or N03AB02 and J05AB02 or N03AB03 and J05AB02 or N03AB04 and J05AB02 or N03AB05 and J05AB02 or N03AB52 and J05AB02 or N03AB54 and J05AB02 or N03AC01 and J05AB02 or N03AC02 and J05AB02 or N03AC03 and J05AB02 or N03AD01 and J05AB02 or N03AD02 and J05AB02 or N03AD03 and J05AB02 or N03AD51 and J05AB02 or N03AE01 and J05AB02 or N03AF01 and J05AB02 or N03AF02 and J05AB02 or N03AF03 and J05AB02 or N03AF04 and J05AB02 or N03AG01 and J05AB02 or N03AG02 and J05AB02 or N03AG03 and J05AB02 or N03AG04 and J05AB02 or N03AG05 and J05AB02 or N03AG06 and J05AB02 or N03AX03 and J05AB02 or N03AX07 and J05AB02 or N03AX09 and J05AB02 or N03AX10 and J05AB02 or N03AX11 and J05AB02 or N03AX12 and J05AB02 or N03AX13 and J05AB02 or N03AX14 and J05AB02 or N03AX15 and J05AB02 or N03AX16 and J05AB02 or N03AX17 and J05AB02 or N03AX18 and J05AB02 or N03AX19 and J05AB02 or N03AX21 and J05AB02 or N03AX22 and J05AB02 or N03AX23 and J05AB02 or N03AX24 and J05AB02 or N03AX30 and J05AB02 or N03AA01 and J05AB03 or N03AA02 and J05AB03 or N03AA03 and J05AB03 or N03AA04 and J05AB03 or N03AA30 and J05AB03 or N03AB01 and J05AB03 or N03AB02 and J05AB03 or N03AB03 and J05AB03 or N03AB04 and J05AB03 or N03AB05 and J05AB03 or N03AB52 and J05AB03 or N03AB54 and J05AB03 or N03AC01 and J05AB03 or N03AC02 and J05AB03 or N03AC03 and J05AB03 or N03AD01 and J05AB03 or N03AD02 and J05AB03 or N03AD03 and J05AB03 or N03AD51 and J05AB03 or N03AE01 and J05AB03 or N03AF01 and J05AB03 or N03AF02 and J05AB03 or N03AF03 and J05AB03 or N03AF04 and J05AB03 or N03AG01 and J05AB03 or N03AG02 and J05AB03 or N03AG03 and J05AB03 or N03AG04 and J05AB03 or N03AG05 and J05AB03 or N03AG06 and J05AB03 or N03AX03 and J05AB03 or N03AX07 and J05AB03 or N03AX09 and J05AB03 or N03AX10 and J05AB03 or N03AX11 and J05AB03 or N03AX12 and J05AB03 or N03AX13 and J05AB03 or N03AX14 and J05AB03 or N03AX15 and J05AB03 or N03AX16 and J05AB03 or N03AX17 and J05AB03 or N03AX18 and J05AB03 or N03AX19 and J05AB03 or N03AX21 and J05AB03 or N03AX22 and J05AB03 or N03AX23 and J05AB03 or N03AX24 and J05AB03 or N03AX30 and J05AB03 or N03AA01 and J05AB06 or N03AA02 and J05AB06 or N03AA03 and J05AB06 or N03AA04 and J05AB06 or N03AA30 and J05AB06 or N03AB01 and J05AB06 or N03AB02 and J05AB06 or N03AB03 and J05AB06 or N03AB04 and J05AB06 or N03AB05 and J05AB06 or N03AB52 and J05AB06 or N03AB54 and J05AB06 or N03AC01 and J05AB06 or N03AC02 and J05AB06 or N03AC03 and J05AB06 or N03AD01 and J05AB06 or N03AD02 and J05AB06 or N03AD03 and J05AB06 or N03AD51 and J05AB06 or N03AE01 and J05AB06 or N03AF01 and J05AB06 or N03AF02 and J05AB06 or N03AF03 and J05AB06 or N03AF04 and J05AB06 or N03AG01 and J05AB06 or N03AG02 and J05AB06 or N03AG03 and J05AB06 or N03AG04 and J05AB06 or N03AG05 and J05AB06 or N03AG06 and J05AB06 or N03AX03 and J05AB06 or N03AX07 and J05AB06 or N03AX09 and J05AB06 or N03AX10 and J05AB06 or N03AX11 and J05AB06 or N03AX12 and J05AB06 or N03AX13 and J05AB06 or N03AX14 and J05AB06 or N03AX15 and J05AB06 or N03AX16 and J05AB06 or N03AX17 and J05AB06 or N03AX18 and J05AB06 or N03AX19 and J05AB06 or N03AX21 and J05AB06 or N03AX22 and J05AB06 or N03AX23 and J05AB06 or N03AX24 and J05AB06 or N03AX30 and J05AB06 or N03AA01 and J05AB09 or N03AA02 and J05AB09 or N03AA03 and J05AB09 or N03AA04 and J05AB09 or N03AA30 and J05AB09 or N03AB01 and J05AB09 or N03AB02 and J05AB09 or N03AB03 and J05AB09 or N03AB04 and J05AB09 or N03AB05 and J05AB09 or N03AB52 and J05AB09 or N03AB54 and J05AB09 or N03AC01 and J05AB09 or N03AC02 and J05AB09 or N03AC03 and J05AB09 or N03AD01 and J05AB09 or N03AD02 and J05AB09 or N03AD03 and J05AB09 or N03AD51 and J05AB09 or N03AE01 and J05AB09 or N03AF01 and J05AB09 or N03AF02 and J05AB09 or N03AF03 and J05AB09 or N03AF04 and J05AB09 or N03AG01 and J05AB09 or N03AG02 and J05AB09 or N03AG03 and J05AB09 or N03AG04 and J05AB09 or N03AG05 and J05AB09 or N03AG06 and J05AB09 or N03AX03 and J05AB09 or N03AX07 and J05AB09 or N03AX09 and J05AB09 or N03AX10 and J05AB09 or N03AX11 and J05AB09 or N03AX12 and J05AB09 or N03AX13 and J05AB09 or N03AX14 and J05AB09 or N03AX15 and J05AB09 or N03AX16 and J05AB09 or N03AX17 and J05AB09 or N03AX18 and J05AB09 or N03AX19 and J05AB09 or N03AX21 and J05AB09 or N03AX22 and J05AB09 or N03AX23 and J05AB09 or N03AX24 and J05AB09 or N03AX30 and J05AB09 or N03AA01 and J05AB11 or N03AA02 and J05AB11 or N03AA03 and J05AB11 or N03AA04 and J05AB11 or N03AA30 and J05AB11 or N03AB01 and J05AB11 or N03AB02 and J05AB11 or N03AB03 and J05AB11 or N03AB04 and J05AB11 or N03AB05 and J05AB11 or N03AB52 and J05AB11 or N03AB54 and J05AB11or N03AC01 and J05AB11 or N03AC02 and J05AB11 or N03AC03 and J05AB11 or N03AD01 and J05AB11 or N03AD02 and J05AB11 or N03AD03 and J05AB11 or N03AD51 and J05AB11 or N03AE01 and J05AB11 or N03AF01 and J05AB11 or N03AF02 and J05AB11 or N03AF03 and J05AB11 or N03AF04 and J05AB11 or N03AG01 and J05AB11 or N03AG02 and J05AB11 or N03AG03 and J05AB11 or N03AG04 and J05AB11 or N03AG05 and J05AB11 or N03AG06 and J05AB11 or N03AX03 and J0SAB11 or N03AX07 and J05AB11 or N03AX09 and J05AB11 or N03AX10 and J0SAB11 or N03AX11 and J0SAB11 or N03AX12 and J0SAB11 or N03AX13 and J05AB11 or N03AX14 and J05AB11 or N03AX15 and J05AB11 or N03AX16 and J0SAB11 or N03AX17 and J0SAB11 or N03AX18 and J0SAB11 or N03AX19 and J0SAB11 or N03AX21 and J0SAB11 or N03AX22 and J0SAB11 or N03AX23 and J05AB11 or N03AX24 and J05AB11 or N03AX30 and J05AB11 or N03AA01 and J05AB12 or N03AA02 and J05AB12 or N03AA03 and J05AB12 or N03AA04 and J05AB12 or N03AA30 and J05AB12 or N03AB01 and J05AB12 or N03AB02 and J05AB12 or N03AB03 and J05AB12 or N03AB04 and J05AB12 or N03AB05 and J05AB12 or N03AB52 and J05AB12 or N03AB54 and J05AB12 or N03AC01 and J05AB12 or N03AC02 and J05AB12 or N03AC03 and J05AB12 or N03AD01 and J05AB12 or N03AD02 and J05AB12 or N03AD03 and J05AB12 or N03AD51 and J05AB12 or N03AE01 and J05AB12 or N03AF01 and J05AB12 or N03AF02 and J05AB12 or N03AF03 and J05AB12 or N03AF04 and J05AB12 or N03AG01 and J05AB12 or N03AG02 and J05AB12 or N03AG03 and J05AB12 or N03AG04 and J05AB12 or N03AG05 and J05AB12 or N03AG06 and J05AB12 or N03AX03 and J05AB12 or N03AX07 and J05AB12 or N03AX09 and J05AB12 or N03AX10 and J05AB12 or N03AX11 and J05AB12 or N03AX12 and J05AB12 or N03AX13 and J05AB12 or N03AX14 and J05AB12 or N03AX15 and J05AB12 or N03AX16 and J05AB12 or N03AX17 and J05AB12 or N03AX18 and J05AB12 or N03AX19 and J05AB12 or N03AX21 and J05AB12 or N03AX22 and J05AB12 or N03AX23 and J05AB12 or N03AX24 and J05AB12 or N03AX30 and J05AB12 or N03AA01 and J05AB13 or N03AA02 and J05AB13 or N03AA03 and J05AB13 or N03AA04 and J05AB13 or N03AA30 and J05AB13 or N03AB01 and J05AB13 or N03AB02 and J05AB13 or N03AB03 and J05AB13 or N03AB04 and J05AB13 or N03AB05 and J05AB13 or N03AB52 and J05AB13 or N03AB54 and J05AB13 or N03AC01 and J05AB13 or N03AC02 and J05AB13 or N03AC03 and J05AB13 or N03AD01 and J05AB13 or N03AD02 and J05AB13 or N03AD03 and J05AB13 or N03AD51 and J05AB13 or N03AE01 and J05AB13 or N03AF01 and J05AB13 or N03AF02 and J05AB13 or N03AF03 and J05AB13 or N03AF04 and J05AB13 or N03AG01 and J05AB13 or N03AG02 and J05AB13 or N03AG03 and J05AB13 or N03AG04 and J05AB13 or N03AG05 and J05AB13 or N03AG06 and J05AB13 or N03AX03 and J05AB13 or N03AX07 and J05AB13 or N03AX09 and J05AB13 or N03AX10 and J05AB13 or N03AX11 and J05AB13 or N03AX12 and J05AB13 or N03AX13 and J05AB13 or N03AX14 and J05AB13 or N03AX15 and J05AB13 or N03AX16 and J05AB13 or N03AX17 and J05AB13 or N03AX18 and J05AB13 or N03AX19 and J05AB13 or N03AX21 and J05AB13 or N03AX22 and J05AB13 or N03AX23 and J05AB13 or N03AX24 and J05AB13 or N03AX30 and J05AB13 or N03AA01 and J05AB14 or N03AA02 and J05AB14 or N03AA03 and J05AB14 or N03AA04 and J05AB14 or N03AA30 and J05AB14 or N03AB01 and J05AB14 or N03AB02 and J05AB14 or N03AB03 and J05AB14 or N03AB04 and J05AB14 or N03AB05 and J05AB14 or N03AB52 and J05AB14 or N03AB54 and J05AB14 or N03AC01 and J05AB14 or N03AC02 and J05AB14 or N03AC03 and J05AB14 or N03AD01 and J05AB14 or N03AD02 and J05AB14 or N03AD03 and J05AB14 or N03AD51 and J05AB14 or N03AE01 and J05AB14 or N03AF01 and J05AB14 or N03AF02 and J05AB14 or N03AF03 and J05AB14 or N03AF04 and J05AB14 or N03AG01 and J05AB14 or N03AG02 and J05AB14 or N03AG03 and J05AB14 or N03AG04 and J05AB14 or N03AG05 and J05AB14 or N03AG06 and J05AB14 or N03AX03 and J05AB14 or N03AX07 and J05AB14 or N03AX09 and J05AB14 or N03AX10 and J05AB14 or N03AX11 and J05AB14 or N03AX12 and J05AB14 or N03AX13 and J05AB14 or N03AX14 and J05AB14 or N03AX15 and J05AB14 or N03AX16 and J05AB14 or N03AX17 and J05AB14 or N03AX18 and J05AB14 or N03AX19 and J05AB14 or N03AX21 and J05AB14 or N03AX22 and J05AB14 or N03AX23 and J05AB14 or N03AX24 and J05AB14 or N03AX30 and J05AB14 or N03AA01 and J05AB15 or N03AA02 and J05AB15 or N03AA03 and J05AB15 or N03AA04 and J05AB15 or N03AA30 and J05AB15 or N03AB01 and J05AB15 or N03AB02 and J05AB15 or N03AB03 and J05AB15 or N03AB04 and J05AB15 or N03AB05 and J05AB15 or N03AB52 and J05AB15 or N03AB54 and J05AB15 or N03AC01 and J05AB15 or N03AC02 and J05AB15 or N03AC03 and J05AB15 or N03AD01 and J05AB15 or N03AD02 and J05AB15 or N03AD03 and J05AB15 or N03AD51 and J05AB15 or N03AE01 and J05AB15 or N03AF01 and J05AB15 or N03AF02 and J05AB15 or N03AF03 and J05AB15 or N03AF04 and J05AB15 or N03AG01 and J05AB15 or N03AG02 and J05AB15 or N03AG03 and J05AB15 or N03AG04 and J05AB15 or N03AG05 and J05AB15 or N03AG06 and J05AB15 or N03AX03 and J05AB15 or N03AX07 and J05AB15 or N03AX09 and J05AB15 or N03AX10 and J05AB15 or N03AX11 and J05AB15 or N03AX12 and J05AB15 or N03AX13 and J05AB15 or N03AX14 and J05AB15 or N03AX15 and J05AB15 or N03AX16 and J05AB15 or N03AX17 and J05AB15 or N03AX18 and J05AB15 or N03AX19 and J05AB15 or N03AX21 and J05AB15 or N03AX22 and J05AB15 or N03AX23 and J05AB15 or N03AX24 and J05AB15 or N03AX30 and J05AB15 or N03AA01 and J05AC02 or N03AA02 and J05AC02 or N03AA03 and J05AC02 or N03AA04 and J05AC02 or N03AA30 and J05AC02 or N03AB01 and J05AC02 or N03AB02 and J05AC02 or N03AB03 and J05AC02 or N03AB04 and J05AC02 or N03AB05 and J05AC02 or N03AB52 and J05AC02 or N03AB54 and J05AC02 or N03AC01 and J05AC02 or N03AC02 and J05AC02 or N03AC03 and J05AC02 or N03AD01 and J05AC02 or N03AD02 and J05AC02 or N03AD03 and J05AC02 or N03AD51 and J05AC02 or N03AE01 and J05AC02 or N03AF01 and J05AC02 or N03AF02 and J05AC02 or N03AF03 and J05AC02 or N03AF04 and J05AC02 or N03AG01 and J05AC02 or N03AG02 and J05AC02 or N03AG03 and J05AC02 or N03AG04 and J05AC02 or N03AG05 and J05AC02 or N03AG06 and J05AC02 or N03AX03 and J05AC02 or N03AX07 and J05AC02 or N03AX09 and J05AC02 or N03AX10 and J05AC02 or N03AX11 and J05AC02 or N03AX12 and J05AC02 or N03AX13 and J05AC02 or N03AX14 and J05AC02 or N03AX15 and J05AC02 or N03AX16 and J05AC02 or N03AX17 and J05AC02 or N03AX18 and J05AC02 or N03AX19 and J05AC02 or N03AX21 and J05AC02 or N03AX22 and J05AC02 or N03AX23 and J05AC02 or N03AX24 and J05AC02 or N03AX30 and J05AC02 or N03AA01 and J05AC03 or N03AA02 and J05AC03 or N03AA03 and J05AC03 or N03AA04 and J05AC03 or N03AA30 and J05AC03 or N03AB01 and J05AC03 or N03AB02 and J05AC03 or N03AB03 and J05AC03 or N03AB04 and J05AC03 or N03AB05 and J05AC03 or N03AB52 and J05AC03 or N03AB54 and J05AC03 or N03AC01 and J05AC03 or N03AC02 and J05AC03 or N03AC03 and J05AC03 or N03AD01 and J05AC03 or N03AD02 and J05AC03 or N03AD03 and J05AC03 or N03AD51 and J05AC03 or N03AE01 and J05AC03 or N03AF01 and J05AC03 or N03AF02 and J05AC03 or N03AF03 and J05AC03 or N03AF04 and J05AC03 or N03AG01 and J05AC03 or N03AG02 and J05AC03 or N03AG03 and J05AC03 or N03AG04 and J05AC03 or N03AG05 and J05AC03 or N03AG06 and J05AC03 or N03AX03 and J05AC03 or N03AX07 and J05AC03 or N03AX09 and J05AC03 or N03AX10 and J05AC03 or N03AX11 and J05AC03 or N03AX12 and J05AC03 or N03AX13 and J05AC03 or N03AX14 and J05AC03 or N03AX15 and J05AC03 or N03AX16 and J05AC03 or N03AX17 and J05AC03 or N03AX18 and J05AC03 or N03AX19 and J05AC03 or N03AX21 and J05AC03 or N03AX22 and J05AC03 or N03AX23 and J05AC03 or N03AX24 and J05AC03 or N03AX30 and J05AC03 or N03AA01 and J05AD01 or N03AA02 and J05AD01 or N03AA03 and J05AD01 or N03AA04 and J05AD01 or N03AA30 and J05AD01 or N03AB01 and J05AD01 or N03AB02 and J05AD01 or N03AB03 and J05AD01 or N03AB04 and J05AD01 or N03AB05 and J05AD01 or N03AB52 and J05AD01 or N03AB54 and J05AD01 or N03AC01 and J05AD01 or N03AC02 and J05AD01 or N03AC03 and J05AD01 or N03AD01 and J05AD01 or N03AD02 and J05AD01 or N03AD03 and J05AD01 or N03AD51 and J05AD01 or N03AE01 and J05AD01 or N03AF01 and J05AD01 or N03AF02 and J05AD01 or N03AF03 and J05AD01 or N03AF04 and J05AD01 or N03AG01 and J05AD01 or N03AG02 and J05AD01 or N03AG03 and J05AD01 or N03AG04 and J05AD01 or N03AG05 and J05AD01 or N03AG06 and J05AD01 or N03AX03 and J05AD01 or N03AX07 and J05AD01 or N03AX09 and J05AD01 or N03AX10 and J05AD01 or N03AX11 and J05AD01 or N03AX12 and J05AD01 or N03AX13 and J05AD01 or N03AX14 and J05AD01 or N03AX15 and J05AD01 or N03AX16 and J05AD01 or N03AX17 and J05AD01 or N03AX18 and J05AD01 or N03AX19 and J05AD01 or N03AX21 and J05AD01 or N03AX22 and J05AD01 or N03AX23 and J05AD01 or N03AX24 and J05AD01 or N03AX30 and J05AD01 or N03AA01 and J05AD02 or N03AA02 and J05AD02 or N03AA03 and J05AD02 or N03AA04 and J05AD02 or N03AA30 and J05AD02 or N03AB01 and J05AD02 or N03AB02 and J05AD02 or N03AB03 and J05AD02 or N03AB04 and J05AD02 or N03AB05 and J05AD02 or N03AB52 and J05AD02 or N03AB54 and J05AD02 or N03AC01 and J05AD02 or N03AC02 and J05AD02 or N03AC03 and J05AD02 or N03AD01 and J05AD02 or N03AD02 and J05AD02 or N03AD03 and J05AD02 or N03AD51 and J05AD02 or N03AE01 and J05AD02 or N03AF01 and J05AD02 or N03AF02 and J05AD02 or N03AF03 and J05AD02 or N03AF04 and J05AD02 or N03AG01 and J05AD02 or N03AG02 and J05AD02 or N03AG03 and J05AD02 or N03AG04 and J05AD02 or N03AG05 and J05AD02 or N03AG06 and J05AD02 or N03AX03 and J05AD02 or N03AX07 and J05AD02 or N03AX09 and J05AD02 or N03AX10 and J05AD02 or N03AX11 and J05AD02 or N03AX12 and J05AD02 or N03AX13 and J05AD02 or N03AX14 and J05AD02 or N03AX15 and J05AD02 or N03AX16 and J05AD02 or N03AX17 and J05AD02 or N03AX18 and J05AD02 or N03AX19 and J05AD02 or N03AX21 and J05AD02 or N03AX22 and J05AD02 or N03AX23 and J05AD02 or N03AX24 and J05AD02 or N03AX30 and J05AD02 or N03AA01 and J05AE01 or N03AA02 and J05AE01 or N03AA03 and J05AE01 or N03AA04 and J05AE01 or N03AA30 and J05AE01 or N03AB01 and J05AE01 or N03AB02 and J05AE01 or N03AB03 and J05AE01 or N03AB04 and J05AE01 or N03AB05 and J05AE01 or N03AB52 and J05AE01 or N03AB54 and J05AE01 or N03AC01 and J05AE01 or N03AC02 and J05AE01 or N03AC03 and J05AE01 or N03AD01 and J05AE01 or N03AD02 and J05AE01 or N03AD03 and J05AE01 or N03AD51 and J05AE01 or N03AE01 and J05AE01 or N03AF01 and J05AE01 or N03AF02 and J05AE01 or N03AF03 and J05AE01 or N03AF04 and J05AE01 or N03AG01 and J05AE01 or N03AG02 and J0SAE01 or N03AG03 and J05AE01 or N03AG04 and J05AE01 or N03AG05 and J05AE01 or N03AG06 and J05AE01 or N03AX03 and J05AE01 or N03AX07 and J05AE01 or N03AX09 and J05AE01 or N03AX10 and J05AE01 or N03AX11 and J05AE01 or N03AX12 and J05AE01 or N03AX13 and J05AE01 or N03AX14 and J05AE01 or N03AX15 and J05AE01 or N03AX16 and J05AE01 or N03AX17 and J05AE01 or N03AX18 and J05AE01 or N03AX19 and J05AE01 or N03AX21 and J05AE01 or N03AX22 and J05AE01 or N03AX23 and J05AE01 or N03AX24 and J05AE01 or N03AX30 and J05AE01 or N03AA01 and J05AE02 or N03AA02 and J05AE02 or N03AA03 and J05AE02 or N03AA04 and J05AE02 or N03AA30 and J05AE02 or N03AB01 and J05AE02 or N03AB02 and J05AE02 or N03AB03 and J05AE02 or N03AB04 and J05AE02 or N03AB05 and J05AE02 or N03AB52 and J05AE02 or N03AB54 and J05AE02 or N03AC01 and J05AE02 or N03AC02 and J05AE02 or N03AC03 and J05AE02 or N03AD01 and J05AE02 or N03AD02 and J05AE02 or N03AD03 and J05AE02 or N03AD51 and J05AE02 or N03AE01 and J05AE02 or N03AF01 and J05AE02 or N03AF02 and J05AE02 or N03AF03 and J05AE02 or N03AF04 and J05AE02 or N03AG01 and J05AE02 or N03AG02 and J05AE02 or N03AG03 and J05AE02 or N03AG04 and J05AE02 or N03AG05 and J05AE02 or N03AG06 and J05AE02 or N03AX03 and J05AE02 or N03AX07 and J05AE02 or N03AX09 and J05AE02 or N03AX10 and J05AE02 or N03AX11 and J05AE02 or N03AX12 and J05AE02 or N03AX13 and J05AE02 or N03AX14 and J05AE02 or N03AX15 and J05AE02 or N03AX16 and J05AE02 or N03AX17 and J05AE02 or N03AX18 and J05AE02 or N03AX19 and J05AE02 or N03AX21 and J05AE02 or N03AX22 and J05AE02 or N03AX23 and J05AE02 or N03AX24 and J05AE02 or N03AX30 and J05AE02 or N03AA01 and J05AE03 or N03AA02 and J05AE03 or N03AA03 and J05AE03 or N03AA04 and J05AE03 or N03AA30 and J05AE03 or N03AB01 and J05AE03 or N03AB02 and J05AE03 or N03AB03 and J05AE03 or N03AB04 and J05AE03 or N03AB05 and J05AE03 or N03AB52 and J05AE03 or N03AB54 and J05AE03 or N03AC01 and J05AE03 or N03AC02 and J05AE03 or N03AC03 and J05AE03 or N03AD01 and J05AE03 or N03AD02 and J05AE03 or N03AD03 and J05AE03 or N03AD51 and J05AE03 or N03AE01 and J05AE03 or N03AF01 and J05AE03 or N03AF02 and J05AE03 or N03AF03 and J05AE03 or N03AF04 and J05AE03 or N03AG01 and J05AE03 or N03AG02 and J05AE03 or N03AG03 and J05AE03 or N03AG04 and J05AE03 or N03AG05 and J05AE03 or N03AG06 and J05AE03 or N03AX03 and J05AE03 or N03AX07 and J05AE03 or N03AX09 and J05AE03 or N03AX10 and J05AE03 or N03AX11 and J05AE03 or N03AX12 and J05AE03 or N03AX13 and J05AE03 or N03AX14 and J05AE03 or N03AX15 and J05AE03 or N03AX16 and J05AE03 or N03AX17 and J05AE03 or N03AX18 and J05AE03 or N03AX19 and J05AE03 or N03AX21 and J05AE03 or N03AX22 and J05AE03 or N03AX23 and J05AE03 or N03AX24 and J05AE03 or N03AX30 and J05AE03 or N03AA01 and J05AE04 or N03AA02 and J05AE04 or N03AA03 and J05AE04 or N03AA04 and J05AE04 or N03AA30 and J05AE04 or N03AB01 and J05AE04 or N03AB02 and J05AE04 or N03AB03 and J05AE04 or N03AB04 and J05AE04 or N03AB05 and J05AE04 or N03AB52 and J05AE04 or N03AB54 and J05AE04 or N03AC01 and J05AE04 or N03AC02 and J05AE04 or N03AC03 and J05AE04 or N03AD01 and J05AE04 or N03AD02 and J05AE04 or N03AD03 and J05AE04 or N03AD51 and J05AE04 or N03AE01 and J05AE04 or N03AF01 and J05AE04 or N03AF02 and J05AE04 or N03AF03 and J05AE04 or N03AF04 and J05AE04 or N03AG01 and J05AE04 or N03AG02 and J05AE04 or N03AG03 and J05AE04 or N03AG04 and J05AE04 or N03AG05 and J05AE04 or N03AG06 and J05AE04 or N03AX03 and J05AE04 or N03AX07 and J05AE04 or N03AX09 and J05AE04 or N03AX10 and J05AE04 or N03AX11 and J05AE04 or N03AX12 and J05AE04 or N03AX13 and J05AE04 or N03AX14 and J05AE04 or N03AX15 and J05AE04 or N03AX16 and J05AE04 or N03AX17 and J05AE04 or N03AX18 and J05AE04 or N03AX19 and J05AE04 or N03AX21 and J05AE04 or N03AX22 and J05AE04 or N03AX23 and J05AE04 or N03AX24 and J05AE04 or N03AX30 and J05AE04 or N03AA01 and J05AE05 or N03AA02 and J05AE05 or N03AA03 and J05AE05 or N03AA04 and J05AE05 or N03AA30 and J05AE05 or N03AB01 and J05AE05 or N03AB02 and J05AE05 or N03AB03 and J05AE05 or N03AB04 and J05AE05 or N03AB05 and J05AE05 or N03AB52 and J05AE05 or N03AB54 and J05AE05 or N03AC01 and J05AE05 or N03AC02 and J05AE05 or N03AC03 and J05AE05 or N03AD01 and J05AE05 or N03AD02 and J05AE05 or N03AD03 and J05AE05 or N03AD51 and J05AE05 or N03AE01 and J05AE05 or N03AF01 and J05AE05 or N03AF02 and J05AE05 or N03AF03 and J05AE05 or N03AF04 and J05AE05 or N03AG01 and J05AE05 or N03AG02 and J05AE05 or N03AG03 and J05AE05 or N03AG04 and J05AE05 or N03AG05 and J05AE05 or N03AG06 and J05AE05 or N03AX03 and J05AE05 or N03AX07 and J05AE05 or N03AX09 and J05AE05 or N03AX10 and J05AE05 or N03AX11 and J05AE05 or N03AX12 and J05AE05 or N03AX13 and J05AE05 or N03AX14 and J05AE05 or N03AX15 and J05AE05 or N03AX16 and J05AE05 or N03AX17 and J05AE05 or N03AX18 and J05AE05 or N03AX19 and J05AE05 or N03AX21 and J05AE05 or N03AX22 and J05AE05 or N03AX23 and J05AE05 or N03AX24 and J05AE05 or N03AX30 and J05AE05 or N03AA01 and J05AE07 or N03AA02 and J05AE07 or N03AA03 and J05AE07 or N03AA04 and J05AE07 or N03AA30 and J05AE07 or N03AB01 and J05AE07 or N03AB02 and J05AE07 or N03AB03 and J05AE07 or N03AB04 and J05AE07 or N03AB05 and J05AE07 or N03AB52 and J05AE07 or N03AB54 and J05AE07 or N03AC01 and J05AE07 or N03AC02 and J05AE07 or N03AC03 and J05AE07 or N03AD01 and J05AE07 or N03AD02 and J05AE07 or N03AD03 and J05AE07 or N03AD51 and J05AE07 or N03AE01 and J05AE07 or N03AF01 and J05AE07 or N03AF02 and J05AE07 or N03AF03 and J05AE07 or N03AF04 and J05AE07 or N03AG01 and J05AE07 or N03AG02 and J05AE07 or N03AG03 and J05AE07 or N03AG04 and J05AE07 or N03AG05 and J05AE07 or N03AG06 and J05AE07 or N03AX03 and J05AE07 or N03AX07 and J05AE07 or N03AX09 and J05AE07 or N03AX10 and J05AE07 or N03AX11 and J05AE07 or N03AX12 and J05AE07 or N03AX13 and J05AE07 or N03AX14 and J05AE07 or N03AX15 and J05AE07 or N03AX16 and J05AE07 or N03AX17 and J05AE07 or N03AX18 and J05AE07 or N03AX19 and J05AE07 or N03AX21 and J05AE07 or N03AX22 and J05AE07 or N03AX23 and J05AE07 or N03AX24 and J05AE07 or N03AX30 and J05AE07 or N03AA01 and J05AE08 or N03AA02 and J05AE08 or N03AA03 and J05AE08 or N03AA04 and J05AE08 or N03AA30 and J05AE08 or N03AB01 and J05AE08 or N03AB02 and J05AE08 or N03AB03 and J05AE08 or N03AB04 and J05AE08 or N03AB05 and J05AE08 or N03AB52 and J05AE08 or N03AB54 and J05AE08 or N03AC01 and J05AE08 or N03AC02 and J05AE08 or N03AC03 and J05AE08 or N03AD01 and J05AE08 or N03AD02 and J05AE08 or N03AD03 and J05AE08 or N03AD51 and J05AE08 or N03AE01 and J05AE08 or N03AF01 and J05AE08 or N03AF02 and J05AE08 or N03AF03 and J05AE08 or N03AF04 and J05AE08 or N03AG01 and J05AE08 or N03AG02 and J05AE08 or N03AG03 and J05AE08 or N03AG04 and J05AE08 or N03AG05 and J05AE08 or N03AG06 and J05AE08 or N03AX03 and J05AE08 or N03AX07 and J05AE08 or N03AX09 and J05AE08 or N03AX10 and J05AE08 or N03AX11 and J05AE08 or N03AX12 and J05AE08 or N03AX13 and J05AE08 or N03AX14 and J05AE08 or N03AX15 and J05AE08 or N03AX16 and J05AE08 or N03AX17 and J05AE08 or N03AX18 and J05AE08 or N03AX19 and J05AE08 or N03AX21 and J05AE08 or N03AX22 and J05AE08 or N03AX23 and J05AE08 or N03AX24 and J05AE08 or N03AX30 and J05AE08 or N03AA01 and J05AE09 or N03AA02 and J05AE09 or N03AA03 and J05AE09 or N03AA04 and J05AE09 or N03AA30 and J05AE09 or N03AB01 and J05AE09 or N03AB02 and J05AE09 or N03AB03 and J05AE09 or N03AB04 and J05AE09 or N03AB05 and J05AE09 or N03AB52 and J05AE09 or N03AB54 and J05AE09 or N03AC01 and J05AE09 or N03AC02 and J05AE09 or N03AC03 and J05AE09 or N03AD01 and J05AE09 or N03AD02 and J05AE09 or N03AD03 and J05AE09 or N03AD51 and J05AE09 or N03AE01 and J05AE09 or N03AF01 and J05AE09 or N03AF02 and J05AE09 or N03AF03 and J05AE09 or N03AF04 and J05AE09 or N03AG01 and J05AE09 or N03AG02 and J05AE09 or N03AG03 and J05AE09 or N03AG04 and J05AE09 or N03AG05 and J05AE09 or N03AG06 and J05AE09 or N03AX03 and J05AE09 or N03AX07 and J05AE09 or N03AX09 and J05AE09 or N03AX10 and J05AE09 or N03AX11 and J05AE09 or N03AX12 and J05AE09 or N03AX13 and J05AE09 or N03AX14 and J05AE09 or N03AX15 and J05AE09 or N03AX16 and J05AE09 or N03AX17 and J05AE09 or N03AX18 and J05AE09 or N03AX19 and J05AE09 or N03AX21 and J05AE09 or N03AX22 and J05AE09 or N03AX23 and J05AE09 or N03AX24 and J05AE09 or N03AX30 and J05AE09 or N03AA01 and J05AE10 or N03AA02 and J05AE10 or N03AA03 and J05AE10 or N03AA04 and J05AE10 or N03AA30 and J05AE10 or N03AB01 and J05AE10 or N03AB02 and J05AE10 or N03AB03 and J05AE10 or N03AB04 and J05AE10 or N03AB05 and J05AE10 or N03AB52 and J05AE10 or N03AB54 and J05AE10 or N03AC01 and J05AE10 or N03AC02 and J05AE10 or N03AC03 and J05AE10 or N03AD01 and J05AE10 or N03AD02 and J05AE10 or N03AD03 and J05AE10 or N03AD51 and J05AE10 or N03AE01 and J05AE10 or N03AF01 and J05AE10 or N03AF02 and J05AE10 or N03AF03 and J05AE10 or N03AF04 and J05AE10 or N03AG01 and J05AE10 or N03AG02 and J05AE10 or N03AG03 and J05AE10 or N03AG04 and J05AE10 or N03AG05 and J05AE10 or N03AG06 and J05AE10 or N03AX03 and J05AE10 or N03AX07 and J05AE10 or N03AX09 and J05AE10 or N03AX10 and J05AE10 or N03AX11 and J05AE10 or N03AX12 and J05AE10 or N03AX13 and J05AE10 or N03AX14 and J05AE10 or N03AX15 and J05AE10 or N03AX16 and J05AE10 or N03AX17 and J05AE10 or N03AX18 and J05AE10 or N03AX19 and J05AE10 or N03AX21 and J05AE10 or N03AX22 and J05AE10 or N03AX23 and J05AE10 or N03AX24 and J05AE10 or N03AX30 and J05AE10 or N03AA01 and J05AF01 or N03AA02 and J05AF01 or N03AA03 and J05AF01 or N03AA04 and J05AF01 or N03AA30 and J05AF01 or N03AB01 and J05AF01 or N03AB02 and J05AF01 or N03AB03 and J05AF01 or N03AB04 and J05AF01 or N03AB05 and J05AF01 or N03AB52 and J05AF01 or N03AB54 and J05AF01 or N03AC01 and J05AF01 or N03AC02 and J05AF01 or N03AC03 and J05AF01 or N03AD01 and J05AF01 or N03AD02 and J05AF01 or N03AD03 and J05AF01 or N03AD51 and J05AF01 or N03AE01 and J05AF01 or N03AF01 and J05AF01 or N03AF02 and J05AF01 or N03AF03 and J05AF01 or N03AF04 and J05AF01 or N03AG01 and J05AF01 or N03AG02 and J05AF01 or N03AG03 and J05AF01 or N03AG04 and J05AF01 or N03AG05 and J05AF01 or N03AG06 and J05AF01 or N03AX03 and J05AF01 or N03AX07 and J05AF01 or N03AX09 and J05AF01 or N03AX10 and J05AF01 or N03AX11 and J05AF01 or N03AX12 and J05AF01 or N03AX13 and J05AF01 or N03AX14 and J05AF01 or N03AX15 and J05AF01 or N03AX16 and J05AF01 or N03AX17 and J05AF01 or N03AX18 and J05AF01 or N03AX19 and J05AF01 or N03AX21 and J05AF01 or N03AX22 and J05AF01 or N03AX23 and J05AF01 or N03AX24 and J05AF01 or N03AX30 and J05AF01 or N03AA01 and J05AF02 or N03AA02 and J05AF02 or N03AA03 and J05AF02 or N03AA04 and J05AF02 or N03AA30 and J05AF02 or N03AB01 and J05AF02 or N03AB02 and J05AF02 or N03AB03 and J05AF02 or N03AB04 and J05AF02 or N03AB05 and J05AF02 or N03AB52 and J05AF02 or N03AB54 and J05AF02 or N03AC01 and J05AF02 or N03AC02 and J05AF02 or N03AC03 and J05AF02 or N03AD01 and J05AF02 or N03AD02 and J05AF02 or N03AD03 and J05AF02 or N03AD51 and J05AF02 or N03AE01 and J05AF02 or N03AF01 and J05AF02 or N03AF02 and J05AF02 or N03AF03 and J05AF02 or N03AF04 and J05AF02 or N03AG01 and J05AF02 or N03AG02 and J05AF02 or N03AG03 and J05AF02 or N03AG04 and J05AF02 or N03AG05 and J05AF02 or N03AG06 and J05AF02 or N03AX03 and J05AF02 or N03AX07 and J05AF02 or N03AX09 and J05AF02 or N03AX10 and J05AF02 or N03AX11 and J05AF02 or N03AX12 and J05AF02 or N03AX13 and J05AF02 or N03AX14 and J05AF02 or N03AX15 and J05AF02 or N03AX16 and J05AF02 or N03AX17 and J05AF02 or N03AX18 and J05AF02 or N03AX19 and J05AF02 or N03AX21 and J05AF02 or N03AX22 and J05AF02 or N03AX23 and J05AF02 or N03AX24 and J05AF02 or N03AX30 and J05AF02 or N03AA01 and J05AF03 or N03AA02 and J05AF03 or N03AA03 and J05AF03 or N03AA04 and J05AF03 or N03AA30 and J05AF03 or N03AB01 and J05AF03 or N03AB02 and J05AF03 or N03AB03 and J05AF03 or N03AB04 and J05AF03 or N03AB05 and J05AF03 or N03AB52 and J05AF03 or N03AB54 and J05AF03 or N03AC01 and J05AF03 or N03AC02 and J05AF03 or N03AC03 and J05AF03 or N03AD01 and J05AF03 or N03AD02 and J05AF03 or N03AD03 and J05AF03 or N03AD51 and J05AF03 or N03AE01 and J05AF03 or N03AF01 and J05AF03 or N03AF02 and J05AF03 or N03AF03 and J05AF03 or N03AF04 and J05AF03 or N03AG01 and J05AF03 or N03AG02 and J05AF03 or N03AG03 and J05AF03 or N03AG04 and J05AF03 or N03AG05 and J05AF03 or N03AG06 and J05AF03 or N03AX03 and J05AF03 or N03AX07 and J05AF03 or N03AX09 and J05AF03 or N03AX10 and J05AF03 or N03AX11 and J05AF03 or N03AX12 and J05AF03 or N03AX13 and J05AF03 or N03AX14 and J05AF03 or N03AX15 and J05AF03 or N03AX16 and J05AF03 or N03AX17 and J05AF03 or N03AX18 and J05AF03 or N03AX19 and J05AF03 or N03AX21 and J05AF03 or N03AX22 and J05AF03 or N03AX23 and J05AF03 or N03AX24 and J05AF03 or N03AX30 and J05AF03 or N03AA01 and J05AF04 or N03AA02 and J05AF04 or N03AA03 and J05AF04 or N03AA04 and J05AF04 or N03AA30 and J05AF04 or N03AB01 and J05AF04 or N03AB02 and J05AF04 or N03AB03 and J05AF04 or N03AB04 and J05AF04 or N03AB05 and J05AF04 or N03AB52 and J05AF04 or N03AB54 and J05AF04 or N03AC01 and J05AF04 or N03AC02 and J05AF04 or N03AC03 and J05AF04 or N03AD01 and J05AF04 or N03AD02 and J05AF04 or N03AD03 and J05AF04 or N03AD51 and J05AF04 or N03AE01 and J05AF04 or N03AF01 and J05AF04 or N03AF02 and J05AF04 or N03AF03 and J05AF04 or N03AF04 and J05AF04 or N03AG01 and J05AF04 or N03AG02 and J05AF04 or N03AG03 and J05AF04 or N03AG04 and J05AF04 or N03AG05 and J05AF04 or N03AG06 and J05AF04 or N03AX03 and J05AF04 or N03AX07 and J05AF04 or N03AX09 and J05AF04 or N03AX10 and J05AF04 or N03AX11 and J05AF04 or N03AX12 and J05AF04 or N03AX13 and J05AF04 or N03AX14 and J05AF04 or N03AX15 and J05AF04 or N03AX16 and J05AF04 or N03AX17 and J05AF04 or N03AX18 and J05AF04 or N03AX19 and J05AF04 or N03AX21 and J05AF04 or N03AX22 and J05AF04 or N03AX23 and J05AF04 or N03AX24 and J05AF04 or N03AX30 and J05AF04 or N03AA01 and J05AF05 or N03AA02 and J05AF05 or N03AA03 and J05AF05 or N03AA04 and J05AF05 or N03AA30 and J05AF05 or N03AB01 and J05AF05 or N03AB02 and J05AF05 or N03AB03 and J05AF05 or N03AB04 and J05AF05 or N03AB05 and J05AF05 or N03AB52 and J05AF05 or N03AB54 and J05AF05 or N03AC01 and J05AF05 or N03AC02 and J05AF05 or N03AC03 and J05AF05 or N03AD01 and J05AF05 or N03AD02 and J05AF05 or N03AD03 and J05AF05 or N03AD51 and J05AF05 or N03AE01 and J05AF05 or N03AF01 and J05AF05 or N03AF02 and J05AF05 or N03AF03 and J05AF05 or N03AF04 and J05AF05 or N03AG01 and J05AF05 or N03AG02 and J05AF05 or N03AG03 and J05AF05 or N03AG04 and J05AF05 or N03AG05 and J05AF05 or N03AG06 and J05AF05 or N03AX03 and J05AF05 or N03AX07 and J05AF05 or N03AX09 and J05AF05 or N03AX10 and J05AF05 or N03AX11 and J05AF05 or N03AX12 and J05AF05 or N03AX13 and J05AF05 or N03AX14 and J05AF05 or N03AX15 and J05AF05 or N03AX16 and J05AF05 or N03AX17 and J05AF05 or N03AX18 and J05AF05 or N03AX19 and J05AF05 or N03AX21 and J05AF05 or N03AX22 and J05AF05 or N03AX23 and J05AF05 or N03AX24 and J05AF05 or N03AX30 and J05AF05 or N03AA01 and J05AF06 or N03AA02 and J05AF06 or N03AA03 and J05AF06 or N03AA04 and J05AF06 or N03AA30 and J05AF06 or N03AB01 and J05AF06 or N03AB02 and J05AF06 or N03AB03 and J05AF06 or N03AB04 and J05AF06 or N03AB05 and J05AF06 or N03AB52 and J05AF06 or N03AB54 and J05AF06 or N03AC01 and J05AF06 or N03AC02 and J05AF06 or N03AC03 and J05AF06 or N03AD01 and J05AF06 or N03AD02 and J05AF06 or N03AD03 and J05AF06 or N03AD51 and J05AF06 or N03AE01 and J05AF06 or N03AF01 and J05AF06 or N03AF02 and J05AF06 or N03AF03 and J05AF06 or N03AF04 and J05AF06 or N03AG01 and J05AF06 or N03AG02 and J05AF06 or N03AG03 and J05AF06 or N03AG04 and J05AF06 or N03AG05 and J05AF06 or N03AG06 and J05AF06 or N03AX03 and J05AF06 or N03AX07 and J05AF06 or N03AX09 and J05AF06 or N03AX10 and J05AF06 or N03AX11 and J05AF06 or N03AX12 and J05AF06 or N03AX13 and J05AF06 or N03AX14 and J05AF06 or N03AX15 and J05AF06 or N03AX16 and J05AF06 or N03AX17 and J05AF06 or N03AX18 and J05AF06 or N03AX19 and J05AF06 or N03AX21 and J05AF06 or N03AX22 and J05AF06 or N03AX23 and J05AF06 or N03AX24 and J05AF06 or N03AX30 and J05AF06 or N03AA01 and J05AF07 or N03AA02 and J05AF07 or N03AA03 and J05AF07 or N03AA04 and J05AF07 or N03AA30 and J05AF07 or N03AB01 and J05AF07 or N03AB02 and J05AF07 or N03AB03 and J05AF07 or N03AB04 and J05AF07 or N03AB05 and J05AF07 or N03AB52 and J05AF07 or N03AB54 and J05AF07 or N03AC01 and J05AF07 or N03AC02 and J05AF07 or N03AC03 and J05AF07 or N03AD01 and J05AF07 or N03AD02 and J05AF07 or N03AD03 and J05AF07 or N03AD51 and J05AF07 or N03AE01 and J05AF07 or N03AF01 and J05AF07 or N03AF02 and J05AF07 or N03AF03 and J05AF07 or N03AF04 and J05AF07 or N03AG01 and J05AF07 or N03AG02 and J05AF07 or N03AG03 and J05AF07 or N03AG04 and J05AF07 or N03AG05 and J05AF07 or N03AG06 and J05AF07 or N03AX03 and J05AF07 or N03AX07 and J05AF07 or N03AX09 and J05AF07 or N03AX10 and J05AF07 or N03AX11 and J05AF07 or N03AX12 and J05AF07 or N03AX13 and J05AF07 or N03AX14 and J05AF07 or N03AX15 and J05AF07 or N03AX16 and J05AF07 or N03AX17 and J05AF07 or N03AX18 and J05AF07 or N03AX19 and J05AF07 or N03AX21 and J05AF07 or N03AX22 and J05AF07 or N03AX23 and J05AF07 or N03AX24 and J05AF07 or N03AX30 and J05AF07 or N03AA01 and J05AF08 or N03AA02 and J05AF08 or N03AA03 and J05AF08 or N03AA04 and J05AF08 or N03AA30 and J05AF08 or N03AB01 and J05AF08 or N03AB02 and J05AF08 or N03AB03 and J05AF08 or N03AB04 and J05AF08 or N03AB05 and J05AF08 or N03AB52 and J05AF08 or N03AB54 and J05AF08 or N03AC01 and J05AF08 or N03AC02 and J05AF08 or N03AC03 and J05AF08 or N03AD01 and J05AF08 or N03AD02 and J05AF08 or N03AD03 and J05AF08 or N03AD51 and J05AF08 or N03AE01 and J05AF08 or N03AF01 and J05AF08 or N03AF02 and J05AF08 or N03AF03 and J05AF08 or N03AF04 and J05AF08 or N03AG01 and J05AF08 or N03AG02 and J05AF08 or N03AG03 and J05AF08 or N03AG04 and J05AF08 or N03AG05 and J05AF08 or N03AG06 and J05AF08 or N03AX03 and J05AF08 or N03AX07 and J05AF08 or N03AX09 and J05AF08 or N03AX10 and J05AF08 or N03AX11 and J05AF08 or N03AX12 and J05AF08 or N03AX13 and J05AF08 or N03AX14 and J05AF08 or N03AX15 and J05AF08 or N03AX16 and J05AF08 or N03AX17 and J05AF08 or N03AX18 and J05AF08 or N03AX19 and J05AF08 or N03AX21 and J05AF08 or N03AX22 and J05AF08 or N03AX23 and J05AF08 or N03AX24 and J05AF08 or N03AX30 and J05AF08 or N03AA01 and J05AF09 or N03AA02 and J05AF09 or N03AA03 and J05AF09 or N03AA04 and J05AF09 or N03AA30 and J05AF09 or N03AB01 and J05AF09 or N03AB02 and J05AF09 or N03AB03 and J05AF09 or N03AB04 and J05AF09 or N03AB05 and J05AF09 or N03AB52 and J05AF09 or N03AB54 and J05AF09 or N03AC01 and J05AF09 or N03AC02 and J05AF09 or N03AC03 and J05AF09 or N03AD01 and J05AF09 or N03AD02 and J05AF09 or N03AD03 and J05AF09 or N03AD51 and J05AF09 or N03AE01 and J05AF09 or N03AF01 and J05AF09 or N03AF02 and J05AF09 or N03AF03 and J05AF09 or N03AF04 and J05AF09 or N03AG01 and J05AF09 or N03AG02 and J05AF09 or N03AG03 and J05AF09 or N03AG04 and J05AF09 or N03AG05 and J05AF09 or N03AG06 and J05AF09 or N03AX03 and J05AF09 or N03AX07 and J05AF09 or N03AX09 and J05AF09 or N03AX10 and J05AF09 or N03AX11 and J05AF09 or N03AX12 and J05AF09 or N03AX13 and J05AF09 or N03AX14 and J05AF09 or N03AX15 and J05AF09 or N03AX16 and J05AF09 or N03AX17 and J05AF09 or N03AX18 and J05AF09 or N03AX19 and J05AF09 or N03AX21 and J05AF09 or N03AX22 and J05AF09 or N03AX23 and J05AF09 or N03AX24 and J05AF09 or N03AX30 and J05AF09 or N03AA01 and J05AF10 or N03AA02 and J05AF10 or N03AA03 and J05AF10 or N03AA04 and J05AF10 or N03AA30 and J05AF10 or N03AB01 and J05AF10 or N03AB02 and J05AF10 or N03AB03 and J05AF10 or N03AB04 and J05AF10 or N03AB05 and J05AF10 or N03AB52 and J05AF10 or N03AB54 and J05AF10 or N03AC01 and J05AF10 or N03AC02 and J05AF10 or N03AC03 and J05AF10 or N03AD01 and J05AF10 or N03AD02 and J05AF10 or N03AD03 and J05AF10 or N03AD51 and J05AF10 or N03AE01 and J05AF10 or N03AF01 and J05AF10 or N03AF02 and J05AF10 or N03AF03 and J05AF10 or N03AF04 and J05AF10 or N03AG01 and J05AF10 or N03AG02 and J05AF10 or N03AG03 and J05AF10 or N03AG04 and J05AF10 or N03AG05 and J05AF10 or N03AG06 and J05AF10 or N03AX03 and J05AF10 or N03AX07 and J05AF10 or N03AX09 and J05AF10 or N03AX10 and J05AF10 or N03AX11 and J05AF10 or N03AX12 and J05AF10 or N03AX13 and J05AF10 or N03AX14 and J05AF10 or N03AX15 and J05AF10 or N03AX16 and J05AF10 or N03AX17 and J05AF10 or N03AX18 and J05AF10 or N03AX19 and J05AF10 or N03AX21 and J05AF10 or N03AX22 and J05AF10 or N03AX23 and J05AF10 or N03AX24 and J05AF10 or N03AX30 and J05AF10 or N03AA01 and J05AF11 or N03AA02 and J05AF11 or N03AA03 and J05AF11 or N03AA04 and J0SAF11 or N03AA30 and J05AF11 or N03AB01 and J05AF11 or N03AB02 and J05AF11 or N03AB03 and J05AF11 or N03AB04 and J05AF11 or N03AB05 and J05AF11 or N03AB52 and J05AF11 or N03AB54 and J05AF11 or N03AC01 and J05AF11 or N03AC02 and J0SAF11 or N03AC03 and J05AF11 or N03AD01 and J05AF11 or N03AD02 and J05AF11 or N03AD03 and J05AF11 or N03AD51 and J05AF11 or N03AE01 and J05AF11 or N03AF01 and J0SAF11 or N03AF02 and J05AF11 or N03AF03 and J05AF11 or N03AF04 and J05AF11 or N03AG01 and J05AF11 or N03AG02 and J05AF11 or N03AG03 and J0SAF11 or N03AG04 and J05AF11 or N03AG05 and J05AF11 or N03AG06 and J05AF11 or N03AX03 and J05AF11 or N03AX07 and J05AF11 or N03AX09 and J05AF11 or N03AX10 and J05AF11 or N03AX11 and J05AF11 or N03AX12 and J0SAF11 or N03AX13 and J05AF11 or N03AX14 and J05AF11 or N03AX15 and J05AF11 or N03AX16 and J05AF11 or N03AX17 and J05AF11 or N03AX18 and J05AF11 or N03AX19 and J05AF11 or N03AX21 and J05AF11 or N03AX22 and J05AF11 or N03AX23 and J05AF11 or N03AX24 and J05AF11 or N03AX30 and J05AF11 or N03AA01 and J05AF12 or N03AA02 and J05AF12 or N03AA03 and J05AF12 or N03AA04 and J05AF12 or N03AA30 and J05AF12 or N03AB01 and J05AF12 or N03AB02 and J05AF12 or N03AB03 and J05AF12 or N03AB04 and J05AF12 or N03AB05 and J05AF12 or N03AB52 and J05AF12 or N03AB54 and J05AF12 or N03AC01 and J05AF12 or N03AC02 and J05AF12 or N03AC03 and J05AF12 or N03AD01 and J05AF12 or N03AD02 and J05AF12 or N03AD03 and J05AF12 or N03AD51 and J05AF12 or N03AE01 and J05AF12 or N03AF01 and J05AF12 or N03AF02 and J05AF12 or N03AF03 and J05AF12 or N03AF04 and J05AF12 or N03AG01 and J05AF12 or N03AG02 and J05AF12 or N03AG03 and J05AF12 or N03AG04 and J05AF12 or N03AG05 and J05AF12 or N03AG06 and J05AF12 or N03AX03 and J05AF12 or N03AX07 and J05AF12 or N03AX09 and J05AF12 or N03AX10 and J05AF12 or N03AX11 and J05AF12 or N03AX12 and J05AF12 or N03AX13 and J05AF12 or N03AX14 and J05AF12 or N03AX15 and J05AF12 or N03AX16 and J05AF12 or N03AX17 and J05AF12 or N03AX18 and J05AF12 or N03AX19 and J05AF12 or N03AX21 and J05AF12 or N03AX22 and J05AF12 or N03AX23 and J05AF12 or N03AX24 and J05AF12 or N03AX30 and J05AF12 or N03AA01 and J05AF13 or N03AA02 and J05AF13 or N03AA03 and J05AF13 or N03AA04 and J05AF13 or N03AA30 and J05AF13 or N03AB01 and J05AF13 or N03AB02 and J05AF13 or N03AB03 and J05AF13 or N03AB04 and J05AF13 or N03AB05 and J05AF13 or N03AB52 and J05AF13 or N03AB54 and J05AF13 or N03AC01 and J05AF13 or N03AC02 and J05AF13 or N03AC03 and J05AF13 or N03AD01 and J05AF13 or N03AD02 and J05AF13 or N03AD03 and J05AF13 or N03AD51 and J05AF13 or N03AE01 and J05AF13 or N03AF01 and J05AF13 or N03AF02 and J05AF13 or N03AF03 and J05AF13 or N03AF04 and J05AF13 or N03AG01 and J05AF13 or N03AG02 and J05AF13 or N03AG03 and J05AF13 or N03AG04 and J05AF13 or N03AG05 and J05AF13 or N03AG06 and J05AF13 or N03AX03 and J05AF13 or N03AX07 and J05AF13 or N03AX09 and J05AF13 or N03AX10 and J05AF13 or N03AX11 and J05AF13 or N03AX12 and J05AF13 or N03AX13 and J05AF13 or N03AX14 and J05AF13 or N03AX15 and J05AF13 or N03AX16 and J05AF13 or N03AX17 and J05AF13 or N03AX18 and J05AF13 or N03AX19 and J05AF13 or N03AX21 and J05AF13 or N03AX22 and J05AF13 or N03AX23 and J05AF13 or N03AX24 and J05AF13 or N03AX30 and J05AF13 or N03AA01 and J05AG01 or N03AA02 and J05AG01 or N03AA03 and J05AG01 or N03AA04 and J05AG01 or N03AA30 and J05AG01 or N03AB01 and J05AG01 or N03AB02 and J05AG01 or N03AB03 and J05AG01 or N03AB04 and J05AG01 or N03AB05 and J05AG01 or N03AB52 and J05AG01 or N03AB54 and J05AG01 or N03AC01 and J05AG01 or N03AC02 and J05AG01 or N03AC03 and J05AG01 or N03AD01 and J05AG01 or N03AD02 and J05AG01 or N03AD03 and J05AG01 or N03AD51 and J05AG01 or N03AE01 and J05AG01 or N03AF01 and J05AG01 or N03AF02 and J05AG01 or N03AF03 and J05AG01 or N03AF04 and J05AG01 or N03AG01 and J05AG01 or N03AG02 and J05AG01 or N03AG03 and J05AG01 or N03AG04 and J05AG01 or N03AG05 and J05AG01 or N03AG06 and J05AG01 or N03AX03 and J05AG01 or N03AX07 and J05AG01 or N03AX09 and J05AG01 or N03AX10 and J05AG01 or N03AX11 and J05AG01 or N03AX12 and J05AG01 or N03AX13 and J05AG01 or N03AX14 and J05AG01 or N03AX15 and J05AG01 or N03AX16 and J05AG01 or N03AX17 and J05AG01 or N03AX18 and J05AG01 or N03AX19 and J05AG01 or N03AX21 and J05AG01 or N03AX22 and J05AG01 or N03AX23 and J05AG01 or N03AX24 and J05AG01 or N03AX30 and J05AG01 or N03AA01 and J05AG02 or N03AA02 and J05AG02 or N03AA03 and J05AG02 or N03AA04 and J05AG02 or N03AA30 and J05AG02 or N03AB01 and J05AG02 or N03AB02 and J05AG02 or N03AB03 and J05AG02 or N03AB04 and J05AG02 or N03AB05 and J05AG02 or N03AB52 and J05AG02 or N03AB54 and J05AG02 or N03AC01 and J05AG02 or N03AC02 and J05AG02 or N03AC03 and J05AG02 or N03AD01 and J05AG02 or N03AD02 and J05AG02 or N03AD03 and J05AG02 or N03AD51 and J05AG02 or N03AE01 and J05AG02 or N03AF01 and J05AG02 or N03AF02 and J05AG02 or N03AF03 and J05AG02 or N03AF04 and J05AG02 or N03AG01 and J05AG02 or N03AG02 and J05AG02 or N03AG03 and J05AG02 or N03AG04 and J05AG02 or N03AG05 and J05AG02 or N03AG06 and J05AG02 or N03AX03 and J05AG02 or N03AX07 and J05AG02 or N03AX09 and J05AG02 or N03AX10 and J05AG02 or N03AX11 and J05AG02 or N03AX12 and J05AG02 or N03AX13 and J05AG02 or N03AX14 and J05AG02 or N03AX15 and J05AG02 or N03AX16 and J05AG02 or N03AX17 and J05AG02 or N03AX18 and J05AG02 or N03AX19 and J05AG02 or N03AX21 and J05AG02 or N03AX22 and J05AG02 or N03AX23 and J05AG02 or N03AX24 and J05AG02 or N03AX30 and J05AG02 or N03AA01 and J05AG03 or N03AA02 and J05AG03 or N03AA03 and J05AG03 or N03AA04 and J05AG03 or N03AA30 and J05AG03 or N03AB01 and J05AG03 or N03AB02 and J05AG03 or N03AB03 and J05AG03 or N03AB04 and J05AG03 or N03AB05 and J05AG03 or N03AB52 and J05AG03 or N03AB54 and J05AG03 or N03AC01 and J05AG03 or N03AC02 and J05AG03 or N03AC03 and J05AG03 or N03AD01 and J05AG03 or N03AD02 and J05AG03 or N03AD03 and J05AG03 or N03AD51 and J05AG03 or N03AE01 and J05AG03 or N03AF01 and J05AG03 or N03AF02 and J05AG03 or N03AF03 and J05AG03 or N03AF04 and J05AG03 or N03AG01 and J05AG03 or N03AG02 and J05AG03 or N03AG03 and J05AG03 or N03AG04 and J05AG03 or N03AG05 and J05AG03 or N03AG06 and J05AG03 or N03AX03 and J05AG03 or N03AX07 and J05AG03 or N03AX09 and J05AG03 or N03AX10 and J05AG03 or N03AX11 and J05AG03 or N03AX12 and J05AG03 or N03AX13 and J05AG03 or N03AX14 and J05AG03 or N03AX15 and J05AG03 or N03AX16 and J05AG03 or N03AX17 and J05AG03 or N03AX18 and J05AG03 or N03AX19 and J05AG03 or N03AX21 and J05AG03 or N03AX22 and J05AG03 or N03AX23 and J05AG03 or N03AX24 and J05AG03 or N03AX30 and J05AG03 or N03AA01 and J05AG04 or N03AA02 and J05AG04 or N03AA03 and J05AG04 or N03AA04 and J05AG04 or N03AA30 and J05AG04 or N03AB01 and J05AG04 or N03AB02 and J05AG04 or N03AB03 and J05AG04 or N03AB04 and J05AG04 or N03AB05 and J05AG04 or N03AB52 and J05AG04 or N03AB54 and J05AG04 or N03AC01 and J05AG04 or N03AC02 and J05AG04 or N03AC03 and J05AG04 or N03AD01 and J05AG04 or N03AD02 and J05AG04 or N03AD03 and J05AG04 or N03AD51 and J05AG04 or N03AE01 and J05AG04 or N03AF01 and J05AG04 or N03AF02 and J05AG04 or N03AF03 and J05AG04 or N03AF04 and J05AG04 or N03AG01 and J05AG04 or N03AG02 and J05AG04 or N03AG03 and J05AG04 or N03AG04 and J05AG04 or N03AG05 and J05AG04 or N03AG06 and J05AG04 or N03AX03 and J05AG04 or N03AX07 and J05AG04 or N03AX09 and J05AG04 or N03AX10 and J05AG04 or N03AX11 and J05AG04 or N03AX12 and J05AG04 or N03AX13 and J05AG04 or N03AX14 and J05AG04 or N03AX15 and J05AG04 or N03AX16 and J05AG04 or N03AX17 and J05AG04 or N03AX18 and J05AG04 or N03AX19 and J05AG04 or N03AX21 and J05AG04 or N03AX22 and J05AG04 or N03AX23 and J05AG04 or N03AX24 and J05AG04 or N03AX30 and J05AG04 or N03AA01 and J05AG05 or N03AA02 and J05AG05 or N03AA03 and J05AG05 or N03AA04 and J05AG05 or N03AA30 and J05AG05 or N03AB01 and J05AG05 or N03AB02 and J05AG05 or N03AB03 and J05AG05 or N03AB04 and J05AG05 or N03AB05 and J05AG05 or N03AB52 and J05AG05 or N03AB54 and J05AG05 or N03AC01 and J05AG05 or N03AC02 and J05AG05 or N03AC03 and J05AG05 or N03AD01 and J05AG05 or N03AD02 and J05AG05 or N03AD03 and J05AG05 or N03AD51 and J05AG05 or N03AE01 and J05AG05 or N03AF01 and J05AG05 or N03AF02 and J05AG05 or N03AF03 and J05AG05 or N03AF04 and J05AG05 or N03AG01 and J05AG05 or N03AG02 and J05AG05 or N03AG03 and J05AG05 or N03AG04 and J05AG05 or N03AG05 and J05AG05 or N03AG06 and J05AG05 or N03AX03 and J05AG05 or N03AX07 and J05AG05 or N03AX09 and J05AG05 or N03AX10 and J05AG05 or N03AX11 and J05AG05 or N03AX12 and J05AG05 or N03AX13 and J05AG05 or N03AX14 and J05AG05 or N03AX15 and J05AG05 or N03AX16 and J05AG05 or N03AX17 and J05AG05 or N03AX18 and J05AG05 or N03AX19 and J05AG05 or N03AX21 and J05AG05 or N03AX22 and J05AG05 or N03AX23 and J05AG05 or N03AX24 and J05AG05 or N03AX30 and J05AG05 or N03AA01 and J05AG06 or N03AA02 and J05AG06 or N03AA03 and J05AG06 or N03AA04 and J05AG06 or N03AA30 and J05AG06 or N03AB01 and J05AG06 or N03AB02 and J05AG06 or N03AB03 and J05AG06 or N03AB04 and J05AG06 or N03AB05 and J05AG06 or N03AB52 and J05AG06 or N03AB54 and J05AG06 or N03AC01 and J05AG06 or N03AC02 and J05AG06 or N03AC03 and J05AG06 or N03AD01 and J05AG06 or N03AD02 and J05AG06 or N03AD03 and J05AG06 or N03AD51 and J05AG06 or N03AE01 and J05AG06 or N03AF01 and J05AG06 or N03AF02 and J05AG06 or N03AF03 and J05AG06 or N03AF04 and J05AG06 or N03AG01 and J05AG06 or N03AG02 and J05AG06 or N03AG03 and J05AG06 or N03AG04 and J05AG06 or N03AG05 and J05AG06 or N03AG06 and J05AG06 or N03AX03 and J05AG06 or N03AX07 and J05AG06 or N03AX09 and J05AG06 or N03AX10 and J05AG06 or N03AX11 and J05AG06 or N03AX12 and J05AG06 or N03AX13 and J05AG06 or N03AX14 and J05AG06 or N03AX15 and J05AG06 or N03AX16 and J05AG06 or N03AX17 and J05AG06 or N03AX18 and J05AG06 or N03AX19 and J05AG06 or N03AX21 and J05AG06 or N03AX22 and J05AG06 or N03AX23 and J05AG06 or N03AX24 and J05AG06 or N03AX30 and J05AG06 or N03AA01 and J05AH01 or N03AA02 and J05AH01 or N03AA03 and J05AH01 or N03AA04 and J05AH01 or N03AA30 and J05AH01 or N03AB01 and J05AH01 or N03AB02 and J05AH01 or N03AB03 and J05AH01 or N03AB04 and J05AH01 or N03AB05 and J05AH01 or N03AB52 and J05AH01 or N03AB54 and J05AH01 or N03AC01 and J05AH01 or N03AC02 and J05AH01 or N03AC03 and J05AH01 or N03AD01 and J05AH01 or N03AD02 and J05AH01 or N03AD03 and J05AH01 or N03AD51 and J05AH01 or N03AE01 and J05AH01 or N03AF01 and J05AH01 or N03AF02 and J05AH01 or N03AF03 and J05AH01 or N03AF04 and J05AH01 or N03AG01 and J05AH01 or N03AG02 and J05AH01 or N03AG03 and J05AH01 or N03AG04 and J05AH01 or N03AG05 and J05AH01 or N03AG06 and J05AH01 or N03AX03 and J05AH01 or N03AX07 and J05AH01 or N03AX09 and J05AH01 or N03AX10 and J05AH01 or N03AX11 and J05AH01 or N03AX12 and J05AH01 or N03AX13 and J05AH01 or N03AX14 and J05AH01 or N03AX15 and J05AH01 or N03AX16 and J05AH01 or N03AX17 and J05AH01 or N03AX18 and J05AH01 or N03AX19 and J05AH01 or N03AX21 and J05AH01 or N03AX22 and J05AH01 or N03AX23 and J05AH01 or N03AX24 and J05AH01 or N03AX30 and J05AH01 or N03AA01 and J05AH02 or N03AA02 and J05AH02 or N03AA03 and J05AH02 or N03AA04 and J05AH02 or N03AA30 and J05AH02 or N03AB01 and J05AH02 or N03AB02 and J05AH02 or N03AB03 and J05AH02 or N03AB04 and J05AH02 or N03AB05 and J05AH02 or N03AB52 and J05AH02 or N03AB54 and J05AH02 or N03AC01 and J05AH02 or N03AC02 and J05AH02 or N03AC03 and J05AH02 or N03AD01 and J05AH02 or N03AD02 and J05AH02 or N03AD03 and J05AH02 or N03AD51 and J05AH02 or N03AE01 and J05AH02 or N03AF01 and J05AH02 or N03AF02 and J05AH02 or N03AF03 and J05AH02 or N03AF04 and J05AH02 or N03AG01 and J05AH02 or N03AG02 and J05AH02 or N03AG03 and J05AH02 or N03AG04 and J05AH02 or N03AG05 and J05AH02 or N03AG06 and J05AH02 or N03AX03 and J05AH02 or N03AX07 and J05AH02 or N03AX09 and J05AH02 or N03AX10 and J05AH02 or N03AX11 and J05AH02 or N03AX12 and J05AH02 or N03AX13 and J05AH02 or N03AX14 and J05AH02 or N03AX15 and J05AH02 or N03AX16 and J05AH02 or N03AX17 and J05AH02 or N03AX18 and J05AH02 or N03AX19 and J05AH02 or N03AX21 and J05AH02 or N03AX22 and J05AH02 or N03AX23 and J05AH02 or N03AX24 and J05AH02 or N03AX30 and J05AH02 or N03AA01 and J05AH03 or N03AA02 and J05AH03 or N03AA03 and J05AH03 or N03AA04 and J05AH03 or N03AA30 and J05AH03 or N03AB01 and J05AH03 or N03AB02 and J05AH03 or N03AB03 and J05AH03 or N03AB04 and J05AH03 or N03AB05 and J05AH03 or N03AB52 and J05AH03 or N03AB54 and J05AH03 or N03AC01 and J05AH03 or N03AC02 and J05AH03 or N03AC03 and J05AH03 or N03AD01 and J05AH03 or N03AD02 and J05AH03 or N03AD03 and J05AH03 or N03AD51 and J05AH03 or N03AE01 and J05AH03 or N03AF01 and J05AH03 or N03AF02 and J05AH03 or N03AF03 and J05AH03 or N03AF04 and J05AH03 or N03AG01 and J05AH03 or N03AG02 and J05AH03 or N03AG03 and J05AH03 or N03AG04 and J05AH03 or N03AG05 and J05AH03 or N03AG06 and J05AH03 or N03AX03 and J05AH03 or N03AX07 and J05AH03 or N03AX09 and J05AH03 or N03AX10 and J05AH03 or N03AX11 and J05AH03 or N03AX12 and J05AH03 or N03AX13 and J05AH03 or N03AX14 and J05AH03 or N03AX15 and J05AH03 or N03AX16 and J05AH03 or N03AX17 and J05AH03 or N03AX18 and J05AH03 or N03AX19 and J05AH03 or N03AX21 and J05AH03 or N03AX22 and J05AH03 or N03AX23 and J05AH03 or N03AX24 and J05AH03 or N03AX30 and J05AH03 or N03AA01 and J05AP01 or N03AA02 and J05AP01 or N03AA03 and J05AP01 or N03AA04 and J05AP01 or N03AA30 and J05AP01 or N03AB01 and J05AP01 or N03AB02 and J05AP01 or N03AB03 and J05AP01 or N03AB04 and J05AP01 or N03AB05 and J05AP01 or N03AB52 and J05AP01 or N03AB54 and J05AP01 or N03AC01 and J05AP01 or N03AC02 and J05AP01 or N03AC03 and J05AP01 or N03AD01 and J05AP01 or N03AD02 and J05AP01 or N03AD03 and J05AP01 or N03AD51 and J05AP01 or N03AE01 and J05AP01 or N03AF01 and J05AP01 or N03AF02 and J05AP01 or N03AF03 and J05AP01 or N03AF04 and J05AP01 or N03AG01 and J05AP01 or N03AG02 and J05AP01 or N03AG03 and J05AP01 or N03AG04 and J05AP01 or N03AG05 and J05AP01 or N03AG06 and J05AP01 or N03AX03 and J05AP01 or N03AX07 and J05AP01 or N03AX09 and J05AP01 or N03AX10 and J05AP01 or N03AX11 and J05AP01 or N03AX12 and J05AP01 or N03AX13 and J05AP01 or N03AX14 and J05AP01 or N03AX15 and J05AP01 or N03AX16 and J05AP01 or N03AX17 and J05AP01 or N03AX18 and J05AP01 or N03AX19 and J05AP01 or N03AX21 and J05AP01 or N03AX22 and J05AP01 or N03AX23 and J05AP01 or N03AX24 and J05AP01 or N03AX30 and J05AP01 or N03AA01 and J05AP02 or N03AA02 and J05AP02 or N03AA03 and J05AP02 or N03AA04 and J05AP02 or N03AA30 and J05AP02 or N03AB01 and J05AP02 or N03AB02 and J05AP02 or N03AB03 and J05AP02 or N03AB04 and J05AP02 or N03AB05 and J05AP02 or N03AB52 and J05AP02 or N03AB54 and J05AP02 or N03AC01 and J05AP02 or N03AC02 and J05AP02 or N03AC03 and J05AP02 or N03AD01 and J05AP02 or N03AD02 and J05AP02 or N03AD03 and J05AP02 or N03AD51 and J05AP02 or N03AE01 and J05AP02 or N03AF01 and J05AP02 or N03AF02 and J05AP02 or N03AF03 and J05AP02 or N03AF04 and J05AP02 or N03AG01 and J05AP02 or N03AG02 and J05AP02 or N03AG03 and J05AP02 or N03AG04 and J05AP02 or N03AG05 and J05AP02 or N03AG06 and J05AP02 or N03AX03 and J05AP02 or N03AX07 and J05AP02 or N03AX09 and J05AP02 or N03AX10 and J05AP02 or N03AX11 and J05AP02 or N03AX12 and J05AP02 or N03AX13 and J05AP02 or N03AX14 and J05AP02 or N03AX15 and J05AP02 or N03AX16 and J05AP02 or N03AX17 and J05AP02 or N03AX18 and J05AP02 or N03AX19 and J05AP02 or N03AX21 and J05AP02 or N03AX22 and J05AP02 or N03AX23 and J05AP02 or N03AX24 and J05AP02 or N03AX30 and J05AP02 or N03AA01 and J05AP03 or N03AA02 and J05AP03 or N03AA03 and J05AP03 or N03AA04 and J05AP03 or N03AA30 and J05AP03 or N03AB01 and J05AP03 or N03AB02 and J05AP03 or N03AB03 and J05AP03 or N03AB04 and J05AP03 or N03AB05 and J05AP03 or N03AB52 and J05AP03 or N03AB54 and J05AP03 or N03AC01 and J05AP03 or N03AC02 and J05AP03 or N03AC03 and J05AP03 or N03AD01 and J05AP03 or N03AD02 and J05AP03 or N03AD03 and J05AP03 or N03AD51 and J05AP03 or N03AE01 and J05AP03 or N03AF01 and J05AP03 or N03AF02 and J05AP03 or N03AF03 and J05AP03 or N03AF04 and J05AP03 or N03AG01 and J05AP03 or N03AG02 and J05AP03 or N03AG03 and J05AP03 or N03AG04 and J05AP03 or N03AG05 and J05AP03 or N03AG06 and J05AP03 or N03AX03 and J05AP03 or N03AX07 and J05AP03 or N03AX09 and J05AP03 or N03AX10 and J05AP03 or N03AX11 and J05AP03 or N03AX12 and J05AP03 or N03AX13 and J05AP03 or N03AX14 and J05AP03 or N03AX15 and J05AP03 or N03AX16 and J05AP03 or N03AX17 and J05AP03 or N03AX18 and J05AP03 or N03AX19 and J05AP03 or N03AX21 and J05AP03 or N03AX22 and J05AP03 or N03AX23 and J05AP03 or N03AX24 and J05AP03 or N03AX30 and J05AP03 or N03AA01 and J05AP04 or N03AA02 and J05AP04 or N03AA03 and J05AP04 or N03AA04 and J05AP04 or N03AA30 and J05AP04 or N03AB01 and J05AP04 or N03AB02 and J05AP04 or N03AB03 and J05AP04 or N03AB04 and J05AP04 or N03AB05 and J05AP04 or N03AB52 and J05AP04 or N03AB54 and J05AP04 or N03AC01 and J05AP04 or N03AC02 and J05AP04 or N03AC03 and J05AP04 or N03AD01 and J05AP04 or N03AD02 and J05AP04 or N03AD03 and J05AP04 or N03AD51 and J05AP04 or N03AE01 and J05AP04 or N03AF01 and J05AP04 or N03AF02 and J05AP04 or N03AF03 and J05AP04 or N03AF04 and J05AP04 or N03AG01 and J05AP04 or N03AG02 and J05AP04 or N03AG03 and J05AP04 or N03AG04 and J05AP04 or N03AG05 and J05AP04 or N03AG06 and J05AP04 or N03AX03 and J05AP04 or N03AX07 and J05AP04 or N03AX09 and J05AP04 or N03AX10 and J05AP04 or N03AX11 and J05AP04 or N03AX12 and J05AP04 or N03AX13 and J05AP04 or N03AX14 and J05AP04 or N03AX15 and J05AP04 or N03AX16 and J05AP04 or N03AX17 and J05AP04 or N03AX18 and J05AP04 or N03AX19 and J05AP04 or N03AX21 and J05AP04 or N03AX22 and J05AP04 or N03AX23 and J05AP04 or N03AX24 and J05AP04 or N03AX30 and J05AP04 or N03AA01 and J05AP05 or N03AA02 and J05AP05 or N03AA03 and J05AP05 or N03AA04 and J05AP05 or N03AA30 and J05AP05 or N03AB01 and J05AP05 or N03AB02 and J05AP05 or N03AB03 and J05AP05 or N03AB04 and J05AP05 or N03AB05 and J05AP05 or N03AB52 and J05AP05 or N03AB54 and J05AP05 or N03AC01 and J05AP05 or N03AC02 and J05AP05 or N03AC03 and J05AP05 or N03AD01 and J05AP05 or N03AD02 and J05AP05 or N03AD03 and J05AP05 or N03AD51 and J05AP05 or N03AE01 and J05AP05 or N03AF01 and J05AP05 or N03AF02 and J05AP05 or N03AF03 and J05AP05 or N03AF04 and J05AP05 or N03AG01 and J05AP05 or N03AG02 and J05AP05 or N03AG03 and J05AP05 or N03AG04 and J05AP05 or N03AG05 and J05AP05 or N03AG06 and J05AP05 or N03AX03 and J05AP05 or N03AX07 and J05AP05 or N03AX09 and J05AP05 or N03AX10 and J05AP05 or N03AX11 and J05AP05 or N03AX12 and J05AP05 or N03AX13 and J05AP05 or N03AX14 and J05AP05 or N03AX15 and J05AP05 or N03AX16 and J05AP05 or N03AX17 and J05AP05 or N03AX18 and J05AP05 or N03AX19 and J05AP05 or N03AX21 and J05AP05 or N03AX22 and J05AP05 or N03AX23 and J05AP05 or N03AX24 and J05AP05 or N03AX30 and J05AP05 or N03AA01 and J05AP06 or N03AA02 and J05AP06 or N03AA03 and J05AP06 or N03AA04 and J05AP06 or N03AA30 and J05AP06 or N03AB01 and J05AP06 or N03AB02 and J05AP06 or N03AB03 and J05AP06 or N03AB04 and J05AP06 or N03AB05 and J05AP06 or N03AB52 and J05AP06 or N03AB54 and J05AP06 or N03AC01 and J05AP06 or N03AC02 and J05AP06 or N03AC03 and J05AP06 or N03AD01 and J05AP06 or N03AD02 and J05AP06 or N03AD03 and J05AP06 or N03AD51 and J05AP06 or N03AE01 and J05AP06 or N03AF01 and J05AP06 or N03AF02 and J05AP06 or N03AF03 and J05AP06 or N03AF04 and J05AP06 or N03AG01 and J05AP06 or N03AG02 and J05AP06 or N03AG03 and J05AP06 or N03AG04 and J05AP06 or N03AG05 and J05AP06 or N03AG06 and J05AP06 or N03AX03 and J05AP06 or N03AX07 and J05AP06 or N03AX09 and J05AP06 or N03AX10 and J05AP06 or N03AX11 and J05AP06 or N03AX12 and J05AP06 or N03AX13 and J05AP06 or N03AX14 and J05AP06 or N03AX15 and J05AP06 or N03AX16 and J05AP06 or N03AX17 and J05AP06 or N03AX18 and J05AP06 or N03AX19 and J05AP06 or N03AX21 and J05AP06 or N03AX22 and J05AP06 or N03AX23 and J05AP06 or N03AX24 and J05AP06 or N03AX30 and J05AP06 or N03AA01 and J05AP07 or N03AA02 and J05AP07 or N03AA03 and J05AP07 or N03AA04 and J05AP07 or N03AA30 and J05AP07 or N03AB01 and J05AP07 or N03AB02 and J05AP07 or N03AB03 and J05AP07 or N03AB04 and J05AP07 or N03AB05 and J05AP07 or N03AB52 and J05AP07 or N03AB54 and J05AP07 or N03AC01 and J05AP07 or N03AC02 and J05AP07 or N03AC03 and J05AP07 or N03AD01 and J05AP07 or N03AD02 and J05AP07 or N03AD03 and J05AP07 or N03AD51 and J05AP07 or N03AE01 and J05AP07 or N03AF01 and J05AP07 or N03AF02 and J05AP07 or N03AF03 and J05AP07 or N03AF04 and J05AP07 or N03AG01 and J05AP07 or N03AG02 and J05AP07 or N03AG03 and J05AP07 or N03AG04 and J05AP07 or N03AG05 and J05AP07 or N03AG06 and J05AP07 or N03AX03 and J05AP07 or N03AX07 and J05AP07 or N03AX09 and J05AP07 or N03AX10 and J05AP07 or N03AX11 and J05AP07 or N03AX12 and J05AP07 or N03AX13 and J05AP07 or N03AX14 and J05AP07 or N03AX15 and J05AP07 or N03AX16 and J05AP07 or N03AX17 and J05AP07 or N03AX18 and J05AP07 or N03AX19 and J05AP07 or N03AX21 and J05AP07 or N03AX22 and J05AP07 or N03AX23 and J05AP07 or N03AX24 and J05AP07 or N03AX30 and J05AP07 or N03AA01 and J05AP08 or N03AA02 and J05AP08 or N03AA03 and J05AP08 or N03AA04 and J05AP08 or N03AA30 and J05AP08 or N03AB01 and J05AP08 or N03AB02 and J05AP08 or N03AB03 and J05AP08 or N03AB04 and J05AP08 or N03AB05 and J05AP08 or N03AB52 and J05AP08 or N03AB54 and J05AP08 or N03AC01 and J05AP08 or N03AC02 and J05AP08 or N03AC03 and J05AP08 or N03AD01 and J05AP08 or N03AD02 and J05AP08 or N03AD03 and J05AP08 or N03AD51 and J05AP08 or N03AE01 and J05AP08 or N03AF01 and J05AP08 or N03AF02 and J05AP08 or N03AF03 and J05AP08 or N03AF04 and J05AP08 or N03AG01 and J05AP08 or N03AG02 and J05AP08 or N03AG03 and J05AP08 or N03AG04 and J05AP08 or N03AG05 and J05AP08 or N03AG06 and J05AP08 or N03AX03 and J05AP08 or N03AX07 and J05AP08 or N03AX09 and J05AP08 or N03AX10 and J05AP08 or N03AX11 and J05AP08 or N03AX12 and J05AP08 or N03AX13 and J05AP08 or N03AX14 and J05AP08 or N03AX15 and J05AP08 or N03AX16 and J05AP08 or N03AX17 and J05AP08 or N03AX18 and J05AP08 or N03AX19 and J05AP08 or N03AX21 and J05AP08 or N03AX22 and J05AP08 or N03AX23 and J05AP08 or N03AX24 and J05AP08 or N03AX30 and J05AP08 or N03AA01 and J05AP09 or N03AA02 and J05AP09 or N03AA03 and J05AP09 or N03AA04 and J05AP09 or N03AA30 and J05AP09 or N03AB01 and J05AP09 or N03AB02 and J05AP09 or N03AB03 and J05AP09 or N03AB04 and J05AP09 or N03AB05 and J05AP09 or N03AB52 and J05AP09 or N03AB54 and J05AP09 or N03AC01 and J05AP09 or N03AC02 and J05AP09 or N03AC03 and J05AP09 or N03AD01 and J05AP09 or N03AD02 and J05AP09 or N03AD03 and J05AP09 or N03AD51 and J05AP09 or N03AE01 and J05AP09 or N03AF01 and J05AP09 or N03AF02 and J05AP09 or N03AF03 and J05AP09 or N03AF04 and J05AP09 or N03AG01 and J05AP09 or N03AG02 and J05AP09 or N03AG03 and J05AP09 or N03AG04 and J05AP09 or N03AG05 and J05AP09 or N03AG06 and J05AP09 or N03AX03 and J05AP09 or N03AX07 and J05AP09 or N03AX09 and J05AP09 or N03AX10 and J05AP09 or N03AX11 and J05AP09 or N03AX12 and J05AP09 or N03AX13 and J05AP09 or N03AX14 and J05AP09 or N03AX15 and J05AP09 or N03AX16 and J05AP09 or N03AX17 and J05AP09 or N03AX18 and J05AP09 or N03AX19 and J05AP09 or N03AX21 and J05AP09 or N03AX22 and J05AP09 or N03AX23 and J05AP09 or N03AX24 and J05AP09 or N03AX30 and J05AP09 or N03AA01 and J05AP51 or N03AA02 and J05AP51 or N03AA03 and J05AP51 or N03AA04 and J05AP51 or N03AA30 and J05AP51 or N03AB01 and J05AP51 or N03AB02 and J05AP51 or N03AB03 and J05AP51 or N03AB04 and J05AP51 or N03AB05 and J05AP51 or N03AB52 and J05AP51 or N03AB54 and J05AP51 or N03AC01 and J05AP51 or N03AC02 and J05AP51 or N03AC03 and J05AP51 or N03AD01 and J05AP51 or N03AD02 and J05AP51 or N03AD03 and J05AP51 or N03AD51 and J05AP51 or N03AE01 and J05AP51 or N03AF01 and J05AP51 or N03AF02 and J05AP51 or N03AF03 and J05AP51 or N03AF04 and J05AP51 or N03AG01 and J05AP51 or N03AG02 and J05AP51 or N03AG03 and J05AP51 or N03AG04 and J05AP51 or N03AG05 and J05AP51 or N03AG06 and J05AP51 or N03AX03 and J05AP51 or N03AX07 and J05AP51 or N03AX09 and J05AP51 or N03AX10 and J05AP51 or N03AX11 and J05AP51 or N03AX12 and J05AP51 or N03AX13 and J05AP51 or N03AX14 and J05AP51 or N03AX15 and J05AP51 or N03AX16 and J05AP51 or N03AX17 and J05AP51 or N03AX18 and J05AP51 or N03AX19 and J05AP51 or N03AX21 and J05AP51 or N03AX22 and J05AP51 or N03AX23 and J05AP51 or N03AX24 and J05AP51 or N03AX30 and J05AP51 or N03AA01 and J05AP52 or N03AA02 and J05AP52 or N03AA03 and J05AP52 or N03AA04 and J05AP52 or N03AA30 and J05AP52 or N03AB01 and J05AP52 or N03AB02 and J05AP52 or N03AB03 and J05AP52 or N03AB04 and J05AP52 or N03AB05 and J05AP52 or N03AB52 and J05AP52 or N03AB54 and J05AP52 or N03AC01 and J05AP52 or N03AC02 and J05AP52 or N03AC03 and J05AP52 or N03AD01 and J05AP52 or N03AD02 and J05AP52 or N03AD03 and J05AP52 or N03AD51 and J05AP52 or N03AE01 and J05AP52 or N03AF01 and J05AP52 or N03AF02 and J05AP52 or N03AF03 and J05AP52 or N03AF04 and J05AP52 or N03AG01 and J05AP52 or N03AG02 and J05AP52 or N03AG03 and J05AP52 or N03AG04 and J05AP52 or N03AG05 and J05AP52 or N03AG06 and J05AP52 or N03AX03 and J05AP52 or N03AX07 and J05AP52 or N03AX09 and J05AP52 or N03AX10 and J05AP52 or N03AX11 and J05AP52 or N03AX12 and J05AP52 or N03AX13 and J05AP52 or N03AX14 and J05AP52 or N03AX15 and J05AP52 or N03AX16 and J05AP52 or N03AX17 and J05AP52 or N03AX18 and J05AP52 or N03AX19 and J05AP52 or N03AX21 and J05AP52 or N03AX22 and J05AP52 or N03AX23 and J05AP52 or N03AX24 and J05AP52 or N03AX30 and J05AP52 or N03AA01 and J05AP53 or N03AA02 and J05AP53 or N03AA03 and J05AP53 or N03AA04 and J05AP53 or N03AA30 and J05AP53 or N03AB01 and J05AP53 or N03AB02 and J05AP53 or N03AB03 and J05AP53 or N03AB04 and J05AP53 or N03AB05 and J05AP53 or N03AB52 and J05AP53 or N03AB54 and J05AP53 or N03AC01 and J05AP53 or N03AC02 and J05AP53 or N03AC03 and J05AP53 or N03AD01 and J05AP53 or N03AD02 and J05AP53 or N03AD03 and J05AP53 or N03AD51 and J05AP53 or N03AE01 and J05AP53 or N03AF01 and J05AP53 or N03AF02 and J05AP53 or N03AF03 and J05AP53 or N03AF04 and J05AP53 or N03AG01 and J05AP53 or N03AG02 and J05AP53 or N03AG03 and J05AP53 or N03AG04 and J05AP53 or N03AG05 and J05AP53 or N03AG06 and J05AP53 or N03AX03 and J05AP53 or N03AX07 and J05AP53 or N03AX09 and J05AP53 or N03AX10 and J05AP53 or N03AX11 and J05AP53 or N03AX12 and J05AP53 or N03AX13 and J05AP53 or N03AX14 and J05AP53 or N03AX15 and J05AP53 or N03AX16 and J05AP53 or N03AX17 and J05AP53 or N03AX18 and J05AP53 or N03AX19 and J05AP53 or N03AX21 and J05AP53 or N03AX22 and J05AP53 or N03AX23 and J05AP53 or N03AX24 and J05AP53 or N03AX30 and J05AP53 or N03AA01 and J05AP54 or N03AA02 and J05AP54 or N03AA03 and J05AP54 or N03AA04 and J05AP54 or N03AA30 and J05AP54 or N03AB01 and J05AP54 or N03AB02 and J05AP54 or N03AB03 and J05AP54 or N03AB04 and J05AP54 or N03AB05 and J05AP54 or N03AB52 and J05AP54 or N03AB54 and J05AP54 or N03AC01 and J05AP54 or N03AC02 and J05AP54 or N03AC03 and J05AP54 or N03AD01 and J05AP54 or N03AD02 and J05AP54 or N03AD03 and J05AP54 or N03AD51 and J05AP54 or N03AE01 and J05AP54 or N03AF01 and J05AP54 or N03AF02 and J05AP54 or N03AF03 and J05AP54 or N03AF04 and J05AP54 or N03AG01 and J05AP54 or N03AG02 and J05AP54 or N03AG03 and J05AP54 or N03AG04 and J05AP54 or N03AG05 and J05AP54 or N03AG06 and J05AP54 or N03AX03 and J05AP54 or N03AX07 and J05AP54 or N03AX09 and J05AP54 or N03AX10 and J05AP54 or N03AX11 and J05AP54 or N03AX12 and J05AP54 or N03AX13 and J05AP54 or N03AX14 and J05AP54 or N03AX15 and J05AP54 or N03AX16 and J05AP54 or N03AX17 and J05AP54 or N03AX18 and J05AP54 or N03AX19 and J05AP54 or N03AX21 and J05AP54 or N03AX22 and J05AP54 or N03AX23 and J05AP54 or N03AX24 and J05AP54 or N03AX30 and J05AP54 or N03AA01 and J05AP55 or N03AA02 and J05AP55 or N03AA03 and J05AP55 or N03AA04 and J05AP55 or N03AA30 and J05AP55 or N03AB01 and J05AP55 or N03AB02 and J05AP55 or N03AB03 and J05AP55 or N03AB04 and J05AP55 or N03AB05 and J05AP55 or N03AB52 and J05AP55 or N03AB54 and J05AP55 or N03AC01 and J05AP55 or N03AC02 and J05AP55 or N03AC03 and J05AP55 or N03AD01 and J05AP55 or N03AD02 and J05AP55 or N03AD03 and J05AP55 or N03AD51 and J05AP55 or N03AE01 and J05AP55 or N03AF01 and J05AP55 or N03AF02 and J05AP55 or N03AF03 and J05AP55 or N03AF04 and J05AP55 or N03AG01 and J05AP55 or N03AG02 and J05AP55 or N03AG03 and J05AP55 or N03AG04 and J05AP55 or N03AG05 and J05AP55 or N03AG06 and J05AP55 or N03AX03 and J05AP55 or N03AX07 and J05AP55 or N03AX09 and J05AP55 or N03AX10 and J05AP55 or N03AX11 and J05AP55 or N03AX12 and J05AP55 or N03AX13 and J05AP55 or N03AX14 and J05AP55 or N03AX15 and J05AP55 or N03AX16 and J05AP55 or N03AX17 and J05AP55 or N03AX18 and J05AP55 or N03AX19 and J05AP55 or N03AX21 and J05AP55 or N03AX22 and J05AP55 or N03AX23 and J05AP55 or N03AX24 and J05AP55 or N03AX30 and J05AP55 or N03AA01 and J05AP56 or N03AA02 and J05AP56 or N03AA03 and J05AP56 or N03AA04 and J05AP56 or N03AA30 and J05AP56 or N03AB01 and J05AP56 or N03AB02 and J05AP56 or N03AB03 and J05AP56 or N03AB04 and J05AP56 or N03AB05 and J05AP56 or N03AB52 and J05AP56 or N03AB54 and J05AP56 or N03AC01 and J05AP56 or N03AC02 and J05AP56 or N03AC03 and J05AP56 or N03AD01 and J05AP56 or N03AD02 and J05AP56 or N03AD03 and J05AP56 or N03AD51 and J05AP56 or N03AE01 and J05AP56 or N03AF01 and J05AP56 or N03AF02 and J05AP56 or N03AF03 and J05AP56 or N03AF04 and J05AP56 or N03AG01 and J05AP56 or N03AG02 and J05AP56 or N03AG03 and J05AP56 or N03AG04 and J05AP56 or N03AG05 and J05AP56 or N03AG06 and J05AP56 or N03AX03 and J05AP56 or N03AX07 and J05AP56 or N03AX09 and J05AP56 or N03AX10 and J05AP56 or N03AX11 and J05AP56 or N03AX12 and J05AP56 or N03AX13 and J05AP56 or N03AX14 and J05AP56 or N03AX15 and J05AP56 or N03AX16 and J05AP56 or N03AX17 and J05AP56 or N03AX18 and J05AP56 or N03AX19 and J05AP56 or N03AX21 and J05AP56 or N03AX22 and J05AP56 or N03AX23 and J05AP56 or N03AX24 and J05AP56 or N03AX30 and J05AP56 or N03AA01 and J05AP57 or N03AA02 and J05AP57 or N03AA03 and J05AP57 or N03AA04 and J05AP57 or N03AA30 and J05AP57 or N03AB01 and J05AP57 or N03AB02 and J05AP57 or N03AB03 and J05AP57 or N03AB04 and J05AP57 or N03AB05 and J05AP57 or N03AB52 and J05AP57 or N03AB54 and J05AP57 or N03AC01 and J05AP57 or N03AC02 and J05AP57 or N03AC03 and J05AP57 or N03AD01 and J05AP57 or N03AD02 and J05AP57 or N03AD03 and J05AP57 or N03AD51 and J05AP57 or N03AE01 and J05AP57 or N03AF01 and J05AP57 or N03AF02 and J05AP57 or N03AF03 and J05AP57 or N03AF04 and J05AP57 or N03AG01 and J05AP57 or N03AG02 and J05AP57 or N03AG03 and J05AP57 or N03AG04 and J05AP57 or N03AG05 and J05AP57 or N03AG06 and J05AP57 or N03AX03 and J05AP57 or N03AX07 and J05AP57 or N03AX09 and J05AP57 or N03AX10 and J05AP57 or N03AX11 and J05AP57 or N03AX12 and J05AP57 or N03AX13 and J05AP57 or N03AX14 and J05AP57 or N03AX15 and J05AP57 or N03AX16 and J05AP57 or N03AX17 and J05AP57 or N03AX18 and J05AP57 or N03AX19 and J05AP57 or N03AX21 and J05AP57 or N03AX22 and J05AP57 or N03AX23 and J05AP57 or N03AX24 and J05AP57 or N03AX30 and J05AP57 or N03AA01 and J05AR01 or N03AA02 and J05AR01 or N03AA03 and J05AR01 or N03AA04 and J05AR01 or N03AA30 and J05AR01 or N03AB01 and J05AR01 or N03AB02 and J05AR01 or N03AB03 and J05AR01 or N03AB04 and J05AR01 or N03AB05 and J05AR01 or N03AB52 and J05AR01 or N03AB54 and J05AR01 or N03AC01 and J05AR01 or N03AC02 and J05AR01 or N03AC03 and J05AR01 or N03AD01 and J05AR01 or N03AD02 and J05AR01 or N03AD03 and J05AR01 or N03AD51 and J05AR01 or N03AE01 and J05AR01 or N03AF01 and J05AR01 or N03AF02 and J05AR01 or N03AF03 and J05AR01 or N03AF04 and J05AR01 or N03AG01 and J05AR01 or N03AG02 and J05AR01 or N03AG03 and J05AR01 or N03AG04 and J05AR01 or N03AG05 and J05AR01 or N03AG06 and J05AR01 or N03AX03 and J05AR01 or N03AX07 and J05AR01 or N03AX09 and J05AR01 or N03AX10 and J05AR01 or N03AX11 and J05AR01 or N03AX12 and J05AR01 or N03AX13 and J05AR01 or N03AX14 and J05AR01 or N03AX15 and J05AR01 or N03AX16 and J05AR01 or N03AX17 and J05AR01 or N03AX18 and J05AR01 or N03AX19 and J05AR01 or N03AX21 and J05AR01 or N03AX22 and J05AR01 or N03AX23 and J05AR01 or N03AX24 and J05AR01 or N03AX30 and J05AR01 or N03AA01 and J05AR02 or N03AA02 and J05AR02 or N03AA03 and J05AR02 or N03AA04 and J05AR02 or N03AA30 and J05AR02 or N03AB01 and J05AR02 or N03AB02 and J05AR02 or N03AB03 and J05AR02 or N03AB04 and J05AR02 or N03AB05 and J05AR02 or N03AB52 and J05AR02 or N03AB54 and J05AR02 or N03AC01 and J05AR02 or N03AC02 and J05AR02 or N03AC03 and J05AR02 or N03AD01 and J05AR02 or N03AD02 and J05AR02 or N03AD03 and J05AR02 or N03AD51 and J05AR02 or N03AE01 and J05AR02 or N03AF01 and J05AR02 or N03AF02 and J05AR02 or N03AF03 and J05AR02 or N03AF04 and J05AR02 or N03AG01 and J05AR02 or N03AG02 and J05AR02 or N03AG03 and J05AR02 or N03AG04 and J05AR02 or N03AG05 and J05AR02 or N03AG06 and J05AR02 or N03AX03 and J05AR02 or N03AX07 and J05AR02 or N03AX09 and J05AR02 or N03AX10 and J05AR02 or N03AX11 and J05AR02 or N03AX12 and J05AR02 or N03AX13 and J05AR02 or N03AX14 and J05AR02 or N03AX15 and J05AR02 or N03AX16 and J05AR02 or N03AX17 and J05AR02 or N03AX18 and J05AR02 or N03AX19 and J05AR02 or N03AX21 and J05AR02 or N03AX22 and J05AR02 or N03AX23 and J05AR02 or N03AX24 and J05AR02 or N03AX30 and J05AR02 or N03AA01 and J05AR03 or N03AA02 and J05AR03 or N03AA03 and J05AR03 or N03AA04 and J05AR03 or N03AA30 and J05AR03 or N03AB01 and J05AR03 or N03AB02 and J05AR03 or N03AB03 and J05AR03 or N03AB04 and J05AR03 or N03AB05 and J05AR03 or N03AB52 and J05AR03 or N03AB54 and J05AR03 or N03AC01 and J05AR03 or N03AC02 and J05AR03 or N03AC03 and J05AR03 or N03AD01 and J05AR03 or N03AD02 and J05AR03 or N03AD03 and J05AR03 or N03AD51 and J05AR03 or N03AE01 and J05AR03 or N03AF01 and J05AR03 or N03AF02 and J05AR03 or N03AF03 and J05AR03 or N03AF04 and J05AR03 or N03AG01 and J05AR03 or N03AG02 and J05AR03 or N03AG03 and J05AR03 or N03AG04 and J05AR03 or N03AG05 and J05AR03 or N03AG06 and J05AR03 or N03AX03 and J05AR03 or N03AX07 and J05AR03 or N03AX09 and J05AR03 or N03AX10 and J05AR03 or N03AX11 and J05AR03 or N03AX12 and J05AR03 or N03AX13 and J05AR03 or N03AX14 and J05AR03 or N03AX15 and J05AR03 or N03AX16 and J05AR03 or N03AX17 and J05AR03 or N03AX18 and J05AR03 or N03AX19 and J05AR03 or N03AX21 and J05AR03 or N03AX22 and J05AR03 or N03AX23 and J05AR03 or N03AX24 and J05AR03 or N03AX30 and J05AR03 or N03AA01 and J05AR04 or N03AA02 and J05AR04 or N03AA03 and J05AR04 or N03AA04 and J05AR04 or N03AA30 and J05AR04 or N03AB01 and J05AR04 or N03AB02 and J05AR04 or N03AB03 and J05AR04 or N03AB04 and J05AR04 or N03AB05 and J05AR04 or N03AB52 and J05AR04 or N03AB54 and J05AR04 or N03AC01 and J05AR04 or N03AC02 and J05AR04 or N03AC03 and J05AR04 or N03AD01 and J05AR04 or N03AD02 and J05AR04 or N03AD03 and J05AR04 or N03AD51 and J05AR04 or N03AE01 and J05AR04 or N03AF01 and J05AR04 or N03AF02 and J05AR04 or N03AF03 and J05AR04 or N03AF04 and J05AR04 or N03AG01 and J05AR04 or N03AG02 and J05AR04 or N03AG03 and J05AR04 or N03AG04 and J05AR04 or N03AG05 and J05AR04 or N03AG06 and J05AR04 or N03AX03 and J05AR04 or N03AX07 and J05AR04 or N03AX09 and J05AR04 or N03AX10 and J05AR04 or N03AX11 and J05AR04 or N03AX12 and J05AR04 or N03AX13 and J05AR04 or N03AX14 and J05AR04 or N03AX15 and J05AR04 or N03AX16 and J05AR04 or N03AX17 and J05AR04 or N03AX18 and J05AR04 or N03AX19 and J05AR04 or N03AX21 and J05AR04 or N03AX22 and J05AR04 or N03AX23 and J05AR04 or N03AX24 and J05AR04 or N03AX30 and J05AR04 or N03AA01 and J05AR05 or N03AA02 and J05AR05 or N03AA03 and J05AR05 or N03AA04 and J05AR05 or N03AA30 and J05AR05 or N03AB01 and J05AR05 or N03AB02 and J05AR05 or N03AB03 and J05AR05 or N03AB04 and J05AR05 or N03AB05 and J05AR05 or N03AB52 and J05AR05 or N03AB54 and J05AR05 or N03AC01 and J05AR05 or N03AC02 and J05AR05 or N03AC03 and J05AR05 or N03AD01 and J05AR05 or N03AD02 and J05AR05 or N03AD03 and J05AR05 or N03AD51 and J05AR05 or N03AE01 and J05AR05 or N03AF01 and J05AR05 or N03AF02 and J05AR05 or N03AF03 and J05AR05 or N03AF04 and J05AR05 or N03AG01 and J05AR05 or N03AG02 and J05AR05 or N03AG03 and J05AR05 or N03AG04 and J05AR05 or N03AG05 and J05AR05 or N03AG06 and J05AR05 or N03AX03 and J05AR05 or N03AX07 and J05AR05 or N03AX09 and J05AR05 or N03AX10 and J05AR05 or N03AX11 and J05AR05 or N03AX12 and J05AR05 or N03AX13 and J05AR05 or N03AX14 and J05AR05 or N03AX15 and J05AR05 or N03AX16 and J05AR05 or N03AX17 and J05AR05 or N03AX18 and J05AR05 or N03AX19 and J05AR05 or N03AX21 and J05AR05 or N03AX22 and J05AR05 or N03AX23 and J05AR05 or N03AX24 and J05AR05 or N03AX30 and J05AR05 or N03AA01 and J05AR06 or N03AA02 and J05AR06 or N03AA03 and J05AR06 or N03AA04 and J05AR06 or N03AA30 and J05AR06 or N03AB01 and J05AR06 or N03AB02 and J05AR06 or N03AB03 and J05AR06 or N03AB04 and J05AR06 or N03AB05 and J05AR06 or N03AB52 and J05AR06 or N03AB54 and J05AR06 or N03AC01 and J05AR06 or N03AC02 and J05AR06 or N03AC03 and J05AR06 or N03AD01 and J05AR06 or N03AD02 and J05AR06 or N03AD03 and J05AR06 or N03AD51 and J05AR06 or N03AE01 and J05AR06 or N03AF01 and J05AR06 or N03AF02 and J05AR06 or N03AF03 and J05AR06 or N03AF04 and J05AR06 or N03AG01 and J05AR06 or N03AG02 and J05AR06 or N03AG03 and J05AR06 or N03AG04 and J05AR06 or N03AG05 and J05AR06 or N03AG06 and J05AR06 or N03AX03 and J05AR06 or N03AX07 and J05AR06 or N03AX09 and J05AR06 or N03AX10 and J05AR06 or N03AX11 and J05AR06 or N03AX12 and J05AR06 or N03AX13 and J05AR06 or N03AX14 and J05AR06 or N03AX15 and J05AR06 or N03AX16 and J05AR06 or N03AX17 and J05AR06 or N03AX18 and J05AR06 or N03AX19 and J05AR06 or N03AX21 and J05AR06 or N03AX22 and J05AR06 or N03AX23 and J05AR06 or N03AX24 and J05AR06 or N03AX30 and J05AR06 or N03AA01 and J05AR07 or N03AA02 and J05AR07 or N03AA03 and J05AR07 or N03AA04 and J05AR07 or N03AA30 and J05AR07 or N03AB01 and J05AR07 or N03AB02 and J05AR07 or N03AB03 and J05AR07 or N03AB04 and J05AR07 or N03AB05 and J05AR07 or N03AB52 and J05AR07 or N03AB54 and J05AR07 or N03AC01 and J05AR07 or N03AC02 and J05AR07 or N03AC03 and J05AR07 or N03AD01 and J05AR07 or N03AD02 and J05AR07 or N03AD03 and J05AR07 or N03AD51 and J05AR07 or N03AE01 and J05AR07 or N03AF01 and J05AR07 or N03AF02 and J05AR07 or N03AF03 and J05AR07 or N03AF04 and J05AR07 or N03AG01 and J05AR07 or N03AG02 and J05AR07 or N03AG03 and J05AR07 or N03AG04 and J05AR07 or N03AG05 and J05AR07 or N03AG06 and J05AR07 or N03AX03 and J05AR07 or N03AX07 and J05AR07 or N03AX09 and J05AR07 or N03AX10 and J05AR07 or N03AX11 and J05AR07 or N03AX12 and J05AR07 or N03AX13 and J05AR07 or N03AX14 and J05AR07 or N03AX15 and J05AR07 or N03AX16 and J05AR07 or N03AX17 and J05AR07 or N03AX18 and J05AR07 or N03AX19 and J05AR07 or N03AX21 and J05AR07 or N03AX22 and J05AR07 or N03AX23 and J05AR07 or N03AX24 and J05AR07 or N03AX30 and J05AR07 or N03AA01 and J05AR08 or N03AA02 and J05AR08 or N03AA03 and J05AR08 or N03AA04 and J05AR08 or N03AA30 and J05AR08 or N03AB01 and J05AR08 or N03AB02 and J05AR08 or N03AB03 and J05AR08 or N03AB04 and J05AR08 or N03AB05 and J05AR08 or N03AB52 and J05AR08 or N03AB54 and J05AR08 or N03AC01 and J05AR08 or N03AC02 and J05AR08 or N03AC03 and J05AR08 or N03AD01 and J05AR08 or N03AD02 and J05AR08 or N03AD03 and J05AR08 or N03AD51 and J05AR08 or N03AE01 and J05AR08 or N03AF01 and J05AR08 or N03AF02 and J05AR08 or N03AF03 and J05AR08 or N03AF04 and J05AR08 or N03AG01 and J05AR08 or N03AG02 and J05AR08 or N03AG03 and J05AR08 or N03AG04 and J05AR08 or N03AG05 and J05AR08 or N03AG06 and J05AR08 or N03AX03 and J05AR08 or N03AX07 and J05AR08 or N03AX09 and J05AR08 or N03AX10 and J05AR08 or N03AX11 and J05AR08 or N03AX12 and J05AR08 or N03AX13 and J05AR08 or N03AX14 and J05AR08 or N03AX15 and J05AR08 or N03AX16 and J05AR08 or N03AX17 and J05AR08 or N03AX18 and J05AR08 or N03AX19 and J05AR08 or N03AX21 and J05AR08 or N03AX22 and J05AR08 or N03AX23 and J05AR08 or N03AX24 and J05AR08 or N03AX30 and J05AR08 or N03AA01 and J05AR09 or N03AA02 and J05AR09 or N03AA03 and J05AR09 or N03AA04 and J05AR09 or N03AA30 and J05AR09 or N03AB01 and J05AR09 or N03AB02 and J05AR09 or N03AB03 and J05AR09 or N03AB04 and J05AR09 or N03AB05 and J05AR09 or N03AB52 and J05AR09 or N03AB54 and J05AR09 or N03AC01 and J05AR09 or N03AC02 and J05AR09 or N03AC03 and J05AR09 or N03AD01 and J05AR09 or N03AD02 and J05AR09 or N03AD03 and J05AR09 or N03AD51 and J05AR09 or N03AE01 and J05AR09 or N03AF01 and J05AR09 or N03AF02 and J05AR09 or N03AF03 and J05AR09 or N03AF04 and J05AR09 or N03AG01 and J05AR09 or N03AG02 and J05AR09 or N03AG03 and J05AR09 or N03AG04 and J05AR09 or N03AG05 and J05AR09 or N03AG06 and J05AR09 or N03AX03 and J05AR09 or N03AX07 and J05AR09 or N03AX09 and J05AR09 or N03AX10 and J05AR09 or N03AX11 and J05AR09 or N03AX12 and J05AR09 or N03AX13 and J05AR09 or N03AX14 and J05AR09 or N03AX15 and J05AR09 or N03AX16 and J05AR09 or N03AX17 and J05AR09 or N03AX18 and J05AR09 or N03AX19 and J05AR09 or N03AX21 and J05AR09 or N03AX22 and J05AR09 or N03AX23 and J05AR09 or N03AX24 and J05AR09 or N03AX30 and J05AR09 or N03AA01 and J05AR10 or N03AA02 and J05AR10 or N03AA03 and J05AR10 or N03AA04 and J05AR10 or N03AA30 and J05AR10 or N03AB01 and J05AR10 or N03AB02 and J05AR10 or N03AB03 and J05AR10 or N03AB04 and J05AR10 or N03AB05 and J05AR10 or N03AB52 and J05AR10 or N03AB54 and J05AR10 or N03AC01 and J05AR10 or N03AC02 and J05AR10 or N03AC03 and J05AR10 or N03AD01 and J05AR10 or N03AD02 and J05AR10 or N03AD03 and J05AR10 or N03AD51 and J05AR10 or N03AE01 and J05AR10 or N03AF01 and J05AR10 or N03AF02 and J05AR10 or N03AF03 and J05AR10 or N03AF04 and J05AR10 or N03AG01 and J05AR10 or N03AG02 and J05AR10 or N03AG03 and J05AR10 or N03AG04 and J05AR10 or N03AG05 and J05AR10 or N03AG06 and J05AR10 or N03AX03 and J05AR10 or N03AX07 and J05AR10 or N03AX09 and J05AR10 or N03AX10 and J05AR10 or N03AX11 and J05AR10 or N03AX12 and J05AR10 or N03AX13 and J05AR10 or N03AX14 and J05AR10 or N03AX15 and J05AR10 or N03AX16 and J05AR10 or N03AX17 and J05AR10 or N03AX18 and J05AR10 or N03AX19 and J05AR10 or N03AX21 and J05AR10 or N03AX22 and J05AR10 or N03AX23 and J05AR10 or N03AX24 and J05AR10 or N03AX30 and J05AR10 or N03AA01 and J05AR11 or N03AA02 and J05AR11 or N03AA03 and J05AR11 or N03AA04 and J05AR11 or N03AA30 and J05AR11 or N03AB01 and J05AR11 or N03AB02 and J05AR11 or N03AB03 and J05AR11 or N03AB04 and J05AR11 or N03AB05 and J05AR11 or N03AB52 and J05AR11 or N03AB54 and J05AR11 or N03AC01 and J05AR11 or N03AC02 and J05AR11 or N03AC03 and J05AR11 or N03AD01 and J05AR11 or N03AD02 and J05AR11 or N03AD03 and J05AR11 or N03AD51 and J05AR11 or N03AE01 and J05AR11 or N03AF01 and J05AR11 or N03AF02 and J05AR11 or N03AF03 and J05AR11 or N03AF04 and J05AR11 or N03AG01 and J05AR11 or N03AG02 and J05AR11 or N03AG03 and J05AR11 or N03AG04 and J05AR11 or N03AG05 and J05AR11 or N03AG06 and J05AR11 or N03AX03 and J05AR11 or N03AX07 and J05AR11 or N03AX09 and J05AR11 or N03AX10 and J05AR11 or N03AX11 and J05AR11 or N03AX12 and J05AR11 or N03AX13 and J05AR11 or N03AX14 and J05AR11 or N03AX15 and J05AR11 or N03AX16 and J05AR11 or N03AX17 and J05AR11 or N03AX18 and J05AR11 or N03AX19 and J05AR11 or N03AX21 and J05AR11 or N03AX22 and J05AR11 or N03AX23 and J05AR11 or N03AX24 and J05AR11 or N03AX30 and J05AR11 or N03AA01 and J05AR12 or N03AA02 and J05AR12 or N03AA03 and J05AR12 or N03AA04 and J05AR12 or N03AA30 and J05AR12 or N03AB01 and J05AR12 or N03AB02 and J05AR12 or N03AB03 and J05AR12 or N03AB04 and J05AR12 or N03AB05 and J05AR12 or N03AB52 and J05AR12 or N03AB54 and J05AR12 or N03AC01 and J05AR12 or N03AC02 and J05AR12 or N03AC03 and J05AR12 or N03AD01 and J05AR12 or N03AD02 and J05AR12 or N03AD03 and J05AR12 or N03AD51 and J05AR12 or N03AE01 and J05AR12 or N03AF01 and J05AR12 or N03AF02 and J05AR12 or N03AF03 and J05AR12 or N03AF04 and J05AR12 or N03AG01 and J05AR12 or N03AG02 and J05AR12 or N03AG03 and J05AR12 or N03AG04 and J05AR12 or N03AG05 and J05AR12 or N03AG06 and J05AR12 or N03AX03 and J05AR12 or N03AX07 and J05AR12 or N03AX09 and J05AR12 or N03AX10 and J05AR12 or N03AX11 and J05AR12 or N03AX12 and J05AR12 or N03AX13 and J05AR12 or N03AX14 and J05AR12 or N03AX15 and J05AR12 or N03AX16 and J05AR12 or N03AX17 and J05AR12 or N03AX18 and J05AR12 or N03AX19 and J05AR12 or N03AX21 and J05AR12 or N03AX22 and J05AR12 or N03AX23 and J05AR12 or N03AX24 and J05AR12 or N03AX30 and J05AR12 or N03AA01 and J05AR13 or N03AA02 and J05AR13 or N03AA03 and J05AR13 or N03AA04 and J05AR13 or N03AA30 and J05AR13 or N03AB01 and J05AR13 or N03AB02 and J05AR13 or N03AB03 and J05AR13 or N03AB04 and J05AR13 or N03AB05 and J05AR13 or N03AB52 and J05AR13 or N03AB54 and J05AR13 or N03AC01 and J05AR13 or N03AC02 and J05AR13 or N03AC03 and J05AR13 or N03AD01 and J05AR13 or N03AD02 and J05AR13 or N03AD03 and J05AR13 or N03AD51 and J05AR13 or N03AE01 and J05AR13 or N03AF01 and J05AR13 or N03AF02 and J05AR13 or N03AF03 and J05AR13 or N03AF04 and J05AR13 or N03AG01 and J05AR13 or N03AG02 and J05AR13 or N03AG03 and J05AR13 or N03AG04 and J05AR13 or N03AG05 and J05AR13 or N03AG06 and J05AR13 or N03AX03 and J05AR13 or N03AX07 and J05AR13 or N03AX09 and J05AR13 or N03AX10 and J05AR13 or N03AX11 and J05AR13 or N03AX12 and J05AR13 or N03AX13 and J05AR13 or N03AX14 and J05AR13 or N03AX15 and J05AR13 or N03AX16 and J05AR13 or N03AX17 and J05AR13 or N03AX18 and J05AR13 or N03AX19 and J05AR13 or N03AX21 and J05AR13 or N03AX22 and J05AR13 or N03AX23 and J05AR13 or N03AX24 and J05AR13 or N03AX30 and J05AR13 or N03AA01 and J05AR14 or N03AA02 and J05AR14 or N03AA03 and J05AR14 or N03AA04 and J05AR14 or N03AA30 and J05AR14 or N03AB01 and J05AR14 or N03AB02 and J05AR14 or N03AB03 and J05AR14 or N03AB04 and J05AR14 or N03AB05 and J05AR14 or N03AB52 and J05AR14 or N03AB54 and J05AR14 or N03AC01 and J05AR14 or N03AC02 and J05AR14 or N03AC03 and J05AR14 or N03AD01 and J05AR14 or N03AD02 and J05AR14 or N03AD03 and J05AR14 or N03AD51 and J05AR14 or N03AE01 and J05AR14 or N03AF01 and J05AR14 or N03AF02 and J05AR14 or N03AF03 and J05AR14 or N03AF04 and J05AR14 or N03AG01 and J05AR14 or N03AG02 and J05AR14 or N03AG03 and J05AR14 or N03AG04 and J05AR14 or N03AG05 and J05AR14 or N03AG06 and J05AR14 or N03AX03 and J05AR14 or N03AX07 and J05AR14 or N03AX09 and J05AR14 or N03AX10 and J05AR14 or N03AX11 and J05AR14 or N03AX12 and J05AR14 or N03AX13 and J05AR14 or N03AX14 and J05AR14 or N03AX15 and J05AR14 or N03AX16 and J05AR14 or N03AX17 and J05AR14 or N03AX18 and J05AR14 or N03AX19 and J05AR14 or N03AX21 and J05AR14 or N03AX22 and J05AR14 or N03AX23 and J05AR14 or N03AX24 and J05AR14 or N03AX30 and J05AR14 or N03AA01 and J05AR15 or N03AA02 and J05AR15 or N03AA03 and J05AR15 or N03AA04 and J05AR15 or N03AA30 and J05AR15 or N03AB01 and J05AR15 or N03AB02 and J05AR15 or N03AB03 and J05AR15 or N03AB04 and J05AR15 or N03AB05 and J05AR15 or N03AB52 and J05AR15 or N03AB54 and J05AR15 or N03AC01 and J05AR15 or N03AC02 and J05AR15 or N03AC03 and J05AR15 or N03AD01 and J05AR15 or N03AD02 and J05AR15 or N03AD03 and J05AR15 or N03AD51 and J05AR15 or N03AE01 and J05AR15 or N03AF01 and J05AR15 or N03AF02 and J05AR15 or N03AF03 and J05AR15 or N03AF04 and J05AR15 or N03AG01 and J05AR15 or N03AG02 and J05AR15 or N03AG03 and J05AR15 or N03AG04 and J05AR15 or N03AG05 and J05AR15 or N03AG06 and J05AR15 or N03AX03 and J05AR15 or N03AX07 and J05AR15 or N03AX09 and J05AR15 or N03AX10 and J05AR15 or N03AX11 and J05AR15 or N03AX12 and J05AR15 or N03AX13 and J05AR15 or N03AX14 and J05AR15 or N03AX15 and J05AR15 or N03AX16 and J05AR15 or N03AX17 and J05AR15 or N03AX18 and J05AR15 or N03AX19 and J05AR15 or N03AX21 and J05AR15 or N03AX22 and J05AR15 or N03AX23 and J05AR15 or N03AX24 and J05AR15 or N03AX30 and J05AR15 or N03AA01 and J05AR16 or N03AA02 and J05AR16 or N03AA03 and J05AR16 or N03AA04 and J05AR16 or N03AA30 and J05AR16 or N03AB01 and J05AR16 or N03AB02 and J05AR16 or N03AB03 and J05AR16 or N03AB04 and J05AR16 or N03AB05 and J05AR16 or N03AB52 and J05AR16 or N03AB54 and J05AR16 or N03AC01 and J05AR16 or N03AC02 and J05AR16 or N03AC03 and J05AR16 or N03AD01 and J05AR16 or N03AD02 and J05AR16 or N03AD03 and J05AR16 or N03AD51 and J05AR16 or N03AE01 and J05AR16 or N03AF01 and J05AR16 or N03AF02 and J05AR16 or N03AF03 and J05AR16 or N03AF04 and J05AR16 or N03AG01 and J05AR16 or N03AG02 and J05AR16 or N03AG03 and J05AR16 or N03AG04 and J05AR16 or N03AG05 and J05AR16 or N03AG06 and J05AR16 or N03AX03 and J05AR16 or N03AX07 and J05AR16 or N03AX09 and J05AR16 or N03AX10 and J05AR16 or N03AX11 and J05AR16 or N03AX12 and J05AR16 or N03AX13 and J05AR16 or N03AX14 and J05AR16 or N03AX15 and J05AR16 or N03AX16 and J05AR16 or N03AX17 and J05AR16 or N03AX18 and J05AR16 or N03AX19 and J05AR16 or N03AX21 and J05AR16 or N03AX22 and J05AR16 or N03AX23 and J05AR16 or N03AX24 and J05AR16 or N03AX30 and J05AR16 or N03AA01 and J05AR17 or N03AA02 and J05AR17 or N03AA03 and J05AR17 or N03AA04 and J05AR17 or N03AA30 and J05AR17 or N03AB01 and J05AR17 or N03AB02 and J05AR17 or N03AB03 and J05AR17 or N03AB04 and J05AR17 or N03AB05 and J05AR17 or N03AB52 and J05AR17 or N03AB54 and J05AR17 or N03AC01 and J05AR17 or N03AC02 and J05AR17 or N03AC03 and J05AR17 or N03AD01 and J05AR17 or N03AD02 and J05AR17 or N03AD03 and J05AR17 or N03AD51 and J05AR17 or N03AE01 and J05AR17 or N03AF01 and J05AR17 or N03AF02 and J05AR17 or N03AF03 and J05AR17 or N03AF04 and J05AR17 or N03AG01 and J05AR17 or N03AG02 and J05AR17 or N03AG03 and J05AR17 or N03AG04 and J05AR17 or N03AG05 and J05AR17 or N03AG06 and J05AR17 or N03AX03 and J05AR17 or N03AX07 and J05AR17 or N03AX09 and J05AR17 or N03AX10 and J05AR17 or N03AX11 and J05AR17 or N03AX12 and J05AR17 or N03AX13 and J05AR17 or N03AX14 and J05AR17 or N03AX15 and J05AR17 or N03AX16 and J05AR17 or N03AX17 and J05AR17 or N03AX18 and J05AR17 or N03AX19 and J05AR17 or N03AX21 and J05AR17 or N03AX22 and J05AR17 or N03AX23 and J05AR17 or N03AX24 and J05AR17 or N03AX30 and J05AR17 or N03AA01 and J05AR18 or N03AA02 and J05AR18 or N03AA03 and J05AR18 or N03AA04 and J05AR18 or N03AA30 and J05AR18 or N03AB01 and J05AR18 or N03AB02 and J05AR18 or N03AB03 and J05AR18 or N03AB04 and J05AR18 or N03AB05 and J05AR18 or N03AB52 and J05AR18 or N03AB54 and J05AR18 or N03AC01 and J05AR18 or N03AC02 and J05AR18 or N03AC03 and J05AR18 or N03AD01 and J05AR18 or N03AD02 and J05AR18 or N03AD03 and J05AR18 or N03AD51 and J05AR18 or N03AE01 and J05AR18 or N03AF01 and J05AR18 or N03AF02 and J05AR18 or N03AF03 and J05AR18 or N03AF04 and J05AR18 or N03AG01 and J05AR18 or N03AG02 and J05AR18 or N03AG03 and J05AR18 or N03AG04 and J05AR18 or N03AG05 and J05AR18 or N03AG06 and J05AR18 or N03AX03 and J05AR18 or N03AX07 and J05AR18 or N03AX09 and J05AR18 or N03AX10 and J05AR18 or N03AX11 and J05AR18 or N03AX12 and J05AR18 or N03AX13 and J05AR18 or N03AX14 and J05AR18 or N03AX15 and J05AR18 or N03AX16 and J05AR18 or N03AX17 and J05AR18 or N03AX18 and J05AR18 or N03AX19 and J05AR18 or N03AX21 and J05AR18 or N03AX22 and J05AR18 or N03AX23 and J05AR18 or N03AX24 and J05AR18 or N03AX30 and J05AR18 or N03AA01 and J05AR19 or N03AA02 and J05AR19 or N03AA03 and J05AR19 or N03AA04 and J05AR19 or N03AA30 and J05AR19 or N03AB01 and J05AR19 or N03AB02 and J05AR19 or N03AB03 and J05AR19 or N03AB04 and J05AR19 or N03AB05 and J05AR19 or N03AB52 and J05AR19 or N03AB54 and J05AR19 or N03AC01 and J05AR19 or N03AC02 and J05AR19 or N03AC03 and J05AR19 or N03AD01 and J05AR19 or N03AD02 and J05AR19 or N03AD03 and J05AR19 or N03AD51 and J05AR19 or N03AE01 and J05AR19 or N03AF01 and J05AR19 or N03AF02 and J05AR19 or N03AF03 and J05AR19 or N03AF04 and J05AR19 or N03AG01 and J05AR19 or N03AG02 and J05AR19 or N03AG03 and J05AR19 or N03AG04 and J05AR19 or N03AG05 and J05AR19 or N03AG06 and J05AR19 or N03AX03 and J05AR19 or N03AX07 and J05AR19 or N03AX09 and J05AR19 or N03AX10 and J05AR19 or N03AX11 and J05AR19 or N03AX12 and J05AR19 or N03AX13 and J05AR19 or N03AX14 and J05AR19 or N03AX15 and J05AR19 or N03AX16 and J05AR19 or N03AX17 and J05AR19 or N03AX18 and J05AR19 or N03AX19 and J05AR19 or N03AX21 and J05AR19 or N03AX22 and J05AR19 or N03AX23 and J05AR19 or N03AX24 and J05AR19 or N03AX30 and J05AR19 or N03AA01 and J05AR20 or N03AA02 and J05AR20 or N03AA03 and J05AR20 or N03AA04 and J05AR20 or N03AA30 and J05AR20 or N03AB01 and J05AR20 or N03AB02 and J05AR20 or N03AB03 and J05AR20 or N03AB04 and J05AR20 or N03AB05 and J05AR20 or N03AB52 and J05AR20 or N03AB54 and J05AR20 or N03AC01 and J05AR20 or N03AC02 and J05AR20 or N03AC03 and J05AR20 or N03AD01 and J05AR20 or N03AD02 and J05AR20 or N03AD03 and J05AR20 or N03AD51 and J05AR20 or N03AE01 and J05AR20 or N03AF01 and J05AR20 or N03AF02 and J05AR20 or N03AF03 and J05AR20 or N03AF04 and J05AR20 or N03AG01 and J05AR20 or N03AG02 and J05AR20 or N03AG03 and J05AR20 or N03AG04 and J05AR20 or N03AG05 and J05AR20 or N03AG06 and J05AR20 or N03AX03 and J05AR20 or N03AX07 and J05AR20 or N03AX09 and J05AR20 or N03AX10 and J05AR20 or N03AX11 and J05AR20 or N03AX12 and J05AR20 or N03AX13 and J05AR20 or N03AX14 and J05AR20 or N03AX15 and J05AR20 or N03AX16 and J05AR20 or N03AX17 and J05AR20 or N03AX18 and J05AR20 or N03AX19 and J05AR20 or N03AX21 and J05AR20 or N03AX22 and J05AR20 or N03AX23 and J05AR20 or N03AX24 and J05AR20 or N03AX30 and J05AR20 or N03AA01 and J05AR21 or N03AA02 and J05AR21 or N03AA03 and J05AR21 or N03AA04 and J05AR21 or N03AA30 and J05AR21 or N03AB01 and J05AR21 or N03AB02 and J05AR21 or N03AB03 and J05AR21 or N03AB04 and J05AR21 or N03AB05 and J05AR21 or N03AB52 and J05AR21 or N03AB54 and J05AR21 or N03AC01 and J05AR21 or N03AC02 and J05AR21 or N03AC03 and J05AR21 or N03AD01 and J05AR21 or N03AD02 and J05AR21 or N03AD03 and J05AR21 or N03AD51 and J05AR21 or N03AE01 and J05AR21 or N03AF01 and J05AR21 or N03AF02 and J05AR21 or N03AF03 and J05AR21 or N03AF04 and J05AR21 or N03AG01 and J05AR21 or N03AG02 and J05AR21 or N03AG03 and J05AR21 or N03AG04 and J05AR21 or N03AG05 and J05AR21 or N03AG06 and J05AR21 or N03AX03 and J05AR21 or N03AX07 and J05AR21 or N03AX09 and J05AR21 or N03AX10 and J05AR21 or N03AX11 and J05AR21 or N03AX12 and J05AR21 or N03AX13 and J05AR21 or N03AX14 and J05AR21 or N03AX15 and J05AR21 or N03AX16 and J05AR21 or N03AX17 and J05AR21 or N03AX18 and J05AR21 or N03AX19 and J05AR21 or N03AX21 and J05AR21 or N03AX22 and J05AR21 or N03AX23 and J05AR21 or N03AX24 and J05AR21 or N03AX30 and J05AR21 or N03AA01 and J05AR22 or N03AA02 and J05AR22 or N03AA03 and J05AR22 or N03AA04 and J05AR22 or N03AA30 and J05AR22 or N03AB01 and J05AR22 or N03AB02 and J05AR22 or N03AB03 and J05AR22 or N03AB04 and J05AR22 or N03AB05 and J05AR22 or N03AB52 and J05AR22 or N03AB54 and J05AR22 or N03AC01 and J05AR22 or N03AC02 and J05AR22 or N03AC03 and J05AR22 or N03AD01 and J05AR22 or N03AD02 and J05AR22 or N03AD03 and J05AR22 or N03AD51 and J05AR22 or N03AE01 and J05AR22 or N03AF01 and J05AR22 or N03AF02 and J05AR22 or N03AF03 and J05AR22 or N03AF04 and J05AR22 or N03AG01 and J05AR22 or N03AG02 and J05AR22 or N03AG03 and J05AR22 or N03AG04 and J05AR22 or N03AG05 and J05AR22 or N03AG06 and J05AR22 or N03AX03 and J05AR22 or N03AX07 and J05AR22 or N03AX09 and J05AR22 or N03AX10 and J05AR22 or N03AX11 and J05AR22 or N03AX12 and J05AR22 or N03AX13 and J05AR22 or N03AX14 and J05AR22 or N03AX15 and J05AR22 or N03AX16 and J05AR22 or N03AX17 and J05AR22 or N03AX18 and J05AR22 or N03AX19 and J05AR22 or N03AX21 and J05AR22 or N03AX22 and J05AR22 or N03AX23 and J05AR22 or N03AX24 and J05AR22 or N03AX30 and J05AR22 or N03AA01 and J05AR23 or N03AA02 and J05AR23 or N03AA03 and J05AR23 or N03AA04 and J05AR23 or N03AA30 and J05AR23 or N03AB01 and J05AR23 or N03AB02 and J05AR23 or N03AB03 and J05AR23 or N03AB04 and J05AR23 or N03AB05 and J05AR23 or N03AB52 and J05AR23 or N03AB54 and J05AR23 or N03AC01 and J05AR23 or N03AC02 and J05AR23 or N03AC03 and J05AR23 or N03AD01 and J05AR23 or N03AD02 and J05AR23 or N03AD03 and J05AR23 or N03AD51 and J05AR23 or N03AE01 and J05AR23 or N03AF01 and J05AR23 or N03AF02 and J05AR23 or N03AF03 and J05AR23 or N03AF04 and J05AR23 or N03AG01 and J05AR23 or N03AG02 and J05AR23 or N03AG03 and J05AR23 or N03AG04 and J05AR23 or N03AG05 and J05AR23 or N03AG06 and J05AR23 or N03AX03 and J05AR23 or N03AX07 and J05AR23 or N03AX09 and J05AR23 or N03AX10 and J05AR23 or N03AX11 and J05AR23 or N03AX12 and J05AR23 or N03AX13 and J05AR23 or N03AX14 and J05AR23 or N03AX15 and J05AR23 or N03AX16 and J05AR23 or N03AX17 and J05AR23 or N03AX18 and J05AR23 or N03AX19 and J05AR23 or N03AX21 and J05AR23 or N03AX22 and J05AR23 or N03AX23 and J05AR23 or N03AX24 and J05AR23 or N03AX30 and J05AR23 or N03AA01 and J05AR24 or N03AA02 and J05AR24 or N03AA03 and J05AR24 or N03AA04 and J05AR24 or N03AA30 and J05AR24 or N03AB01 and J05AR24 or N03AB02 and J05AR24 or N03AB03 and J05AR24 or N03AB04 and J05AR24 or N03AB05 and J05AR24 or N03AB52 and J05AR24 or N03AB54 and J05AR24 or N03AC01 and J05AR24 or N03AC02 and J05AR24 or N03AC03 and J05AR24 or N03AD01 and J05AR24 or N03AD02 and J05AR24 or N03AD03 and J05AR24 or N03AD51 and J05AR24 or N03AE01 and J05AR24 or N03AF01 and J05AR24 or N03AF02 and J05AR24 or N03AF03 and J05AR24 or N03AF04 and J05AR24 or N03AG01 and J05AR24 or N03AG02 and J05AR24 or N03AG03 and J05AR24 or N03AG04 and J05AR24 or N03AG05 and J05AR24 or N03AG06 and J05AR24 or N03AX03 and J05AR24 or N03AX07 and J05AR24 or N03AX09 and J05AR24 or N03AX10 and J05AR24 or N03AX11 and J05AR24 or N03AX12 and J05AR24 or N03AX13 and J05AR24 or N03AX14 and J05AR24 or N03AX15 and J05AR24 or N03AX16 and J05AR24 or N03AX17 and J05AR24 or N03AX18 and J05AR24 or N03AX19 and J05AR24 or N03AX21 and J05AR24 or N03AX22 and J05AR24 or N03AX23 and J05AR24 or N03AX24 and J05AR24 or N03AX30 and J05AR24 or N03AA01 and J05AX01 or N03AA02 and J05AX01 or N03AA03 and J05AX01 or N03AA04 and J05AX01 or N03AA30 and J05AX01 or N03AB01 and J05AX01 or N03AB02 and J05AX01 or N03AB03 and J05AX01 or N03AB04 and J05AX01 or N03AB05 and J05AX01 or N03AB52 and J05AX01 or N03AB54 and J05AX01 or N03AC01 and J05AX01 or N03AC02 and J05AX01 or N03AC03 and J05AX01 or N03AD01 and J05AX01 or N03AD02 and J05AX01 or N03AD03 and J05AX01 or N03AD51 and J05AX01 or N03AE01 and J05AX01 or N03AF01 and J05AX01 or N03AF02 and J05AX01 or N03AF03 and J05AX01 or N03AF04 and J05AX01 or N03AG01 and J05AX01 or N03AG02 and J05AX01 or N03AG03 and J05AX01 or N03AG04 and J05AX01 or N03AG05 and J05AX01 or N03AG06 and J05AX01 or N03AX03 and J05AX01 or N03AX07 and J05AX01 or N03AX09 and J05AX01 or N03AX10 and J05AX01 or N03AX11 and J05AX01 or N03AX12 and J05AX01 or N03AX13 and J05AX01 or N03AX14 and J05AX01 or N03AX15 and J05AX01 or N03AX16 and J05AX01 or N03AX17 and J05AX01 or N03AX18 and J05AX01 or N03AX19 and J05AX01 or N03AX21 and J05AX01 or N03AX22 and J05AX01 or N03AX23 and J05AX01 or N03AX24 and J05AX01 or N03AX30 and J05AX01 or N03AA01 and J05AX02 or N03AA02 and J05AX02 or N03AA03 and J05AX02 or N03AA04 and J05AX02 or N03AA30 and J05AX02 or N03AB01 and J05AX02 or N03AB02 and J05AX02 or N03AB03 and J05AX02 or N03AB04 and J05AX02 or N03AB05 and J05AX02 or N03AB52 and J05AX02 or N03AB54 and J05AX02 or N03AC01 and J05AX02 or N03AC02 and J05AX02 or N03AC03 and J05AX02 or N03AD01 and J05AX02 or N03AD02 and J05AX02 or N03AD03 and J05AX02 or N03AD51 and J05AX02 or N03AE01 and J05AX02 or N03AF01 and J05AX02 or N03AF02 and J05AX02 or N03AF03 and J05AX02 or N03AF04 and J05AX02 or N03AG01 and J05AX02 or N03AG02 and J05AX02 or N03AG03 and J05AX02 or N03AG04 and J05AX02 or N03AG05 and J05AX02 or N03AG06 and J05AX02 or N03AX03 and J05AX02 or N03AX07 and J05AX02 or N03AX09 and J05AX02 or N03AX10 and J05AX02 or N03AX11 and J05AX02 or N03AX12 and J05AX02 or N03AX13 and J05AX02 or N03AX14 and J05AX02 or N03AX15 and J05AX02 or N03AX16 and J05AX02 or N03AX17 and J05AX02 or N03AX18 and J05AX02 or N03AX19 and J05AX02 or N03AX21 and J05AX02 or N03AX22 and J05AX02 or N03AX23 and J05AX02 or N03AX24 and J05AX02 or N03AX30 and J05AX02 or N03AA01 and J05AX05 or N03AA02 and J05AX05 or N03AA03 and J05AX05 or N03AA04 and J05AX05 or N03AA30 and J05AX05 or N03AB01 and J05AX05 or N03AB02 and J05AX05 or N03AB03 and J05AX05 or N03AB04 and J05AX05 or N03AB05 and J05AX05 or N03AB52 and J05AX05 or N03AB54 and J05AX05 or N03AC01 and J05AX05 or N03AC02 and J05AX05 or N03AC03 and J05AX05 or N03AD01 and J05AX05 or N03AD02 and J05AX05 or N03AD03 and J05AX05 or N03AD51 and J05AX05 or N03AE01 and J05AX05 or N03AF01 and J05AX05 or N03AF02 and J05AX05 or N03AF03 and J05AX05 or N03AF04 and J05AX05 or N03AG01 and J05AX05 or N03AG02 and J05AX05 or N03AG03 and J05AX05 or N03AG04 and J05AX05 or N03AG05 and J05AX05 or N03AG06 and J05AX05 or N03AX03 and J05AX05 or N03AX07 and J05AX05 or N03AX09 and J05AX05 or N03AX10 and J05AX05 or N03AX11 and J05AX05 or N03AX12 and J05AX05 or N03AX13 and J05AX05 or N03AX14 and J05AX05 or N03AX15 and J05AX05 or N03AX16 and J05AX05 or N03AX17 and J05AX05 or N03AX18 and J05AX05 or N03AX19 and J05AX05 or N03AX21 and J05AX05 or N03AX22 and J05AX05 or N03AX23 and J05AX05 or N03AX24 and J05AX05 or N03AX30 and J05AX05 or N03AA01 and J05AX06 or N03AA02 and J05AX06 or N03AA03 and J05AX06 or N03AA04 and J05AX06 or N03AA30 and J05AX06 or N03AB01 and J05AX06 or N03AB02 and J05AX06 or N03AB03 and J05AX06 or N03AB04 and J05AX06 or N03AB05 and J05AX06 or N03AB52 and J05AX06 or N03AB54 and J05AX06 or N03AC01 and J05AX06 or N03AC02 and J05AX06 or N03AC03 and J05AX06 or N03AD01 and J05AX06 or N03AD02 and J05AX06 or N03AD03 and J05AX06 or N03AD51 and J05AX06 or N03AE01 and J05AX06 or N03AF01 and J05AX06 or N03AF02 and J05AX06 or N03AF03 and J05AX06 or N03AF04 and J05AX06 or N03AG01 and J05AX06 or N03AG02 and J05AX06 or N03AG03 and J05AX06 or N03AG04 and J05AX06 or N03AG05 and J05AX06 or N03AG06 and J05AX06 or N03AX03 and J05AX06 or N03AX07 and J05AX06 or N03AX09 and J05AX06 or N03AX10 and J05AX06 or N03AX11 and J05AX06 or N03AX12 and J05AX06 or N03AX13 and J05AX06 or N03AX14 and J05AX06 or N03AX15 and J05AX06 or N03AX16 and J05AX06 or N03AX17 and J05AX06 or N03AX18 and J05AX06 or N03AX19 and J05AX06 or N03AX21 and J05AX06 or N03AX22 and J05AX06 or N03AX23 and J05AX06 or N03AX24 and J05AX06 or N03AX30 and J05AX06 or N03AA01 and J05AX07 or N03AA02 and J05AX07 or N03AA03 and J05AX07 or N03AA04 and J05AX07 or N03AA30 and J05AX07 or N03AB01 and J05AX07 or N03AB02 and J05AX07 or N03AB03 and J05AX07 or N03AB04 and J05AX07 or N03AB05 and J05AX07 or N03AB52 and J05AX07 or N03AB54 and J05AX07 or N03AC01 and J05AX07 or N03AC02 and J05AX07 or N03AC03 and J05AX07 or N03AD01 and J05AX07 or N03AD02 and J05AX07 or N03AD03 and J05AX07 or N03AD51 and J05AX07 or N03AE01 and J05AX07 or N03AF01 and J05AX07 or N03AF02 and J05AX07 or N03AF03 and J05AX07 or N03AF04 and J05AX07 or N03AG01 and J05AX07 or N03AG02 and J05AX07 or N03AG03 and J05AX07 or N03AG04 and J05AX07 or N03AG05 and J05AX07 or N03AG06 and J05AX07 or N03AX03 and J05AX07 or N03AX07 and J05AX07 or N03AX09 and J05AX07 or N03AX10 and J05AX07 or N03AX11 and J05AX07 or N03AX12 and J05AX07 or N03AX13 and J05AX07 or N03AX14 and J05AX07 or N03AX15 and J05AX07 or N03AX16 and J05AX07 or N03AX17 and J05AX07 or N03AX18 and J05AX07 or N03AX19 and J05AX07 or N03AX21 and J05AX07 or N03AX22 and J05AX07 or N03AX23 and J05AX07 or N03AX24 and J05AX07 or N03AX30 and J05AX07 or N03AA01 and J05AX08 or N03AA02 and J05AX08 or N03AA03 and J05AX08 or N03AA04 and J05AX08 or N03AA30 and J05AX08 or N03AB01 and J05AX08 or N03AB02 and J05AX08 or N03AB03 and J05AX08 or N03AB04 and J05AX08 or N03AB05 and J05AX08 or N03AB52 and J05AX08 or N03AB54 and J05AX08 or N03AC01 and J05AX08 or N03AC02 and J05AX08 or N03AC03 and J05AX08 or N03AD01 and J05AX08 or N03AD02 and J05AX08 or N03AD03 and J05AX08 or N03AD51 and J05AX08 or N03AE01 and J05AX08 or N03AF01 and J05AX08 or N03AF02 and J05AX08 or N03AF03 and J05AX08 or N03AF04 and J05AX08 or N03AG01 and J05AX08 or N03AG02 and J05AX08 or N03AG03 and J05AX08 or N03AG04 and J05AX08 or N03AG05 and J05AX08 or N03AG06 and J05AX08 or N03AX03 and J05AX08 or N03AX07 and J05AX08 or N03AX09 and J05AX08 or N03AX10 and J05AX08 or N03AX11 and J05AX08 or N03AX12 and J05AX08 or N03AX13 and J05AX08 or N03AX14 and J05AX08 or N03AX15 and J05AX08 or N03AX16 and J05AX08 or N03AX17 and J05AX08 or N03AX18 and J05AX08 or N03AX19 and J05AX08 or N03AX21 and J05AX08 or N03AX22 and J05AX08 or N03AX23 and J05AX08 or N03AX24 and J05AX08 or N03AX30 and J05AX08 or N03AA01 and J05AX09 or N03AA02 and J05AX09 or N03AA03 and J05AX09 or N03AA04 and J05AX09 or N03AA30 and J05AX09 or N03AB01 and J05AX09 or N03AB02 and J05AX09 or N03AB03 and J05AX09 or N03AB04 and J05AX09 or N03AB05 and J05AX09 or N03AB52 and J05AX09 or N03AB54 and J05AX09 or N03AC01 and J05AX09 or N03AC02 and J05AX09 or N03AC03 and J05AX09 or N03AD01 and J05AX09 or N03AD02 and J05AX09 or N03AD03 and J05AX09 or N03AD51 and J05AX09 or N03AE01 and J05AX09 or N03AF01 and J05AX09 or N03AF02 and J05AX09 or N03AF03 and J05AX09 or N03AF04 and J05AX09 or N03AG01 and J05AX09 or N03AG02 and J05AX09 or N03AG03 and J05AX09 or N03AG04 and J05AX09 or N03AG05 and J05AX09 or N03AG06 and J05AX09 or N03AX03 and J05AX09 or N03AX07 and J05AX09 or N03AX09 and J05AX09 or N03AX10 and J05AX09 or N03AX11 and J05AX09 or N03AX12 and J05AX09 or N03AX13 and J05AX09 or N03AX14 and J05AX09 or N03AX15 and J05AX09 or N03AX16 and J05AX09 or N03AX17 and J05AX09 or N03AX18 and J05AX09 or N03AX19 and J05AX09 or N03AX21 and J05AX09 or N03AX22 and J05AX09 or N03AX23 and J05AX09 or N03AX24 and J05AX09 or N03AX30 and J05AX09 or N03AA01 and J05AX10 or N03AA02 and J05AX10 or N03AA03 and J05AX10 or N03AA04 and J05AX10 or N03AA30 and J05AX10 or N03AB01 and J05AX10 or N03AB02 and J05AX10 or N03AB03 and J05AX10 or N03AB04 and J05AX10 or N03AB05 and J05AX10 or N03AB52 and J05AX10 or N03AB54 and J05AX10 or N03AC01 and J05AX10 or N03AC02 and J05AX10 or N03AC03 and J05AX10 or N03AD01 and J05AX10 or N03AD02 and J05AX10 or N03AD03 and J05AX10 or N03AD51 and J05AX10 or N03AE01 and J05AX10 or N03AF01 and J05AX10 or N03AF02 and J05AX10 or N03AF03 and J05AX10 or N03AF04 and J05AX10 or N03AG01 and J05AX10 or N03AG02 and J05AX10 or N03AG03 and J05AX10 or N03AG04 and J05AX10 or N03AG05 and J05AX10 or N03AG06 and J05AX10 or N03AX03 and J05AX10 or N03AX07 and J05AX10 or N03AX09 and J05AX10 or N03AX10 and J05AX10 or N03AX11 and J05AX10 or N03AX12 and J05AX10 or N03AX13 and J05AX10 or N03AX14 and J05AX10 or N03AX15 and J05AX10 or N03AX16 and J05AX10 or N03AX17 and J05AX10 or N03AX18 and J05AX10 or N03AX19 and J05AX10 or N03AX21 and J05AX10 or N03AX22 and J05AX10 or N03AX23 and J05AX10 or N03AX24 and J05AX10 or N03AX30 and J05AX10 or N03AA01 and J05AX11 or N03AA02 and J05AX11 or N03AA03 and J05AX11 or N03AA04 and J05AX11 or N03AA30 and J05AX11 or N03AB01 and J05AX11 or N03AB02 and J05AX11 or N03AB03 and J05AX11 or N03AB04 and J05AX11 or N03AB05 and J05AX11 or N03AB52 and J05AX11 or N03AB54 and J05AX11 or N03AC01 and J05AX11 or N03AC02 and J05AX11 or N03AC03 and J05AX11 or N03AD01 and J05AX11 or N03AD02 and J05AX11 or N03AD03 and J05AX11 or N03AD51 and J05AX11 or N03AE01 and J05AX11 or N03AF01 and J05AX11 or N03AF02 and J05AX11 or N03AF03 and J05AX11 or N03AF04 and J05AX11 or N03AG01 and J05AX11 or N03AG02 and J05AX11 or N03AG03 and J05AX11 or N03AG04 and J05AX11 or N03AG05 and J05AX11 or N03AG06 and J05AX11 or N03AX03 and J05AX11 or N03AX07 and J05AX11 or N03AX09 and J05AX11 or N03AX10 and J05AX11 or N03AX11 and J05AX11 or N03AX12 and J05AX11 or N03AX13 and J05AX11 or N03AX14 and J05AX11 or N03AX15 and J05AX11 or N03AX16 and J05AX11 or N03AX17 and J05AX11 or N03AX18 and J05AX11 or N03AX19 and J05AX11 or N03AX21 and J05AX11 or N03AX22 and J05AX11 or N03AX23 and J05AX11 or N03AX24 and J05AX11 or N03AX30 and J05AX11 or N03AA01 and J05AX12 or N03AA02 and J05AX12 or N03AA03 and J05AX12 or N03AA04 and J05AX12 or N03AA30 and J05AX12 or N03AB01 and J05AX12 or N03AB02 and J05AX12 or N03AB03 and J05AX12 or N03AB04 and J05AX12 or N03AB05 and J05AX12 or N03AB52 and J05AX12 or N03AB54 and J05AX12 or N03AC01 and J05AX12 or N03AC02 and J05AX12 or N03AC03 and J05AX12 or N03AD01 and J05AX12 or N03AD02 and J05AX12 or N03AD03 and J05AX12 or N03AD51 and J05AX12 or N03AE01 and J05AX12 or N03AF01 and J05AX12 or N03AF02 and J05AX12 or N03AF03 and J05AX12 or N03AF04 and J05AX12 or N03AG01 and J05AX12 or N03AG02 and J05AX12 or N03AG03 and J05AX12 or N03AG04 and J05AX12 or N03AG05 and J05AX12 or N03AG06 and J05AX12 or N03AX03 and J05AX12 or N03AX07 and J05AX12 or N03AX09 and J05AX12 or N03AX10 and J05AX12 or N03AX11 and J05AX12 or N03AX12 and J05AX12 or N03AX13 and J05AX12 or N03AX14 and J05AX12 or N03AX15 and J05AX12 or N03AX16 and J05AX12 or N03AX17 and J05AX12 or N03AX18 and J05AX12 or N03AX19 and J05AX12 or N03AX21 and J05AX12 or N03AX22 and J05AX12 or N03AX23 and J05AX12 or N03AX24 and J05AX12 or N03AX30 and J05AX12 or N03AA01 and J05AX13 or N03AA02 and J05AX13 or N03AA03 and J05AX13 or N03AA04 and J05AX13 or N03AA30 and J05AX13 or N03AB01 and J05AX13 or N03AB02 and J05AX13 or N03AB03 and J05AX13 or N03AB04 and J05AX13 or N03AB05 and J05AX13 or N03AB52 and J05AX13 or N03AB54 and J05AX13 or N03AC01 and J05AX13 or N03AC02 and J05AX13 or N03AC03 and J05AX13 or N03AD01 and J05AX13 or N03AD02 and J05AX13 or N03AD03 and J05AX13 or N03AD51 and J05AX13 or N03AE01 and J05AX13 or N03AF01 and J05AX13 or N03AF02 and J05AX13 or N03AF03 and J05AX13 or N03AF04 and J05AX13 or N03AG01 and J05AX13 or N03AG02 and J05AX13 or N03AG03 and J05AX13 or N03AG04 and J05AX13 or N03AG05 and J05AX13 or N03AG06 and J05AX13 or N03AX03 and J05AX13 or N03AX07 and J05AX13 or N03AX09 and J05AX13 or N03AX10 and J05AX13 or N03AX11 and J05AX13 or N03AX12 and J05AX13 or N03AX13 and J05AX13 or N03AX14 and J05AX13 or N03AX15 and J05AX13 or N03AX16 and J05AX13 or N03AX17 and J05AX13 or N03AX18 and J05AX13 or N03AX19 and J05AX13 or N03AX21 and J05AX13 or N03AX22 and J05AX13 or N03AX23 and J05AX13 or N03AX24 and J05AX13 or N03AX30 and J05AX13 or N03AA01 and J05AX17 or N03AA02 and J05AX17 or N03AA03 and J05AX17 or N03AA04 and J05AX17 or N03AA30 and J05AX17 or N03AB01 and J05AX17 or N03AB02 and J05AX17 or N03AB03 and J05AX17 or N03AB04 and J05AX17 or N03AB05 and J05AX17 or N03AB52 and J05AX17 or N03AB54 and J05AX17 or N03AC01 and J05AX17 or N03AC02 and J05AX17 or N03AC03 and J05AX17 or N03AD01 and J05AX17 or N03AD02 and J05AX17 or N03AD03 and J05AX17 or N03AD51 and J05AX17 or N03AE01 and J05AX17 or N03AF01 and J05AX17 or N03AF02 and J05AX17 or N03AF03 and J05AX17 or N03AF04 and J05AX17 or N03AG01 and J05AX17 or N03AG02 and J05AX17 or N03AG03 and J05AX17 or N03AG04 and J05AX17 or N03AG05 and J05AX17 or N03AG06 and J05AX17 or N03AX03 and J05AX17 or N03AX07 and J05AX17 or N03AX09 and J05AX17 or N03AX10 and J05AX17 or N03AX11 and J05AX17 or N03AX12 and J05AX17 or N03AX13 and J05AX17 or N03AX14 and J05AX17 or N03AX15 and J05AX17 or N03AX16 and J05AX17 or N03AX17 and J05AX17 or N03AX18 and J05AX17 or N03AX19 and J05AX17 or N03AX21 and J05AX17 or N03AX22 and J05AX17 or N03AX23 and J05AX17 or N03AX24 and J05AX17 or N03AX30 and J05AX17 or N03AA01 and J05AX18 or N03AA02 and J05AX18 or N03AA03 and J05AX18 or N03AA04 and J05AX18 or N03AA30 and J05AX18 or N03AB01 and J05AX18 or N03AB02 and J05AX18 or N03AB03 and J05AX18 or N03AB04 and J05AX18 or N03AB05 and J05AX18 or N03AB52 and J05AX18 or N03AB54 and J05AX18 or N03AC01 and J05AX18 or N03AC02 and J05AX18 or N03AC03 and J05AX18 or N03AD01 and J05AX18 or N03AD02 and J05AX18 or N03AD03 and J05AX18 or N03AD51 and J05AX18 or N03AE01 and J05AX18 or N03AF01 and J05AX18 or N03AF02 and J05AX18 or N03AF03 and J05AX18 or N03AF04 and J05AX18 or N03AG01 and J05AX18 or N03AG02 and J05AX18 or N03AG03 and J05AX18 or N03AG04 and J05AX18 or N03AG05 and J05AX18 or N03AG06 and J05AX18 or N03AX03 and J05AX18 or N03AX07 and J05AX18 or N03AX09 and J05AX18 or N03AX10 and J05AX18 or N03AX11 and J05AX18 or N03AX12 and J05AX18 or N03AX13 and J05AX18 or N03AX14 and J05AX18 or N03AX15 and J05AX18 or N03AX16 and J05AX18 or N03AX17 and J05AX18 or N03AX18 and J05AX18 or N03AX19 and J05AX18 or N03AX21 and J05AX18 or N03AX22 and J05AX18 or N03AX23 and J05AX18 or N03AX24 and J05AX18 or N03AX30 and J05AX18 or N03AA01 and J05AX19 or N03AA02 and J05AX19 or N03AA03 and J05AX19 or N03AA04 and J05AX19 or N03AA30 and J05AX19 or N03AB01 and J05AX19 or N03AB02 and J05AX19 or N03AB03 and J05AX19 or N03AB04 and J05AX19 or N03AB05 and J05AX19 or N03AB52 and J05AX19 or N03AB54 and J05AX19 or N03AC01 and J05AX19 or N03AC02 and J05AX19 or N03AC03 and J05AX19 or N03AD01 and J05AX19 or N03AD02 and J05AX19 or N03AD03 and J05AX19 or N03AD51 and J05AX19 or N03AE01 and J05AX19 or N03AF01 and J05AX19 or N03AF02 and J05AX19 or N03AF03 and J05AX19 or N03AF04 and J05AX19 or N03AG01 and J05AX19 or N03AG02 and J05AX19 or N03AG03 and J05AX19 or N03AG04 and J05AX19 or N03AG05 and J05AX19 or N03AG06 and J05AX19 or N03AX03 and J05AX19 or N03AX07 and J05AX19 or N03AX09 and J05AX19 or N03AX10 and J05AX19 or N03AX11 and J05AX19 or N03AX12 and J05AX19 or N03AX13 and J05AX19 or N03AX14 and J05AX19 or N03AX15 and J05AX19 or N03AX16 and J05AX19 or N03AX17 and J05AX19 or N03AX18 and J05AX19 or N03AX19 and J05AX19 or N03AX21 and J05AX19 or N03AX22 and J05AX19 or N03AX23 and J05AX19 or N03AX24 and J05AX19 or N03AX30 and J05AX19 or N03AA01 and J05AX21 or N03AA02 and J05AX21 or N03AA03 and J05AX21 or N03AA04 and J05AX21 or N03AA30 and J05AX21 or N03AB01 and J05AX21 or N03AB02 and J05AX21 or N03AB03 and J05AX21 or N03AB04 and J05AX21 or N03AB05 and J05AX21 or N03AB52 and J05AX21 or N03AB54 and J05AX21 or N03AC01 and J05AX21 or N03AC02 and J05AX21 or N03AC03 and J05AX21 or N03AD01 and J05AX21 or N03AD02 and J05AX21 or N03AD03 and J05AX21 or N03AD51 and J05AX21 or N03AE01 and J05AX21 or N03AF01 and J05AX21 or N03AF02 and J05AX21 or N03AF03 and J05AX21 or N03AF04 and J05AX21 or N03AG01 and J05AX21 or N03AG02 and J05AX21 or N03AG03 and J05AX21 or N03AG04 and J05AX21 or N03AG05 and J05AX21 or N03AG06 and J05AX21 or N03AX03 and J05AX21 or N03AX07 and J05AX21 or N03AX09 and J05AX21 or N03AX10 and J05AX21 or N03AX11 and J05AX21 or N03AX12 and J05AX21 or N03AX13 and J05AX21 or N03AX14 and J05AX21 or N03AX15 and J05AX21 or N03AX16 and J05AX21 or N03AX17 and J05AX21 or N03AX18 and J05AX21 or N03AX19 and J05AX21 or N03AX21 and J05AX21 or N03AX22 and J05AX21 or N03AX23 and J05AX21 or N03AX24 and J05AX21 or N03AX30 and J05AX21 or N03AA01 and J05AX23 or N03AA02 and J05AX23 or N03AA03 and J05AX23 or N03AA04 and J05AX23 or N03AA30 and J05AX23 or N03AB01 and J05AX23 or N03AB02 and J05AX23 or N03AB03 and J05AX23 or N03AB04 and J05AX23 or N03AB05 and J05AX23 or N03AB52 and J05AX23 or N03AB54 and J05AX23 or N03AC01 and J05AX23 or N03AC02 and J05AX23 or N03AC03 and J05AX23 or N03AD01 and J05AX23 or N03AD02 and J05AX23 or N03AD03 and J05AX23 or N03AD51 and J05AX23 or N03AE01 and J05AX23 or N03AF01 and J05AX23 or N03AF02 and J05AX23 or N03AF03 and J05AX23 or N03AF04 and J05AX23 or N03AG01 and J05AX23 or N03AG02 and J05AX23 or N03AG03 and J05AX23 or N03AG04 and J05AX23 or N03AG05 and J05AX23 or N03AG06 and J05AX23 or N03AX03 and J05AX23 or N03AX07 and J05AX23 or N03AX09 and J05AX23 or N03AX10 and J05AX23 or N03AX11 and J05AX23 or N03AX12 and J05AX23 or N03AX13 and J05AX23 or N03AX14 and J05AX23 or N03AX15 and J05AX23 or N03AX16 and J05AX23 or N03AX17 and J05AX23 or N03AX18 and J05AX23 or N03AX19 and J05AX23 or N03AX21 and J05AX23 or N03AX22 and J05AX23 or N03AX23 and J05AX23 or N03AX24 and J05AX23 or N03AX30 and J05AX23.

8. A composition according to any of the previous claims, **characterised in that** it is a composition composed of any combination of active substances selected from the NO3 ATC group and active substances selected from the J05 ATC group.

9. The use of J05 ATC group substances for treatment of nervous tissue dysfunction, in particular viral latency.

10. A composition according to claim 9, **characterised in that** it is a J05 ATC group substance: metisazone or aciclovir or idoxuridine or vidarabine or ganciclovir or famciclovir or valaciclovir or cidofovir or penciclovir or valganciclovir or brivudine or rimantadine or tromantadine or foscarnet or fosfonet or saquinavir or indinavir or ritonavir or nelfinavir or amprenavir or fosamprenavir or atazanavir or tipranavir or darunavir or zidovudine or didanosine or zalcitabine or stavudine or lamivudine or abacavir or tenofovir disoproxil or adefovir dipivoxil or emtricitabine or entecavir or telbivudine or clevudine or tenofovir alafenamide or nevirapine or delavirdine or efavirenz or etravirine or rilpivirine or doravirine or zanamivir or oseltamivir or peramivir or ribavirin or telaprevir or boceprevir or faldaprevir or simeprevir or asunaprevir or daclatasvir or sofosbuvir or dasabuvir or sofosbuvir and ledipasvir or dasabuvir, ombitasvir, paritaprevir and ritonavir or ombitasvir, paritaprevir and ritonavir or elbasvir and grazoprevir or sofosbuvir and velpatasvir or sofosbuvir, velpatasvir and voxilaprevir or glecaprevir and pibrentasvir or zidovudine and lamivudine or lamivudine and abacavir or tenofovir disoproxil and emtricitabine or zidovudine, lamivudine and abacavir or zidovudine, lamivudine and nevirapine or emtricitabine, tenofovir disoproxil and efavirenz or stavudine, lamivudine and nevirapine or emtricitabine, tenofovir disoproxil and rilpivirine or emtricitabine, tenofovir disoproxil, elvitegravir and cobicistat lopinavir and ritonavir or lamivudine, tenofovir disoproxil and efavirenz or lamivudine and tenofovir disoproxil or lamivudine, abacavir and dolutegravir or darunavir and cobicistat or atazanavir and cobicistat or lamivudine and raltegravir or emtricitabine and tenofovir alafenamide or emtricitabine, tenofovir alafenamide, elvitegravir and cobicistat or emtricitabine or tenofovir alafenamide and rilpivirine or emtricitabine, tenofovir alafenamide and bictegravir or dolutegravir and rilpivirine or emtricitabine, tenofovir or alafenamide, darunavir and cobicistat or atazanavir and ritonavir or lamivudine, tenofovir disoproxil and doravirine or moroxydine or lysozyme or inosine pranobex or pleconaril or enfuvirtide or raltegravir or maraviroc or maribavir or elvitegravir or dolutegravir or umifenovir or enisamium iodide or letermovir or tilorone or pentanedioic acid imidazolyl ethanamide or ibalizumab.

## Amended claims

### Amended claims in accordance with Rule 137(2) EPC.

1. A pharmacological composition used in treatment of nervous tissue dysfunction, in particular viral latency, **characterised in that** consistent with the invention, contains at least one active substance with antiepileptic effect selected from the NO3 ATC group and at least one active substance with antiviral effect selected from the J05 ATC group.

2. A composition according to claim 1, **characterised in that** the active substance of the NO3 ATC group is: methylphenobarbital or phenobarbital or primidone or barbexaclone or metharbital or ethotoin or phenytoin or amino(diphenylhydantoin) valeric acid or mephenytoin or fosphenytoin or phenytoin combinations or mephenytoin combinations or paramethadione or trimethadione or ethadione or ethosuximide or phensuximide or mesuximide or ethosuximide combinations or clonazepam or carbamazepine or oxcarbazepine or rufinamide or eslicarbazepine or valproic acid or valpromide or aminobutyric acid or vigabatrin or progabide or tiagabine or sultiame or phenacemide or lamotrigine or felbamate or topiramate or gabapentin or pheneturide or levetiracetam or zonisamide or pregabalin or stiripentol or lacosamide or carisbamate or retigabine or perampanel or brivaracetam or cannabidiol or beclamide.

3. A composition according to claim 1, **characterised in that** the active substance of the J05 ATC group is: metisazone or aciclovir or idoxuridine or vidarabine or ganciclovir or famciclovir or valaciclovir or cidofovir or penciclovir or valganciclovir or brivudine or rimantadine or tromantadine or foscarnet or fosfonet or saquinavir or indinavir or ritonavir or nelfinavir or amprenavir or fosamprenavir or atazanavir or tipranavir or darunavir or zidovudine or didanosine or zalcitabine or stavudine or lamivudine or abacavir or tenofovir disoproxil or adefovir dipivoxil or emtricitabine or entecavir or telbivudine or clevudine or tenofovir alafenamide or nevirapine or delavirdine or efavirenz or etravirine or rilpivirine or doravirine or zanamivir or oseltamivir or peramivir or ribavirin or telaprevir or boceprevir or faldaprevir or simeprevir or asunaprevir or daclatasvir or sofosbuvir or dasabuvir or sofosbuvir and ledipasvir or dasabuvir, ombitasvir, paritaprevir and ritonavir or ombitasvir, paritaprevir and ritonavir or elbasvir and grazoprevir or sofosbuvir and velpatasvir or sofosbuvir, velpatasvir and voxilaprevir or glecaprevir and pibrentasvir or zidovudine and lamivudine or lamivudine and abacavir or tenofovir disoproxil and emtricitabine or zidovudine, lamivudine and abacavir or zidovudine, lamivudine and nevirapine or emtricitabine, tenofovir disoproxil and efavirenz or stavudine, lamivudine and nevirapine or emtricitabine, tenofovir disoproxil and rilpivirine or emtricitabine, tenofovir disoproxil, elvitegravir and cobicistat lopinavir and ritonavir or lamivudine, tenofovir disoproxil and efavirenz or lamivudine and tenofovir disoproxil or lamivudine, abacavir and dolutegravir or darunavir and cobicistat or atazanavir and cobicistat or lamivudine and raltegravir or emtricitabine and tenofovir alafenamide or emtricitabine, tenofovir alafenamide, elvitegravir and cobicistat or emtricitabine or tenofovir alafenamide and rilpivirine or emtricitabine, tenofovir alafenamide and bictegravir or dolutegravir and rilpivirine or emtricitabine, tenofovir or alafenamide, darunavir and cobicistat or atazanavir and ritonavir or lamivudine, tenofovir disoproxil and doravirine or moroxydine or lysozyme or inosine pranobex or pleconaril or enfuvirtide or raltegravir or maraviroc or maribavir or elvitegravir or dolutegravir or umifenovir or enisamium iodide or letermovir or tilorone or pentanedioic acid imidazolyl ethanamide or ibalizumab.

4. A composition according to claims 1 or 2, **characterised in that** the preferable active substance with antiepileptic effect is valproic acid (sodium valproate) or vigabatrin, combined with inosine pranobex as the active substance with antiepileptic effect.

5. A composition according to any of the previous claims, **characterised in that** the weight ratio in [mg] of active substances selected from the NO3 ATC group to active substances selected from the J05 ATC group is as follows: 10.000.000 to 1:1; 1.000.000 to 1:1; 100.000 to 1:1; 10.000 to 1:1; 1.000 to 1:1; 100 to 1 :1; 10 to 1:1; 1 :1 to 10.000.000; 1:1 to 1.000.000; 1:1 to 100. 000; 1:1 to 10.000; 1 to 1: 1.000; 1:1 to 100; 1:1 to 10 preferably 1:2, most preferably 1:2.13.

6. A composition according to any of the previous claims, **characterised in that** the preferable combination of active substances in the composition is:
N03AA01 and J05AA01 or N03AA02 and J05AA01 or N03AA03 and J05AA01 or N03AA04 and J05AA01 or N03AA30 and J05AA01 or N03AB01 and J05AA01 or N03AB02 and J05AA01 or N03AB03 and J05AA01 or N03AB04 and J05AA01 or N03AB05 and J05AA01 or N03AB52 and J05AA01 or N03AB54 and J05AA01 or N03AC01 and J05AA01 or N03AC02 and J05AA01 or N03AC03 and J05AA01 or N03AD01 and J05AA01 or N03AD02 and J05AA01 or N03AD03 and J05AA01 or N03AD51 and J05AA01 or N03AE01 and J05AA01 or N03AF01 and J05AA01 or N03AF02 and J05AA01 or N03AF03 and J05AA01 or N03AF04 and J05AA01 or N03AG01 and J05AA01 or N03AG02 and J05AA01 or N03AG03 and J05AA01 or N03AG04 and J05AA01 or N03AG05 and J05AA01 or N03AG06 and J05AA01 or N03AX03 and J05AA01 or N03AX07 and J05AA01 or N03AX09 and J05AA01 or N03AX10 and J05AA01 or N03AX11 and J05AA01 or N03AX12 and J05AA01 or N03AX13 and J05AA01 or N03AX14 and J05AA01 or N03AX15 and J05AA01 or N03AX16 and J05AA01 or N03AX17 and J05AA01 or N03AX18 and J05AA01 or N03AX19 and J05AA01 or N03AX21 and J05AA01 or N03AX22 and J05AA01 or N03AX23 and J05AA01 or N03AX24 and J05AA01 or N03AX30 and J05AA01 or N03AA01 and J05AB01 or N03AA02 and J05AB01 or N03AA03 and J05AB01 or N03AA04 and J05AB01 or N03AA30 and J05AB01 or N03AB01 and J05AB01 or N03AB02 and J05AB01 or N03AB03 and J05AB01 or N03AB04 and J05AB01 or N03AB05 and J05AB01 or N03AB52 and J05AB01 or N03AB54 and J05AB01 or N03AC01 and J05AB01 or N03AC02 and J05AB01 or N03AC03 and J05AB01 or N03AD01 and J05AB01 or N03AD02 and J05AB01 or N03AD03 and J05AB01 or N03AD51 and J05AB01 or N03AE01 and J05AB01 or N03AF01 and J05AB01 or N03AF02 and J05AB01 or N03AF03 and J05AB01 or N03AF04 and J05AB01 or N03AG01 and J05AB01 or N03AG02 and J05AB01 or N03AG03 and J05AB01 or N03AG04 and J05AB01 or N03AG05 and J05AB01 or N03AG06 and J05AB01 or N03AX03 and J05AB01 or N03AX07 and J05AB01 or N03AX09 and J05AB01 or N03AX10 and J05AB01 or N03AX11 and J05AB01 or N03AX12 and J05AB01 or N03AX13 and J0SAB01 or N03AX14 and J05AB01 or N03AX15 and J05AB01 or N03AX16 and J05AB01 or N03AX17 and J05AB01 or N03AX18 and J05AB01 or N03AX19 and J05AB01 or N03AX21 and J05AB01 or N03AX22 and J05AB01 or N03AX23 and J05AB01 or N03AX24 and J05AB01 or N03AX30 and J05AB01 or N03AA01 and J05AB02 or N03AA02 and J05AB02 or N03AA03 and J05AB02 or N03AA04 and J05AB02 or N03AA30 and J05AB02 or N03AB01 and J05AB02 or N03AB02 and J05AB02 or N03AB03 and J05AB02 or N03AB04 and J05AB02 or N03AB05 and J05AB02 or N03AB52 and J05AB02 or N03AB54 and J05AB02 or N03AC01 and J05AB02 or N03AC02 and J05AB02 or N03AC03 and J05AB02 or N03AD01 and J05AB02 or N03AD02 and J05AB02 or N03AD03 and J05AB02 or N03AD51 and J05AB02 or N03AE01 and J05AB02 or N03AF01 and J05AB02 or N03AF02 and J05AB02 or N03AF03 and J05AB02 or N03AF04 and J05AB02 or N03AG01 and J05AB02 or N03AG02 and J05AB02 or N03AG03 and J05AB02 or N03AG04 and J05AB02 or N03AG05 and J05AB02 or N03AG06 and J05AB02 or N03AX03 and J05AB02 or N03AX07 and J05AB02 or N03AX09 and J05AB02 or N03AX10 and J05AB02 or N03AX11 and J05AB02 or N03AX12 and J05AB02 or N03AX13 and J05AB02 or N03AX14 and J05AB02 or N03AX15 and J05AB02 or N03AX16 and J05AB02 or N03AX17 and J05AB02 or N03AX18 and J05AB02 or N03AX19 and J05AB02 or N03AX21 and J05AB02 or N03AX22 and J05AB02 or N03AX23 and J05AB02 or N03AX24 and J05AB02 or N03AX30 and J05AB02 or N03AA01 and J05AB03 or N03AA02 and J05AB03 or N03AA03 and J05AB03 or N03AA04 and J05AB03 or N03AA30 and J05AB03 or N03AB01 and J05AB03 or N03AB02 and J05AB03 or N03AB03 and J05AB03 or N03AB04 and J05AB03 or N03AB05 and J05AB03 or N03AB52 and J05AB03 or N03AB54 and J05AB03 or N03AC01 and J05AB03 or N03AC02 and J05AB03 or N03AC03 and J05AB03 or N03AD01 and J05AB03 or N03AD02 and J05AB03 or N03AD03 and J05AB03 or N03AD51 and J05AB03 or N03AE01 and J05AB03 or N03AF01 and J05AB03 or N03AF02 and J05AB03 or N03AF03 and J05AB03 or N03AF04 and J05AB03 or N03AG01 and J05AB03 or N03AG02 and J05AB03 or N03AG03 and J05AB03 or N03AG04 and J05AB03 or N03AG05 and J05AB03 or N03AG06 and J05AB03 or N03AX03 and J05AB03 or N03AX07 and J05AB03 or N03AX09 and J05AB03 or N03AX10 and J05AB03 or N03AX11 and J05AB03 or N03AX12 and J05AB03 or N03AX13 and J05AB03 or N03AX14 and J05AB03 or N03AX15 and J05AB03 or N03AX16 and J05AB03 or N03AX17 and J05AB03 or N03AX18 and J05AB03 or N03AX19 and J05AB03 or N03AX21 and J05AB03 or N03AX22 and J05AB03 or N03AX23 and J05AB03 or N03AX24 and J05AB03 or N03AX30 and J05AB03 or N03AA01 and J05AB06 or N03AA02 and J05AB06 or N03AA03 and J05AB06 or N03AA04 and J05AB06 or N03AA30 and J05AB06 or N03AB01 and J05AB06 or N03AB02 and J05AB06 or N03AB03 and J05AB06 or N03AB04 and J05AB06 or N03AB05 and J05AB06 or N03AB52 and J05AB06 or N03AB54 and J05AB06 or N03AC01 and J05AB06 or N03AC02 and J05AB06 or N03AC03 and J05AB06 or N03AD01 and J05AB06 or N03AD02 and J05AB06 or N03AD03 and J05AB06 or N03AD51 and J05AB06 or N03AE01 and J05AB06 or N03AF01 and J05AB06 or N03AF02 and J05AB06 or N03AF03 and J05AB06 or N03AF04 and J05AB06 or N03AG01 and J05AB06 or N03AG02 and J05AB06 or N03AG03 and J05AB06 or N03AG04 and J05AB06 or N03AG05 and J05AB06 or N03AG06 and J05AB06 or N03AX03 and J05AB06 or N03AX07 and J05AB06 or N03AX09 and J05AB06 or N03AX10 and J05AB06 or N03AX11 and J05AB06 or N03AX12 and J05AB06 or N03AX13 and J05AB06 or N03AX14 and J05AB06 or N03AX15 and J05AB06 or N03AX16 and J05AB06 or N03AX17 and J05AB06 or N03AX18 and J05AB06 or N03AX19 and J05AB06 or N03AX21 and J05AB06 or N03AX22 and J05AB06 or N03AX23 and J05AB06 or N03AX24 and J05AB06 or N03AX30 and J05AB06 or N03AA01 and J05AB09 or N03AA02 and J05AB09 or N03AA03 and J05AB09 or N03AA04 and J05AB09 or N03AA30 and J05AB09 or N03AB01 and J05AB09 or N03AB02 and J05AB09 or N03AB03 and J05AB09 or N03AB04 and J05AB09 or N03AB05 and J05AB09 or N03AB52 and J05AB09 or N03AB54 and J05AB09 or N03AC01 and J05AB09 or N03AC02 and J05AB09 or N03AC03 and J05AB09 or N03AD01 and J05AB09 or N03AD02 and J05AB09 or N03AD03 and J05AB09 or N03AD51 and J05AB09 or N03AE01 and J05AB09 or N03AF01 and J05AB09 or N03AF02 and J05AB09 or N03AF03 and J05AB09 or N03AF04 and J05AB09 or N03AG01 and J05AB09 or N03AG02 and J05AB09 or N03AG03 and J05AB09 or N03AG04 and J05AB09 or N03AG05 and J05AB09 or N03AG06 and J05AB09 or N03AX03 and J05AB09 or N03AX07 and J05AB09 or N03AX09 and J05AB09 or N03AX10 and J05AB09 or N03AX11 and J05AB09 or N03AX12 and J05AB09 or N03AX13 and J05AB09 or N03AX14 and J05AB09 or N03AX15 and J05AB09 or N03AX16 and J05AB09 or N03AX17 and J05AB09 or N03AX18 and J05AB09 or N03AX19 and J05AB09 or N03AX21 and J05AB09 or N03AX22 and J05AB09 or N03AX23 and J05AB09 or N03AX24 and J05AB09 or N03AX30 and J05AB09 or N03AA01 and J05AB11 or N03AA02 and J05AB11 or N03AA03 and J05AB11 or N03AA04 and J05AB11 or N03AA30 and J05AB11 or N03AB01 and J05AB11 or N03AB02 and J05AB11 or N03AB03 and J05AB11 or N03AB04 and J05AB11 or N03AB05 and J05AB11 or N03AB52 and J05AB11 or N03AB54 and J05AB11 or N03AC01 and J05AB11 or N03AC02 and J05AB11 or N03AC03 and J05AB11 or N03AD01 and J05AB11 or N03AD02 and J05AB11 or N03AD03 and J05AB11 or N03AD51 and J05AB11 or N03AE01 and J05AB11 or N03AF01 and J05AB11 or N03AF02 and J05AB11 or N03AF03 and J05AB11 or N03AF04 and J05AB11 or N03AG01 and J05AB11 or N03AG02 and J05AB11 or N03AG03 and J05AB11 or N03AG04 and J05AB11 or N03AG05 and J05AB11 or N03AG06 and J05AB11 or N03AX03 and J05AB11 or N03AX07 and J0SAB11 or N03AX09 and J05AB11 or N03AX10 and J05AB11 or N03AX11 and J05AB11 or N03AX12 and J05AB11 or N03AX13 and J05AB11 or N03AX14 and J05AB11 or N03AX15 and J05AB11 or N03AX16 and J05AB11 or N03AX17 and J05AB11 or N03AX18 and J05AB11 or N03AX19 and J05AB11 or N03AX21 and J05AB11 or N03AX22 and J05AB11 or N03AX23 and J05AB11 or N03AX24 and J05AB11 or N03AX30 and J05AB11 or N03AA01 and J05AB12 or N03AA02 and J05AB12 or N03AA03 and J05AB12 or N03AA04 and J05AB12 or N03AA30 and J05AB12 or N03AB01 and J05AB12 or N03AB02 and J05AB12 or N03AB03 and J05AB12 or N03AB04 and J05AB12 or N03AB05 and J05AB12 or N03AB52 and J05AB12 or N03AB54 and J05AB12 or N03AC01 and J05AB12 or N03AC02 and J05AB12 or N03AC03 and J05AB12 or N03AD01 and J05AB12 or N03AD02 and J05AB12 or N03AD03 and J05AB12 or N03AD51 and J05AB12 or N03AE01 and J05AB12 or N03AF01 and J05AB12 or N03AF02 and J05AB12 or N03AF03 and J05AB12 or N03AF04 and J05AB12 or N03AG01 and J05AB12 or N03AG02 and J05AB12 or N03AG03 and J05AB12 or N03AG04 and J05AB12 or N03AG05 and J05AB12 or N03AG06 and J05AB12 or N03AX03 and J05AB12 or N03AX07 and J05AB12 or N03AX09 and J05AB12 or N03AX10 and J05AB12 or N03AX11 and J05AB12 or N03AX12 and J05AB12 or N03AX13 and J05AB12 or N03AX14 and J05AB12 or N03AX15 and J05AB12 or N03AX16 and J05AB12 or N03AX17 and J05AB12 or N03AX18 and J05AB12 or N03AX19 and J05AB12 or N03AX21 and J05AB12 or N03AX22 and J05AB12 or N03AX23 and J05AB12 or N03AX24 and J05AB12 or N03AX30 and J05AB12 or N03AA01 and J05AB13 or N03AA02 and J05AB13 or N03AA03 and J05AB13 or N03AA04 and J05AB13 or N03AA30 and J05AB13 or N03AB01 and J05AB13 or N03AB02 and J05AB13 or N03AB03 and J05AB13 or N03AB04 and J05AB13 or N03AB05 and J05AB13 or N03AB52 and J05AB13 or N03AB54 and J05AB13 or N03AC01 and J05AB13 or N03AC02 and J05AB13 or N03AC03 and J05AB13 or N03AD01 and J05AB13 or N03AD02 and J05AB13 or N03AD03 and J05AB13 or N03AD51 and J05AB13 or N03AE01 and J05AB13 or N03AF01 and J05AB13 or N03AF02 and J05AB13 or N03AF03 and J05AB13 or N03AF04 and J05AB13 or N03AG01 and J05AB13 or N03AG02 and J05AB13 or N03AG03 and J05AB13 or N03AG04 and J05AB13 or N03AG05 and J05AB13 or N03AG06 and J05AB13 or N03AX03 and J05AB13 or N03AX07 and J05AB13 or N03AX09 and J05AB13 or N03AX10 and J05AB13 or N03AX11 and J05AB13 or N03AX12 and J05AB13 or N03AX13 and J05AB13 or N03AX14 and J05AB13 or N03AX15 and J05AB13 or N03AX16 and J05AB13 or N03AX17 and J05AB13 or N03AX18 and J05AB13 or N03AX19 and J05AB13 or N03AX21 and J05AB13 or N03AX22 and J05AB13 or N03AX23 and J05AB13 or N03AX24 and J05AB13 or N03AX30 and J05AB13 or N03AA01 and J05AB14 or N03AA02 and J05AB14 or N03AA03 and J05AB14 or N03AA04 and J05AB14 or N03AA30 and J05AB14 or N03AB01 and J05AB14 or N03AB02 and J05AB14 or N03AB03 and J05AB14 or N03AB04 and J05AB14 or N03AB05 and J05AB14 or N03AB52 and J05AB14 or N03AB54 and J05AB14 or N03AC01 and J05AB14 or N03AC02 and J05AB14 or N03AC03 and J05AB14 or N03AD01 and J05AB14 or N03AD02 and J05AB14 or N03AD03 and J05AB14 or N03AD51 and J05AB14 or N03AE01 and J05AB14 or N03AF01 and J05AB14 or N03AF02 and J05AB14 or N03AF03 and J05AB14 or N03AF04 and J05AB14 or N03AG01 and J05AB14 or N03AG02 and J05AB14 or N03AG03 and J05AB14 or N03AG04 and J05AB14 or N03AG05 and J05AB14 or N03AG06 and J05AB14 or N03AX03 and J05AB14 or N03AX07 and J05AB14 or N03AX09 and J05AB14 or N03AX10 and J05AB14 or N03AX11 and J05AB14 or N03AX12 and J05AB14 or N03AX13 and J05AB14 or N03AX14 and J05AB14 or N03AX15 and J05AB14 or N03AX16 and J05AB14 or N03AX17 and J05AB14 or N03AX18 and J05AB14 or N03AX19 and J05AB14 or N03AX21 and J05AB14 or N03AX22 and J05AB14 or N03AX23 and J05AB14 orN03AX24 and J05AB14 or N03AX30 and J05AB14 or N03AA01 and J05AB15 or N03AA02 and J05AB15 or N03AA03 and J05AB15 or N03AA04 and J05AB15 or N03AA30 and J05AB15 or N03AB01 and J05AB15 or N03AB02 and J05AB15 or N03AB03 and J05AB15 or N03AB04 and J05AB15 or N03AB05 and J05AB15 or N03AB52 and J05AB15 or N03AB54 and J05AB15 or N03AC01 and J05AB15 or N03AC02 and J05AB15 or N03AC03 and J05AB15 or N03AD01 and J05AB15 or N03AD02 and J05AB15 or N03AD03 and J05AB15 or N03AD51 and J05AB15 or N03AE01 and J05AB15 or N03AF01 and J05AB15 or N03AF02 and J05AB15 or N03AF03 and J05AB15 or N03AF04 and J05AB15 or N03AG01 and J05AB15 or N03AG02 and J05AB15 or N03AG03 and J05AB15 or N03AG04 and J05AB15 or N03AG05 and J05AB15 or N03AG06 and J05AB15 or N03AX03 and J05AB15 or N03AX07 and J05AB15 or N03AX09 and J05AB15 or N03AX10 and J05AB15 or N03AX11 and J05AB15 or N03AX12 and J05AB15 or N03AX13 and J05AB15 or N03AX14 and J05AB15 or N03AX15 and J05AB15 or N03AX16 and J05AB15 or N03AX17 and J05AB15 or N03AX18 and J05AB15 or N03AX19 and J05AB15 or N03AX21 and J05AB15 or N03AX22 and J05AB15 or N03AX23 and J05AB15 or N03AX24 and J05AB15 or N03AX30 and J05AB15 or N03AA01 and J05AC02 or N03AA02 and J05AC02 or N03AA03 and J05AC02 or N03AA04 and J05AC02 or N03AA30 and J05AC02 or N03AB01 and J05AC02 or N03AB02 and J05AC02 or N03AB03 and J05AC02 or N03AB04 and J05AC02 or N03AB05 and J05AC02 or N03AB52 and J05AC02 or N03AB54 and J05AC02 or N03AC01 and J05AC02 or N03AC02 and J05AC02 or N03AC03 and J05AC02 or N03AD01 and J05AC02 or N03AD02 and J05AC02 or N03AD03 and J05AC02 or N03AD51 and J05AC02 or N03AE01 and J05AC02 or N03AF01 and J05AC02 or N03AF02 and J05AC02 or N03AF03 and J05AC02 or N03AF04 and J05AC02 or N03AG01 and J05AC02 or N03AG02 and J05AC02 or N03AG03 and J05AC02 or N03AG04 and J05AC02 or N03AG05 and J05AC02 or N03AG06 and J05AC02 or N03AX03 and J05AC02 or N03AX07 and J05AC02 or N03AX09 and J05AC02 or N03AX10 and J05AC02 or N03AX11 and J05AC02 or N03AX12 and J05AC02 or N03AX13 and J05AC02 or N03AX14 and J05AC02 or N03AX15 and J05AC02 or N03AX16 and J05AC02 or N03AX17 and J05AC02 or N03AX18 and J05AC02 or N03AX19 and J05AC02 or N03AX21 and J05AC02 or N03AX22 and J05AC02 or N03AX23 and J05AC02 or N03AX24 and J05AC02 or N03AX30 and J05AC02 or N03AA01 and J05AC03 or N03AA02 and J05AC03 or N03AA03 and J05AC03 or N03AA04 and J05AC03 or N03AA30 and J05AC03 or N03AB01 and J05AC03 or N03AB02 and J05AC03 or N03AB03 and J05AC03 or N03AB04 and J05AC03 or N03AB05 and J05AC03 or N03AB52 and J05AC03 or N03AB54 and J05AC03 or N03AC01 and J05AC03 or N03AC02 and J05AC03 or N03AC03 and J05AC03 or N03AD01 and J05AC03 or N03AD02 and J05AC03 or N03AD03 and J05AC03 or N03AD51 and J05AC03 or N03AE01 and J05AC03 or N03AF01 and J05AC03 or N03AF02 and J05AC03 or N03AF03 and J05AC03 or N03AF04 and J05AC03 or N03AG01 and J05AC03 or N03AG02 and J05AC03 or N03AG03 and J05AC03 or N03AG04 and J05AC03 or N03AG05 and J05AC03 or N03AG06 and J05AC03 or N03AX03 and J05AC03 or N03AX07 and J05AC03 or N03AX09 and J05AC03 or N03AX10 and J05AC03 or N03AX11 and J05AC03 or N03AX12 and J05AC03 or N03AX13 and J05AC03 or N03AX14 and J05AC03 or N03AX15 and J05AC03 or N03AX16 and J05AC03 or N03AX17 and J05AC03 or N03AX18 and J05AC03 or N03AX19 and J05AC03 or N03AX21 and J05AC03 or N03AX22 and J05AC03 or N03AX23 and J05AC03 or N03AX24 and J05AC03 or N03AX30 and J05AC03 or N03AA01 and J05AD01 or N03AA02 and J05AD01 or N03AA03 and J05AD01 or N03AA04 and J05AD01 or N03AA30 and J05AD01 or N03AB01 and J05AD01 or N03AB02 and J05AD01 or N03AB03 and J05AD01 or N03AB04 and J05AD01 or N03AB05 and J05AD01 or N03AB52 and J05AD01 or N03AB54 and J05AD01 or N03AC01 and J05AD01 or N03AC02 and J05AD01 or N03AC03 and J05AD01 or N03AD01 and J05AD01 or N03AD02 and J05AD01 or N03AD03 and J05AD01 or N03AD51 and J05AD01 or N03AE01 and J05AD01 or N03AF01 and J05AD01 or N03AF02 and J05AD01 or N03AF03 and J05AD01 or N03AF04 and J05AD01 or N03AG01 and J05AD01 or N03AG02 and J05AD01 or N03AG03 and J05AD01 or N03AG04 and J05AD01 or N03AG05 and J05AD01 or N03AG06 and J05AD01 or N03AX03 and J05AD01 or N03AX07 and J05AD01 or N03AX09 and J05AD01 or N03AX10 and J05AD01 or N03AX11 and J05AD01 or N03AX12 and J05AD01 or N03AX13 and J05AD01 or N03AX14 and J05AD01 or N03AX15 and J05AD01 or N03AX16 and J05AD01 or N03AX17 and J05AD01 or N03AX18 and J05AD01 or N03AX19 and J05AD01 or N03AX21 and J05AD01 or N03AX22 and J05AD01 or N03AX23 and J05AD01 or N03AX24 and J05AD01 or N03AX30 and J05AD01 or N03AA01 and J05AD02 or N03AA02 and J05AD02 or N03AA03 and J05AD02 or N03AA04 and J05AD02 or N03AA30 and J05AD02 or N03AB01 and J05AD02 or N03AB02 and J05AD02 or N03AB03 and J05AD02 or N03AB04 and J05AD02 or N03AB05 and J05AD02 or N03AB52 and J05AD02 or N03AB54 and J05AD02 or N03AC01 and J05AD02 or N03AC02 and J05AD02 or N03AC03 and J05AD02 or N03AD01 and J05AD02 or N03AD02 and J05AD02 or N03AD03 and J05AD02 or N03AD51 and J05AD02 or N03AE01 and J05AD02 or N03AF01 and J05AD02 or N03AF02 and J05AD02 or N03AF03 and J05AD02 or N03AF04 and J05AD02 or N03AG01 and J05AD02 or N03AG02 and J05AD02 or N03AG03 and J05AD02 or N03AG04 and J05AD02 or N03AG05 and J05AD02 or N03AG06 and J05AD02 or N03AX03 and J05AD02 or N03AX07 and J05AD02 or N03AX09 and J05AD02 or N03AX10 and J05AD02 or N03AX11 and J05AD02 or N03AX12 and J05AD02 or N03AX13 and J05AD02 or N03AX14 and J05AD02 or N03AX15 and J05AD02 or N03AX16 and J05AD02 or N03AX17 and J05AD02 or N03AX18 and J05AD02 or N03AX19 and J05AD02 or N03AX21 and J05AD02 or N03AX22 and J05AD02 or N03AX23 and J05AD02 or N03AX24 and J05AD02 or N03AX30 and J05AD02 or N03AA01 and J05AE01 or N03AA02 and J05AE01 or N03AA03 and J05AE01 or N03AA04 and J05AE01 or N03AA30 and J05AE01 or N03AB01 and J05AE01 or N03AB02 and J05AE01 or N03AB03 and J05AE01 or N03AB04 and J05AE01 or N03AB05 and J05AE01 or N03AB52 and J05AE01 or N03AB54 and J05AE01 or N03AC01 and J05AE01 or N03AC02 and J05AE01 or N03AC03 and J05AE01 or N03AD01 and J05AE01 or N03AD02 and J05AE01 or N03AD03 and J05AE01 or N03AD51 and J05AE01 or N03AE01 and J05AE01 or N03AF01 and J05AE01 or N03AF02 and J05AE01 or N03AF03 and J05AE01 or N03AF04 and J05AE01 or N03AG01 and J05AE01 or N03AG02 and J05AE01 or N03AG03 and J05AE01 or N03AG04 and J05AE01 or N03AG05 and J05AE01 or N03AG06 and J05AE01 or N03AX03 and J05AE01 or N03AX07 and J05AE01 or N03AX09 and J05AE01 or N03AX10 and J05AE01 or N03AX11 and J05AE01 or N03AX12 and J05AE01 or N03AX13 and J05AE01 or N03AX14 and J05AE01 or N03AX15 and J05AE01 or N03AX16 and J05AE01 or N03AX17 and J05AE01 or N03AX18 and J05AE01 or N03AX19 and J05AE01 or N03AX21 and J05AE01 or N03AX22 and J05AE01 or N03AX23 and J05AE01 or N03AX24 and J05AE01 or N03AX30 and J05AE01 or N03AA01 and J05AE02 or N03AA02 and J05AE02 or N03AA03 and J05AE02 or N03AA04 and J05AE02 or N03AA30 and J05AE02 or N03AB01 and J05AE02 or N03AB02 and J05AE02 or N03AB03 and J05AE02 or N03AB04 and J05AE02 or N03AB05 and J05AE02 or N03AB52 and J05AE02 or N03AB54 and J05AE02 or N03AC01 and J05AE02 or N03AC02 and J05AE02 or N03AC03 and J05AE02 or N03AD01 and J05AE02 or N03AD02 and J05AE02 or N03AD03 and J05AE02 or N03AD51 and J05AE02 or N03AE01 and J05AE02 or N03AF01 and J05AE02 or N03AF02 and J05AE02 or N03AF03 and J05AE02 or N03AF04 and J05AE02 or N03AG01 and J05AE02 or N03AG02 and J05AE02 or N03AG03 and J05AE02 or N03AG04 and J05AE02 or N03AG05 and J05AE02 or N03AG06 and J05AE02 or N03AX03 and J05AE02 or N03AX07 and J05AE02 or N03AX09 and J05AE02 or N03AX10 and J05AE02 or N03AX11 and J05AE02 or N03AX12 and J05AE02 or N03AX13 and J05AE02 or N03AX14 and J05AE02 or N03AX15 and J05AE02 or N03AX16 and J05AE02 or N03AX17 and J05AE02 or N03AX18 and J05AE02 or N03AX19 and J05AE02 or N03AX21 and J05AE02 or N03AX22 and J05AE02 or N03AX23 and J05AE02 or N03AX24 and J05AE02 or N03AX30 and J05AE02 or N03AA01 and J05AE03 or N03AA02 and J05AE03 or N03AA03 and J05AE03 or N03AA04 and J05AE03 or N03AA30 and J05AE03 or N03AB01 and J05AE03 or N03AB02 and J05AE03 or N03AB03 and J05AE03 or N03AB04 and J05AE03 or N03AB05 and J05AE03 or N03AB52 and J05AE03 or N03AB54 and J05AE03 or N03AC01 and J05AE03 or N03AC02 and J05AE03 or N03AC03 and J05AE03 or N03AD01 and J05AE03 or N03AD02 and J05AE03 or N03AD03 and J05AE03 or N03AD51 and J05AE03 or N03AE01 and J05AE03 or N03AF01 and J05AE03 or N03AF02 and J05AE03 or N03AF03 and J05AE03 or N03AF04 and J05AE03 or N03AG01 and J05AE03 or N03AG02 and J05AE03 or N03AG03 and J05AE03 or N03AG04 and J05AE03 or N03AG05 and J05AE03 or N03AG06 and J05AE03 or N03AX03 and J05AE03 or N03AX07 and J05AE03 or N03AX09 and J05AE03 or N03AX10 and J05AE03 or N03AX11 and J05AE03 or N03AX12 and J05AE03 or N03AX13 and J05AE03 or N03AX14 and J05AE03 or N03AX15 and J05AE03 or N03AX16 and J05AE03 or N03AX17 and J05AE03 or N03AX18 and J05AE03 or N03AX19 and J05AE03 or N03AX21 and J05AE03 or N03AX22 and J05AE03 or N03AX23 and J05AE03 or N03AX24 and J05AE03 or N03AX30 and J05AE03 or N03AA01 and J05AE04 or N03AA02 and J05AE04 or N03AA03 and J05AE04 or N03AA04 and J05AE04 or N03AA30 and J05AE04 or N03AB01 and J05AE04 or N03AB02 and J05AE04 or N03AB03 and J05AE04 or N03AB04 and J05AE04 or N03AB05 and J05AE04 or N03AB52 and J05AE04 or N03AB54 and J05AE04 or N03AC01 and J05AE04 or N03AC02 and J05AE04 or N03AC03 and J05AE04 or N03AD01 and J05AE04 or N03AD02 and J05AE04 or N03AD03 and J05AE04 or N03AD51 and J05AE04 or N03AE01 and J05AE04 or N03AF01 and J05AE04 or N03AF02 and J05AE04 or N03AF03 and J05AE04 or N03AF04 and J05AE04 or N03AG01 and J05AE04 or N03AG02 and J05AE04 or N03AG03 and J05AE04 or N03AG04 and J05AE04 or N03AG05 and J05AE04 or N03AG06 and J05AE04 or N03AX03 and J05AE04 or N03AX07 and J05AE04 or N03AX09 and J05AE04 or N03AX10 and J05AE04 or N03AX11 and J05AE04 or N03AX12 and J05AE04 or N03AX13 and J05AE04 or N03AX14 and J05AE04 or N03AX15 and J05AE04 or N03AX16 and J05AE04 or N03AX17 and J05AE04 or N03AX18 and J05AE04 or N03AX19 and J05AE04 or N03AX21 and J05AE04 or N03AX22 and J05AE04 or N03AX23 and J05AE04 or N03AX24 and J05AE04 or N03AX30 and J05AE04 or N03AA01 and J05AE05 or N03AA02 and J05AE05 or N03AA03 and J05AE05 or N03AA04 and J05AE05 or N03AA30 and J05AE05 or N03AB01 and J05AE05 or N03AB02 and J05AE05 or N03AB03 and J05AE05 or N03AB04 and J05AE05 or N03AB05 and J05AE05 or N03AB52 and J05AE05 or N03AB54 and J05AE05 or N03AC01 and J05AE05 or N03AC02 and J05AE05 or N03AC03 and J05AE05 or N03AD01 and J05AE05 or N03AD02 and J05AE05 or N03AD03 and J05AE05 or N03AD51 and J05AE05 or N03AE01 and J05AE05 or N03AF01 and J05AE05 or N03AF02 and J05AE05 or N03AF03 and J05AE05 or N03AF04 and J05AE05 or N03AG01 and J05AE05 or N03AG02 and J05AE05 or N03AG03 and J05AE05 or N03AG04 and J05AE05 or N03AG05 and J05AE05 or N03AG06 and J05AE05 or N03AX03 and J05AE05 or N03AX07 and J05AE05 or N03AX09 and J05AE05 or N03AX10 and J05AE05 or N03AX11 and J05AE05 or N03AX12 and J05AE05 or N03AX13 and J05AE05 or N03AX14 and J05AE05 or N03AX15 and J05AE05 or N03AX16 and J05AE05 or N03AX17 and J05AE05 or N03AX18 and J05AE05 or N03AX19 and J05AE05 or N03AX21 and J05AE05 or N03AX22 and J05AE05 or N03AX23 and J05AE05 or N03AX24 and J05AE05 or N03AX30 and J05AE05 or N03AA01 and J05AE07 or N03AA02 and J05AE07 or N03AA03 and J05AE07 or N03AA04 and J05AE07 or N03AA30 and J05AE07 or N03AB01 and J05AE07 or N03AB02 and J05AE07 or N03AB03 and J05AE07 or N03AB04 and J05AE07 or N03AB05 and J05AE07 or N03AB52 and J05AE07 or N03AB54 and J05AE07 or N03AC01 and J05AE07 or N03AC02 and J05AE07 or N03AC03 and J05AE07 or N03AD01 and J05AE07 or N03AD02 and J05AE07 or N03AD03 and J05AE07 or N03AD51 and J05AE07 or N03AE01 and J05AE07 or N03AF01 and J05AE07 or N03AF02 and J05AE07 or N03AF03 and J05AE07 or N03AF04 and J05AE07 or N03AG01 and J05AE07 or N03AG02 and J05AE07 or N03AG03 and J05AE07 or N03AG04 and J05AE07 or N03AG05 and J05AE07 or N03AG06 and J05AE07 or N03AX03 and J05AE07 or N03AX07 and J05AE07 or N03AX09 and J05AE07 or N03AX10 and J05AE07 or N03AX11 and J05AE07 or N03AX12 and J05AE07 or N03AX13 and J05AE07 or N03AX14 and J05AE07 or N03AX15 and J05AE07 or N03AX16 and J05AE07 or N03AX17 and J05AE07 or N03AX18 and J05AE07 or N03AX19 and J05AE07 or N03AX21 and J05AE07 or N03AX22 and J05AE07 or N03AX23 and J05AE07 or N03AX24 and J05AE07 or N03AX30 and J05AE07 or N03AA01 and J05AE08 or N03AA02 and J05AE08 or N03AA03 and J05AE08 or N03AA04 and J05AE08 or N03AA30 and J05AE08 or N03AB01 and J05AE08 or N03AB02 and J05AE08 or N03AB03 and J05AE08 or N03AB04 and J05AE08 or N03AB05 and J05AE08 or N03AB52 and J05AE08 or N03AB54 and J05AE08 or N03AC01 and J05AE08 or N03AC02 and J05AE08 or N03AC03 and J05AE08 or N03AD01 and J05AE08 or N03AD02 and J05AE08 or N03AD03 and J05AE08 or N03AD51 and J05AE08 or N03AE01 and J05AE08 or N03AF01 and J05AE08 or N03AF02 and J05AE08 or N03AF03 and J05AE08 or N03AF04 and J05AE08 or N03AG01 and J05AE08 or N03AG02 and J05AE08 or N03AG03 and J05AE08 or N03AG04 and J05AE08 or N03AG05 and J05AE08 or N03AG06 and J05AE08 or N03AX03 and J05AE08 or N03AX07 and J05AE08 or N03AX09 and J05AE08 or N03AX10 and J05AE08 or N03AX11 and J05AE08 or N03AX12 and J05AE08 or N03AX13 and J05AE08 or N03AX14 and J05AE08 or N03AX15 and J05AE08 or N03AX16 and J05AE08 or N03AX17 and J05AE08 or N03AX18 and J05AE08 or N03AX19 and J05AE08 or N03AX21 and J05AE08 or N03AX22 and J05AE08 or N03AX23 and J05AE08 or N03AX24 and J05AE08 or N03AX30 and J05AE08 or N03AA01 and J05AE09 or N03AA02 and J05AE09 or N03AA03 and J05AE09 or N03AA04 and J05AE09 or N03AA30 and J05AE09 or N03AB01 and J05AE09 or N03AB02 and J05AE09 or N03AB03 and J05AE09 or N03AB04 and J05AE09 or N03AB05 and J05AE09 or N03AB52 and J05AE09 or N03AB54 and J05AE09 or N03AC01 and J05AE09 or N03AC02 and J05AE09 or N03AC03 and J05AE09 or N03AD01 and J05AE09 or N03AD02 and J05AE09 or N03AD03 and J05AE09 or N03AD51 and J05AE09 or N03AE01 and J05AE09 or N03AF01 and J05AE09 or N03AF02 and J05AE09 or N03AF03 and J05AE09 or N03AF04 and J05AE09 or N03AG01 and J05AE09 or N03AG02 and J05AE09 or N03AG03 and J05AE09 or N03AG04 and J05AE09 or N03AG05 and J05AE09 or N03AG06 and J05AE09 or N03AX03 and J05AE09 or N03AX07 and J05AE09 or N03AX09 and J05AE09 or N03AX10 and J05AE09 or N03AX11 and J05AE09 or N03AX12 and J05AE09 or N03AX13 and J05AE09 or N03AX14 and J05AE09 or N03AX15 and J05AE09 or N03AX16 and J05AE09 or N03AX17 and J05AE09 or N03AX18 and J05AE09 or N03AX19 and J05AE09 or N03AX21 and J05AE09 or N03AX22 and J05AE09 or N03AX23 and J05AE09 or N03AX24 and J05AE09 or N03AX30 and J05AE09 or N03AA01 and J05AE10 or N03AA02 and J05AE10 or N03AA03 and J05AE10 or N03AA04 and J05AE10 or N03AA30 and J05AE10 or N03AB01 and J05AE10 or N03AB02 and J05AE10 or N03AB03 and J05AE10 or N03AB04 and J05AE10 or N03AB05 and J05AE10 or N03AB52 and J05AE10 or N03AB54 and J05AE10 or N03AC01 and J05AE10 or N03AC02 and J05AE10 or N03AC03 and J05AE10 or N03AD01 and J05AE10 or N03AD02 and J05AE10 or N03AD03 and J05AE10 or N03AD51 and J05AE10 or N03AE01 and J05AE10 or N03AF01 and J05AE10 or N03AF02 and J05AE10 or N03AF03 and J05AE10 or N03AF04 and J05AE10 or N03AG01 and J05AE10 or N03AG02 and J05AE10 or N03AG03 and J05AE10 or N03AG04 and J05AE10 or N03AG05 and J05AE10 or N03AG06 and J05AE10 or N03AX03 and J05AE10 or N03AX07 and J05AE10 or N03AX09 and J05AE10 or N03AX10 and J05AE10 or N03AX11 and J05AE10 or N03AX12 and J05AE10 or N03AX13 and J05AE10 or N03AX14 and J05AE10 or N03AX15 and J05AE10 or N03AX16 and J05AE10 or N03AX17 and J05AE10 or N03AX18 and J05AE10 or N03AX19 and J05AE10 or N03AX21 and J05AE10 or N03AX22 and J05AE10 or N03AX23 and J05AE10 or N03AX24 and J05AE10 or N03AX30 and J05AE10 or N03AA01 and J05AF01 or N03AA02 and J05AF01 or N03AA03 and J05AF01 or N03AA04 and J05AF01 or N03AA30 and J05AF01 or N03AB01 and J05AF01 or N03AB02 and J05AF01 or N03AB03 and J05AF01 or N03AB04 and J05AF01 or N03AB05 and J05AF01 or N03AB52 and J05AF01 or N03AB54 and J05AF01 or N03AC01 and J05AF01 or N03AC02 and J05AF01 or N03AC03 and J05AF01 or N03AD01 and J05AF01 or N03AD02 and J05AF01 or N03AD03 and J05AF01 or N03AD51 and J05AF01 or N03AE01 and J05AF01 or N03AF01 and J05AF01 or N03AF02 and J05AF01 or N03AF03 and J05AF01 or N03AF04 and J05AF01 or N03AG01 and J05AF01 or N03AG02 and J05AF01 or N03AG03 and J05AF01 or N03AG04 and J05AF01 or N03AG05 and J05AF01 or N03AG06 and J05AF01 or N03AX03 and J05AF01 or N03AX07 and J05AF01 or N03AX09 and J05AF01 or N03AX10 and J05AF01 or N03AX11 and J05AF01 or N03AX12 and J05AF01 or N03AX13 and J05AF01 or N03AX14 and J05AF01 or N03AX15 and J05AF01 or N03AX16 and J05AF01 or N03AX17 and J05AF01 or N03AX18 and J05AF01 or N03AX19 and J05AF01 or N03AX21 and J05AF01 or N03AX22 and J05AF01 or N03AX23 and J05AF01 or N03AX24 and J05AF01 or N03AX30 and J05AF01 or N03AA01 and J05AF02 or N03AA02 and J05AF02 or N03AA03 and J05AF02 or N03AA04 and J05AF02 or N03AA30 and J05AF02 or N03AB01 and J05AF02 or N03AB02 and J05AF02 or N03AB03 and J05AF02 or N03AB04 and J05AF02 or N03AB05 and J05AF02 or N03AB52 and J05AF02 or N03AB54 and J05AF02 or N03AC01 and J05AF02 or N03AC02 and J05AF02 or N03AC03 and J05AF02 or N03AD01 and J05AF02 or N03AD02 and J05AF02 or N03AD03 and J05AF02 or N03AD51 and J05AF02 or N03AE01 and J05AF02 or N03AF01 and J05AF02 or N03AF02 and J05AF02 or N03AF03 and J05AF02 or N03AF04 and J05AF02 or N03AG01 and J05AF02 or N03AG02 and J05AF02 or N03AG03 and J05AF02 or N03AG04 and J05AF02 or N03AG05 and J05AF02 or N03AG06 and J05AF02 or N03AX03 and J05AF02 or N03AX07 and J05AF02 or N03AX09 and J05AF02 or N03AX10 and J05AF02 or N03AX11 and J05AF02 or N03AX12 and J05AF02 or N03AX13 and J05AF02 or N03AX14 and J05AF02 or N03AX15 and J05AF02 or N03AX16 and J05AF02 or N03AX17 and J05AF02 or N03AX18 and J05AF02 or N03AX19 and J05AF02 or N03AX21 and J05AF02 or N03AX22 and J05AF02 or N03AX23 and J05AF02 or N03AX24 and J05AF02 or N03AX30 and J05AF02 or N03AA01 and J05AF03 or N03AA02 and J05AF03 or N03AA03 and J05AF03 or N03AA04 and J05AF03 or N03AA30 and J05AF03 or N03AB01 and J05AF03 or N03AB02 and J05AF03 or N03AB03 and J05AF03 or N03AB04 and J05AF03 or N03AB05 and J05AF03 or N03AB52 and J05AF03 or N03AB54 and J05AF03 or N03AC01 and J05AF03 or N03AC02 and J05AF03 or N03AC03 and J05AF03 or N03AD01 and J05AF03 or N03AD02 and J05AF03 or N03AD03 and J05AF03 or N03AD51 and J05AF03 or N03AE01 and J05AF03 or N03AF01 and J05AF03 or N03AF02 and J05AF03 or N03AF03 and J05AF03 or N03AF04 and J05AF03 or N03AG01 and J05AF03 or N03AG02 and J05AF03 or N03AG03 and J05AF03 or N03AG04 and J05AF03 or N03AG05 and J05AF03 or N03AG06 and J05AF03 or N03AX03 and J05AF03 or N03AX07 and J05AF03 or N03AX09 and J05AF03 or N03AX10 and J05AF03 or N03AX11 and J05AF03 or N03AX12 and J05AF03 or N03AX13 and J05AF03 or N03AX14 and J05AF03 or N03AX15 and J05AF03 or N03AX16 and J05AF03 or N03AX17 and J05AF03 or N03AX18 and J05AF03 or N03AX19 and J05AF03 or N03AX21 and J05AF03 or N03AX22 and J05AF03 or N03AX23 and J05AF03 or N03AX24 and J05AF03 or N03AX30 and J05AF03 or N03AA01 and J05AF04 or N03AA02 and J05AF04 or N03AA03 and J05AF04 or N03AA04 and J05AF04 or N03AA30 and J05AF04 or N03AB01 and J05AF04 or N03AB02 and J05AF04 or N03AB03 and J05AF04 or N03AB04 and J05AF04 or N03AB05 and J05AF04 or N03AB52 and J05AF04 or N03AB54 and J05AF04 or N03AC01 and J05AF04 or N03AC02 and J05AF04 or N03AC03 and J05AF04 or N03AD01 and J05AF04 or N03AD02 and J05AF04 or N03AD03 and J05AF04 or N03AD51 and J05AF04 or N03AE01 and J05AF04 or N03AF01 and J05AF04 or N03AF02 and J05AF04 or N03AF03 and J05AF04 or N03AF04 and J05AF04 or N03AG01 and J05AF04 or N03AG02 and J05AF04 or N03AG03 and J05AF04 or N03AG04 and J05AF04 or N03AG05 and J05AF04 or N03AG06 and J05AF04 or N03AX03 and J05AF04 or N03AX07 and J05AF04 or N03AX09 and J05AF04 or N03AX10 and J05AF04 or N03AX11 and J05AF04 or N03AX12 and J05AF04 or N03AX13 and J05AF04 or N03AX14 and J05AF04 or N03AX15 and J05AF04 or N03AX16 and J05AF04 or N03AX17 and J05AF04 or N03AX18 and J05AF04 or N03AX19 and J05AF04 or N03AX21 and J05AF04 or N03AX22 and J05AF04 or N03AX23 and J05AF04 or N03AX24 and J05AF04 or N03AX30 and J05AF04 or N03AA01 and J05AF05 or N03AA02 and J05AF05 or N03AA03 and J05AF05 or N03AA04 and J05AF05 or N03AA30 and J05AF05 or N03AB01 and J05AF05 or N03AB02 and J05AF05 or N03AB03 and J05AF05 or N03AB04 and J05AF05 or N03AB05 and J05AF05 or N03AB52 and J05AF05 or N03AB54 and J05AF05 or N03AC01 and J05AF05 or N03AC02 and J05AF05 or N03AC03 and J05AF05 or N03AD01 and J05AF05 or N03AD02 and J05AF05 or N03AD03 and J05AF05 or N03AD51 and J05AF05 or N03AE01 and J05AF05 or N03AF01 and J05AF05 or N03AF02 and J05AF05 or N03AF03 and J05AF05 or N03AF04 and J05AF05 or N03AG01 and J05AF05 or N03AG02 and J05AF05 or N03AG03 and J05AF05 or N03AG04 and J05AF05 or N03AG05 and J05AF05 or N03AG06 and J05AF05 or N03AX03 and J05AF05 or N03AX07 and J05AF05 or N03AX09 and J05AF05 or N03AX10 and J05AF05 or N03AX11 and J05AF05 or N03AX12 and J05AF05 or N03AX13 and J05AF05 or N03AX14 and J05AF05 or N03AX15 and J05AF05 or N03AX16 and J05AF05 or N03AX17 and J05AF05 or N03AX18 and J05AF05 or N03AX19 and J05AF05 or N03AX21 and J05AF05 or N03AX22 and J05AF05 or N03AX23 and J05AF05 or N03AX24 and J05AF05 or N03AX30 and J05AF05 or N03AA01 and J05AF06 or N03AA02 and J05AF06 or N03AA03 and J05AF06 or N03AA04 and J05AF06 or N03AA30 and J05AF06 or N03AB01 and J05AF06 or N03AB02 and J05AF06 or N03AB03 and J05AF06 or N03AB04 and J05AF06 or N03AB05 and J05AF06 or N03AB52 and J05AF06 or N03AB54 and J05AF06 or N03AC01 and J05AF06 or N03AC02 and J05AF06 or N03AC03 and J05AF06 or N03AD01 and J05AF06 or N03AD02 and J05AF06 or N03AD03 and J05AF06 or N03AD51 and J05AF06 or N03AE01 and J05AF06 or N03AF01 and J05AF06 or N03AF02 and J05AF06 or N03AF03 and J05AF06 or N03AF04 and J05AF06 or N03AG01 and J05AF06 or N03AG02 and J05AF06 or N03AG03 and J05AF06 or N03AG04 and J05AF06 or N03AG05 and J05AF06 or N03AG06 and J05AF06 or N03AX03 and J05AF06 or N03AX07 and J05AF06 or N03AX09 and J05AF06 or N03AX10 and J05AF06 or N03AX11 and J05AF06 or N03AX12 and J05AF06 or N03AX13 and J05AF06 or N03AX14 and J05AF06 or N03AX15 and J05AF06 or N03AX16 and J05AF06 or N03AX17 and J05AF06 or N03AX18 and J05AF06 or N03AX19 and J05AF06 or N03AX21 and J05AF06 or N03AX22 and J05AF06 or N03AX23 and J05AF06 or N03AX24 and J05AF06 or N03AX30 and J05AF06 or N03AA01 and J05AF07 or N03AA02 and J05AF07 or N03AA03 and J05AF07 or N03AA04 and J05AF07 or N03AA30 and J05AF07 or N03AB01 and J05AF07 or N03AB02 and J05AF07 or N03AB03 and J05AF07 or N03AB04 and J05AF07 or N03AB05 and J05AF07 or N03AB52 and J05AF07 or N03AB54 and J05AF07 or N03AC01 and J05AF07 or N03AC02 and J05AF07 or N03AC03 and J05AF07 or N03AD01 and J05AF07 or N03AD02 and J05AF07 or N03AD03 and J05AF07 or N03AD51 and J05AF07 or N03AE01 and J05AF07 or N03AF01 and J05AF07 or N03AF02 and J05AF07 or N03AF03 and J05AF07 or N03AF04 and J05AF07 or N03AG01 and J05AF07 or N03AG02 and J05AF07 or N03AG03 and J05AF07 or N03AG04 and J05AF07 or N03AG05 and J05AF07 or N03AG06 and J05AF07 or N03AX03 and J05AF07 or N03AX07 and J05AF07 or N03AX09 and J05AF07 or N03AX10 and J05AF07 or N03AX11 and J05AF07 or N03AX12 and J05AF07 or N03AX13 and J05AF07 or N03AX14 and J05AF07 or N03AX15 and J05AF07 or N03AX16 and J05AF07 or N03AX17 and J05AF07 or N03AX18 and J05AF07 or N03AX19 and J05AF07 or N03AX21 and J05AF07 or N03AX22 and J05AF07 or N03AX23 and J05AF07 or N03AX24 and J05AF07 or N03AX30 and J05AF07 or N03AA01 and J05AF08 or N03AA02 and J05AF08 or N03AA03 and J05AF08 or N03AA04 and J05AF08 or N03AA30 and J05AF08 or N03AB01 and J05AF08 or N03AB02 and J05AF08 or N03AB03 and J05AF08 or N03AB04 and J05AF08 or N03AB05 and J05AF08 or N03AB52 and J05AF08 or N03AB54 and J05AF08 or N03AC01 and J05AF08 or N03AC02 and J05AF08 or N03AC03 and J05AF08 or N03AD01 and J05AF08 or N03AD02 and J05AF08 or N03AD03 and J05AF08 or N03AD51 and J05AF08 or N03AE01 and J05AF08 or N03AF01 and J05AF08 or N03AF02 and J05AF08 or N03AF03 and J05AF08 or N03AF04 and J05AF08 or N03AG01 and J05AF08 or N03AG02 and J05AF08 or N03AG03 and J05AF08 or N03AG04 and J05AF08 or N03AG05 and J05AF08 or N03AG06 and J05AF08 or N03AX03 and J05AF08 or N03AX07 and J05AF08 or N03AX09 and J05AF08 or N03AX10 and J05AF08 or N03AX11 and J05AF08 or N03AX12 and J05AF08 or N03AX13 and J05AF08 or N03AX14 and J05AF08 or N03AX15 and J05AF08 or N03AX16 and J05AF08 or N03AX17 and J05AF08 or N03AX18 and J05AF08 or N03AX19 and J05AF08 or N03AX21 and J05AF08 or N03AX22 and J05AF08 or N03AX23 and J05AF08 or N03AX24 and J05AF08 or N03AX30 and J05AF08 or N03AA01 and J05AF09 or N03AA02 and J05AF09 or N03AA03 and J05AF09 or N03AA04 and J05AF09 or N03AA30 and J05AF09 or N03AB01 and J05AF09 or N03AB02 and J05AF09 or N03AB03 and J05AF09 or N03AB04 and J05AF09 or N03AB05 and J05AF09 or N03AB52 and J05AF09 or N03AB54 and J05AF09 or N03AC01 and J05AF09 or N03AC02 and J05AF09 or N03AC03 and J05AF09 or N03AD01 and J05AF09 or N03AD02 and J05AF09 or N03AD03 and J05AF09 or N03AD51 and J05AF09 or N03AE01 and J05AF09 or N03AF01 and J05AF09 or N03AF02 and J05AF09 or N03AF03 and J05AF09 or N03AF04 and J05AF09 or N03AG01 and J05AF09 or N03AG02 and J05AF09 or N03AG03 and J05AF09 or N03AG04 and J05AF09 or N03AG05 and J05AF09 or N03AG06 and J05AF09 or N03AX03 and J05AF09 or N03AX07 and J05AF09 or N03AX09 and J05AF09 or N03AX10 and J05AF09 or N03AX11 and J05AF09 or N03AX12 and J05AF09 or N03AX13 and J05AF09 or N03AX14 and J05AF09 or N03AX15 and J05AF09 or N03AX16 and J05AF09 or N03AX17 and J05AF09 or N03AX18 and J05AF09 or N03AX19 and J05AF09 or N03AX21 and J05AF09 or N03AX22 and J05AF09 or N03AX23 and J05AF09 or N03AX24 and J05AF09 or N03AX30 and J05AF09 or N03AA01 and J05AF10 or N03AA02 and J05AF10 or N03AA03 and J05AF10 or N03AA04 and J05AF10 or N03AA30 and J05AF10 or N03AB01 and J05AF10 or N03AB02 and J05AF10 or N03AB03 and J05AF10 or N03AB04 and J05AF10 or N03AB05 and J05AF10 or N03AB52 and J05AF10 or N03AB54 and J05AF10 or N03AC01 and J05AF10 or N03AC02 and J05AF10 or N03AC03 and J05AF10 or N03AD01 and J05AF10 or N03AD02 and J05AF10 or N03AD03 and J05AF10 or N03AD51 and J05AF10 or N03AE01 and J05AF10 or N03AF01 and J05AF10 or N03AF02 and J05AF10 or N03AF03 and J05AF10 or N03AF04 and J05AF10 or N03AG01 and J05AF10 or N03AG02 and J05AF10 or N03AG03 and J05AF10 or N03AG04 and J05AF10 or N03AG05 and J05AF10 or N03AG06 and J05AF10 or N03AX03 and J05AF10 or N03AX07 and J05AF10 or N03AX09 and J05AF10 or N03AX10 and J05AF10 or N03AX11 and J05AF10 or N03AX12 and J05AF10 or N03AX13 and J05AF10 or N03AX14 and J05AF10 or N03AX15 and J05AF10 or N03AX16 and J05AF10 or N03AX17 and J05AF10 or N03AX18 and J05AF10 or N03AX19 and J05AF10 or N03AX21 and J05AF10 or N03AX22 and J05AF10 or N03AX23 and J05AF10 or N03AX24 and J05AF10 or N03AX30 and J05AF10 or N03AA01 and J05AF11 or N03AA02 and J05AF11 or N03AA03 and J05AF11 or N03AA04 and J05AF11 or N03AA30 and J05AF11 or N03AB01 and J05AF11 or N03AB02 and J05AF11 or N03AB03 and J05AF11 or N03AB04 and J05AF11 or N03AB05 and J05AF11 or N03AB52 and J05AF11 or N03AB54 and J05AF11 or N03AC01 and J05AF11 or N03AC02 and J05AF11 or N03AC03 and J05AF11 or N03AD01 and J05AF11 or N03AD02 and J05AF11 or N03AD03 and J05AF11 or N03AD51 and J05AF11 or N03AE01 and J05AF11 or N03AF01 and J05AF11 or N03AF02 and J05AF11 or N03AF03 and J05AF11 or N03AF04 and J05AF11 or N03AG01 and J05AF11 or N03AG02 and J05AF11 or N03AG03 and J05AF11 or N03AG04 and J05AF11 or N03AG05 and J05AF11 or N03AG06 and J05AF11 or N03AX03 and J05AF11 or N03AX07 and J05AF11 or N03AX09 and J05AF11 or N03AX10 and J05AF11 or N03AX11 and J05AF11 or N03AX12 and J05AF11 or N03AX13 and J05AF11 or N03AX14 and J0SAF11 or N03AX15 and J05AF11 or N03AX16 and J05AF11 or N03AX17 and J05AF11 or N03AX18 and J05AF11 or N03AX19 and J05AF11 or N03AX21 and J05AF11 or N03AX22 and J05AF11 or N03AX23 and J05AF11 or N03AX24 and J05AF11 or N03AX30 and J05AF11 or N03AA01 and J05AF12 or N03AA02 and J05AF12 or N03AA03 and J05AF12 or N03AA04 and J05AF12 or N03AA30 and J05AF12 or N03AB01 and J05AF12 or N03AB02 and J05AF12 or N03AB03 and J05AF12 or N03AB04 and J05AF12 or N03AB05 and J05AF12 or N03AB52 and J05AF12 or N03AB54 and J05AF12 or N03AC01 and J05AF12 or N03AC02 and J05AF12 or N03AC03 and J05AF12 or N03AD01 and J05AF12 or N03AD02 and J05AF12 or N03AD03 and J05AF12 or N03AD51 and J05AF12 or N03AE01 and J05AF12 or N03AF01 and J05AF12 or N03AF02 and J05AF12 or N03AF03 and J05AF12 or N03AF04 and J05AF12 or N03AG01 and J05AF12 or N03AG02 and J05AF12 or N03AG03 and J05AF12 or N03AG04 and J05AF12 or N03AG05 and J05AF12 or N03AG06 and J05AF12 or N03AX03 and J05AF12 or N03AX07 and J05AF12 or N03AX09 and J05AF12 or N03AX10 and J05AF12 or N03AX11 and J05AF12 or N03AX12 and J05AF12 or N03AX13 and J05AF12 or N03AX14 and J05AF12 or N03AX15 and J05AF12 or N03AX16 and J05AF12 or N03AX17 and J05AF12 or N03AX18 and J05AF12 or N03AX19 and J05AF12 or N03AX21 and J05AF12 or N03AX22 and J05AF12 or N03AX23 and J05AF12 or N03AX24 and J05AF12 or N03AX30 and J05AF12 or N03AA01 and J05AF13 or N03AA02 and J05AF13 or N03AA03 and J05AF13 or N03AA04 and J05AF13 or N03AA30 and J05AF13 or N03AB01 and J05AF13 or N03AB02 and J05AF13 or N03AB03 and J05AF13 or N03AB04 and J05AF13 or N03AB05 and J05AF13 or N03AB52 and J05AF13 or N03AB54 and J05AF13 or N03AC01 and J05AF13 or N03AC02 and J05AF13 or N03AC03 and J05AF13 or N03AD01 and J05AF13 or N03AD02 and J05AF13 or N03AD03 and J05AF13 or N03AD51 and J05AF13 or N03AE01 and J05AF13 or N03AF01 and J05AF13 or N03AF02 and J05AF13 or N03AF03 and J05AF13 or N03AF04 and J05AF13 or N03AG01 and J05AF13 or N03AG02 and J05AF13 or N03AG03 and J05AF13 or N03AG04 and J05AF13 or N03AG05 and J05AF13 or N03AG06 and J05AF13 or N03AX03 and J05AF13 or N03AX07 and J05AF13 or N03AX09 and J05AF13 or N03AX10 and J05AF13 or N03AX11 and J05AF13 or N03AX12 and J05AF13 or N03AX13 and J05AF13 or N03AX14 and J05AF13 or N03AX15 and J05AF13 or N03AX16 and J05AF13 or N03AX17 and J05AF13 or N03AX18 and J05AF13 or N03AX19 and J05AF13 or N03AX21 and J05AF13 or N03AX22 and J05AF13 or N03AX23 and J05AF13 or N03AX24 and J05AF13 or N03AX30 and J05AF13 or N03AA01 and J05AG01 or N03AA02 and J05AG01 or N03AA03 and J05AG01 or N03AA04 and J05AG01 or N03AA30 and J05AG01 or N03AB01 and J05AG01 or N03AB02 and J05AG01 or N03AB03 and J05AG01 or N03AB04 and J05AG01 or N03AB05 and J05AG01 or N03AB52 and J05AG01 or N03AB54 and J05AG01 or N03AC01 and J05AG01 or N03AC02 and J05AG01 or N03AC03 and J05AG01 or N03AD01 and J05AG01 or N03AD02 and J05AG01 or N03AD03 and J05AG01 or N03AD51 and J05AG01 or N03AE01 and J05AG01 or N03AF01 and J05AG01 or N03AF02 and J05AG01 or N03AF03 and J05AG01 or N03AF04 and J05AG01 or N03AG01 and J05AG01 or N03AG02 and J05AG01 or N03AG03 and J05AG01 or N03AG04 and J05AG01 or N03AG05 and J05AG01 or N03AG06 and J05AG01 or N03AX03 and J05AG01 or N03AX07 and J05AG01 or N03AX09 and J05AG01 or N03AX10 and J05AG01 or N03AX11 and J05AG01 or N03AX12 and J05AG01 or N03AX13 and J05AG01 or N03AX14 and J05AG01 or N03AX15 and J05AG01 or N03AX16 and J05AG01 or N03AX17 and J05AG01 or N03AX18 and J05AG01 or N03AX19 and J05AG01 or N03AX21 and J05AG01 or N03AX22 and J05AG01 or N03AX23 and J05AG01 or N03AX24 and J05AG01 or N03AX30 and J05AG01 or N03AA01 and J05AG02 or N03AA02 and J05AG02 or N03AA03 and J05AG02 or N03AA04 and J05AG02 or N03AA30 and J05AG02 or N03AB01 and J05AG02 or N03AB02 and J05AG02 or N03AB03 and J05AG02 or N03AB04 and J05AG02 or N03AB05 and J05AG02 or N03AB52 and J05AG02 or N03AB54 and J05AG02 or N03AC01 and J05AG02 or N03AC02 and J05AG02 or N03AC03 and J05AG02 or N03AD01 and J05AG02 or N03AD02 and J05AG02 or N03AD03 and J05AG02 or N03AD51 and J05AG02 or N03AE01 and J05AG02 or N03AF01 and J05AG02 or N03AF02 and J05AG02 or N03AF03 and J05AG02 or N03AF04 and J05AG02 or N03AG01 and J05AG02 or N03AG02 and J05AG02 or N03AG03 and J05AG02 or N03AG04 and J05AG02 or N03AG05 and J05AG02 or N03AG06 and J05AG02 or N03AX03 and J05AG02 or N03AX07 and J05AG02 or N03AX09 and J05AG02 or N03AX10 and J05AG02 or N03AX11 and J05AG02 or N03AX12 and J05AG02 or N03AX13 and J05AG02 or N03AX14 and J05AG02 or N03AX15 and J05AG02 or N03AX16 and J05AG02 or N03AX17 and J05AG02 or N03AX18 and J05AG02 or N03AX19 and J05AG02 or N03AX21 and J05AG02 or N03AX22 and J05AG02 or N03AX23 and J05AG02 or N03AX24 and J05AG02 or N03AX30 and J05AG02 or N03AA01 and J05AG03 or N03AA02 and J05AG03 or N03AA03 and J05AG03 or N03AA04 and J05AG03 or N03AA30 and J05AG03 or N03AB01 and J05AG03 or N03AB02 and J05AG03 or N03AB03 and J05AG03 or N03AB04 and J05AG03 or N03AB05 and J05AG03 or N03AB52 and J05AG03 or N03AB54 and J05AG03 or N03AC01 and J05AG03 or N03AC02 and J05AG03 or N03AC03 and J05AG03 or N03AD01 and J05AG03 or N03AD02 and J05AG03 or N03AD03 and J05AG03 or N03AD51 and J05AG03 or N03AE01 and J05AG03 or N03AF01 and J05AG03 or N03AF02 and J05AG03 or N03AF03 and J05AG03 or N03AF04 and J05AG03 or N03AG01 and J05AG03 or N03AG02 and J05AG03 or N03AG03 and J05AG03 or N03AG04 and J05AG03 or N03AG05 and J05AG03 or N03AG06 and J05AG03 or N03AX03 and J05AG03 or N03AX07 and J05AG03 or N03AX09 and J05AG03 or N03AX10 and J05AG03 or N03AX11 and J05AG03 or N03AX12 and J05AG03 or N03AX13 and J05AG03 or N03AX14 and J05AG03 or N03AX15 and J05AG03 or N03AX16 and J05AG03 or N03AX17 and J05AG03 or N03AX18 and J05AG03 or N03AX19 and J05AG03 or N03AX21 and J05AG03 or N03AX22 and J05AG03 or N03AX23 and J05AG03 or N03AX24 and J05AG03 or N03AX30 and J05AG03 or N03AA01 and J05AG04 or N03AA02 and J05AG04 or N03AA03 and J05AG04 or N03AA04 and J05AG04 or N03AA30 and J05AG04 or N03AB01 and J05AG04 or N03AB02 and J05AG04 or N03AB03 and J05AG04 or N03AB04 and J05AG04 or N03AB05 and J05AG04 or N03AB52 and J05AG04 or N03AB54 and J05AG04 or N03AC01 and J05AG04 or N03AC02 and J05AG04 or N03AC03 and J05AG04 or N03AD01 and J05AG04 or N03AD02 and J05AG04 or N03AD03 and J05AG04 or N03AD51 and J05AG04 or N03AE01 and J05AG04 or N03AF01 and J05AG04 or N03AF02 and J05AG04 or N03AF03 and J05AG04 or N03AF04 and J05AG04 or N03AG01 and J05AG04 or N03AG02 and J05AG04 or N03AG03 and J05AG04 or N03AG04 and J05AG04 or N03AG05 and J05AG04 or N03AG06 and J05AG04 or N03AX03 and J05AG04 or N03AX07 and J05AG04 or N03AX09 and J05AG04 or N03AX10 and J05AG04 or N03AX11 and J05AG04 or N03AX12 and J05AG04 or N03AX13 and J05AG04 or N03AX14 and J05AG04 or N03AX15 and J05AG04 or N03AX16 and J05AG04 or N03AX17 and J05AG04 or N03AX18 and J05AG04 or N03AX19 and J05AG04 or N03AX21 and J05AG04 or N03AX22 and J05AG04 or N03AX23 and J05AG04 or N03AX24 and J05AG04 or N03AX30 and J05AG04 or N03AA01 and J05AG05 or N03AA02 and J05AG05 or N03AA03 and J05AG05 or N03AA04 and J05AG05 or N03AA30 and J05AG05 or N03AB01 and J05AG05 or N03AB02 and J05AG05 or N03AB03 and J05AG05 or N03AB04 and J05AG05 or N03AB05 and J05AG05 or N03AB52 and J05AG05 or N03AB54 and J05AG05 or N03AC01 and J05AG05 or N03AC02 and J05AG05 or N03AC03 and J05AG05 or N03AD01 and J05AG05 or N03AD02 and J05AG05 or N03AD03 and J05AG05 or N03AD51 and J05AG05 or N03AE01 and J05AG05 or N03AF01 and J05AG05 or N03AF02 and J05AG05 or N03AF03 and J05AG05 or N03AF04 and J05AG05 or N03AG01 and J05AG05 or N03AG02 and J05AG05 or N03AG03 and J05AG05 or N03AG04 and J05AG05 or N03AG05 and J05AG05 or N03AG06 and J05AG05 or N03AX03 and J05AG05 or N03AX07 and J05AG05 or N03AX09 and J05AG05 or N03AX10 and J05AG05 or N03AX11 and J05AG05 or N03AX12 and J05AG05 or N03AX13 and J05AG05 or N03AX14 and J05AG05 or N03AX15 and J05AG05 or N03AX16 and J05AG05 or N03AX17 and J05AG05 or N03AX18 and J05AG05 or N03AX19 and J05AG05 or N03AX21 and J05AG05 or N03AX22 and J05AG05 or N03AX23 and J05AG05 or N03AX24 and J05AG05 or N03AX30 and J05AG05 or N03AA01 and J05AG06 or N03AA02 and J05AG06 or N03AA03 and J05AG06 or N03AA04 and J05AG06 or N03AA30 and J05AG06 or N03AB01 and J05AG06 or N03AB02 and J05AG06 or N03AB03 and J05AG06 or N03AB04 and J05AG06 or N03AB05 and J05AG06 or N03AB52 and J05AG06 or N03AB54 and J05AG06 or N03AC01 and J05AG06 or N03AC02 and J05AG06 or N03AC03 and J05AG06 or N03AD01 and J05AG06 or N03AD02 and J05AG06 or N03AD03 and J05AG06 or N03AD51 and J05AG06 or N03AE01 and J05AG06 or N03AF01 and J05AG06 or N03AF02 and J05AG06 or N03AF03 and J05AG06 or N03AF04 and J05AG06 or N03AG01 and J05AG06 or N03AG02 and J05AG06 or N03AG03 and J05AG06 or N03AG04 and J05AG06 or N03AG05 and J05AG06 or N03AG06 and J05AG06 or N03AX03 and J05AG06 or N03AX07 and J05AG06 or N03AX09 and J05AG06 or N03AX10 and J05AG06 or N03AX11 and J05AG06 or N03AX12 and J05AG06 or N03AX13 and J05AG06 or N03AX14 and J05AG06 or N03AX15 and J05AG06 or N03AX16 and J05AG06 or N03AX17 and J05AG06 or N03AX18 and J05AG06 or N03AX19 and J05AG06 or N03AX21 and J05AG06 or N03AX22 and J05AG06 or N03AX23 and J05AG06 or N03AX24 and J05AG06 or N03AX30 and J05AG06 or N03AA01 and J05AH01 or N03AA02 and J05AH01 or N03AA03 and J05AH01 or N03AA04 and J05AH01 or N03AA30 and J05AH01 or N03AB01 and J05AH01 or N03AB02 and J05AH01 or N03AB03 and J05AH01 or N03AB04 and J05AH01 or N03AB05 and J05AH01 or N03AB52 and J05AH01 or N03AB54 and J05AH01 or N03AC01 and J05AH01 or N03AC02 and J05AH01 or N03AC03 and J05AH01 or N03AD01 and J05AH01 or N03AD02 and J05AH01 or N03AD03 and J05AH01 or N03AD51 and J05AH01 or N03AE01 and J05AH01 or N03AF01 and J05AH01 or N03AF02 and J05AH01 or N03AF03 and J05AH01 or N03AF04 and J05AH01 or N03AG01 and J05AH01 or N03AG02 and J05AH01 or N03AG03 and J05AH01 or N03AG04 and J05AH01 or N03AG05 and J05AH01 or N03AG06 and J05AH01 or N03AX03 and J05AH01 or N03AX07 and J05AH01 or N03AX09 and J05AH01 or N03AX10 and J05AH01 or N03AX11 and J05AH01 or N03AX12 and J05AH01 or N03AX13 and J05AH01 or N03AX14 and J05AH01 or N03AX15 and J05AH01 or N03AX16 and J05AH01 or N03AX17 and J05AH01 or N03AX18 and J05AH01 or N03AX19 and J05AH01 or N03AX21 and J05AH01 or N03AX22 and J05AH01 or N03AX23 and J05AH01 or N03AX24 and J05AH01 or N03AX30 and J05AH01 or N03AA01 and J05AH02 or N03AA02 and J05AH02 or N03AA03 and J05AH02 or N03AA04 and J05AH02 or N03AA30 and J05AH02 or N03AB01 and J05AH02 or N03AB02 and J05AH02 or N03AB03 and J05AH02 or N03AB04 and J05AH02 or N03AB05 and J05AH02 or N03AB52 and J05AH02 or N03AB54 and J05AH02 or N03AC01 and J05AH02 or N03AC02 and J05AH02 or N03AC03 and J05AH02 or N03AD01 and J05AH02 or N03AD02 and J05AH02 or N03AD03 and J05AH02 or N03AD51 and J05AH02 or N03AE01 and J05AH02 or N03AF01 and J05AH02 or N03AF02 and J05AH02 or N03AF03 and J05AH02 or N03AF04 and J05AH02 or N03AG01 and J05AH02 or N03AG02 and J05AH02 or N03AG03 and J05AH02 or N03AG04 and J05AH02 or N03AG05 and J05AH02 or N03AG06 and J05AH02 or N03AX03 and J05AH02 or N03AX07 and J05AH02 or N03AX09 and J05AH02 or N03AX10 and J05AH02 or N03AX11 and J05AH02 or N03AX12 and J05AH02 or N03AX13 and J05AH02 or N03AX14 and J05AH02 or N03AX15 and J05AH02 or N03AX16 and J05AH02 or N03AX17 and J05AH02 or N03AX18 and J05AH02 or N03AX19 and J05AH02 or N03AX21 and J05AH02 or N03AX22 and J05AH02 or N03AX23 and J05AH02 or N03AX24 and J05AH02 or N03AX30 and J05AH02 or N03AA01 and J05AH03 or N03AA02 and J05AH03 or N03AA03 and J05AH03 or N03AA04 and J05AH03 or N03AA30 and J05AH03 or N03AB01 and J05AH03 or N03AB02 and J05AH03 or N03AB03 and J05AH03 or N03AB04 and J05AH03 or N03AB05 and J05AH03 or N03AB52 and J05AH03 or N03AB54 and J05AH03 or N03AC01 and J05AH03 or N03AC02 and J05AH03 or N03AC03 and J05AH03 or N03AD01 and J05AH03 or N03AD02 and J05AH03 or N03AD03 and J05AH03 or N03AD51 and J05AH03 or N03AE01 and J05AH03 or N03AF01 and J05AH03 or N03AF02 and J05AH03 or N03AF03 and J05AH03 or N03AF04 and J05AH03 or N03AG01 and J05AH03 or N03AG02 and J05AH03 or N03AG03 and J05AH03 or N03AG04 and J05AH03 or N03AG05 and J05AH03 or N03AG06 and J05AH03 or N03AX03 and J05AH03 or N03AX07 and J05AH03 or N03AX09 and J05AH03 or N03AX10 and J05AH03 or N03AX11 and J05AH03 or N03AX12 and J05AH03 or N03AX13 and J05AH03 or N03AX14 and J05AH03 or N03AX15 and J05AH03 or N03AX16 and J05AH03 or N03AX17 and J05AH03 or N03AX18 and J05AH03 or N03AX19 and J05AH03 or N03AX21 and J05AH03 or N03AX22 and J05AH03 or N03AX23 and J05AH03 or N03AX24 and J05AH03 or N03AX30 and J05AH03 or N03AA01 and J05AP01 or N03AA02 and J05AP01 or N03AA03 and J05AP01 or N03AA04 and J05AP01 or N03AA30 and J05AP01 or N03AB01 and J05AP01 or N03AB02 and J05AP01 or N03AB03 and J05AP01 or N03AB04 and J05AP01 or N03AB05 and J05AP01 or N03AB52 and J05AP01 or N03AB54 and J05AP01 or N03AC01 and J05AP01 or N03AC02 and J05AP01 or N03AC03 and J05AP01 or N03AD01 and J05AP01 or N03AD02 and J05AP01 or N03AD03 and J05AP01 or N03AD51 and J05AP01 or N03AE01 and J05AP01 or N03AF01 and J05AP01 or N03AF02 and J05AP01 or N03AF03 and J05AP01 or N03AF04 and J05AP01 or N03AG01 and J05AP01 or N03AG02 and J05AP01 or N03AG03 and J05AP01 or N03AG04 and J05AP01 or N03AG05 and J05AP01 or N03AG06 and J05AP01 or N03AX03 and J05AP01 or N03AX07 and J05AP01 or N03AX09 and J05AP01 or N03AX10 and J05AP01 or N03AX11 and J05AP01 or N03AX12 and J05AP01 or N03AX13 and J05AP01 or N03AX14 and J05AP01 or N03AX15 and J05AP01 or N03AX16 and J05AP01 or N03AX17 and J05AP01 or N03AX18 and J05AP01 or N03AX19 and J05AP01 or N03AX21 and J05AP01 or N03AX22 and J05AP01 or N03AX23 and J05AP01 or N03AX24 and J05AP01 or N03AX30 and J05AP01 or N03AA01 and J05AP02 or N03AA02 and J05AP02 or N03AA03 and J05AP02 or N03AA04 and J05AP02 or N03AA30 and J05AP02 or N03AB01 and J05AP02 or N03AB02 and J05AP02 or N03AB03 and J05AP02 or N03AB04 and J05AP02 or N03AB05 and J05AP02 or N03AB52 and J05AP02 or N03AB54 and J05AP02 or N03AC01 and J05AP02 or N03AC02 and J05AP02 or N03AC03 and J05AP02 or N03AD01 and J05AP02 or N03AD02 and J05AP02 or N03AD03 and J05AP02 or N03AD51 and J05AP02 or N03AE01 and J05AP02 or N03AF01 and J05AP02 or N03AF02 and J05AP02 or N03AF03 and J05AP02 or N03AF04 and J05AP02 or N03AG01 and J05AP02 or N03AG02 and J05AP02 or N03AG03 and J05AP02 or N03AG04 and J05AP02 or N03AG05 and J05AP02 or N03AG06 and J05AP02 or N03AX03 and J05AP02 or N03AX07 and J05AP02 or N03AX09 and J05AP02 or N03AX10 and J05AP02 or N03AX11 and J05AP02 or N03AX12 and J05AP02 or N03AX13 and J05AP02 or N03AX14 and J05AP02 or N03AX15 and J05AP02 or N03AX16 and J05AP02 or N03AX17 and J05AP02 or N03AX18 and J05AP02 or N03AX19 and J05AP02 or N03AX21 and J05AP02 or N03AX22 and J05AP02 or N03AX23 and J05AP02 or N03AX24 and J05AP02 or N03AX30 and J05AP02 or N03AA01 and J05AP03 or N03AA02 and J05AP03 or N03AA03 and J05AP03 or N03AA04 and J05AP03 or N03AA30 and J05AP03 or N03AB01 and J05AP03 or N03AB02 and J05AP03 or N03AB03 and J05AP03 or N03AB04 and J05AP03 or N03AB05 and J05AP03 or N03AB52 and J05AP03 or N03AB54 and J05AP03 or N03AC01 and J05AP03 or N03AC02 and J05AP03 or N03AC03 and J05AP03 or N03AD01 and J05AP03 or N03AD02 and J05AP03 or N03AD03 and J05AP03 or N03AD51 and J05AP03 or N03AE01 and J05AP03 or N03AF01 and J05AP03 or N03AF02 and J05AP03 or N03AF03 and J05AP03 or N03AF04 and J05AP03 or N03AG01 and J05AP03 or N03AG02 and J05AP03 or N03AG03 and J05AP03 or N03AG04 and J05AP03 or N03AG05 and J05AP03 or N03AG06 and J05AP03 or N03AX03 and J05AP03 or N03AX07 and J05AP03 or N03AX09 and J05AP03 or N03AX10 and J05AP03 or N03AX11 and J05AP03 or N03AX12 and J05AP03 or N03AX13 and J05AP03 or N03AX14 and J05AP03 or N03AX15 and J05AP03 or N03AX16 and J05AP03 or N03AX17 and J05AP03 or N03AX18 and J05AP03 or N03AX19 and J05AP03 or N03AX21 and J05AP03 or N03AX22 and J05AP03 or N03AX23 and J05AP03 or N03AX24 and J05AP03 or N03AX30 and J05AP03 or N03AA01 and J05AP04 or N03AA02 and J05AP04 or N03AA03 and J05AP04 or N03AA04 and J05AP04 or N03AA30 and J05AP04 or N03AB01 and J05AP04 or N03AB02 and J05AP04 or N03AB03 and J05AP04 or N03AB04 and J05AP04 or N03AB05 and J05AP04 or N03AB52 and J05AP04 or N03AB54 and J05AP04 or N03AC01 and J05AP04 or N03AC02 and J05AP04 or N03AC03 and J05AP04 or N03AD01 and J05AP04 or N03AD02 and J05AP04 or N03AD03 and J05AP04 or N03AD51 and J05AP04 or N03AE01 and J05AP04 or N03AF01 and J05AP04 or N03AF02 and J05AP04 or N03AF03 and J05AP04 or N03AF04 and J05AP04 or N03AG01 and J05AP04 or N03AG02 and J05AP04 or N03AG03 and J05AP04 or N03AG04 and J05AP04 or N03AG05 and J05AP04 or N03AG06 and J05AP04 or N03AX03 and J05AP04 or N03AX07 and J05AP04 or N03AX09 and J05AP04 or N03AX10 and J05AP04 or N03AX11 and J05AP04 or N03AX12 and J05AP04 or N03AX13 and J05AP04 or N03AX14 and J05AP04 or N03AX15 and J05AP04 or N03AX16 and J05AP04 or N03AX17 and J05AP04 or N03AX18 and J05AP04 or N03AX19 and J05AP04 or N03AX21 and J05AP04 or N03AX22 and J05AP04 or N03AX23 and J05AP04 or N03AX24 and J05AP04 or N03AX30 and J05AP04 or N03AA01 and J05AP05 or N03AA02 and J05AP05 or N03AA03 and J05AP05 or N03AA04 and J05AP05 or N03AA30 and J05AP05 or N03AB01 and J05AP05 or N03AB02 and J05AP05 or N03AB03 and J05AP05 or N03AB04 and J05AP05 or N03AB05 and J05AP05 or N03AB52 and J05AP05 or N03AB54 and J05AP05 or N03AC01 and J05AP05 or N03AC02 and J05AP05 or N03AC03 and J05AP05 or N03AD01 and J05AP05 or N03AD02 and J05AP05 or N03AD03 and J05AP05 or N03AD51 and J05AP05 or N03AE01 and J05AP05 or N03AF01 and J05AP05 or N03AF02 and J05AP05 or N03AF03 and J05AP05 or N03AF04 and J05AP05 or N03AG01 and J05AP05 or N03AG02 and J05AP05 or N03AG03 and J05AP05 or N03AG04 and J05AP05 or N03AG05 and J05AP05 or N03AG06 and J05AP05 or N03AX03 and J05AP05 or N03AX07 and J05AP05 or N03AX09 and J05AP05 or N03AX10 and J05AP05 or N03AX11 and J05AP05 or N03AX12 and J05AP05 or N03AX13 and J05AP05 or N03AX14 and J05AP05 or N03AX15 and J05AP05 or N03AX16 and J05AP05 or N03AX17 and J05AP05 or N03AX18 and J05AP05 or N03AX19 and J05AP05 or N03AX21 and J05AP05 or N03AX22 and J05AP05 or N03AX23 and J05AP05 or N03AX24 and J05AP05 or N03AX30 and J05AP05 or N03AA01 and J05AP06 or N03AA02 and J05AP06 or N03AA03 and J05AP06 or N03AA04 and J05AP06 or N03AA30 and J05AP06 or N03AB01 and J05AP06 or N03AB02 and J05AP06 or N03AB03 and J05AP06 or N03AB04 and J05AP06 or N03AB05 and J05AP06 or N03AB52 and J05AP06 or N03AB54 and J05AP06 or N03AC01 and J05AP06 or N03AC02 and J05AP06 or N03AC03 and J05AP06 or N03AD01 and J05AP06 or N03AD02 and J05AP06 or N03AD03 and J05AP06 or N03AD51 and J05AP06 or N03AE01 and J05AP06 or N03AF01 and J05AP06 or N03AF02 and J05AP06 or N03AF03 and J05AP06 or N03AF04 and J05AP06 or N03AG01 and J05AP06 or N03AG02 and J05AP06 or N03AG03 and J05AP06 or N03AG04 and J05AP06 or N03AG05 and J05AP06 or N03AG06 and J05AP06 or N03AX03 and J05AP06 or N03AX07 and J05AP06 or N03AX09 and J05AP06 or N03AX10 and J05AP06 or N03AX11 and J05AP06 or N03AX12 and J05AP06 or N03AX13 and J05AP06 or N03AX14 and J05AP06 or N03AX15 and J05AP06 or N03AX16 and J05AP06 or N03AX17 and J05AP06 or N03AX18 and J05AP06 or N03AX19 and J05AP06 or N03AX21 and J05AP06 or N03AX22 and J05AP06 or N03AX23 and J05AP06 or N03AX24 and J05AP06 or N03AX30 and J05AP06 or N03AA01 and J05AP07 or N03AA02 and J05AP07 or N03AA03 and J05AP07 or N03AA04 and J05AP07 or N03AA30 and J05AP07 or N03AB01 and J05AP07 or N03AB02 and J05AP07 or N03AB03 and J05AP07 or N03AB04 and J05AP07 or N03AB05 and J05AP07 or N03AB52 and J05AP07 or N03AB54 and J05AP07 or N03AC01 and J05AP07 or N03AC02 and J05AP07 or N03AC03 and J05AP07 or N03AD01 and J05AP07 or N03AD02 and J05AP07 or N03AD03 and J05AP07 or N03AD51 and J05AP07 or N03AE01 and J05AP07 or N03AF01 and J05AP07 or N03AF02 and J05AP07 or N03AF03 and J05AP07 or N03AF04 and J05AP07 or N03AG01 and J05AP07 or N03AG02 and J05AP07 or N03AG03 and J05AP07 or N03AG04 and J05AP07 or N03AG05 and J05AP07 or N03AG06 and J05AP07 or N03AX03 and J05AP07 or N03AX07 and J05AP07 or N03AX09 and J05AP07 or N03AX10 and J05AP07 or N03AX11 and J05AP07 or N03AX12 and J05AP07 or N03AX13 and J05AP07 or N03AX14 and J05AP07 or N03AX15 and J05AP07 or N03AX16 and J05AP07 or N03AX17 and J05AP07 or N03AX18 and J05AP07 or N03AX19 and J05AP07 or N03AX21 and J05AP07 or N03AX22 and J05AP07 or N03AX23 and J05AP07 or N03AX24 and J05AP07 or N03AX30 and J05AP07 or N03AA01 and J05AP08 or N03AA02 and J05AP08 or N03AA03 and J05AP08 or N03AA04 and J05AP08 or N03AA30 and J05AP08 or N03AB01 and J05AP08 or N03AB02 and J05AP08 or N03AB03 and J05AP08 or N03AB04 and J05AP08 or N03AB05 and J05AP08 or N03AB52 and J05AP08 or N03AB54 and J05AP08 or N03AC01 and J05AP08 or N03AC02 and J05AP08 or N03AC03 and J05AP08 or N03AD01 and J05AP08 or N03AD02 and J05AP08 or N03AD03 and J05AP08 or N03AD51 and J05AP08 or N03AE01 and J05AP08 or N03AF01 and J05AP08 or N03AF02 and J05AP08 or N03AF03 and J05AP08 or N03AF04 and J05AP08 or N03AG01 and J05AP08 or N03AG02 and J05AP08 or N03AG03 and J05AP08 or N03AG04 and J05AP08 or N03AG05 and J05AP08 or N03AG06 and J05AP08 or N03AX03 and J05AP08 or N03AX07 and J05AP08 or N03AX09 and J05AP08 or N03AX10 and J05AP08 or N03AX11 and J05AP08 or N03AX12 and J05AP08 or N03AX13 and J05AP08 or N03AX14 and J05AP08 or N03AX15 and J05AP08 or N03AX16 and J05AP08 or N03AX17 and J05AP08 or N03AX18 and J05AP08 or N03AX19 and J05AP08 or N03AX21 and J05AP08 or N03AX22 and J05AP08 or N03AX23 and J05AP08 or N03AX24 and J05AP08 or N03AX30 and J05AP08 or N03AA01 and J05AP09 or N03AA02 and J05AP09 or N03AA03 and J05AP09 or N03AA04 and J05AP09 or N03AA30 and J05AP09 or N03AB01 and J05AP09 or N03AB02 and J05AP09 or N03AB03 and J05AP09 or N03AB04 and J05AP09 or N03AB05 and J05AP09 or N03AB52 and J05AP09 or N03AB54 and J05AP09 or N03AC01 and J05AP09 or N03AC02 and J05AP09 or N03AC03 and J05AP09 or N03AD01 and J05AP09 or N03AD02 and J05AP09 or N03AD03 and J05AP09 or N03AD51 and J05AP09 or N03AE01 and J05AP09 or N03AF01 and J05AP09 or N03AF02 and J05AP09 or N03AF03 and J05AP09 or N03AF04 and J05AP09 or N03AG01 and J05AP09 or N03AG02 and J05AP09 or N03AG03 and J05AP09 or N03AG04 and J05AP09 or N03AG05 and J05AP09 or N03AG06 and J05AP09 or N03AX03 and J05AP09 or N03AX07 and J05AP09 or N03AX09 and J05AP09 or N03AX10 and J05AP09 or N03AX11 and J05AP09 or N03AX12 and J05AP09 or N03AX13 and J05AP09 or N03AX14 and J05AP09 or N03AX15 and J05AP09 or N03AX16 and J05AP09 or N03AX17 and J05AP09 or N03AX18 and J05AP09 or N03AX19 and J05AP09 or N03AX21 and J05AP09 or N03AX22 and J05AP09 or N03AX23 and J05AP09 or N03AX24 and J05AP09 or N03AX30 and J05AP09 or N03AA01 and J05AP51 or N03AA02 and J05AP51 or N03AA03 and J05AP51 or N03AA04 and J05AP51 or N03AA30 and J05AP51 or N03AB01 and J05AP51 or N03AB02 and J05AP51 or N03AB03 and J05AP51 or N03AB04 and J05AP51 or N03AB05 and J05AP51 or N03AB52 and J05AP51 or N03AB54 and J05AP51 or N03AC01 and J05AP51 or N03AC02 and J05AP51 or N03AC03 and J05AP51 or N03AD01 and J05AP51 or N03AD02 and J05AP51 or N03AD03 and J05AP51 or N03AD51 and J05AP51 or N03AE01 and J05AP51 or N03AF01 and J05AP51 or N03AF02 and J05AP51 or N03AF03 and J05AP51 or N03AF04 and J05AP51 or N03AG01 and J05AP51 or N03AG02 and J05AP51 or N03AG03 and J05AP51 or N03AG04 and J05AP51 or N03AG05 and J05AP51 or N03AG06 and J05AP51 or N03AX03 and J05AP51 or N03AX07 and J05AP51 or N03AX09 and J05AP51 or N03AX10 and J05AP51 or N03AX11 and J05AP51 or N03AX12 and J05AP51 or N03AX13 and J05AP51 or N03AX14 and J05AP51 or N03AX15 and J05AP51 or N03AX16 and J05AP51 or N03AX17 and J05AP51 or N03AX18 and J05AP51 or N03AX19 and J05AP51 or N03AX21 and J05AP51 or N03AX22 and J05AP51 or N03AX23 and J05AP51 or N03AX24 and J05AP51 or N03AX30 and J05AP51 or N03AA01 and J05AP52 or N03AA02 and J05AP52 or N03AA03 and J05AP52 or N03AA04 and J05AP52 or N03AA30 and J05AP52 or N03AB01 and J05AP52 or N03AB02 and J05AP52 or N03AB03 and J05AP52 or N03AB04 and J05AP52 or N03AB05 and J05AP52 or N03AB52 and J05AP52 or N03AB54 and J05AP52 or N03AC01 and J05AP52 or N03AC02 and J05AP52 or N03AC03 and J05AP52 or N03AD01 and J05AP52 or N03AD02 and J05AP52 or N03AD03 and J05AP52 or N03AD51 and J05AP52 or N03AE01 and J05AP52 or N03AF01 and J05AP52 or N03AF02 and J05AP52 or N03AF03 and J05AP52 or N03AF04 and J05AP52 or N03AG01 and J05AP52 or N03AG02 and J05AP52 or N03AG03 and J05AP52 or N03AG04 and J05AP52 or N03AG05 and J05AP52 or N03AG06 and J05AP52 or N03AX03 and J05AP52 or N03AX07 and J05AP52 or N03AX09 and J05AP52 or N03AX10 and J05AP52 or N03AX11 and J05AP52 or N03AX12 and J05AP52 or N03AX13 and J05AP52 or N03AX14 and J05AP52 or N03AX15 and J05AP52 or N03AX16 and J05AP52 or N03AX17 and J05AP52 or N03AX18 and J05AP52 or N03AX19 and J05AP52 or N03AX21 and J05AP52 or N03AX22 and J05AP52 or N03AX23 and J05AP52 or N03AX24 and J05AP52 or N03AX30 and J05AP52 or N03AA01 and J05AP53 or N03AA02 and J05AP53 or N03AA03 and J05AP53 or N03AA04 and J05AP53 or N03AA30 and J05AP53 or N03AB01 and J05AP53 or N03AB02 and J05AP53 or N03AB03 and J05AP53 or N03AB04 and J05AP53 or N03AB05 and J05AP53 or N03AB52 and J05AP53 or N03AB54 and J05AP53 or N03AC01 and J05AP53 or N03AC02 and J05AP53 or N03AC03 and J05AP53 or N03AD01 and J05AP53 or N03AD02 and J05AP53 or N03AD03 and J05AP53 or N03AD51 and J05AP53 or N03AE01 and J05AP53 or N03AF01 and J05AP53 or N03AF02 and J05AP53 or N03AF03 and J05AP53 or N03AF04 and J05AP53 or N03AG01 and J05AP53 or N03AG02 and J05AP53 or N03AG03 and J05AP53 or N03AG04 and J05AP53 or N03AG05 and J05AP53 or N03AG06 and J05AP53 or N03AX03 and J05AP53 or N03AX07 and J05AP53 or N03AX09 and J05AP53 or N03AX10 and J05AP53 or N03AX11 and J05AP53 or N03AX12 and J05AP53 or N03AX13 and J05AP53 or N03AX14 and J05AP53 or N03AX15 and J05AP53 or N03AX16 and J05AP53 or N03AX17 and J05AP53 or N03AX18 and J05AP53 or N03AX19 and J05AP53 or N03AX21 and J05AP53 or N03AX22 and J05AP53 or N03AX23 and J05AP53 or N03AX24 and J05AP53 or N03AX30 and J05AP53 or N03AA01 and J05AP54 or N03AA02 and J05AP54 or N03AA03 and J05AP54 or N03AA04 and J05AP54 or N03AA30 and J05AP54 or N03AB01 and J05AP54 or N03AB02 and J05AP54 or N03AB03 and J05AP54 or N03AB04 and J05AP54 or N03AB05 and J05AP54 or N03AB52 and J05AP54 or N03AB54 and J05AP54 or N03AC01 and J05AP54 or N03AC02 and J05AP54 or N03AC03 and J05AP54 or N03AD01 and J05AP54 or N03AD02 and J05AP54 or N03AD03 and J05AP54 or N03AD51 and J05AP54 or N03AE01 and J05AP54 or N03AF01 and J05AP54 or N03AF02 and J05AP54 or N03AF03 and J05AP54 or N03AF04 and J05AP54 or N03AG01 and J05AP54 or N03AG02 and J05AP54 or N03AG03 and J05AP54 or N03AG04 and J05AP54 or N03AG05 and J05AP54 or N03AG06 and J05AP54 or N03AX03 and J05AP54 or N03AX07 and J05AP54 or N03AX09 and J05AP54 or N03AX10 and J05AP54 or N03AX11 and J05AP54 or N03AX12 and J05AP54 or N03AX13 and J05AP54 or N03AX14 and J05AP54 or N03AX15 and J05AP54 or N03AX16 and J05AP54 or N03AX17 and J05AP54 or N03AX18 and J05AP54 or N03AX19 and J05AP54 or N03AX21 and J05AP54 or N03AX22 and J05AP54 or N03AX23 and J05AP54 or N03AX24 and J05AP54 or N03AX30 and J05AP54 or N03AA01 and J05AP55 or N03AA02 and J05AP55 or N03AA03 and J05AP55 or N03AA04 and J05AP55 or N03AA30 and J05AP55 or N03AB01 and J05AP55 or N03AB02 and J05AP55 or N03AB03 and J05AP55 or N03AB04 and J05AP55 or N03AB05 and J05AP55 or N03AB52 and J05AP55 or N03AB54 and J05AP55 or N03AC01 and J05AP55 or N03AC02 and J05AP55 or N03AC03 and J05AP55 or N03AD01 and J05AP55 or N03AD02 and J05AP55 or N03AD03 and J05AP55 or N03AD51 and J05AP55 or N03AE01 and J05AP55 or N03AF01 and J05AP55 or N03AF02 and J05AP55 or N03AF03 and J05AP55 or N03AF04 and J05AP55 or N03AG01 and J05AP55 or N03AG02 and J05AP55 or N03AG03 and J05AP55 or N03AG04 and J05AP55 or N03AG05 and J05AP55 or N03AG06 and J05AP55 or N03AX03 and J05AP55 or N03AX07 and J05AP55 or N03AX09 and J05AP55 or N03AX10 and J05AP55 or N03AX11 and J05AP55 or N03AX12 and J05AP55 or N03AX13 and J05AP55 or N03AX14 and J05AP55 or N03AX15 and J05AP55 or N03AX16 and J05AP55 or N03AX17 and J05AP55 or N03AX18 and J05AP55 or N03AX19 and J05AP55 or N03AX21 and J05AP55 or N03AX22 and J05AP55 or N03AX23 and J05AP55 or N03AX24 and J05AP55 or N03AX30 and J05AP55 or N03AA01 and J05AP56 or N03AA02 and J05AP56 or N03AA03 and J05AP56 or N03AA04 and J05AP56 or N03AA30 and J05AP56 or N03AB01 and J05AP56 or N03AB02 and J05AP56 or N03AB03 and J05AP56 or N03AB04 and J05AP56 or N03AB05 and J05AP56 or N03AB52 and J05AP56 or N03AB54 and J05AP56 or N03AC01 and J05AP56 or N03AC02 and J05AP56 or N03AC03 and J05AP56 or N03AD01 and J05AP56 or N03AD02 and J05AP56 or N03AD03 and J05AP56 or N03AD51 and J05AP56 or N03AE01 and J05AP56 or N03AF01 and J05AP56 or N03AF02 and J05AP56 or N03AF03 and J05AP56 or N03AF04 and J05AP56 or N03AG01 and J05AP56 or N03AG02 and J05AP56 or N03AG03 and J05AP56 or N03AG04 and J05AP56 or N03AG05 and J05AP56 or N03AG06 and J05AP56 or N03AX03 and J05AP56 or N03AX07 and J05AP56 or N03AX09 and J05AP56 or N03AX10 and J05AP56 or N03AX11 and J05AP56 or N03AX12 and J05AP56 or N03AX13 and J05AP56 or N03AX14 and J05AP56 or N03AX15 and J05AP56 or N03AX16 and J05AP56 or N03AX17 and J05AP56 or N03AX18 and J05AP56 or N03AX19 and J05AP56 or N03AX21 and J05AP56 or N03AX22 and J05AP56 or N03AX23 and J05AP56 or N03AX24 and J05AP56 or N03AX30 and J05AP56 or N03AA01 and J05AP57 or N03AA02 and J05AP57 or N03AA03 and J05AP57 or N03AA04 and J05AP57 or N03AA30 and J05AP57 or N03AB01 and J05AP57 or N03AB02 and J05AP57 or N03AB03 and J05AP57 or N03AB04 and J05AP57 or N03AB05 and J05AP57 or N03AB52 and J05AP57 or N03AB54 and J05AP57 or N03AC01 and J05AP57 or N03AC02 and J05AP57 or N03AC03 and J05AP57 or N03AD01 and J05AP57 or N03AD02 and J05AP57 or N03AD03 and J05AP57 or N03AD51 and J05AP57 or N03AE01 and J05AP57 or N03AF01 and J05AP57 or N03AF02 and J05AP57 or N03AF03 and J05AP57 or N03AF04 and J05AP57 or N03AG01 and J05AP57 or N03AG02 and J05AP57 or N03AG03 and J05AP57 or N03AG04 and J05AP57 or N03AG05 and J05AP57 or N03AG06 and J05AP57 or N03AX03 and J05AP57 or N03AX07 and J05AP57 or N03AX09 and J05AP57 or N03AX10 and J05AP57 or N03AX11 and J05AP57 or N03AX12 and J05AP57 or N03AX13 and J05AP57 or N03AX14 and J05AP57 or N03AX15 and J05AP57 or N03AX16 and J05AP57 or N03AX17 and J05AP57 or N03AX18 and J05AP57 or N03AX19 and J05AP57 or N03AX21 and J05AP57 or N03AX22 and J05AP57 or N03AX23 and J05AP57 or N03AX24 and J05AP57 or N03AX30 and J05AP57 or N03AA01 and J05AR01 or N03AA02 and J05AR01 or N03AA03 and J05AR01 or N03AA04 and J05AR01 or N03AA30 and J05AR01 or N03AB01 and J05AR01 or N03AB02 and J05AR01 or N03AB03 and J05AR01 or N03AB04 and J05AR01 or N03AB05 and J05AR01 or N03AB52 and J05AR01 or N03AB54 and J05AR01 or N03AC01 and J05AR01 or N03AC02 and J05AR01 or N03AC03 and J05AR01 or N03AD01 and J05AR01 or N03AD02 and J05AR01 or N03AD03 and J05AR01 or N03AD51 and J05AR01 or N03AE01 and J05AR01 or N03AF01 and J05AR01 or N03AF02 and J05AR01 or N03AF03 and J05AR01 or N03AF04 and J05AR01 or N03AG01 and J05AR01 or N03AG02 and J05AR01 or N03AG03 and J05AR01 or N03AG04 and J05AR01 or N03AG05 and J05AR01 or N03AG06 and J05AR01 or N03AX03 and J05AR01 or N03AX07 and J05AR01 or N03AX09 and J05AR01 or N03AX10 and J05AR01 or N03AX11 and J05AR01 or N03AX12 and J05AR01 or N03AX13 and J05AR01 or N03AX14 and J05AR01 or N03AX15 and J05AR01 or N03AX16 and J05AR01 or N03AX17 and J05AR01 or N03AX18 and J05AR01 or N03AX19 and J05AR01 or N03AX21 and J05AR01 or N03AX22 and J05AR01 or N03AX23 and J05AR01 or N03AX24 and J05AR01 or N03AX30 and JOSAR01 or N03AA01 and J05AR02 or N03AA02 and J05AR02 or N03AA03 and J05AR02 or N03AA04 and J05AR02 or N03AA30 and J05AR02 or N03AB01 and J05AR02 or N03AB02 and J05AR02 or N03AB03 and J05AR02 or N03AB04 and J05AR02 or N03AB05 and J05AR02 or N03AB52 and J05AR02 or N03AB54 and J05AR02 or N03AC01 and J05AR02 or N03AC02 and J05AR02 or N03AC03 and J05AR02 or N03AD01 and J05AR02 or N03AD02 and J05AR02 or N03AD03 and J05AR02 or N03AD51 and J05AR02 or N03AE01 and J05AR02 or N03AF01 and J05AR02 or N03AF02 and J05AR02 or N03AF03 and J05AR02 or N03AF04 and J05AR02 or N03AG01 and J05AR02 or N03AG02 and J05AR02 or N03AG03 and J05AR02 or N03AG04 and J05AR02 or N03AG05 and J05AR02 or N03AG06 and J05AR02 or N03AX03 and J05AR02 or N03AX07 and J05AR02 or N03AX09 and J05AR02 or N03AX10 and J05AR02 or N03AX11 and J05AR02 or N03AX12 and J05AR02 or N03AX13 and J05AR02 or N03AX14 and J05AR02 or N03AX15 and J05AR02 or N03AX16 and J05AR02 or N03AX17 and J05AR02 or N03AX18 and J05AR02 or N03AX19 and J05AR02 or N03AX21 and J05AR02 or N03AX22 and J05AR02 or N03AX23 and J05AR02 or N03AX24 and J05AR02 or N03AX30 and J05AR02 or N03AA01 and J05AR03 or N03AA02 and J05AR03 or N03AA03 and J05AR03 or N03AA04 and J05AR03 or N03AA30 and J05AR03 or N03AB01 and J05AR03 or N03AB02 and J05AR03 or N03AB03 and J05AR03 or N03AB04 and J05AR03 or N03AB05 and J05AR03 or N03AB52 and J05AR03 or N03AB54 and J05AR03 or N03AC01 and J05AR03 or N03AC02 and J05AR03 or N03AC03 and J05AR03 or N03AD01 and J05AR03 or N03AD02 and J05AR03 or N03AD03 and J05AR03 or N03AD51 and J05AR03 or N03AE01 and J05AR03 or N03AF01 and J05AR03 or N03AF02 and J05AR03 or N03AF03 and J05AR03 or N03AF04 and J05AR03 or N03AG01 and J05AR03 or N03AG02 and J05AR03 or N03AG03 and J05AR03 or N03AG04 and J05AR03 or N03AG05 and J05AR03 or N03AG06 and J05AR03 or N03AX03 and J05AR03 or N03AX07 and J05AR03 or N03AX09 and J05AR03 or N03AX10 and J05AR03 or N03AX11 and J05AR03 or N03AX12 and J05AR03 or N03AX13 and J05AR03 or N03AX14 and J05AR03 or N03AX15 and J05AR03 or N03AX16 and J05AR03 or N03AX17 and J05AR03 or N03AX18 and J05AR03 or N03AX19 and J05AR03 or N03AX21 and J05AR03 or N03AX22 and J05AR03 or N03AX23 and J05AR03 or N03AX24 and J05AR03 or N03AX30 and J05AR03 or N03AA01 and J05AR04 or N03AA02 and J05AR04 or N03AA03 and J05AR04 or N03AA04 and J05AR04 or N03AA30 and J05AR04 or N03AB01 and J05AR04 or N03AB02 and J05AR04 or N03AB03 and J05AR04 or N03AB04 and J05AR04 or N03AB05 and J05AR04 or N03AB52 and J05AR04 or N03AB54 and J05AR04 or N03AC01 and J05AR04 or N03AC02 and J05AR04 or N03AC03 and J05AR04 or N03AD01 and J05AR04 or N03AD02 and J05AR04 or N03AD03 and J05AR04 or N03AD51 and J05AR04 or N03AE01 and J05AR04 or N03AF01 and J05AR04 or N03AF02 and J05AR04 or N03AF03 and J05AR04 or N03AF04 and J05AR04 or N03AG01 and J05AR04 or N03AG02 and J05AR04 or N03AG03 and J05AR04 or N03AG04 and J05AR04 or N03AG05 and J05AR04 or N03AG06 and J05AR04 or N03AX03 and J05AR04 or N03AX07 and J05AR04 or N03AX09 and J05AR04 or N03AX10 and J05AR04 or N03AX11 and J05AR04 or N03AX12 and J05AR04 or N03AX13 and J05AR04 or N03AX14 and J05AR04 or N03AX15 and J05AR04 or N03AX16 and J05AR04 or N03AX17 and J05AR04 or N03AX18 and J05AR04 or N03AX19 and J05AR04 or N03AX21 and J05AR04 or N03AX22 and J05AR04 or N03AX23 and J05AR04 or N03AX24 and J05AR04 or N03AX30 and J05AR04 or N03AA01 and J05AR05 or N03AA02 and J05AR05 or N03AA03 and J05AR05 or N03AA04 and J05AR05 or N03AA30 and J05AR05 or N03AB01 and J05AR05 or N03AB02 and J05AR05 or N03AB03 and J05AR05 or N03AB04 and J05AR05 or N03AB05 and J05AR05 or N03AB52 and J05AR05 or N03AB54 and J05AR05 or N03AC01 and J05AR05 or N03AC02 and J05AR05 or N03AC03 and J05AR05 or N03AD01 and J05AR05 or N03AD02 and J05AR05 or N03AD03 and J05AR05 or N03AD51 and J05AR05 or N03AE01 and J05AR05 or N03AF01 and J05AR05 or N03AF02 and J05AR05 or N03AF03 and J05AR05 or N03AF04 and J05AR05 or N03AG01 and J05AR05 or N03AG02 and J05AR05 or N03AG03 and J05AR05 or N03AG04 and J05AR05 or N03AG05 and J05AR05 or N03AG06 and J05AR05 or N03AX03 and J05AR05 or N03AX07 and J05AR05 or N03AX09 and J05AR05 or N03AX10 and J05AR05 or N03AX11 and J05AR05 or N03AX12 and J05AR05 or N03AX13 and J05AR05 or N03AX14 and J05AR05 or N03AX15 and J05AR05 or N03AX16 and J05AR05 or N03AX17 and J05AR05 or N03AX18 and J05AR05 or N03AX19 and J05AR05 or N03AX21 and J05AR05 or N03AX22 and J05AR05 or N03AX23 and J05AR05 or N03AX24 and J05AR05 or N03AX30 and J05AR05 or N03AA01 and J05AR06 or N03AA02 and J05AR06 or N03AA03 and J05AR06 or N03AA04 and J05AR06 or N03AA30 and J05AR06 or N03AB01 and J05AR06 or N03AB02 and J05AR06 or N03AB03 and J05AR06 or N03AB04 and J05AR06 or N03AB05 and J05AR06 or N03AB52 and J05AR06 or N03AB54 and J05AR06 or N03AC01 and J05AR06 or N03AC02 and J05AR06 or N03AC03 and J05AR06 or N03AD01 and J05AR06 or N03AD02 and J05AR06 or N03AD03 and J05AR06 or N03AD51 and J05AR06 or N03AE01 and J05AR06 or N03AF01 and J05AR06 or N03AF02 and J05AR06 or N03AF03 and J05AR06 or N03AF04 and J05AR06 or N03AG01 and J05AR06 or N03AG02 and J05AR06 or N03AG03 and J05AR06 or N03AG04 and J05AR06 or N03AG05 and J05AR06 or N03AG06 and J05AR06 or N03AX03 and J05AR06 or N03AX07 and J05AR06 or N03AX09 and J05AR06 or N03AX10 and J05AR06 or N03AX11 and J05AR06 or N03AX12 and J05AR06 or N03AX13 and J05AR06 or N03AX14 and J05AR06 or N03AX15 and J05AR06 or N03AX16 and J05AR06 or N03AX17 and J05AR06 or N03AX18 and J05AR06 or N03AX19 and J05AR06 or N03AX21 and J05AR06 or N03AX22 and J05AR06 or N03AX23 and J05AR06 or N03AX24 and J05AR06 or N03AX30 and J05AR06 or N03AA01 and J05AR07 or N03AA02 and J05AR07 or N03AA03 and J05AR07 or N03AA04 and J05AR07 or N03AA30 and J05AR07 or N03AB01 and J05AR07 or N03AB02 and J05AR07 or N03AB03 and J05AR07 or N03AB04 and J05AR07 or N03AB05 and J05AR07 or N03AB52 and J05AR07 or N03AB54 and J05AR07 or N03AC01 and J05AR07 or N03AC02 and J05AR07 or N03AC03 and J05AR07 or N03AD01 and J05AR07 or N03AD02 and J05AR07 or N03AD03 and J05AR07 or N03AD51 and J05AR07 or N03AE01 and J05AR07 or N03AF01 and J05AR07 or N03AF02 and J05AR07 or N03AF03 and J05AR07 or N03AF04 and J05AR07 or N03AG01 and J05AR07 or N03AG02 and J05AR07 or N03AG03 and J05AR07 or N03AG04 and J05AR07 or N03AG05 and J05AR07 or N03AG06 and J05AR07 or N03AX03 and J05AR07 or N03AX07 and J05AR07 or N03AX09 and J05AR07 or N03AX10 and J05AR07 or N03AX11 and J05AR07 or N03AX12 and J05AR07 or N03AX13 and J05AR07 or N03AX14 and J05AR07 or N03AX15 and J05AR07 or N03AX16 and J05AR07 or N03AX17 and J05AR07 or N03AX18 and J05AR07 or N03AX19 and J05AR07 or N03AX21 and J05AR07 or N03AX22 and J05AR07 or N03AX23 and J05AR07 or N03AX24 and J05AR07 or N03AX30 and J05AR07 or N03AA01 and J05AR08 or N03AA02 and J05AR08 or N03AA03 and J05AR08 or N03AA04 and J05AR08 or N03AA30 and J05AR08 or N03AB01 and J05AR08 or N03AB02 and J05AR08 or N03AB03 and J05AR08 or N03AB04 and J05AR08 or N03AB05 and J05AR08 or N03AB52 and J05AR08 or N03AB54 and J05AR08 or N03AC01 and J05AR08 or N03AC02 and J05AR08 or N03AC03 and J05AR08 or N03AD01 and J05AR08 or N03AD02 and J05AR08 or N03AD03 and J05AR08 or N03AD51 and J05AR08 or N03AE01 and J05AR08 or N03AF01 and J05AR08 or N03AF02 and J05AR08 or N03AF03 and J05AR08 or N03AF04 and J05AR08 or N03AG01 and J05AR08 or N03AG02 and J05AR08 or N03AG03 and J05AR08 or N03AG04 and J05AR08 or N03AG05 and J05AR08 or N03AG06 and J05AR08 or N03AX03 and J05AR08 or N03AX07 and J05AR08 or N03AX09 and J05AR08 or N03AX10 and J05AR08 or N03AX11 and J05AR08 or N03AX12 and J05AR08 or N03AX13 and J05AR08 or N03AX14 and J05AR08 or N03AX15 and J05AR08 or N03AX16 and J05AR08 or N03AX17 and J05AR08 or N03AX18 and J05AR08 or N03AX19 and J05AR08 or N03AX21 and J05AR08 or N03AX22 and J05AR08 or N03AX23 and J05AR08 or N03AX24 and J05AR08 or N03AX30 and J05AR08 or N03AA01 and J05AR09 or N03AA02 and J05AR09 or N03AA03 and J05AR09 or N03AA04 and J05AR09 or N03AA30 and J05AR09 or N03AB01 and J05AR09 or N03AB02 and J05AR09 or N03AB03 and J05AR09 or N03AB04 and J05AR09 or N03AB05 and J05AR09 or N03AB52 and J05AR09 or N03AB54 and J05AR09 or N03AC01 and J05AR09 or N03AC02 and J05AR09 or N03AC03 and J05AR09 or N03AD01 and J05AR09 or N03AD02 and J05AR09 or N03AD03 and J05AR09 or N03AD51 and J05AR09 or N03AE01 and J05AR09 or N03AF01 and J05AR09 or N03AF02 and J05AR09 or N03AF03 and J05AR09 or N03AF04 and J05AR09 or N03AG01 and J05AR09 or N03AG02 and J05AR09 or N03AG03 and J05AR09 or N03AG04 and J05AR09 or N03AG05 and J05AR09 or N03AG06 and J05AR09 or N03AX03 and J05AR09 or N03AX07 and J05AR09 or N03AX09 and J05AR09 or N03AX10 and J05AR09 or N03AX11 and J05AR09 or N03AX12 and J05AR09 or N03AX13 and J05AR09 or N03AX14 and J05AR09 or N03AX15 and J05AR09 or N03AX16 and J05AR09 or N03AX17 and J05AR09 or N03AX18 and J05AR09 or N03AX19 and J05AR09 or N03AX21 and J05AR09 or N03AX22 and J05AR09 or N03AX23 and J05AR09 or N03AX24 and J05AR09 or N03AX30 and J05AR09 or N03AA01 and J05AR10 or N03AA02 and J05AR10 or N03AA03 and J05AR10 or N03AA04 and J05AR10 or N03AA30 and J05AR10 or N03AB01 and J05AR10 or N03AB02 and J05AR10 or N03AB03 and J05AR10 or N03AB04 and J05AR10 or N03AB05 and J05AR10 or N03AB52 and J05AR10 or N03AB54 and J05AR10 or N03AC01 and J05AR10 or N03AC02 and J05AR10 or N03AC03 and J05AR10 or N03AD01 and J05AR10 or N03AD02 and J05AR10 or N03AD03 and J05AR10 or N03AD51 and J05AR10 or N03AE01 and J05AR10 or N03AF01 and J05AR10 or N03AF02 and J05AR10 or N03AF03 and J05AR10 or N03AF04 and J05AR10 or N03AG01 and J05AR10 or N03AG02 and J05AR10 or N03AG03 and J05AR10 or N03AG04 and J05AR10 or N03AG05 and J05AR10 or N03AG06 and J05AR10 or N03AX03 and J05AR10 or N03AX07 and J05AR10 or N03AX09 and J05AR10 or N03AX10 and J05AR10 or N03AX11 and J05AR10 or N03AX12 and J05AR10 or N03AX13 and J05AR10 or N03AX14 and J05AR10 or N03AX15 and J05AR10 or N03AX16 and J05AR10 or N03AX17 and J05AR10 or N03AX18 and J05AR10 or N03AX19 and J05AR10 or N03AX21 and J05AR10 or N03AX22 and J05AR10 or N03AX23 and J05AR10 or N03AX24 and J05AR10 or N03AX30 and J05AR10 or N03AA01 and J05AR11 or N03AA02 and J05AR11 or N03AA03 and J05AR11 or N03AA04 and J05AR11 or N03AA30 and J05AR11 or N03AB01 and J05AR11 or N03AB02 and J05AR11 or N03AB03 and J05AR11 or N03AB04 and J05AR11 or N03AB05 and J05AR11 or N03AB52 and J05AR11 or N03AB54 and J05AR11 or N03AC01 and J05AR11 or N03AC02 and J05AR11 or N03AC03 and J05AR11 or N03AD01 and J05AR11 or N03AD02 and J05AR11 or N03AD03 and J05AR11 or N03AD51 and J05AR11 or N03AE01 and J05AR11 or N03AF01 and J05AR11 or N03AF02 and J05AR11 or N03AF03 and J05AR11 or N03AF04 and J05AR11 or N03AG01 and J05AR11 or N03AG02 and J05AR11 or N03AG03 and J05AR11 or N03AG04 and J05AR11 or N03AG05 and J05AR11 or N03AG06 and J05AR11 or N03AX03 and J05AR11 or N03AX07 and J05AR11 or N03AX09 and J05AR11 or N03AX10 and J05AR11 or N03AX11 and J05AR11 or N03AX12 and J05AR11 or N03AX13 and J05AR11 or N03AX14 and J05AR11 or N03AX15 and J05AR11 or N03AX16 and J05AR11 or N03AX17 and J05AR11 or N03AX18 and J05AR11 or N03AX19 and J05AR11 or N03AX21 and J05AR11 or N03AX22 and J05AR11 or N03AX23 and J05AR11 or N03AX24 and J05AR11 or N03AX30 and J05AR11 or N03AA01 and J05AR12 or N03AA02 and J05AR12 or N03AA03 and J05AR12 or N03AA04 and J05AR12 or N03AA30 and J05AR12 or N03AB01 and J05AR12 or N03AB02 and J05AR12 or N03AB03 and J05AR12 or N03AB04 and J05AR12 or N03AB05 and J05AR12 or N03AB52 and J05AR12 or N03AB54 and J05AR12 or N03AC01 and J05AR12 or N03AC02 and J05AR12 or N03AC03 and J05AR12 or N03AD01 and J05AR12 or N03AD02 and J05AR12 or N03AD03 and J05AR12 or N03AD51 and J05AR12 or N03AE01 and J05AR12 or N03AF01 and J05AR12 or N03AF02 and J05AR12 or N03AF03 and J05AR12 or N03AF04 and J05AR12 or N03AG01 and J05AR12 or N03AG02 and J05AR12 or N03AG03 and J05AR12 or N03AG04 and J05AR12 or N03AG05 and J05AR12 or N03AG06 and J05AR12 or N03AX03 and J05AR12 or N03AX07 and J05AR12 or N03AX09 and J05AR12 or N03AX10 and J05AR12 or N03AX11 and J05AR12 or N03AX12 and J05AR12 or N03AX13 and J05AR12 or N03AX14 and J05AR12 or N03AX15 and J05AR12 or N03AX16 and J05AR12 or N03AX17 and J05AR12 or N03AX18 and J05AR12 or N03AX19 and J05AR12 or N03AX21 and J05AR12 or N03AX22 and J05AR12 or N03AX23 and J05AR12 or N03AX24 and J05AR12 or N03AX30 and J05AR12 or N03AA01 and J05AR13 or N03AA02 and J05AR13 or N03AA03 and J05AR13 or N03AA04 and J05AR13 or N03AA30 and J05AR13 or N03AB01 and J05AR13 or N03AB02 and J05AR13 or N03AB03 and J05AR13 or N03AB04 and J05AR13 or N03AB05 and J05AR13 or N03AB52 and J05AR13 or N03AB54 and J05AR13 or N03AC01 and J05AR13 or N03AC02 and J05AR13 or N03AC03 and J05AR13 or N03AD01 and J05AR13 or N03AD02 and J05AR13 or N03AD03 and J05AR13 or N03AD51 and J05AR13 or N03AE01 and J05AR13 or N03AF01 and J05AR13 or N03AF02 and J05AR13 or N03AF03 and J05AR13 or N03AF04 and J05AR13 or N03AG01 and J05AR13 or N03AG02 and J05AR13 or N03AG03 and J05AR13 or N03AG04 and J05AR13 or N03AG05 and J05AR13 or N03AG06 and J05AR13 or N03AX03 and J05AR13 or N03AX07 and J05AR13 or N03AX09 and J05AR13 or N03AX10 and J05AR13 or N03AX11 and J05AR13 or N03AX12 and J05AR13 or N03AX13 and J05AR13 or N03AX14 and J05AR13 or N03AX15 and J05AR13 or N03AX16 and J05AR13 or N03AX17 and J05AR13 or N03AX18 and J05AR13 or N03AX19 and J05AR13 or N03AX21 and J05AR13 or N03AX22 and J05AR13 or N03AX23 and J05AR13 or N03AX24 and J05AR13 or N03AX30 and J05AR13 or N03AA01 and J05AR14 or N03AA02 and J05AR14 or N03AA03 and J05AR14 or N03AA04 and J05AR14 or N03AA30 and J05AR14 or N03AB01 and J05AR14 or N03AB02 and J05AR14 or N03AB03 and J05AR14 or N03AB04 and J05AR14 or N03AB05 and J05AR14 or N03AB52 and J05AR14 or N03AB54 and J05AR14 or N03AC01 and J05AR14 or N03AC02 and J05AR14 or N03AC03 and J05AR14 or N03AD01 and J05AR14 or N03AD02 and J05AR14 or N03AD03 and J05AR14 or N03AD51 and J05AR14 or N03AE01 and J05AR14 or N03AF01 and J05AR14 or N03AF02 and J05AR14 or N03AF03 and J05AR14 or N03AF04 and J05AR14 or N03AG01 and J05AR14 or N03AG02 and J05AR14 or N03AG03 and J05AR14 or N03AG04 and J05AR14 or N03AG05 and J05AR14 or N03AG06 and J05AR14 or N03AX03 and J05AR14 or N03AX07 and J05AR14 or N03AX09 and J05AR14 or N03AX10 and J05AR14 or N03AX11 and J05AR14 or N03AX12 and J05AR14 or N03AX13 and J05AR14 or N03AX14 and J05AR14 or N03AX15 and J05AR14 or N03AX16 and J05AR14 or N03AX17 and J05AR14 or N03AX18 and J05AR14 or N03AX19 and J05AR14 or N03AX21 and J05AR14 or N03AX22 and J05AR14 or N03AX23 and J05AR14 or N03AX24 and J05AR14 or N03AX30 and J05AR14 or N03AA01 and J05AR15 or N03AA02 and J05AR15 or N03AA03 and J05AR15 or N03AA04 and J05AR15 or N03AA30 and J05AR15 or N03AB01 and J05AR15 or N03AB02 and J05AR15 or N03AB03 and J05AR15 or N03AB04 and J05AR15 or N03AB05 and J05AR15 or N03AB52 and J05AR15 or N03AB54 and J05AR15 or N03AC01 and J05AR15 or N03AC02 and J05AR15 or N03AC03 and J05AR15 or N03AD01 and J05AR15 or N03AD02 and J05AR15 or N03AD03 and J05AR15 or N03AD51 and J05AR15 or N03AE01 and J05AR15 or N03AF01 and J05AR15 or N03AF02 and J05AR15 or N03AF03 and J05AR15 or N03AF04 and J05AR15 or N03AG01 and J05AR15 or N03AG02 and J05AR15 or N03AG03 and J05AR15 or N03AG04 and J05AR15 or N03AG05 and J05AR15 or N03AG06 and J05AR15 or N03AX03 and J05AR15 or N03AX07 and J05AR15 or N03AX09 and J05AR15 or N03AX10 and J05AR15 or N03AX11 and J05AR15 or N03AX12 and J05AR15 or N03AX13 and J05AR15 or N03AX14 and J05AR15 or N03AX15 and J05AR15 or N03AX16 and J05AR15 or N03AX17 and J05AR15 or N03AX18 and J05AR15 or N03AX19 and J05AR15 or N03AX21 and J05AR15 or N03AX22 and J05AR15 or N03AX23 and J05AR15 or N03AX24 and J05AR15 or N03AX30 and J05AR15 or N03AA01 and J05AR16 or N03AA02 and J05AR16 or N03AA03 and J05AR16 or N03AA04 and J05AR16 or N03AA30 and J05AR16 or N03AB01 and J05AR16 or N03AB02 and J05AR16 or N03AB03 and J05AR16 or N03AB04 and J05AR16 or N03AB05 and J05AR16 or N03AB52 and J05AR16 or N03AB54 and J05AR16 or N03AC01 and J05AR16 or N03AC02 and J05AR16 or N03AC03 and J05AR16 or N03AD01 and J05AR16 or N03AD02 and J05AR16 or N03AD03 and J05AR16 or N03AD51 and J05AR16 or N03AE01 and J05AR16 or N03AF01 and J05AR16 or N03AF02 and J05AR16 or N03AF03 and J05AR16 or N03AF04 and J05AR16 or N03AG01 and J05AR16 or N03AG02 and J05AR16 or N03AG03 and J05AR16 or N03AG04 and J05AR16 or N03AG05 and J05AR16 or N03AG06 and J05AR16 or N03AX03 and J05AR16 or N03AX07 and J05AR16 or N03AX09 and J05AR16 or N03AX10 and J05AR16 or N03AX11 and J05AR16 or N03AX12 and J05AR16 or N03AX13 and J05AR16 or N03AX14 and J05AR16 or N03AX15 and J05AR16 or N03AX16 and J05AR16 or N03AX17 and J05AR16 or N03AX18 and J05AR16 or N03AX19 and J05AR16 or N03AX21 and J05AR16 or N03AX22 and J05AR16 or N03AX23 and J05AR16 or N03AX24 and J05AR16 or N03AX30 and J05AR16 or N03AA01 and J05AR17 or N03AA02 and J05AR17 or N03AA03 and J05AR17 or N03AA04 and J05AR17 or N03AA30 and J05AR17 or N03AB01 and J05AR17 or N03AB02 and J05AR17 or N03AB03 and J05AR17 or N03AB04 and J05AR17 or N03AB05 and J05AR17 or N03AB52 and J05AR17 or N03AB54 and J05AR17 or N03AC01 and J05AR17 or N03AC02 and J05AR17 or N03AC03 and J05AR17 or N03AD01 and J05AR17 or N03AD02 and J05AR17 or N03AD03 and J05AR17 or N03AD51 and J05AR17 or N03AE01 and J05AR17 or N03AF01 and J05AR17 or N03AF02 and J05AR17 or N03AF03 and J05AR17 or N03AF04 and J05AR17 or N03AG01 and J05AR17 or N03AG02 and J05AR17 or N03AG03 and J05AR17 or N03AG04 and J05AR17 or N03AG05 and J05AR17 or N03AG06 and J05AR17 or N03AX03 and J05AR17 or N03AX07 and J05AR17 or N03AX09 and J05AR17 or N03AX10 and J05AR17 or N03AX11 and J05AR17 or N03AX12 and J05AR17 or N03AX13 and J05AR17 or N03AX14 and J05AR17 or N03AX15 and J05AR17 or N03AX16 and J05AR17 or N03AX17 and J05AR17 or N03AX18 and J05AR17 or N03AX19 and J05AR17 or N03AX21 and J05AR17 or N03AX22 and J05AR17 or N03AX23 and J05AR17 or N03AX24 and J05AR17 or N03AX30 and J05AR17 or N03AA01 and J05AR18 or N03AA02 and J05AR18 or N03AA03 and J05AR18 or N03AA04 and J05AR18 or N03AA30 and J05AR18 or N03AB01 and J05AR18 or N03AB02 and J05AR18 or N03AB03 and J05AR18 or N03AB04 and J05AR18 or N03AB05 and J05AR18 or N03AB52 and J05AR18 or N03AB54 and J05AR18 or N03AC01 and J05AR18 or N03AC02 and J05AR18 or N03AC03 and J05AR18 or N03AD01 and J05AR18 or N03AD02 and J05AR18 or N03AD03 and J05AR18 or N03AD51 and J05AR18 or N03AE01 and J05AR18 or N03AF01 and J05AR18 or N03AF02 and J05AR18 or N03AF03 and J05AR18 or N03AF04 and J05AR18 or N03AG01 and J05AR18 or N03AG02 and J05AR18 or N03AG03 and J05AR18 or N03AG04 and J05AR18 or N03AG05 and J05AR18 or N03AG06 and J05AR18 or N03AX03 and J05AR18 or N03AX07 and J05AR18 or N03AX09 and J05AR18 or N03AX10 and J05AR18 or N03AX11 and J05AR18 or N03AX12 and J05AR18 or N03AX13 and J05AR18 or N03AX14 and J05AR18 or N03AX15 and J05AR18 or N03AX16 and J05AR18 or N03AX17 and J05AR18 or N03AX18 and J05AR18 or N03AX19 and J05AR18 or N03AX21 and J05AR18 or N03AX22 and J05AR18 or N03AX23 and J05AR18 or N03AX24 and J05AR18 or N03AX30 and J05AR18 or N03AA01 and J05AR19 or N03AA02 and J05AR19 or N03AA03 and J05AR19 or N03AA04 and J05AR19 or N03AA30 and J05AR19 or N03AB01 and J05AR19 or N03AB02 and J05AR19 or N03AB03 and J05AR19 or N03AB04 and J05AR19 or N03AB05 and J05AR19 or N03AB52 and J05AR19 or N03AB54 and J05AR19 or N03AC01 and J05AR19 or N03AC02 and J05AR19 or N03AC03 and J05AR19 or N03AD01 and J05AR19 or N03AD02 and J05AR19 or N03AD03 and J05AR19 or N03AD51 and J05AR19 or N03AE01 and J05AR19 or N03AF01 and J05AR19 or N03AF02 and J05AR19 or N03AF03 and J05AR19 or N03AF04 and J05AR19 or N03AG01 and J05AR19 or N03AG02 and J05AR19 or N03AG03 and J05AR19 or N03AG04 and J05AR19 or N03AG05 and J05AR19 or N03AG06 and J05AR19 or N03AX03 and J05AR19 or N03AX07 and J05AR19 or N03AX09 and J05AR19 or N03AX10 and J05AR19 or N03AX11 and J05AR19 or N03AX12 and J05AR19 or N03AX13 and J05AR19 or N03AX14 and J05AR19 or N03AX15 and J05AR19 or N03AX16 and J05AR19 or N03AX17 and J05AR19 or N03AX18 and J05AR19 or N03AX19 and J05AR19 or N03AX21 and J05AR19 or N03AX22 and J05AR19 or N03AX23 and J05AR19 or N03AX24 and J05AR19 or N03AX30 and J05AR19 or N03AA01 and J05AR20 or N03AA02 and J05AR20 or N03AA03 and J05AR20 or N03AA04 and J05AR20 or N03AA30 and J05AR20 or N03AB01 and J05AR20 or N03AB02 and J05AR20 or N03AB03 and J05AR20 or N03AB04 and J05AR20 or N03AB05 and J05AR20 or N03AB52 and J05AR20 or N03AB54 and J05AR20 or N03AC01 and J05AR20 or N03AC02 and J05AR20 or N03AC03 and J05AR20 or N03AD01 and J05AR20 or N03AD02 and J05AR20 or N03AD03 and J05AR20 or N03AD51 and J05AR20 or N03AE01 and J05AR20 or N03AF01 and J05AR20 or N03AF02 and J05AR20 or N03AF03 and J05AR20 or N03AF04 and J05AR20 or N03AG01 and J05AR20 or N03AG02 and J05AR20 or N03AG03 and J05AR20 or N03AG04 and J05AR20 or N03AG05 and J05AR20 or N03AG06 and J05AR20 or N03AX03 and J05AR20 or N03AX07 and J05AR20 or N03AX09 and J05AR20 or N03AX10 and J05AR20 or N03AX11 and J05AR20 or N03AX12 and J05AR20 or N03AX13 and J05AR20 or N03AX14 and J05AR20 or N03AX15 and J05AR20 or N03AX16 and J05AR20 or N03AX17 and J05AR20 or N03AX18 and J05AR20 or N03AX19 and J05AR20 or N03AX21 and J05AR20 or N03AX22 and J05AR20 or N03AX23 and J05AR20 or N03AX24 and J05AR20 or N03AX30 and J05AR20 or N03AA01 and J05AR21 or N03AA02 and J05AR21 or N03AA03 and J05AR21 or N03AA04 and J05AR21 or N03AA30 and J05AR21 or N03AB01 and J05AR21 or N03AB02 and J05AR21 or N03AB03 and J05AR21 or N03AB04 and J05AR21 or N03AB05 and J05AR21 or N03AB52 and J05AR21 or N03AB54 and J05AR21 or N03AC01 and J05AR21 or N03AC02 and J05AR21 or N03AC03 and J05AR21 or N03AD01 and J05AR21 or N03AD02 and J05AR21 or N03AD03 and J05AR21 or N03AD51 and J05AR21 or N03AE01 and J05AR21 or N03AF01 and J05AR21 or N03AF02 and J05AR21 or N03AF03 and J05AR21 or N03AF04 and J05AR21 or N03AG01 and J05AR21 or N03AG02 and J05AR21 or N03AG03 and J05AR21 or N03AG04 and J05AR21 or N03AG05 and J05AR21 or N03AG06 and J05AR21 or N03AX03 and J05AR21 or N03AX07 and J05AR21 or N03AX09 and J05AR21 or N03AX10 and J05AR21 or N03AX11 and J05AR21 or N03AX12 and J05AR21 or N03AX13 and J05AR21 or N03AX14 and J05AR21 or N03AX15 and J05AR21 or N03AX16 and J05AR21 or N03AX17 and J05AR21 or N03AX18 and J05AR21 or N03AX19 and J05AR21 or N03AX21 and J05AR21 or N03AX22 and J05AR21 or N03AX23 and J05AR21 or N03AX24 and J05AR21 or N03AX30 and J05AR21 or N03AA01 and J05AR22 or N03AA02 and J05AR22 or N03AA03 and J05AR22 or N03AA04 and J05AR22 or N03AA30 and J05AR22 or N03AB01 and J05AR22 or N03AB02 and J05AR22 or N03AB03 and J05AR22 or N03AB04 and J05AR22 or N03AB05 and J05AR22 or N03AB52 and J05AR22 or N03AB54 and J05AR22 or N03AC01 and J05AR22 or N03AC02 and J05AR22 or N03AC03 and J05AR22 or N03AD01 and J05AR22 or N03AD02 and J05AR22 or N03AD03 and J05AR22 or N03AD51 and J05AR22 or N03AE01 and J05AR22 or N03AF01 and J05AR22 or N03AF02 and J05AR22 or N03AF03 and J05AR22 or N03AF04 and J05AR22 or N03AG01 and J05AR22 or N03AG02 and J05AR22 or N03AG03 and J05AR22 or N03AG04 and J05AR22 or N03AG05 and J05AR22 or N03AG06 and J05AR22 or N03AX03 and J05AR22 or N03AX07 and J05AR22 or N03AX09 and J05AR22 or N03AX10 and J05AR22 or N03AX11 and J05AR22 or N03AX12 and J05AR22 or N03AX13 and J05AR22 or N03AX14 and J05AR22 or N03AX15 and J05AR22 or N03AX16 and J05AR22 or N03AX17 and J05AR22 or N03AX18 and J05AR22 or N03AX19 and J05AR22 or N03AX21 and J05AR22 or N03AX22 and J05AR22 or N03AX23 and J05AR22 or N03AX24 and J05AR22 or N03AX30 and J05AR22 or N03AA01 and J05AR23 or N03AA02 and J05AR23 or N03AA03 and J05AR23 or N03AA04 and J05AR23 or N03AA30 and J05AR23 or N03AB01 and J05AR23 or N03AB02 and J05AR23 or N03AB03 and J05AR23 or N03AB04 and J05AR23 or N03AB05 and J05AR23 or N03AB52 and J05AR23 or N03AB54 and J05AR23 or N03AC01 and J05AR23 or N03AC02 and J05AR23 or N03AC03 and J05AR23 or N03AD01 and J05AR23 or N03AD02 and J05AR23 or N03AD03 and J05AR23 or N03AD51 and J05AR23 or N03AE01 and J05AR23 or N03AF01 and J05AR23 or N03AF02 and J05AR23 or N03AF03 and J05AR23 or N03AF04 and J05AR23 or N03AG01 and J05AR23 or N03AG02 and J05AR23 or N03AG03 and J05AR23 or N03AG04 and J05AR23 or N03AG05 and J05AR23 or N03AG06 and J05AR23 or N03AX03 and J05AR23 or N03AX07 and J05AR23 or N03AX09 and J05AR23 or N03AX10 and J05AR23 or N03AX11 and J05AR23 or N03AX12 and J05AR23 or N03AX13 and J05AR23 or N03AX14 and J05AR23 or N03AX15 and J05AR23 or N03AX16 and J05AR23 or N03AX17 and J05AR23 or N03AX18 and J05AR23 or N03AX19 and J05AR23 or N03AX21 and J05AR23 or N03AX22 and J05AR23 or N03AX23 and J05AR23 or N03AX24 and J05AR23 or N03AX30 and J05AR23 or N03AA01 and J05AR24 or N03AA02 and J05AR24 or N03AA03 and J05AR24 or N03AA04 and J05AR24 or N03AA30 and J05AR24 or N03AB01 and J05AR24 or N03AB02 and J05AR24 or N03AB03 and J05AR24 or N03AB04 and J05AR24 or N03AB05 and J05AR24 or N03AB52 and J05AR24 or N03AB54 and J05AR24 or N03AC01 and J05AR24 or N03AC02 and J05AR24 or N03AC03 and J05AR24 or N03AD01 and J05AR24 or N03AD02 and J05AR24 or N03AD03 and J05AR24 or N03AD51 and J05AR24 or N03AE01 and J05AR24 or N03AF01 and J05AR24 or N03AF02 and J05AR24 or N03AF03 and J05AR24 or N03AF04 and J05AR24 or N03AG01 and J05AR24 or N03AG02 and J05AR24 or N03AG03 and J05AR24 or N03AG04 and J05AR24 or N03AG05 and J05AR24 or N03AG06 and J05AR24 or N03AX03 and J05AR24 or N03AX07 and J05AR24 or N03AX09 and J05AR24 or N03AX10 and J05AR24 or N03AX11 and J05AR24 or N03AX12 and J05AR24 or N03AX13 and J05AR24 or N03AX14 and J05AR24 or N03AX15 and J05AR24 or N03AX16 and J05AR24 or N03AX17 and J05AR24 or N03AX18 and J05AR24 or N03AX19 and J05AR24 or N03AX21 and J05AR24 or N03AX22 and J05AR24 or N03AX23 and J05AR24 or N03AX24 and J05AR24 or N03AX30 and J05AR24 or N03AA01 and J05AX01 or N03AA02 and J05AX01 or N03AA03 and J05AX01 or N03AA04 and J05AX01 or N03AA30 and J05AX01 or N03AB01 and J05AX01 or N03AB02 and J05AX01 or N03AB03 and J05AX01 or N03AB04 and J05AX01 or N03AB05 and J05AX01 or N03AB52 and J05AX01 or N03AB54 and J05AX01 or N03AC01 and J05AX01 or N03AC02 and J05AX01 or N03AC03 and J05AX01 or N03AD01 and J05AX01 or N03AD02 and J05AX01 or N03AD03 and J05AX01 or N03AD51 and J05AX01 or N03AE01 and J05AX01 or N03AF01 and J05AX01 or N03AF02 and J05AX01 or N03AF03 and J05AX01 or N03AF04 and J05AX01 or N03AG01 and J05AX01 or N03AG02 and J05AX01 or N03AG03 and J05AX01 or N03AG04 and J05AX01 or N03AG05 and J05AX01 or N03AG06 and J05AX01 or N03AX03 and J05AX01 or N03AX07 and J05AX01 or N03AX09 and J05AX01 or N03AX10 and J05AX01 or N03AX11 and J05AX01 or N03AX12 and J05AX01 or N03AX13 and J05AX01 or N03AX14 and J05AX01 or N03AX15 and J05AX01 or N03AX16 and J05AX01 or N03AX17 and J05AX01 or N03AX18 and J05AX01 or N03AX19 and J05AX01 or N03AX21 and J05AX01 or N03AX22 and J05AX01 or N03AX23 and J05AX01 or N03AX24 and J05AX01 or N03AX30 and J05AX01 or N03AA01 and J05AX02 or N03AA02 and J05AX02 or N03AA03 and J05AX02 or N03AA04 and J05AX02 or N03AA30 and J05AX02 or N03AB01 and J05AX02 or N03AB02 and J05AX02 or N03AB03 and J05AX02 or N03AB04 and J05AX02 or N03AB05 and J05AX02 or N03AB52 and J05AX02 or N03AB54 and J05AX02 or N03AC01 and J05AX02 or N03AC02 and J05AX02 or N03AC03 and J05AX02 or N03AD01 and J05AX02 or N03AD02 and J05AX02 or N03AD03 and J05AX02 or N03AD51 and J05AX02 or N03AE01 and J05AX02 or N03AF01 and J05AX02 or N03AF02 and J05AX02 or N03AF03 and J05AX02 or N03AF04 and J05AX02 or N03AG01 and J05AX02 or N03AG02 and J05AX02 or N03AG03 and J05AX02 or N03AG04 and J05AX02 or N03AG05 and J05AX02 or N03AG06 and J05AX02 or N03AX03 and J05AX02 or N03AX07 and J05AX02 or N03AX09 and J05AX02 or N03AX10 and J05AX02 or N03AX11 and J05AX02 or N03AX12 and J05AX02 or N03AX13 and J05AX02 or N03AX14 and J05AX02 or N03AX15 and J05AX02 or N03AX16 and J05AX02 or N03AX17 and J05AX02 or N03AX18 and J05AX02 or N03AX19 and J05AX02 or N03AX21 and J05AX02 or N03AX22 and J05AX02 or N03AX23 and J05AX02 or N03AX24 and J05AX02 or N03AX30 and J05AX02 or N03AA01 and J05AX05 or N03AA02 and J05AX05 or N03AA03 and J05AX05 or N03AA04 and J05AX05 or N03AA30 and J05AX05 or N03AB01 and J05AX05 or N03AB02 and J05AX05 or N03AB03 and J05AX05 or N03AB04 and J05AX05 or N03AB05 and J05AX05 or N03AB52 and J05AX05 or N03AB54 and J05AX05 or N03AC01 and J05AX05 or N03AC02 and J05AX05 or N03AC03 and J05AX05 or N03AD01 and J05AX05 or N03AD02 and J05AX05 or N03AD03 and J05AX05 or N03AD51 and J05AX05 or N03AE01 and J05AX05 or N03AF01 and J05AX05 or N03AF02 and J05AX05 or N03AF03 and J05AX05 or N03AF04 and J05AX05 or N03AG01 and J05AX05 or N03AG02 and J05AX05 or N03AG03 and J05AX05 or N03AG04 and J05AX05 or N03AG05 and J05AX05 or N03AG06 and J05AX05 or N03AX03 and J05AX05 or N03AX07 and J05AX05 or N03AX09 and J05AX05 or N03AX10 and J05AX05 or N03AX11 and J05AX05 or N03AX12 and J05AX05 or N03AX13 and J05AX05 or N03AX14 and J05AX05 or N03AX15 and J05AX05 or N03AX16 and J05AX05 or N03AX17 and J05AX05 or N03AX18 and J05AX05 or N03AX19 and J05AX05 or N03AX21 and J05AX05 or N03AX22 and J05AX05 or N03AX23 and J05AX05 or N03AX24 and J05AX05 or N03AX30 and J05AX05 or N03AA01 and J05AX06 or N03AA02 and J05AX06 or N03AA03 and J05AX06 or N03AA04 and J05AX06 or N03AA30 and J05AX06 or N03AB01 and J05AX06 or N03AB02 and J05AX06 or N03AB03 and J05AX06 or N03AB04 and J05AX06 or N03AB05 and J05AX06 or N03AB52 and J05AX06 or N03AB54 and J05AX06 or N03AC01 and J05AX06 or N03AC02 and J05AX06 or N03AC03 and J05AX06 or N03AD01 and J05AX06 or N03AD02 and J05AX06 or N03AD03 and J05AX06 or N03AD51 and J05AX06 or N03AE01 and J05AX06 or N03AF01 and J05AX06 or N03AF02 and J05AX06 or N03AF03 and J05AX06 or N03AF04 and J05AX06 or N03AG01 and J05AX06 or N03AG02 and J05AX06 or N03AG03 and J05AX06 or N03AG04 and J05AX06 or N03AG05 and J05AX06 or N03AG06 and J05AX06 or N03AX03 and J05AX06 or N03AX07 and J05AX06 or N03AX09 and J05AX06 or N03AX10 and J05AX06 or N03AX11 and J05AX06 or N03AX12 and J05AX06 or N03AX13 and J05AX06 or N03AX14 and J05AX06 or N03AX15 and J05AX06 or N03AX16 and J05AX06 or N03AX17 and J05AX06 or N03AX18 and J05AX06 or N03AX19 and J05AX06 or N03AX21 and J05AX06 or N03AX22 and J05AX06 or N03AX23 and J05AX06 or N03AX24 and J05AX06 or N03AX30 and J05AX06 or N03AA01 and J05AX07 or N03AA02 and J05AX07 or N03AA03 and J05AX07 or N03AA04 and J05AX07 or N03AA30 and J05AX07 or N03AB01 and J05AX07 or N03AB02 and J05AX07 or N03AB03 and J05AX07 or N03AB04 and J05AX07 or N03AB05 and J05AX07 or N03AB52 and J05AX07 or N03AB54 and J05AX07 or N03AC01 and J05AX07 or N03AC02 and J05AX07 or N03AC03 and J05AX07 or N03AD01 and J05AX07 or N03AD02 and J05AX07 or N03AD03 and J05AX07 or N03AD51 and J05AX07 or N03AE01 and J05AX07 or N03AF01 and J05AX07 or N03AF02 and J05AX07 or N03AF03 and J05AX07 or N03AF04 and J05AX07 or N03AG01 and J05AX07 or N03AG02 and J05AX07 or N03AG03 and J05AX07 or N03AG04 and J05AX07 or N03AG05 and J05AX07 or N03AG06 and J05AX07 or N03AX03 and J05AX07 or N03AX07 and J05AX07 or N03AX09 and J05AX07 or N03AX10 and J05AX07 or N03AX11 and J05AX07 or N03AX12 and J05AX07 or N03AX13 and J05AX07 or N03AX14 and J05AX07 or N03AX15 and J05AX07 or N03AX16 and J05AX07 or N03AX17 and J05AX07 or N03AX18 and J05AX07 or N03AX19 and J05AX07 or N03AX21 and J05AX07 or N03AX22 and J05AX07 or N03AX23 and J05AX07 or N03AX24 and J05AX07 or N03AX30 and J05AX07 or N03AA01 and J05AX08 or N03AA02 and J05AX08 or N03AA03 and J05AX08 or N03AA04 and J05AX08 or N03AA30 and J05AX08 or N03AB01 and J05AX08 or N03AB02 and J05AX08 or N03AB03 and J05AX08 or N03AB04 and J05AX08 or N03AB05 and J05AX08 or N03AB52 and J05AX08 or N03AB54 and J05AX08 or N03AC01 and J05AX08 or N03AC02 and J05AX08 or N03AC03 and J05AX08 or N03AD01 and J05AX08 or N03AD02 and J05AX08 or N03AD03 and J05AX08 or N03AD51 and J05AX08 or N03AE01 and J05AX08 or N03AF01 and J05AX08 or N03AF02 and J05AX08 or N03AF03 and J05AX08 or N03AF04 and J05AX08 or N03AG01 and J05AX08 or N03AG02 and J05AX08 or N03AG03 and J05AX08 or N03AG04 and J05AX08 or N03AG05 and J05AX08 or N03AG06 and J05AX08 or N03AX03 and J05AX08 or N03AX07 and J05AX08 or N03AX09 and J05AX08 or N03AX10 and J05AX08 or N03AX11 and J05AX08 or N03AX12 and J05AX08 or N03AX13 and J05AX08 or N03AX14 and J05AX08 or N03AX15 and J05AX08 or N03AX16 and J05AX08 or N03AX17 and J05AX08 or N03AX18 and J05AX08 or N03AX19 and J05AX08 or N03AX21 and J05AX08 or N03AX22 and J05AX08 or N03AX23 and J05AX08 or N03AX24 and J05AX08 or N03AX30 and J05AX08 or N03AA01 and J05AX09 or N03AA02 and J05AX09 or N03AA03 and J05AX09 or N03AA04 and J05AX09 or N03AA30 and J05AX09 or N03AB01 and J05AX09 or N03AB02 and J05AX09 or N03AB03 and J05AX09 or N03AB04 and J05AX09 or N03AB05 and J05AX09 or N03AB52 and J05AX09 or N03AB54 and J05AX09 or N03AC01 and J05AX09 or N03AC02 and J05AX09 or N03AC03 and J05AX09 or N03AD01 and J05AX09 or N03AD02 and J05AX09 or N03AD03 and J05AX09 or N03AD51 and J05AX09 or N03AE01 and J05AX09 or N03AF01 and J05AX09 or N03AF02 and J05AX09 or N03AF03 and J05AX09 or N03AF04 and J05AX09 or N03AG01 and J05AX09 or N03AG02 and J05AX09 or N03AG03 and J05AX09 or N03AG04 and J05AX09 or N03AG05 and J05AX09 or N03AG06 and J05AX09 or N03AX03 and J05AX09 or N03AX07 and J05AX09 or N03AX09 and J05AX09 or N03AX10 and J05AX09 or N03AX11 and J05AX09 or N03AX12 and J05AX09 or N03AX13 and J05AX09 or N03AX14 and J05AX09 or N03AX15 and J05AX09 or N03AX16 and J05AX09 or N03AX17 and J05AX09 or N03AX18 and J05AX09 or N03AX19 and J05AX09 or N03AX21 and J05AX09 or N03AX22 and J05AX09 or N03AX23 and J05AX09 or N03AX24 and J05AX09 or N03AX30 and J05AX09 or N03AA01 and J05AX10 or N03AA02 and J05AX10 or N03AA03 and J05AX10 or N03AA04 and J05AX10 or N03AA30 and J05AX10 or N03AB01 and J05AX10 or N03AB02 and J05AX10 or N03AB03 and J05AX10 or N03AB04 and J05AX10 or N03AB05 and J05AX10 or N03AB52 and J05AX10 or N03AB54 and J05AX10 or N03AC01 and J05AX10 or N03AC02 and J05AX10 or N03AC03 and J05AX10 or N03AD01 and J05AX10 or N03AD02 and J05AX10 or N03AD03 and J05AX10 or N03AD51 and J05AX10 or N03AE01 and J05AX10 or N03AF01 and J05AX10 or N03AF02 and J05AX10 or N03AF03 and J05AX10 or N03AF04 and J05AX10 or N03AG01 and J05AX10 or N03AG02 and J05AX10 or N03AG03 and J05AX10 or N03AG04 and J05AX10 or N03AG05 and J05AX10 or N03AG06 and J05AX10 or N03AX03 and J05AX10 or N03AX07 and J05AX10 or N03AX09 and J05AX10 or N03AX10 and J05AX10 or N03AX11 and J05AX10 or N03AX12 and J05AX10 or N03AX13 and J05AX10 or N03AX14 and J05AX10 or N03AX15 and J05AX10 or N03AX16 and J05AX10 or N03AX17 and J05AX10 or N03AX18 and J05AX10 or N03AX19 and J05AX10 or N03AX21 and J05AX10 or N03AX22 and J05AX10 or N03AX23 and J05AX10 or N03AX24 and J05AX10 or N03AX30 and J05AX10 or N03AA01 and J05AX11 or N03AA02 and J05AX11 or N03AA03 and J05AX11 or N03AA04 and J05AX11 or N03AA30 and J05AX11 or N03AB01 and J05AX11 or N03AB02 and J05AX11 or N03AB03 and J05AX11 or N03AB04 and J05AX11 or N03AB05 and J05AX11 or N03AB52 and J05AX11 or N03AB54 and J05AX11 or N03AC01 and J05AX11 or N03AC02 and J05AX11 or N03AC03 and J05AX11 or N03AD01 and J05AX11 or N03AD02 and J05AX11 or N03AD03 and J05AX11 or N03AD51 and J05AX11 or N03AE01 and J05AX11 or N03AF01 and J05AX11 or N03AF02 and J05AX11 or N03AF03 and J05AX11 or N03AF04 and J05AX11 or N03AG01 and J05AX11 or N03AG02 and J05AX11 or N03AG03 and J05AX11 or N03AG04 and J05AX11 or N03AG05 and J05AX11 or N03AG06 and J05AX11 or N03AX03 and J05AX11 or N03AX07 and J05AX11 or N03AX09 and J05AX11 or N03AX10 and J05AX11 or N03AX11 and J05AX11 or N03AX12 and J05AX11 or N03AX13 and J05AX11 or N03AX14 and J05AX11 or N03AX15 and J05AX11 or N03AX16 and J05AX11 or N03AX17 and J05AX11 or N03AX18 and J05AX11 or N03AX19 and J05AX11 or N03AX21 and J0SAX11 or N03AX22 and J05AX11 or N03AX23 and J05AX11 or N03AX24 and J05AX11 or N03AX30 and J05AX11 or N03AA01 and J05AX12 or N03AA02 and J05AX12 or N03AA03 and J05AX12 or N03AA04 and J05AX12 or N03AA30 and J05AX12 or N03AB01 and J05AX12 or N03AB02 and J05AX12 or N03AB03 and J05AX12 or N03AB04 and J05AX12 or N03AB05 and J05AX12 or N03AB52 and J05AX12 or N03AB54 and J05AX12 or N03AC01 and J05AX12 or N03AC02 and J05AX12 or N03AC03 and J05AX12 or N03AD01 and J05AX12 or N03AD02 and J05AX12 or N03AD03 and J05AX12 or N03AD51 and J05AX12 or N03AE01 and J05AX12 or N03AF01 and J05AX12 or N03AF02 and J05AX12 or N03AF03 and J05AX12 or N03AF04 and J05AX12 or N03AG01 and J05AX12 or N03AG02 and J05AX12 or N03AG03 and J05AX12 or N03AG04 and J05AX12 or N03AG05 and J05AX12 or N03AG06 and J05AX12 or N03AX03 and J05AX12 or N03AX07 and J05AX12 or N03AX09 and J05AX12 or N03AX10 and J05AX12 or N03AX11 and J05AX12 or N03AX12 and J05AX12 or N03AX13 and J05AX12 or N03AX14 and J05AX12 or N03AX15 and J05AX12 or N03AX16 and J05AX12 or N03AX17 and J05AX12 or N03AX18 and J05AX12 or N03AX19 and J05AX12 or N03AX21 and J05AX12 or N03AX22 and J05AX12 or N03AX23 and J05AX12 or N03AX24 and J05AX12 or N03AX30 and J05AX12 or N03AA01 and J05AX13 or N03AA02 and J05AX13 or N03AA03 and J05AX13 or N03AA04 and J05AX13 or N03AA30 and J05AX13 or N03AB01 and J05AX13 or N03AB02 and J05AX13 or N03AB03 and J05AX13 or N03AB04 and J05AX13 or N03AB05 and J05AX13 or N03AB52 and J05AX13 or N03AB54 and J05AX13 or N03AC01 and J05AX13 or N03AC02 and J05AX13 or N03AC03 and J05AX13 or N03AD01 and J05AX13 or N03AD02 and J05AX13 or N03AD03 and J05AX13 or N03AD51 and J05AX13 or N03AE01 and J05AX13 or N03AF01 and J05AX13 or N03AF02 and J05AX13 or N03AF03 and J05AX13 or N03AF04 and J05AX13 or N03AG01 and J05AX13 or N03AG02 and J05AX13 or N03AG03 and J05AX13 or N03AG04 and J05AX13 or N03AG05 and J05AX13 or N03AG06 and J05AX13 or N03AX03 and J05AX13 or N03AX07 and J05AX13 or N03AX09 and J05AX13 or N03AX10 and J05AX13 or N03AX11 and J05AX13 or N03AX12 and J05AX13 or N03AX13 and J05AX13 or N03AX14 and J05AX13 or N03AX15 and J05AX13 or N03AX16 and J05AX13 or N03AX17 and J05AX13 or N03AX18 and J05AX13 or N03AX19 and J05AX13 or N03AX21 and J05AX13 or N03AX22 and J05AX13 or N03AX23 and J05AX13 or N03AX24 and J05AX13 or N03AX30 and J05AX13 or N03AA01 and J05AX17 or N03AA02 and J05AX17 or N03AA03 and J05AX17 or N03AA04 and J05AX17 or N03AA30 and J05AX17 or N03AB01 and J05AX17 or N03AB02 and J05AX17 or N03AB03 and J05AX17 or N03AB04 and J05AX17 or N03AB05 and J05AX17 or N03AB52 and J05AX17 or N03AB54 and J05AX17 or N03AC01 and J05AX17 or N03AC02 and J05AX17 or N03AC03 and J05AX17 or N03AD01 and J05AX17 or N03AD02 and J05AX17 or N03AD03 and J05AX17 or N03AD51 and J05AX17 or N03AE01 and J05AX17 or N03AF01 and J05AX17 or N03AF02 and J05AX17 or N03AF03 and J05AX17 or N03AF04 and J05AX17 or N03AG01 and J05AX17 or N03AG02 and J05AX17 or N03AG03 and J05AX17 or N03AG04 and J05AX17 or N03AG05 and J05AX17 or N03AG06 and J05AX17 or N03AX03 and J05AX17 or N03AX07 and J05AX17 or N03AX09 and J05AX17 or N03AX10 and J05AX17 or N03AX11 and J05AX17 or N03AX12 and J05AX17 or N03AX13 and J05AX17 or N03AX14 and J05AX17 or N03AX15 and J05AX17 or N03AX16 and J05AX17 or N03AX17 and J05AX17 or N03AX18 and J05AX17 or N03AX19 and J05AX17 or N03AX21 and J05AX17 or N03AX22 and J05AX17 or N03AX23 and J05AX17 or N03AX24 and J05AX17 or N03AX30 and J05AX17 or N03AA01 and J05AX18 or N03AA02 and J05AX18 or N03AA03 and J05AX18 or N03AA04 and J05AX18 or N03AA30 and J05AX18 or N03AB01 and J05AX18 or N03AB02 and J05AX18 or N03AB03 and J05AX18 or N03AB04 and J05AX18 or N03AB05 and J05AX18 or N03AB52 and J05AX18 or N03AB54 and J05AX18 or N03AC01 and J05AX18 or N03AC02 and J05AX18 or N03AC03 and J05AX18 or N03AD01 and J05AX18 or N03AD02 and J05AX18 or N03AD03 and J05AX18 or N03AD51 and J05AX18 or N03AE01 and J05AX18 or N03AF01 and J05AX18 or N03AF02 and J05AX18 or N03AF03 and J05AX18 or N03AF04 and J05AX18 or N03AG01 and J05AX18 or N03AG02 and J05AX18 or N03AG03 and J05AX18 or N03AG04 and J05AX18 or N03AG05 and J05AX18 or N03AG06 and J05AX18 or N03AX03 and J05AX18 or N03AX07 and J05AX18 or N03AX09 and J05AX18 or N03AX10 and J05AX18 or N03AX11 and J05AX18 or N03AX12 and J05AX18 or N03AX13 and J05AX18 or N03AX14 and J05AX18 or N03AX15 and J05AX18 or N03AX16 and J05AX18 or N03AX17 and J05AX18 or N03AX18 and J05AX18 or N03AX19 and J05AX18 or N03AX21 and J05AX18 or N03AX22 and J05AX18 or N03AX23 and J05AX18 or N03AX24 and J05AX18 or N03AX30 and J05AX18 or N03AA01 and J05AX19 or N03AA02 and J05AX19 or N03AA03 and J05AX19 or N03AA04 and J05AX19 or N03AA30 and J05AX19 or N03AB01 and J05AX19 or N03AB02 and J05AX19 or N03AB03 and J05AX19 or N03AB04 and J05AX19 or N03AB05 and J05AX19 or N03AB52 and J05AX19 or N03AB54 and J05AX19 or N03AC01 and J05AX19 or N03AC02 and J05AX19 or N03AC03 and J05AX19 or N03AD01 and J05AX19 or N03AD02 and J05AX19 or N03AD03 and J05AX19 or N03AD51 and J05AX19 or N03AE01 and J05AX19 or N03AF01 and J05AX19 or N03AF02 and J05AX19 or N03AF03 and J05AX19 or N03AF04 and J05AX19 or N03AG01 and J05AX19 or N03AG02 and J05AX19 or N03AG03 and J05AX19 or N03AG04 and J05AX19 or N03AG05 and J05AX19 or N03AG06 and J05AX19 or N03AX03 and J05AX19 or N03AX07 and J05AX19 or N03AX09 and J05AX19 or N03AX10 and J05AX19 or N03AX11 and J05AX19 or N03AX12 and J05AX19 or N03AX13 and J05AX19 or N03AX14 and J05AX19 or N03AX15 and J05AX19 or N03AX16 and J05AX19 or N03AX17 and J05AX19 or N03AX18 and J05AX19 or N03AX19 and J05AX19 or N03AX21 and J05AX19 or N03AX22 and J05AX19 or N03AX23 and J05AX19 or N03AX24 and J05AX19 or N03AX30 and J05AX19 or N03AA01 and J05AX21 or N03AA02 and J05AX21 or N03AA03 and J05AX21 or N03AA04 and J05AX21 or N03AA30 and J05AX21 or N03AB01 and J05AX21 or N03AB02 and J05AX21 or N03AB03 and J05AX21 or N03AB04 and J05AX21 or N03AB05 and J05AX21 or N03AB52 and J05AX21 or N03AB54 and J05AX21 or N03AC01 and J05AX21 or N03AC02 and J05AX21 or N03AC03 and J05AX21 or N03AD01 and J05AX21 or N03AD02 and J05AX21 or N03AD03 and J05AX21 or N03AD51 and J05AX21 or N03AE01 and J05AX21 or N03AF01 and J05AX21 or N03AF02 and J05AX21 or N03AF03 and J05AX21 or N03AF04 and J05AX21 or N03AG01 and J05AX21 or N03AG02 and J05AX21 or N03AG03 and J05AX21 or N03AG04 and J05AX21 or N03AG05 and J05AX21 or N03AG06 and J05AX21 or N03AX03 and J05AX21 or N03AX07 and J05AX21 or N03AX09 and J05AX21 or N03AX10 and J05AX21 or N03AX11 and J05AX21 or N03AX12 and J05AX21 or N03AX13 and J05AX21 or N03AX14 and J05AX21 or N03AX15 and J05AX21 or N03AX16 and J05AX21 or N03AX17 and J05AX21 or N03AX18 and J05AX21 or N03AX19 and J05AX21 or N03AX21 and J05AX21 or N03AX22 and J05AX21 or N03AX23 and J05AX21 or N03AX24 and J05AX21 or N03AX30 and J05AX21 or N03AA01 and J05AX23 or N03AA02 and J05AX23 or N03AA03 and J05AX23 or N03AA04 and J05AX23 or N03AA30 and J05AX23 or N03AB01 and J05AX23 or N03AB02 and J05AX23 or N03AB03 and J05AX23 or N03AB04 and J05AX23 or N03AB05 and J05AX23 or N03AB52 and J05AX23 or N03AB54 and J05AX23 or N03AC01 and J05AX23 or N03AC02 and J05AX23 or N03AC03 and J05AX23 or N03AD01 and J05AX23 or N03AD02 and J05AX23 or N03AD03 and J05AX23 or N03AD51 and J05AX23 or N03AE01 and J05AX23 or N03AF01 and J05AX23 or N03AF02 and J05AX23 or N03AF03 and J05AX23 or N03AF04 and J05AX23 or N03AG01 and J05AX23 or N03AG02 and J05AX23 or N03AG03 and J05AX23 or N03AG04 and J05AX23 or N03AG05 and J05AX23 or N03AG06 and J05AX23 or N03AX03 and J05AX23 or N03AX07 and J05AX23 or N03AX09 and J05AX23 or N03AX10 and J05AX23 or N03AX11 and J05AX23 or N03AX12 and J05AX23 or N03AX13 and J05AX23 or N03AX14 and J05AX23 or N03AX15 and J05AX23 or N03AX16 and J05AX23 or N03AX17 and J05AX23 or N03AX18 and J05AX23 or N03AX19 and J05AX23 or N03AX21 and J05AX23 or N03AX22 and J05AX23 or N03AX23 and J05AX23 or N03AX24 and J05AX23 or N03AX30 and J05AX23.

7. A composition according to any of the previous claims, **characterised in that** it is composed of two active substances, each of which is any combination of substances selected from the NO3 ATC group and substances selected from the J05 ATC group.

8. An active substance of J05ATC group for treatment of nervous tissue dysfunction, in particular viral latency.

9. An active substance according to claim 8, **characterised in that** it is a: metisazone or aciclovir or idoxuridine or vidarabine or ganciclovir or famciclovir or valaciclovir or cidofovir or penciclovir or valganciclovir or brivudine or rimantadine or tromantadine or foscarnet or fosfonet or saquinavir or indinavir or ritonavir or nelfinavir or amprenavir or fosamprenavir or atazanavir or tipranavir or darunavir or zidovudine or didanosine or zalcitabine or stavudine or lamivudine or abacavir or tenofovir disoproxil or adefovir dipivoxil or emtricitabine or entecavir or telbivudine or clevudine or tenofovir alafenamide or nevirapine or delavirdine or efavirenz or etravirine or rilpivirine or doravirine or zanamivir or oseltamivir or peramivir or ribavirin or telaprevir or boceprevir or faldaprevir or simeprevir or asunaprevir or daclatasvir or sofosbuvir or dasabuvir or sofosbuvir and ledipasvir or dasabuvir, ombitasvir, paritaprevir and ritonavir or ombitasvir, paritaprevir and ritonavir or elbasvir and grazoprevir or sofosbuvir and velpatasvir or sofosbuvir, velpatasvir and voxilaprevir or glecaprevir and pibrentasvir or zidovudine and lamivudine or lamivudine and abacavir or tenofovir disoproxil and emtricitabine or zidovudine, lamivudine and abacavir or zidovudine, lamivudine and nevirapine or emtricitabine, tenofovir disoproxil and efavirenz or stavudine, lamivudine and nevirapine or emtricitabine, tenofovir disoproxil and rilpivirine or emtricitabine, tenofovir disoproxil, elvitegravir and cobicistat lopinavir and ritonavir or lamivudine, tenofovir disoproxil and efavirenz or lamivudine and tenofovir disoproxil or lamivudine, abacavir and dolutegravir or darunavir and cobicistat or atazanavir and cobicistat or lamivudine and raltegravir or emtricitabine and tenofovir alafenamide or emtricitabine, tenofovir alafenamide, elvitegravir and cobicistat or emtricitabine or tenofovir alafenamide and rilpivirine or emtricitabine, tenofovir alafenamide and bictegravir or dolutegravir and rilpivirine or emtricitabine, tenofovir or alafenamide, darunavir and cobicistat or atazanavir and ritonavir or lamivudine, tenofovir disoproxil and doravirine or moroxydine or lysozyme or inosine pranobex or pleconaril or enfuvirtide or raltegravir or maraviroc or maribavir or elvitegravir or dolutegravir or umifenovir or enisamium iodide or letermovir or tilorone or pentanedioic acid imidazolyl ethanamide or ibalizumab.
